# EUROPEAN PATENT APPLICATION

(11) **EP 1 422 293 A1**
(43) Date of publication of application: **26.05.2004**
(21) Application number: 03026169.7
(22) Date of filing: 17.11.2003
(51) Int. Cl.: C12N 9/48

(54) **Crystal structure of dipeptidyl peptidase IV**

(30) Priority: 25.11.2002 EP 02026367
(71) Applicant: F. HOFFMANN-LA ROCHE AG, 4070 Basel (CH)
(72) Inventor: Hennig, Michael, 79576 Weil am Rhein (DE); Loeffler, Bernd Michael, 79206 Oberrimsingen (DE); Thoma, Ralf, 79639 Grenzach-Wyhlen (DE)

(57) **Abstract**

The present invention relates to crystal structure information obtained from crystals of the dipeptidyl-peptidase DPP-IV, to methods of preparing such crystals, and to their use for the identification and/or design of inhibitors of DPP-IV activity. A further subject matter of the invention are methods for the identification and/or design of inhibitor compounds of DPP-IV activity, the inhibitor compounds of DPP-IV activity identified by these methods and their use in pharmaceutical compositions for the treatment and/or prevention of diseases comprising diabetes types I and II, IGT, obesity and cancer.

## Description

The present invention relates to crystal structure information obtained from crystals of the dipeptidyl-peptidase DPP-IV, to methods of preparing such crystals, and to their use for the identification and/or design of inhibitors of DPP-IV activity. A further subject matter of the invention are methods for the identification and/or design of inhibitor compounds of DPP-IV activity, the inhibitor compounds of DPP-IV activity identified by these methods and their use in pharmaceutical compositions for the treatment and/or prevention of diseases comprising diabetes types I and II, IGT and obesity.

Dipeptidyl peptidase (DPP-IV; T-cell activation antigen CD26 or adenosine binding protein) is a multifunctional type II cell surface glycoprotein. The protein is widely expressed in a variety of cell types, particularly on differential epithelial cells of the intestine, liver, prostate tissue, corpus luteum, and kidney proximal tubles (Hartel, S., Gossrau, R., Hanski, C. & Reutter, W. (1988). Dipeptidyl peptidase (DPP) IV in rat organs. Comparison of immunohistochemistry and activity histochemistry. *Histochemistry* 89, 151-161; McCaughan, G.W., Wickson, J.E., Creswick, P.F. & Gorrell, M.D. (1990). Identification of the bile canalicular cell surface molecule GP110 as the ectopeptidase dipeptidyl peptidase IV: an analysis by tissue distribution, purification and N-terminal amino acid sequence. *Hepatology* **11**, 534-544) as well as leukocyte subsets (Gorrell, M.D., Wickson, J. & McCaughan, G.W. (1991). Expression of the rat CD26 antigen (dipeptidyl peptidase IV) on subpopulations of rat lymphocytes. *Cell. Immunol.* **134**, 205-215), such as T-helper lymphocytes, and subsets of macrophages (Bühling, F., Kunz, D., Reinhold, D., Ulmer, A.J., Ernst, M., Flad, H.D. & Ansorge, S. (1994). Expression and functional role of dipeptidyl peptidase IV (CD26) on human natural killer cells. *Nat. Immun.* **13**, 270-279) and a soluble form is reported to be present in plasma and urine (Iwaki-Egawa, S., Watanabe, Y., Kikuya, Y. & Fujimoto, Y. (1998). Dipeptidyl peptidase IV from human serum: purification, characterization, and N-terminal amino acid sequence. J. *Biochem.* **124**, 428-433). Human DPP-IV has a short cytoplasmatic tail of six amino acids, a 22 amino acid hydrophobic transmembrane region and a 738 amino acid extracellular domain with ten potential glycosylation sites (Tanaka, T., Camerini, D., Seed, B., Torimoto, Y., Dang, N.H., Kameoka, J., Dahlberg, H.N., Schlossman, S.F. & Morimoto, C. (1992). Cloning and functional expression of the T cell activation antigen CD26. *J. Immunol.* **149**, 481-486).

DPP-IV is involved in many biological processes, including a membrane-anchoring function for the localization of the extracellular enzyme adenosine deaminase (ADA) (Franco, R., Valenzuela, A., Lluis, C. & Blanco, J. (1998). Enzymatic and extraenzymatic role of ecto-adenosine deaminase in lymphocytes. *Immunol. Rev.* **161**, 27-42), participation in cell matrix adhesion by binding to collagen and fibronectin (Loster, K., Zeilinger, K., Schuppan, D. & Reutter, W. (1995). The cysteine-rich region of dipeptidyl peptidase IV (CD 26) is the collagen-binding site. *Biochem. Biophys. Res. Commun.* **217**, 341-348), interaction as a co-receptor for the HIV envelope protein gp 120 (Ohtsuki, T., Tsuda, H. & Morimoto, C. (2000). Good or evil: CD26 and HIV infection. *J. Dermatol. Sci.* **22**, 152-160) and co-stimulatory function during T-cell activation and proliferation (von Bonin, A., Huhn, J. & Fleischer, B. (1998). Dipeptidyl-peptidase IV/CD26 on T cells: analysis of an alternative T-cell activation pathway. *Immunol. Rev.* **161**, 43-53) by interaction with the protein tyrosine phosphatase (CD45) (Torimoto, Y., Dang, N.H., Vivier, E., Tanaka, T., Schlossman, S.F. & Morimoto, C. (1991). Coassociation of CD26 (dipeptidyl peptidase IV) with CD45 on the surface of human T lymphocytes. J. *Immunol.* **147**, 2514-2517).

DPP-IV (EC 3.4.14.5) has postproline dipeptidyl amino peptidase activity, preferentially cleaving X-proline or X-alanine dipeptides from the N-terminus of polypeptides (Hopsu-Havu, V.K. & Glenner, G.G. (1966). A new dipeptide naphthylamidase hydrolyzing glycyl-prolyl-beta-naphthylamide. *Histochemie* **7**, 197-201.) and belongs to the prolyl oligopeptidase family, a group of atypical serine proteases able to hydrolyse the prolyl bond (Cunningham, D.F. & O'Connor, B. (1997). Proline specific peptidases. *Biochim. Biophys. Acta* **1343**, 160-186). It possesses a novel orientation of its catalytic triad residues (Ser-Asp-His) (Ikehara, Y., Ogata, S. & Misumi, Y. (1994). Dipeptidyl-peptidase IV from rat liver. *Methods Enzymol.* **244**, 215-227.), inverse to that found in classical serine proteases (His-Asp-Ser). The cleavage of N-terminal peptides with Pro in the second position is a rate limiting step in the degradation of peptides. The natural substrates of DPP-IV include several chemokines, cytokines, neuropeptides, circulating hormones and bioactive peptides (Lambeir, A.M., Durinx, C., Proost, P., Van Damme, J., Scharpe, S. & De Meester, I. (2001). Kinetic study of the processing by dipeptidyl-peptidase IV/CD26 of neuropeptides involved in pancreatic insulin secretion. *FEBS Lett.* **507**, 327-330.). The wide range of substrates suggests a key regulatory role in the metabolism of peptide hormones and in amino acid transport (Hildebrandt, M., Reutter, W., Arck, P., Rose, M. & Klapp, B.F. (2000). A guardian angel: the involvement of dipeptidyl peptidase IV in psychoneuroendocrine function, nutrition and immune defence. *Clin Sci* **99**, 93-104). Its physiological relevance has been investigated by (Hinke, S.A., Pospisilik, J.A., Demuth, H.U., Mannhart, S., Kuhn-Wache, K., Hoffmann, T., Nishimura, E., Pederson, R.A. & McIntosh, C.H. (2000). Dipeptidyl peptidase IV (DPIV/CD26) degradation of glucagon. Characterization of glucagon degradation products and DPIV-resistant analogs. *J. Biol. Chem.* **275**, 3827-3834).

The finding that DPP-IV is responsible for more than 95% of the degradation of GLP-1 led to an elevated interest in inhibition of this enzyme for the treatment of diabetes type II. Experiments in rats and humans have provided evidence that specific DPP-IV inhibition increased Cₘₐₓ, T_{1/2} and total circulating GLP-1 and decreased plasma glucose. It has been demonstrated that patients with impaired glucose-tolerance (IGT), type-II diabetes and with a secondary failure to respond to sulfonylurea treatment benefit from increased levels of GLP peptides. In addition GLP-1 is effective in type-I diabetic patients due to its glucagono-static effect. More recent investigations show a delay of gastric emptying that could have beneficial effects on satiety and might be relevant for the treatment of obesity. Protection of functional GLP-1 by inhibition of DPP-IV and concomitant activation of the GLP-1 receptor might therefore have a synergistic potential in anti-diabetic drug research (Holst, J.J. & Deacon, C.F. (1998). Inhibition of the activity of dipeptidyl-peptidase IV as a treatment for type 2 diabetes. *Diabetes* **47**, 1663-1670.). Selective and orally available small molecule inhibitors of DPP-IV have been discovered and are now in clinical trials (Villhauer, E.B., Brinkman, J.A., Naderi, G.B., Dunning, B.E., Mangold, B.L., Mone, M.D., Russell, M.E., Weldon, S.C. & Hughes, T.E. (2002).1-[2-[(5-Cyanopyridin-2-yl)amino]ethylamino]acetyl-2-(S)-pyrrolidinecarbon nitrile: a potent, selective, and orally bioavailable dipeptidyl peptidase IV inhibitor with antihyperglycemic properties. J. Med. Chem. **45**, 2362-2365; Pospisilik, J.A., Stafford, S.G., Demuth, H.U., McIntosh, C.H. & Pederson, R.A. (2002). Long-term treatment with dipeptidyl peptidase IV inhibitor improves hepatic and peripheral insulin sensitivity in the VDF zucker rat: a euglycemic-hyperinsulinemic clamp study. *Diabetes* **51**, 2677-2683).

Therefore, the present invention provides a solution to the problem of identifying and/or designing inhibitors of DPP-IV activity by providing crystals of the extracellular domain of DPP-IV and their crystal structure information, methods of preparing such crystals, and methods of identifying and/or designing inhibitors of DPP-IV with these crystals by structure based drug design.

The present invention relates to crystal structure information obtained from crystalline preparations of the dipeptidyl-peptidase DPP-IV, to methods of preparing such crystals, and to their use for the identification and/or design of inhibitors of DPP-IV activity. A further subject matter of the invention are methods for the identification and/or design of inhibitor compounds of DPP-IV activity, the inhibitor compounds of DPP-IV activity identified by these methods and their use in pharmaceutical compositions for the treatment and/or prevention of diseases comprising diabetes types I and II, obesity and cancer.

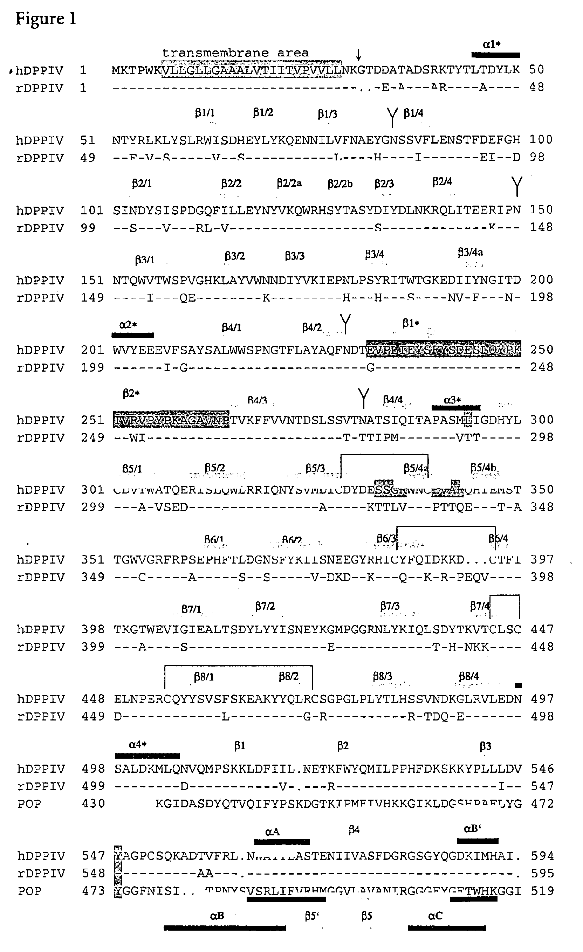

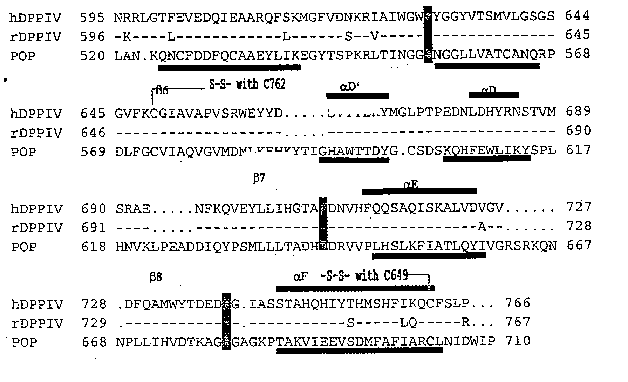

Figure 1. Sequence alignment of DPP-IV and POP: Amino acid sequence alignment of DPP-IV from human (hDPP-IV) and rat (rDPP-IV, only different residues are shown). The alignment of POP from pork was performed using structural superposition for the α/β-hydrolase domain only, because of a lack of structural homology for the β-propeller domain. The top line gives additional information about the secondary structure of DPP-IV (yellow arrows and red bars), the glycosylation sites with visible electron density (Y), the potential glycosylation sites (marked in red), the disulphide bonds (green lines between cysteins that are involved) and an arrow that indicates the start of the cloned ectodomain. Sequences are highlighted light gray for the transmembrane part, gray for the part of the β-propeller involved in dimerization, green for residues involved in adenosine deaminase binding, blue for the tyrosine that is involved in the stabilization of the oxyanion of the catalytic intermediate and pink for the catalytic residues.

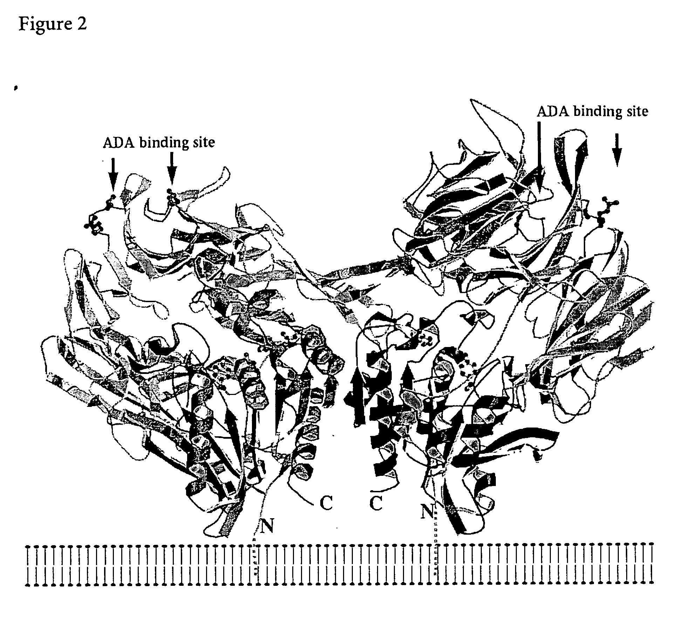

Figure 2. Overall Structure of DPP-IV: Ribbon diagram of DPP-IV viewed perpendicular to the two-fold axis. The domains are colored dark green and light green for the α/β hydrolase and β-propeller domains of subunit A and dark/light blue for the other subunit, respectively. The overall dimension of the molecule is about 125 x 80 x 60 Å³. The active site is highlighted by the catalytic residues in ball and stick representation as well as residues that are identified by mutagenesis data to be important for ADA binding. The proposed location at the cell surface is shown by the schematic drawing of the membrane. This figure was prepared using Molscript (Kraulis, P.J. (1991). MOLSCRIPT: A program to produce both detailed and schematic plots of protein structures. *J. Applied Crystallogr.* **24**, 946-950) and rendered with Raster3D (Merrit, E.A. & Bacon, D.J. (1997). Raster3D: photorealistic molecular graphics. *Methods Enzymol.* **277**, 505-524).

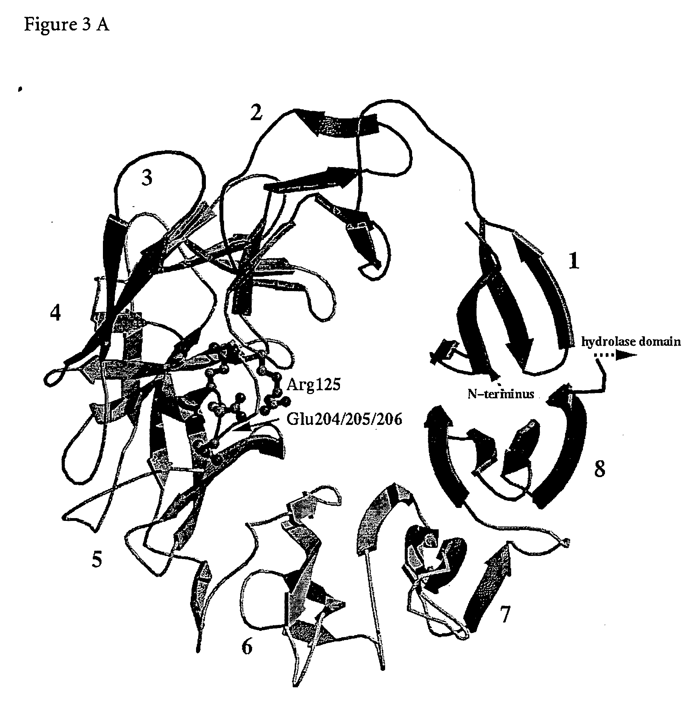

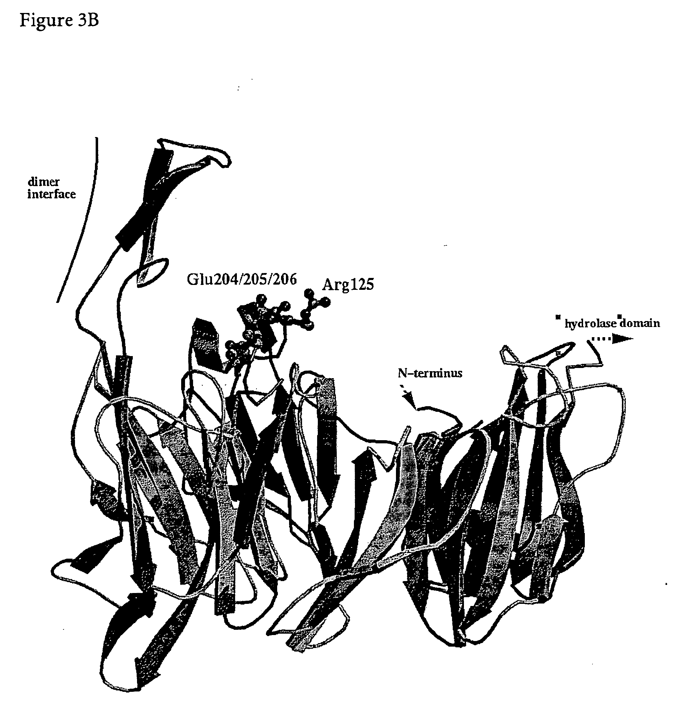

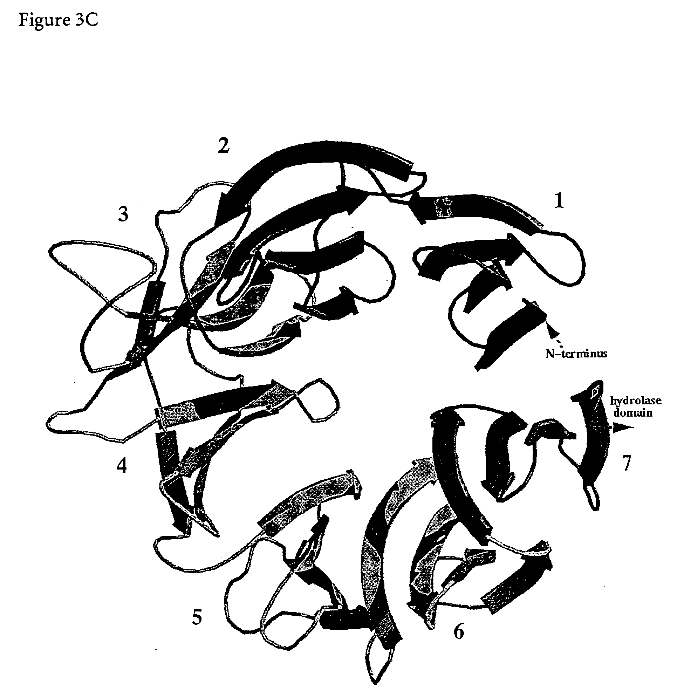

Figure 3. Ribbon drawing of the β-propeller domains of DPP-IV and POP: A: DPP-IV has 8 repeats of a structural motif that consists of four antiparallel β-strands or blades (blades are numbered 1 to 8). Additional secondary structural elements are colored magenta: An antiparallel β-sheet (β2/2a and β2/2b in Figure 1) that is an extension of blade 2 with Arg125 at the tip of the turn that is involved in the substrate binding. An α-helix (α2* in Figure 1) with the C-terminal glutamate rich loop that contributes to substrate recognition and specificity (Glu204/205/206). The antiparallel β-sheet that forms a main part of the dimer interface (β1* and β2* in Figure 1). The latter structural elements are extensions of the blade 4.
B: β-propeller domain of DPP-IV rotated 90°
C: POP has 7 blades and no notable deviations from the β-propeller structure. The blades are numbered 1 to 7.

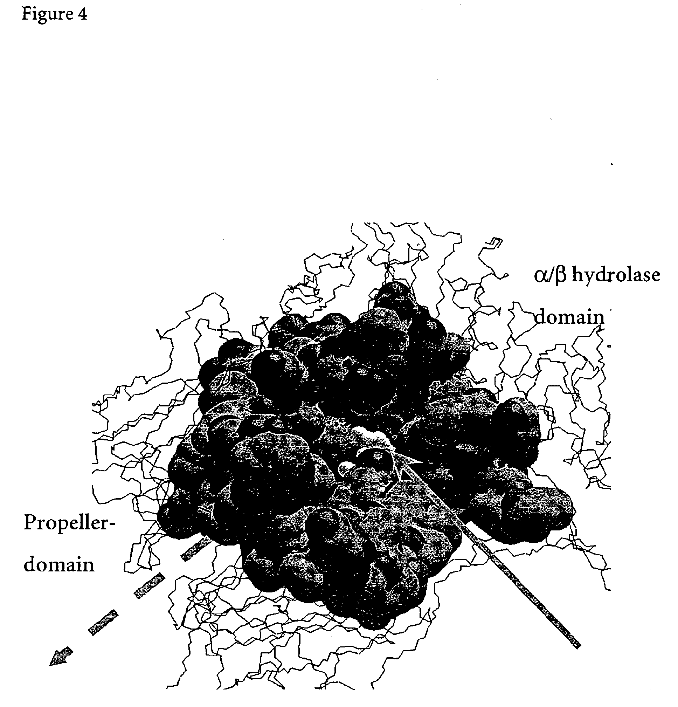

Figure 4. Access to the active site: Schematic view on the subunit of DPP-IV with the active site surface coloured according to the atom types. The substrate Diprotin A is shown with white carbons indicating the substrate binding site. Arrows illustrate that the substrate may enter the active site at the well accessible and open active site cleft and the dipeptidic product of the catalytic reaction may leave the active site cavity via the more narrow tunnel that is formed by the β-propeller.

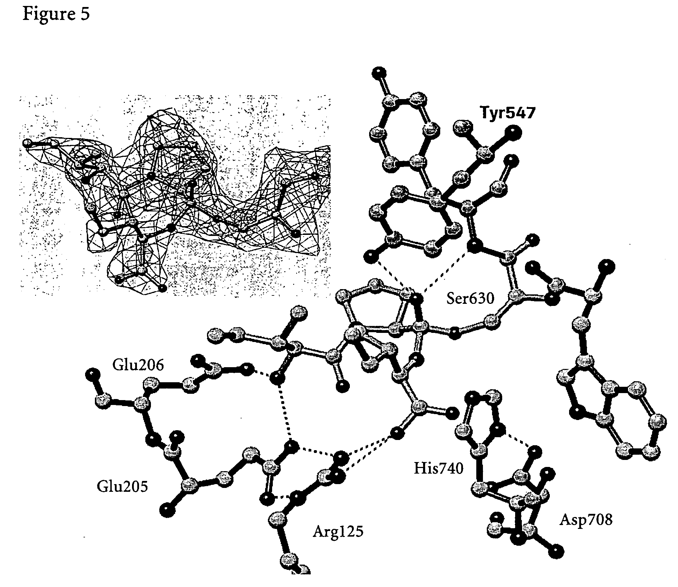

Figure 5. Active site of DPP-IV with Diprotin A (Ile-Pro-Ile): The substrate Diprotin A is trapped as tetrahedral intermediate covalently bound to the active site Ser630. Dashed lines indicate hydrogen bonds. Bonds are dark blue for the protein and light blue for the ligand as well as the active site Ser630. Drawn with MOLOC (Gerber, P.R. (1992). Peptide mechanics: a force field for peptides and proteins working with entire residues as small unites. *Biopolymers* **32**, 1003-1017).The insert shows the omit electron density (ligand and Ser630 were omitted from the calculations) contoured at 2.5 σ (green) and 4 σ (yellow).

The present invention relates to crystals of mammalian DPP-IV, with or without a ligand bound in the active site, where the crystals are of sufficient quality and size to allow for the determination of the three-dimensional X-ray diffraction at atomic resolution. The invention also relates to methods for producing and crystallizing the mammalian DPP-IV. The crystals of mammalian DPP-IV, as well as information derived from their crystal structures can be used to analyze and modify mammalian DPP-IV activity as well as to identify compounds that interact with DPP-IV.

In one aspect the present invention provides a crystal of the extracellular domain of mammalian DPP-IV, preferably having the orthorhombic space group symmetry P2₁2₁2₁ and one homodimer of DPP-IV in the asymmetric unit. Preferably, the crystal includes a unit cell having dimensions a, b, and c; wherein a is from 63Å to 67Å, b is from 66 Å to 70 Å, and c is from 416 Å to 424 Å; and α = β = γ = 90°. Preferably, the crystal includes atoms arranged in a spatial relationship represented by the atomic structure coordinates listed in Table 4. Preferably, the crystal includes DPP-IV comprising the amino acid sequence from Gly31 to Pro766 of the native protein as well as shorter variants thereof comprising all amino acids necessary for forming the active site. Preferably, the crystal includes DPP-IV as set forth in SEQ ID NO:2 as well as shorter variants thereof comprising all amino acids necessary for forming the active site.

The crystals of the invention include apo crystals and co-crystals. The apo crystals of the invention refer to crystals of mammalian DPP-IV formed without a bound active site or allosteric ligand. The co-crystals generally comprise DPP-IV with a ligand bound to the active site or to an allosteric site. The "active site" refers in general to the site where the enzymatic reaction catalyzed by the enzyme takes place. An active site ligand refers to any compound which specifically binds to the active site of a mammalian DPP-IV.

Preferably, the co-crystal of the present invention is characterized as having an orthorhombic space group of P2₁2₁2₁ (space group No. 19) and one homodimer of DPP-IV in the asymmetric unit.

More preferably, the co-crystal has unit cell dimensions of a is from 63 Å to 67Å, b is from 66 Å to 70 Å, and c is from 416 Å to 424 Å.; and α = β = γ = 90° and a P2₁2₁2₁ symmetry.

The co-crystals of the invention generally comprise a crystalline DPP-IV polypeptide in association with one or more compounds at an active or allosteric binding site of the polypeptide. The association may be covalent or non-covalent.

The DPP-IV (dipeptidyl-peptidase, DPP-IV; T-cell activation antigen CD26 or adenosine binding protein) of the present invention may be a mammalian DPP-IV. Preferably, the DPP-IV of the present invention is a human DPP-IV. More preferably, the DPP-IV of the present invention is the extracellular domain of DPP-IV. Even more preferred is the extracellular domain of DPP-IV which is soluble. Most preferably, the human DPP-IV comprises the amino acid sequence from Gly31 to Pro766 of the native protein as well as shorter variants thereof comprising all amino acids necessary for forming the active site. Preferably, DPP-IV comprises the amino acid sequence as set forth in SEQ. ID NO:2 as well as shorter variants thereof comprising all amino acids necessary for forming the active site.

It is to be understood that the crystals of DPP-IV of the invention are not limited to naturally occurring or native DPP-IV. Indeed, the crystals of the invention include mutants of the native DPP-IV. Mutants of native DPP-IV are obtained by replacing at least one amino acid residue in a native DPP-IV domain with a different amino acid residue, or by adding or deleting amino acid residues within the native polypeptide or at the N- or C- terminus of the native polypeptide, and have substantially the same three-dimensional structure as the native DPP-IV from which the mutant is derived.

By having substantially the same three-dimensional structure is meant having a set of atomic structure coordinates from an apo- or co-crystal that have a root mean square deviation of less than or equal to about 1.5 Å when superimposed with the atomic structure coordinates of the native DPP-IV when at least 50% of the alpha carbon atoms of DPP-IV are included in the superposition.

In some instances, it may be particularly advantageous or convenient to substitute, delete and/or add amino acid residues to a native DPP-IV domain in order to provide convenient cloning sites in cDNA encoding the polypeptide, to aid in purification of the polypeptide, etc. Such substitutions, deletions and/or additions which do not substantially alter the three dimensional structure of the native DPP-IV will be apparent to those having skills in the art.

It should be noted that the mutants contemplated herein need not exhibit DPP-IV activity. Indeed, amino acid substitutions, additions or deletions that interfere with the peptidase activity of the DPP-IV but which do not significantly alter the three-dimensional structure of the domain are specifically contemplated by the invention. Such crystalline polypeptides, or the atomic structure coordinates obtained therefrom, can be used to identify compounds that bind to the native domain. These compounds may affect the activity or the native domain.

The derivative crystals of the invention generally comprise a crystalline DPP-IV polypeptide in covalent association with one or more heavy metal atoms. The polypeptide may correspond to a native or a mutated DPP-IV. Heavy metal atoms useful for providing derivative crystals include, by way of example and not limitation, gold and mercury. Alternatively, derivative crystals can be formed from proteins which have heavy atoms incorporated into one or more amino acids, such as seleno-methionine substitutions for methionine.

Therefore, in a preferred embodiment of the present invention the co-crystal is a co-crystal of the extracellular domain of mammalian DPP-IV and HgCl₂.

The native and mutated DPP-IV polypeptides described herein may be isolated from natural sources or produced by methods well known to those skilled in the art of molecular biology. Expression vectors to be used may contain a native or mutated DPP-IV polypeptide coding sequence and appropriate transcriptional and/or translational control signals. These methods include *in vitro* recombinant DNA techniques, synthetic techniques and *in vivo* recombination/genetic recombination. See, for example, the techniques described in Maniatis et al., 1989, *Molecular Cloning: A Laboratory Manual,* Cold Spring Harbor Laboratory, NY; and Ausubel et al., 1989, *Current Protocols in Molecular Biology,* Greene Publishing Associates and Wiley Interscience, NY.

A variety of host-expression vector systems may be utilized to express the DPP-IV coding sequence. These include but are not limited to microorganisms such as bacteria transformed with recombinant bacteriophage DNA, plasmid DNA or cosmid DNA expression vectors containing the DPP-IV coding sequence; yeast transformed with recombinant yeast expression vectors containing the DPP-IV coding sequence; insect cell systems infected with recombinant virus expression vectors (e.g. baculovirus) containing the DPP-IV coding sequence; plant cell systems infected with recombinant virus expression vectors (e.g., cauliflower mosaic virus, CaMV; tobacco mosiac virus, TMV) or transformed with recombinant plasmid expression vectors (e.g., Ti plasmid) containing the DPP-IV coding sequence; or animal cell systems. The expression elements of these systems vary in their strength and specificities. Depending on the host/vector system utilized, any of a number of suitable transcription and translation elements, including constitutive and inducible promoters such as pL of bacteriophage µ, plac, ptrp, ptac (ptrp-lac hybrid promoter) and the like may be used; when cloning in insect cell systems, promoters such as the baculovirus polyhedrin promoter may be used; when cloning in plant cell systems, promoters derived from the genome of plant cells (e.g., heat shock promoters; the promoter for the small subunit of RUBISCO; the promoter for the chlorophyll a/b binding protein) or from plant viruses (e.g., the 35 S RNA promoter of CaMV; the coat protein promoter of TMV) may be used; when cloning in mammalian cell systems, promoters derived from the genome of mammalian cells (e.g., metallothionein promoter) or from mammalian viruses (e.g., the adenovirus late promoter; the vaccinia virus 7.5K promoter) may be used; when generating cell lines that contain multiple copies of the DPP-IV coding sequence, SV40-, BPV- and EBV-based vectors may be used with an appropriate selectable marker.

In a preferred embodiment of the present invention, an isolated nucleic acid sequence encoding the soluble extracellular domain of DPP-IV comprising the nucleotide sequence of SEQ ID NO:1 is provided.

Additionally, an expression vector containing an isolated nucleic acid sequence encoding the soluble extracellular domain of DPP-IV comprising the nucleotide sequence of SEQ ID NO:1 is provided. Preferably, the expression vector for the expression of proteins in P. pastoris which are to be secreted. Furthermore, a host cell transformed with the said expression vector is provided. Preferably, the host cell is *Pichia pastoris.*

A further aspect of the present invention relates to a method of producing the soluble extracellular domain of DPP-IV comprising culturing the host cell with the said expression vector under conditions permitting the expression of the soluble extracellular domain of DPP-IV by the host cell. Preferably, the host cell is P. pastoris. The present invention also provides the soluble extracellular domain of DPP-IV produced by this method.

Furthermore, the present invention relates to a polypeptide comprising the soluble extracellular domain of DPP-IV as set forth in SEQ ID NO:2.

The apo-, derivative and co-crystals of the invention can be obtained by techniques well-known in the art of protein crystallography, including batch, liquid bridge, dialysis, vapor diffusion and hanging drop methods (see e.g. McPherson, 1982, *Preparation and Analysis of Protein Crystals,* John Wiley, NY; McPherson, 1990, *Eur. J. Biochem.* 189:1-23; Webber, 1991, *Adv. Protein Chem.* 41:1-36; Crystallization of Nucleic Acids and Proteins, Edited by Arnaud Ducruix and Richard Giege, Oxford University Press; Protein Crystallization Techniques, Strategies, and Tips, Edited by Terese Bergfors, International University Line, 1999). Generally, the apo- or co-crystals of the invention are grown by placing a substantially pure DPP-IV polypeptide in an aqueous buffer containing a precipitant at a concentration just below that necessary to precipitate the protein. Water is then removed from the solution by controlled evaporation to produce crystallizing conditions, which are maintained until crystal growth ceases.

Preferably, the crystals are produced by a method for crystallizing mammalian DPP-IV, the method comprising (a) providing a buffered, aqueous solution of pH 7 to 8.5 with a concentration of 7 mg/ml to 22 mg/ml of the extracellular domain of mammalian DPP-IV; and (b) growing crystals by vapor diffusion using a buffered reservoir solution with between 10% and 30% PEG, between 10% and 20% glycerol, wherein PEG has an average molecular weight between 1000 and 20000. More preferably, the extracellular domain of mammalian DPP-IV of step (a) of the method is produced in the yeast *Pichia pastoris* (*P. pastoris*) and then deglycosylated. For deglycosylation, different enzymes may be used comprising Endoglycosidase F or PNGase.

Preferably, co-crystals are produced by a method for co-crystallizing mammalian DPP-IV and an active site ligand, the method comprising (a) providing a buffered, aqueous solution of pH 7 to 8.5 with a concentration of 7 mg/ml to 22 mg/ml of the extracellular domain of mammalian DPP-IV; (b) adding a molar excess of the active site ligand to the aqueous solution of mammalian DPP-IV; (c) growing crystals by vapor diffusion using a buffered reservoir solution with between 10% and 30% PEG, between 10% and 20% glycerol, wherein PEG has an average molecular weight between 1000 and 20000. More preferably, the extracellular domain of mammalian DPP-IV of step (a) of the method is produced in P. pastoris and then deglycosylated.

A further aspect of the present invention relates to a crystal produced by the methods for crystallizing or co-crystallizing DPP-IV of the present invention.

Crystals may be frozen prior to data collection.

The mosaic spread of the frozen crystals could sometimes be reduced by annealing, wherein the stream of cold nitrogen gas is briefly blocked, allowing the frozen crystal to thaw momentarily before re-freezing in the nitrogen gas stream.

Diffraction data typically extending to 2.7 Å was collected from the frozen crystals at the synchrotron beamline x06 at the Swiss light source (SLS), Villigen Switzerland. Under optimum conditions, data extending to 2.1 Å was recorded. Preferably, the the data is collected at a resolution of 3.5 Å to 2.1 Å or better. More preferably, the data is collected at a resolution of 2.7 Å to 2.1 Å or better.

Derivative crystals of the invention can be obtained by soaking apo or co-crystals in mother liquor containing salts of heavy metal atoms, according to procedures known to those of skill in the art of X-ray crystallography.

Co-crystals of the invention can be obtained by soaking an apo crystal in mother liquor containing a ligand that binds to the active site, or can be obtained by co-crystallizing the DPP-IV polypeptide in the presence of one or more ligands that bind to the active site or to an allosteric site. Preferably, co-crystals are formed with an active site DPP-IV ligand which is slowly hydrolysable and forms a covalent bond. One example for such an active site ligand is Diprotin A.

In a further embodiment of the present invention a method for determining the three-dimensional structure of a crystallized extracellular domain of mammalian DPP-IV to a resolution of 3.5 Å to 2.1 Å or better is provided, the method comprising
(a) crystallizing an extracellular domain of mammalian DPP-IV; and
(b) analyzing the extracellular domain of mammalian DPP-IV by X-ray diffraction to determine the three-dimensional structure of the crystallized extracellular domain of mammalian DPP-IV, whereby the three-dimensional structure of a crystallized extracellular domain of mammalian DPP-IV is determined to a resolution of about 3.5 Å to 2.1 Å or better.

The present invention further relates to a machine-readable data storage medium comprising a data storage material encoded with machine readable data which, when using a machine programmed with instructions for using said data, displays a graphical three-dimensional representation of a molecule or molecular complex comprising at least a portion of the extracellular domain of mammalian DPP-IV comprising the amino acids of SEQ ID NO:2, the extracellular domain comprising the ligand binding active site being defined by a set of points having a root mean square deviation of less than about 1.5Å from points representing the backbone atoms of said amino acids as represented by structure coordinates listed in Table 4.

The crystals of the invention, and particularly the atomic structure coordinates obtained therefrom, have a wide variety of uses. For example, the crystals and structure coordinates described herein are particularly useful for identifying compounds that interact with DPP-IV as an approach towards developing new therapeutic agents. Pharmaceutical compositions of said compounds can be developed, and said compounds can be used for the manufacture of a medicament comprising said compound for the treatment of IGT, type I and type II diabetes, obesity and cancer.

Therefore, the present invention also relates to the use of a crystal or a co-crystal of the invention for the identification and/or design of inhibitors of DPP-IV activity.

Moreover, the present invention relates to a method for identifying a compound that interacts with DPP-IV, comprising the steps of
(a) generating a three-dimensional model of DPP-IV using the structure coordinates listed in Table 4, a root mean square deviation from the backbone atoms of said amino acids of less than 1.5Å; and
(b) employing said three-dimensional model to design or select a compound that interacts with DPP-IV.
   In another aspect, the method further comprises the steps of
(c) obtaining the identified compound; and
(d) contacting the obtained compound with DPP-IV in order to determine the effect the compound has on DPP-IV activity.

The compound in these methods may be a compound that interacts with the active site of DPP-IV or may be a compound that interacts with an allosteric site of DPP-IV. Preferred are compounds which interact with the active site of DPP-IV. Even more preferred are compounds, which show an inhibitory effect on DPP-IV activity in step (d) of the methods of the present invention.

In a further aspect of the present invention the method for identifying a compound that interacts with DPP-IV is a computer-assisted method. Preferably, determining whether the compound is expected to bind to or interfere with the molecule or molecular complex includes performing a fitting operation between the compound and a binding site or substrate binding surface of the molecule or molecular complex, followed by computationally analyzing the results of the fitting operation to quantify the association between, or the interference with, the compound and the binding site. Optionally, the method further includes screening a library of compound. Optionally, the method further includes supplying or synthesizing the compound, then assaying the compound to determine whether it interacts with and has an effect on mammalian DPP-IV activity.

The present invention also relates to the compounds identified by the said methods for identifying a compound that interacts with DPP-IV.

The structure coordinates described herein can be used as phasing models in determining the crystal structures of additional native or mutated DPP-IV, as well as the structures of co-crystals of such DPP-IV with active site inhibitors or activators bound. The structure coordinates, as well as models of the three-dimensional structures obtained therefrom, can also be used to aid the elucidation of solution-based structures of native or mutated DPP-IVs, such as those obtained via NMR. Thus, the crystals and atomic structure coordinates of the invention provide a convenient means for elucidating the structures and functions of DPP-IV or other prolyl oligopeptidases.

For purposes of clarity and discussion, the crystals of the invention will be described by reference to specific DPP-IV exemplary apo crystals and co-crystals. Those skilled in the art will appreciate that the principles described herein are generally applicable to crystals of any mammalian DPP-IV, including, but not limited to DPP-IV.

Increased levels of glucagon like peptide 1 (GLP1) are beneficial for the decrease of plasma glucose in humans. The finding that DPP-IV is responsible for more than 95% of the degradation of GLP-1 led to an elevated interest in inhibition of this enzyme for the treatment of diabetes type II. Experiments in rats and humans have provided evidence that specific DPP-IV inhibition increased Cₘₐₓ, T_{1/2} and total circulating GLP-1 and decreased plasma glucose. It has been demonstrated that patients with impaired glucose-tolerance (IGT), type-II diabetes and with a secondary failure to respond to sulfonylurea treatment benefit from increased levels of GLP1 peptides. In addition GLP-1 is effective in type-I diabetic patients due to its glucagono-static effect. More recent investigations show a delay of gastric emptying that could have beneficial effects on satiety and might be relevant for the treatment of obesity. Protection of functional GLP-1 by inhibition of DPP-IV and concomitant activation of the GLP-1 receptor might therefore have a synergistic potential in anti-diabetic drug research (Holst, J.J. & Deacon, C.F. (1998). Inhibition of the activity of dipeptidyl-peptidase IV as a treatment for type 2 diabetes. *Diabetes* **47**, 1663-1670). Selective and orally available small molecule inhibitors of DPP-IV have been discovered and are now in clinical trials.

Therefore, in a further aspect of the present invention a pharmaceutical composition comprising the compound identified by the methods of the present invention as having an effect on DPP-IV activity, or pharmaceutically acceptable salts thereof, and a pharmaceutically acceptable carrier is provided.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, benzenesulfonic, toluenesulfonic, methanesulfonic, ethane disulfonic, oxalic, isethionic, and the like.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

"Stable compound" and "stable structure" are meant to indicate a compound that is sufficiently robust to survive isolation to a useful degree of purity from a reaction mixture, and formulation into an efficacious therapeutic agent.

Furthermore, a compound identified by the methods of the present invention as having an effect on DPP-IV activity for use as a therapeutic active substance, in particular for the treatment of diabetes type I, diabetes type II, IGT, obesity and cancer, is provided.

A further aspect of the present invention relates to the use of a compound identified by the methods of the present invention as having an effect on DPP-IV activity for the manufacture of a medicament for the treatment of diabetes type-I, diabetes type-II, IG, obesity, and cancer.

Having now generally described this invention, the same will become better understood by reference to the specific examples, which are included herein for purpose of illustration only and are not intended to be limiting unless otherwise specified, in connection with the following figures.

### Examples

Commercially available reagents referred to in the examples were used according to manufacturer's instructions unless otherwise indicated.

### Example 1

### DNA manipulation and sequence analysis

Preparation of DNA probes, digestion with restriction endonucleases, DNA ligation and transformation of E.coli strains were performed as described (Sambrook, J., Fritsch, E.F. & Maniatis, T. (1989). *Molecular Cloning: A Laboratory Manual.* Cold Spring Harbor Laboratory Press: Cold Spring Harbor, NY.). For DNA sequencing, the ABI PRISM BigDye Terminator Cycle Sequencing Ready Reaction Kit and ABI PRISM 310 Genetic analyzer were used. PCR were performed in the T3 Thermocycler (Whatman Biometra), using the Pfu polymerase (Stratagene).

### Production and Purification of recombinant human sDPP-IV in P. pastoris

The ectodomain of DPP-IV, residues 31-766 (sDPP-IV), was amplified by PCR using a cDNA and the oligonucleotides 5'-TGCTGGAATTCGGCACAGATGATGCTAC-3' (with an EcoRI site in bold) and 5'-GCA TGG TAC CTT GAG GTG CTA AG -3' (with a KpnI site in bold). Using the two new restriction sites, the amplified DNA fragment (SEQ ID NO:1) was cloned into pPICZα-A vector (Invitrogen) to create a fusion with the α-mating factor signal sequence for the secretion of the protein. The use of the EcoRI restriction site added the amino acids glutamine and phenylalanine to the N-terminus of sDPP-IV. The sequence was confirmed by sequencing. pPICZα-sDPP-IV was linearized with SacI, transformed by electroporation in P. pastoris strain GS115 and the phenotype of the colonies obtained was checked as recommended by the distributor Invitrogen.

Eight transformants with phenotype MutS were screened for the expression of DPP-IV. Colonies were grown at 30°C in YPD medium (1% yeast extract, 2% peptone, 2% glucose) with zeocin (100 µg/ml) to an OD₆₀₀ of 8-10. Cells were collected by centrifugation and resuspended in YP medium plus 2% methanol. The same amount of methanol was added every 24 h. After 48 h the medium of each clone was tested for activity (see below). sDPP-IV was then produced in a large scale culture using the transformed cell line with the highest activity per volume as described (Dale, G.E., D'Arcy, B., Yuvaniyama, C., Wipf, B., Oefner, C. & D'Arcy, A. (2000). Purification and crystallization of the extracellular domain of human neutral endopeptidase (neprilysin) expressed in Pichia pastoris. *Acta Crystallogr. D* **56**, 894-897).

Ten liters of the collected sDPP-IV supernatant of the selected transformed P. pastoris cell line was filtered and concentrated to 180 ml by crossflow ultrafiltration (skannette) using a 30 kDA filtration module (AGT Technology corporation). The concentrate was passed over a Sephacryl 200 XK 50/100 size exclusion column (5 x 95 cm, Pharmacia) equilibrated with 50 mM Tris-HCl pH 7.8 and 100 mM NaCl (S-buffer). Collected fractions were screened on SDS-PAGE and for activity. Fractions containing sDPP-IV were dialysed against 50 mM Tris-HCl pH 7.9. The protein solution was loaded on a Fractogel-TMAE column (2.6 x 13 cm, Merck) equilibrated with 50 mM Tris-HCl pH 7.9, washed with two column volumes of the same buffer and eluted with 500 ml of a linear gradient from 0 to 200 mM NaCl. Fractions containing sDPP-IV were dialysed against 20 mM sodium acetat pH 4.8. The protein solution was loaded on a Fractogel-COO⁻ column (1 x 12 cm, Merck) equilibrated with the same buffer and washed with two column volumes of this buffer. Bound proteins were eluted with 200 ml of a linear gradient from 50 to 500 M NaCl. The elution profile showed a major peak at 250 mM NaCl. Preparation of enzymatically deglycosylated sDPP-IV (sDPPIV_{deglycos}) was carried out prior to loading on the last gelfiltration column. 0.1% EndoF1-GST was added to the pooled fractions of DPP-IV and incubated for 20 h at 21°C. The concentrated protein solution was loaded on a Biosec size exclusion column (1.6 x 60 cm, Merck), that was equilibrated with S-buffer. Fractions were analyzed by SDS-PAGE, showing a purity > 95%. N-terminal sequencing showed that the protein was efficiently processed by the STE13 signal peptidase which cleaves off the α-mating factor. Preparation of the sDPPIV_{deglyco}:ADA-complex was performed by addition of a two times excess of ADA (Sigma Type IV, from calf intestinal Mucosa) and purification using a Biosec-size exclusion column.

The soluble extracellular domain of human dipeptidyl peptidase IV (sDPP-IV; residues 31-766) was expressed in the yeast Pichia pastoris. The protein was secreted at the low level of 1 mg/l as estimated from the total activity. As a first purification step the concentrated protein was passed through a size-exclusion column which removed the main fraction of contaminating peptides from the yeast-peptone medium. Sequential chromatography on anion- and cation-exchanger and a second size exclusion chromatography were used to get protein of 95% purity as judged by SDS-PAGE. The yield of pure protein was 0.3 mg/l growth medium. The purified protein shows essentially identical kinetic parameters and inhibition constants for known inhibitors of DPP-IV to those reported for the enzyme purified from human serum (Tables 1 and 2).

### Analytical methods

Purification of sDPP-IV was followed by electrophoresis on 10-20% Tricine SDS polyacrylamide gradient gels (Lammli, U.K. (1970). Cleavage of structural proteins during assembly of the head of bacteriophage T4. *Nature* **227**, 680-685). Protein concentrations were determined according to Bradford (Bradford, M.M. (1976). A rapid and sensitive method for the quantitation of microgram quantities of protein utilizing the principle of protein-dye binding. *Anal. Biochem.* **72**, 248-254) or for pure protein by absorption spectroscopy using the calculated molecular extinction coefficient at 280 nm of 193'920 M⁻¹cm⁻¹ (A₂₈₀ ^{0.1%}= 2.27cm²/mg; Pace, C.N., Vajdos, F., Fee, L., Grimsley, G. & Gray, T. (1995). How to measure and predict the molar absorption coefficient of a protein. *Protein Sci* **4**, 2411-2423). Analytical gel filtration chromatography was performed on a Superdex 200 12 HR 10/30 column (Pharmacia) equilibrated with S-buffer. The eluate was monitored with a miniDAWN multi-angle laser light scattering detector (Wyatt) and a refractive index-detector (Shodex), which allows the determination of the molecular weight and dispersity over the elution peak (Wyatt, P.J. (1993). Light scattering and the absolute characterisation of macromolecules. *Analytica Chimica Acta* **272**, 1-40). Sedimentation equilibrium runs in a Beckman analytical ultracentrifuge (model Optima XL A) were performed at 20°C and 9000 rpm sDPP-IV_{deglycos} and at 7000 rpm for sDPP-IV_{deglycos}:ADA-complex. The initial protein concentrations were 0.22 to 0.25 mg/ml in S-buffer. The absorption was followed at 280 nm. Assumed partial specific volumes for sDPP-IV of 0.729 cm³/g and ADA of 0.735 cm³/g were used to determine the molecular masses.

Free sulfhydryl groups were determined according the procedure described by Ellman (Ellman, G.L. (1959). Tissue sulfhydryl groups. *Arch. Biochem. Biophys.* **82**, 70-77) under denaturing conditions (0.3% SDS in 50 mM Tris pH 8.0).

### Thermostability measurements

The irreversible loss of activity after incubation at various temperatures was used as an operational criterion of the thermostability of sDPP-IV. Kinetics of irreversible heat inactivation were performed as described by Sterner et al. (Sterner, R., Kleemann, G.R., Szadkowski, H., Lustig, A., Hennig, M. & Kirschner, K. (1996). Phosphoribosyl anthranilate isomerase from Thermotoga maritima is an extremely stable and active homodimer. *Protein Sci.* **5**, 2000-2008) with a final protein concentration of 20 µg/ml in 50 mM potassium phosphate buffer at pH 7.5, containing 100 mM NaCl. The residual activity was determined by recording the initial velocity at 25°C of the enzyme-catalyzed reaction (see below) and the averaged values obtained were plotted against the incubation temperature.

### Biacore

DPP-IV was immobilized on a CM5 surface plasmon resonance sensor (Biacore) using standard amide coupling chemistry. The organic adlayer on this sensor type consists of carboxymethylated dextran (MW ≈100 kDA). After activation of the carboxylic acid groups using carbodiimide/N-hydroxysuccinimide solutions, the surface was contacted with a DPP-IV solution (80 µl) containing = 100 µg/ml protein in acetate buffer (10 mM, pH 4.5). The amount immobilized corresponded to a sensor response of roughly 10 000 RU. The surfaces of two flow cells were modified with protein. To suppress baseline drift - possibly due to slow dimer dissociation - the protein of one cell was cross-linked by short contact with carbodiimide/N-hydroxysuccinimide solution. This treatment did not influence the protein activity since binding constants determined with cross - linked protein were similar to those determined with non-cross-linked protein. Hepes buffer (0.01 M Hepes, pH 7.4, 0.15 M NaCl, 3 mM EDTA, 0.005% polysorbate 20 (v/v)) was used as the running buffer. Diprotin-A was disolved directly in this buffer. NVP-DPP728 was first dissolved in pure DMSO and then diluted into running buffer. The final inhibitor solution contained less than 0.1% DMSO. Binding experiments were carried out by contacting the immobilized protein surfaces with inhibitor solutions of varying concentrations at a flow rate of 10 µl/min or 30 µl/min. After each contact with inhibitor, the protein surfaces were regenerated by extensively washing with running buffer.

### Activity assay

The activity assay is based on the increase of fluorescence of products compared to the substrate Ala-Pro-7-amido-4-trifluoromethylcoumarin (Calbiochem, Smith, R.E., Reynolds, C.J. & Elder, E.A. (1992). The evolution of proteinase substrates with special reference to dipeptidylpeptidase IV. *Histochem. J.* **24**, 637-647). A 20 mM stock solution in 10 % DMF is stored at -20°C until use. Purification was followed by using a final substrate concentration of 50 µM and for the determination of kinetic parameters it was varied between 1.5 µM and 500 µM in the assay. DPP-IV activity assays were performed in 96 well plates in a total assay volume of 100 µl. The assay buffer consists of S-Buffer containing 0.1 mg/ml BSA. Fluorescence is detected in a Luminescence Spectrometer LS 50B (Perkin Elmer) at an excitation wavelength of 400 nm and an emission wavelength of 505 nm. Initial rate constants are calculated by best fit linear regression.

### Example 2

### Crystallization and Structure determination

For crystallization trials, sDPP-IV_{deglycos} was concentrated to approximately 10 mg/ml. A reduced factorial screen was carried out using the vapour diffusion method. Crystals were obtained with 20-25% PEG 3350, 200 mM MgCl₂, Tris pH 8.5 and 15% glycerol. The crystals were flash-frozen in liquid nitrogen and exhibit the orthorhombic space group P2₁2₁2₁ with cell dimensions of about 65 Å, 68 Å and 420 Å and one dimer per asymmetric unit. They diffract to a maximum of 2.3 Å resolution using synchrotron radiation and show rather high mosaicity (0.5-1.2°). Addition of 1 mM Diprotin-A prior to crystallization led to crystals of the complex. The mercury derivative was produced by cocrystallization with 0.1 mM HgCl₂.

Data collection was performed using synchrotron radiation (Swiss light source, SLS Villigen, Switzerland and ID14, ESRF Grenoble, France) as well as in-house facilities (search for heavy atom derivatives, evaluation of crystal quality) and processed with DENZO (Otwinowski, Z. (1993). Oscillation data reduction program. In *Proceedings of the CCP4 Study Weekend: Data Collection and Processing* (Wawyey, L., Isaacs, N. & Bailey, S., eds.). pp. 56-62, SERC Daresbury Laboratory, UK). Details of the data collection statistics are given in Table 3. All programs used are part of the CCP4 (CCP4 (Collaborative Computational Project, Number 4) (1994). The CCP4 suite: programs for protein crystallography. *Acta Crystallogr. D,* 760-763) suite, except where indicated. The structure was determined by multiwavelength anomalous dispersion (MAD) of the mercury derivative. One major mercury binding site per subunit (Cys 551, one of the two free SH-groups Cys301 and Cys551 that are located near the active site) was identified by inspection of the difference patterson maps calculated from the peak wavelength data and was subsequently refined using SHARP (De la Fortelle, E. & Bricogne, G. (1997). Maximum likelihood heavy-atom parameter refinement for multiple isomorphus replacement and multiwavelength anomalous diffraction methods. *Methods Enzymol.* **276**, 472-494). Location of the twofold non-crystallographic axis was performed using this mercury site and the program find2folds (Dunten, P. & Hennig, M. (2002). Locating non-crystallographic symmetry elements: The program Find2Folds. *Acta Crystallogr.* A58, C76). Further analysis revealed another site per subunit (Cys301) with less occupancy and the site branched in two positions with about 2.4Å distance. Subsequently the phases were improved by application of twofold averaging combined with solvent flattening and histogram matching as implemented in DM. The initial electron density at 2.6 Å resolution was readily interpretable and about 90% of the polypeptide chain could be built. The molecular model was refined against 2.3 Å data. Subsequent rounds of manual rebuilding and refinement with REFMAC (Murshudov, G.N., Vagin, A.A., Lebedev, A., Wilson, K.S. & Dodson, E.J. (1999). Efficient anisotropic refinement of macromolecular structures using FFT. *Acta Crystallogr. D* **55**, 247-255) led to a complete molecular structure of the polypeptide chain from residues Ser39 to Pro766. Details of the refined structures are reported in Table 3. Coordinates have been deposited in the Protein Data Bank PDB.

### Overall structure

The structure of human DPP-IV was solved by multiple anomalous dispersion (MAD) using a mercury derivative (see Table 3) and subsequently refined to an R-factor of 21.5 % at 2.1 Å resolution. The current model consists of all residues from Ser39 to Pro766 of the amino acid sequence of the expressed ectodomain of the protein.

A homodimer of DPP-IV is situated in the asymmetric unit (Figure 2). Dimerization is also observed in solution under various conditions and is required for activity. Each subunit is made of two domains, the catalytic domain with an α/β hydrolase fold containing the catalytic triad (Ser630, Asp708, His740) and a domain with an eight-bladed β-propeller fold, the β-propeller domain (Figure 2). The assignment of the secondary structure is given in Figures 1 and 2. The only other known crystal structure of this class of enzyme is prolyl-oligopeptidase (POP) determined by Fülop (Fülop, V., Bocskei, Z. & Polgar, L. (1998). Prolyl oligopeptidase: an unusual beta-propeller domain regulates proteolysis. *Cell* **94**, 161-170; pdb entry 1qfm) POP also has an α/β-hydrolase and a β-propeller domain, but is monomeric and the β-propeller consists of seven repeats only (Figure 3C).

### Catalytic Domain

The catalytic domain is built up of residues Gln508 to Pro766 and contains a central eight-stranded parallel β-sheet that is flanked by 12 helices known as α/β hydrolase fold. 21% sequence identity to POP indicates significant structural homology (Figure 1) and superposition of the central α-helix, carrying the catalytic Ser630 on its first turn, with the corresponding structure of POP gives an r.m.s deviation of 2.5 Å² for 238 residues. The catalytic domain is connected to the β-propeller by an N-terminal 15 residue linker, which is considerably shorter than the corresponding 76 residue region in POP. The residues lacking in DPP-IV are, however, replaced structurally and functionally by the C-terminal part of the catalytic domain of the second subunit of the dimer.

### β-propeller domain

The β-propeller domain is formed by the residues Lys56 to Asn497. The preceding N-terminal residues Ser39 to Leu55 form a loop structure with a small α-helix (α1*, Figure 1) at the surface and in close proximity to the first residues of the catalytic domain. The β-propeller domain consists of an eight-fold repeat of a four-stranded antiparallel β-sheet motif (blade, Figure 3). The blades are in circular arrangement such that they form a solvent filled tunnel with a diameter of about 13 Å.

The β-propeller domain in DPP-IV does not form a joint β-sheet motif (described as molecular "velcro"; Fülop, V. & Jones, D.T. (1999). Beta propellers: structural rigidity and functional diversity. *Curr. Opin. Struct. Biol.* **9**, 715-721; Paoli, M. (2001). Protein folds propelled by diversity. *Prog. Biophys. Mol. Biol.* **76**, 103-130), but rather the blades show a regular arrangement (β1/1 to β7/4 or β8/4) (Figure 3A) around the central axis forming a ring system that is not closed.

DPP-IV deviates from the regular β-propeller fold by additional secondary structural elements. An anti-parallel β-sheet is inserted in blade two between the strands one and two. The tip of the turn carries the residues Arg125 that forms a salt bridge with Glu205, that is situated at the C-terminal turn of an α-helix (residues Trp154 to Thr199), that is inserted between the first and second strands of blade 4. Arg125, Glu205 and the neighboring Glu204 form a significant part of the substrate binding site and are mainly responsible for the substrate specificity. An further anti-parallel β-sheet motif formed by residues Asp230 to Asn263 is inserted between the strands three and four of blade four (Figure 3B). This structural element forms a significant part of the dimer interface (see below).

Whereas the N-terminal β-sheet structure of the propeller has shorter strands and is somewhat tilted, the loop connecting the first and second β-sheet is longer, shows high temperature factors and may reduce the rigidity of the propeller architecture. The reduced stability of the circular domain structure at this position might be compensated by an extended hydrophobic cluster that consists of Ile63, Leu69, Ile76, Phe89, Leu90, Phe95, Phe98, Ile107, Ile114, Tyr135, Leu137 and Leu142, and a salt bridge between Arg61 and Asp104 and a hydrogen bond between the main chain NH of Arg61 and Tyr105. This distortion leads to a reduced height of the propeller at the positions between blade one and two (Figure 3B).

As no residues from the α/β hydrolase domain fill this up, a cleft between the two domains of the DPP-IV molecule is formed with a diameter of about 15 Å enabling access to the catalytic site (Figure 4). Therefore, we propose that DPP-IV has two independent ways for the substrate and product to access and leave the active site, a cleft between the domains and the tunnel through the β-propeller. The open cleft may enable large peptides and partially folded proteins to access the active site. The more narrow tunnel could be an exit for the cleaved dipeptides (Figure 4). The crystal structure of POP shows that the cleft between the two domains does not exist and the tunnel through the β-propeller is more narrow with about 4 Å compared to about 13 Å for DPP-IV (Figure 3A and 3C). This structural difference is supported by the observation that DPP-IV can process much larger substrates compared to POP. Peptides with a length of up to about 80 residues appear to be good substrates of DPP-IV. Larger proteins may also be cleaved depending on their tertiary structure. POP is reported to hydrolyse substrates with a maximum size of about 30 residues, only (Polgar, L. (1992). Unusual secondary specificity of prolyl oligopeptidase and the different reactivities of its two forms toward charged substrates. *Biochemistry* **31**, 7729-7735.). As the diameter of the β-propeller tunnel in POP is significantly smaller, it is conceivable that the structure of DPP-IV represents a more open and active enzyme.

The β-propeller motif has been found in several further proteins, but no or only low sequence homology could be demonstrated (Polgar, L. (1992). Unusual secondary specificity of prolyl oligopeptidase and the different reactivities of its two forms toward charged substrates. *Biochemistry* **31**, 7729-7735.). A search of the PDB for homologous structures gave the best results for clathrin (7 blades, ter Haar, E., Musacchio, A., Harrison, S.C. & Kirchhausen, T. (1998). Atomic structure of clathrin: a beta propeller terminal domain joins an alpha zigzag linker. *Cell* **95**, 563-573), methylamine dehydrogenase (7 blades, Chen, L., Doi, M., Durley, R.C., Chistoserdov, A.Y., Lidstrom, M.E., Davidson, V.L. & Mathews, F.S. (1998). Refined crystal structure of methylamine dehydrogenase from Paracoccus denitrificans at 1.75 Å resolution. J. *Mol. Biol.* **276**, 131-149) and nitrite reductase (8 blades, Nurizzo, D., Cutruzzola, F., Arese, M., Bourgeois, D., Brunori, M., Cambillau, C. & Tegoni, M. (1998). Conformational changes occurring upon reduction and NO binding in nitrite reductase from Pseudomonas aeruginosa. *Biochemistry* **37**, 13987-13996), but no DPP-IV related function can be expected.

### Active site

The catalytic triad (Ser630, Asp708, His740) is located in a large cavity at the interface of the two domains. Ser630 is found at the tip of a very sharp turn between β-strand 5 and helix C, called the nucleophile elbow, which is a characteristic of hydrolases of the α/β type (Ollis, D.L., Cheah, E., Cygler, M., Dijkstra, B., Frolow, F., Franken, S.M., Harel, M., Remington, S.J., Silman, I., Schrag, J. & et al. (1992). The alpha/beta hydrolase fold. *Protein Eng.* **5**, 197-211). The serine hydroxy group is well exposed to solvent and hydrogen bonded to the catalytic imidazole group of His740 on one side (2.6 Å) and accessible to the substrate on the other side. His740 is found in the middle of a loop between β-strand 8 and helix F. With a distance of 2.75 Å to Nε of the imidazole ring, one of the oxygen atoms of Asp708 is hydrogen bonded to His740 and completes the catalytic triad (Figure 5). The other oxygen atom of the carboxylate group of Asp708 is coordinated by two main chain NH-groups (Val711 and Asn710). Thus, the location and geometry of the triad are very similar to that found in other α/β hydrolases with the "handedness" opposite to the classical serine peptidases.

The negatively charged oxyanion of the tetrahedral intermediate is stabilized by the main chain NH-group of Tyr631 and by the hydroxy group of Tyr547 (Figure 5). Furthermore, the structure shows that the two Gly628 and Gly632 are important for the formation of the sharp turn to bring the catalytic residue Ser630 in the correct position. This is in accordance with mutagenesis studies on rat DPP-IV (Ogata, S., Misumi, Y., Tsuji, E., Takami, N., Oda, K. & Ikehara, Y. (1992). Identification of the active site residues in dipeptidyl peptidase IV by affinity labeling and site-directed mutagenesis. *Biochemistry* **31**, 2582-2587) showing that the sequence Gly₆₂₈-X-Ser₆₃₀-Tyr₆₃₁-Gly₆₃₂ is essential for DPP-IV activity.

### Substrate binding

The substrate binding site of DPP-IV is indicated by the inhibitor Diprotin-A (Ile-Pro-Ile). It is a slowly hydrolysable substrate with k_{cat}/K_{M} a factor of 10 less than Ile-Pro-4-nitroanilides (Rahfeld, J., Schierhorn, M., Hartrodt, B., Neubert, K. & Heins, J. (1991). Are diprotin A (Ile-Pro-Ile) and diprotin B (Val-Pro-Leu) inhibitors or substrates of dipeptidyl peptidase IV? *Biochim. Biophys. Acta* **1076**, 314-316). Inspection of the electron density map shows the ligand covalently bound to the active site Ser630 of the enzyme in both subunits. The N-terminal Ile (P2) and Pro residues (P1) are well defined and enable a detailed analysis of the interaction with the substrate binding site (according to the notation of Schechter; Schechter, I. & Berger, A. (1968). On the active site of proteases. 3. Mapping the active site of papain; specific peptide inhibitors of papain. *Biochem. Biophys. Res. Commun.* **32**, 898-902). Less well defined electron density is found for the C-terminal Ile (P1'), but in subunit B the conformation of this part of the ligand could also be observed (Figure 5). The side chain Nε of the catalytic His740 is in hydrogen bonding distance to the NH-group of P1' (2.90 Å) and to the Oy of the Ser630 side chain (2.74 Å).

DPP-IV hydrolyzes oligopeptides and proteins from the N-terminus, cleaving dipeptide units when the second residue is proline, hydroxyproline, dehydroproline, pipecolic acid or alanine. In both subunits the proline in position P1 of Diprotin-A is in the trans-configuration and fits optimally into the pocket of the active site as expected (Fischer, G., Heins, J. & Barth, A. (1983). The conformation around the peptide bond between the P1- and P2-positions is important for catalytic activity of some proline-specific proteases. *Biochim. Biophys. Acta* **742**, 452-462). The S1 pocket is formed by Val711, Val656, Tyr662, Tyr666, Tyr659 and Tyr631 which shape a well defined hydrophobic pocket that would be filled by proline much better than by alanine. Gly is also accepted, but with very low k_{cat}/K_{M} values (Brandt, W., Lehmann, T., Thondorf, I., Born, I., Schutkowski, M., Rahfeld, J.U., Neubert, K. & Barth, A. (1995). A model of the active site of dipeptidyl peptidase IV predicted by comparative molecular field analysis and molecular modelling simulations. *Int. J. Pept. Protein Res.* **46**, 494-507). All other naturally ocurring amino acids residues cannot occupy position P1. Either the side chains are too bulky or hydrophilic. The side chains of the residues P2 and P1' point into the solvent and no interaction with the protein occurs. This explains the large diversity of amino acids accepted in substrates at these positions.

Essential for substrate binding and catalysis is the N-terminus of the substrates, which has to be unprotected and protonated (Brandt, W., Ludwig, O., Thondorf, I. & Barth, A. (1996). A new mechanism in serine proteases catalysis exhibited by dipeptidyl peptidase IV (DP IV) - Results of PM3 semiempirical thermodynamic studies supported by experimental results. *Eur. J. Biochem.* **236**, 109-114). The Diprotin-A complex shows that the terminal -NH₃⁺ -group is held very precisely in position by strong interactions with the carboxylates of Glu205 and Glu206 (Figure 5). A third glutamate, Glu204, stabilizes this substrate recognition site by an hydrogen bonding network with the backbone NH of Arg125, His126 and Ser127 as well as the hydroxy group of Ser127. Importance of the glutamate residues is confirmed by single point mutations that abolish DPP-IV activity (Abbott, C.A., McCaughan, G.W. & Gorrell, M.D. (1999). Two highly conserved glutamic acid residues in the predicted beta propeller domain of dipeptidyl peptidase IV are required for its enzyme activity. *FEBS Lett.* **458**, 278-284). The double Glu-motif is located at the end of an helical segment (α2* in Figure 1, see also Figure 3) that is highly conserved in the DPP IV-like gene family (Asp-Trp-X-Tyr-Glu-Glu-Glu-X). The helix represents a deviation from the regular β-sheet architecture of the β-propeller domain (Figures 1 and 3A). The superposition of the active sites of the exopeptidase DPP-IV complexed with Diprotin A and the endopeptidase POP complexed with an octapeptide (Fülöp, V., Szeltner, Z., Renner, V. & Polgar, L. (2001). Structures of prolyl oligopeptidase substrate/inhibitor complexes. Use of inhibitor binding for titration of the catalytic histidine residue. J. *Biol. Chem.* **276**, 1262-1266) shows clear differences. The octapeptide substrate of POP coincides with the double Glu-motif in DPP-IV indicating that this additional structural element functions is very important for substrate selection. Thus, the double Glu-motif is a recognition site for the N-terminus of substrates and restricts the cleavage to dipeptides and the S1 pocket provides an optimal binding to proline and alanine residues leading to a highly specific peptidase.

### Mode of inhibition by Diprotin-A

Inspection of the electron density of the bound inhibitor shows a covalent linkage to Ser630 and a sp³-configuration for the C-atom of the former carbonyl-group of the scissile peptide. Consequently, a tetrahedral intermediate is observed in the complex structure with the substrate Diprotin A (Figure 5) with the oxyanion stabilized by hydrogen bonds to the hydroxy group of the side chain of Tyr547 (2.80 Å) and the main chain amine of Tyr631 (3.38 Å). As much catalytic power of serine proteases derives from its preferential binding of this transition state, the tetrahedral intermediate is a well-defined but high energy state with a short lifetime and its accumulation must be a result of a kinetic barrier.

Inspection of the active site structure reveals several structural features that are special to Diprotin A and may lead to the competitive inhibition of this substrate. First, the two hydrophobic isoleucine side chains point into the same direction in proximity and, therefore, this hydrophobic interaction may stabilize the tripeptide in a unsuitable conformation for the progress of the reaction. Second, a large network of salt bridges and hydrogen bonds stabilize the complex. It involves the carboxyl groups of Glu205/206 that interact with the N-terminus of the tripeptide, but Glu205 makes another salt bridge to Arg125 and this in turn interacts with the C-terminal carboxyl group of the tripeptide (Figure 5). It is obvious that this interaction is only present in tripeptidic substrates and may stabilize the observed intermediate by protection of the leaving group.

### Dimerization

The crystal structure as well as analytical ultracentrifugation indicate dimeric oligomerization for deglycosylated sDPP-IV with a molecular weight of 169 kDa and non-crystallographic twofold symmetry (Figure 2). Six percent or 1837 Å² of the total solvent accessible surface area of each subunit is buried in the dimer interface (program XSAE, Broger, C. personal communication). This interface is mainly build up by two extra β-strands (β1* and β2*) in the loop between the strands two and three of the fourth blade of the β-propeller domain (Figure 3A and 3B). Further interaction is provided by the α/β hydrolase domain with helix αE, β-strand β8 and helix αF with mainly hydrophobic interactions. The active site is very close to this dimer interface (Figure 2) with His740 from the catalytic triad located in the loop connecting αF and β7 (Figure 1). Consequently disruption of the dimer interface would also strongly affect the catalytic activity and dimerization is required for activity.

### Stability of DPP-IV

As a cell surface protein DPP-IV is extremely stable. Consequently the recombinant sDPP-IV shows a half life of 5 min at 71°C in irreversible heat inactivation experiments independent of the protein concentration and the degree of glycosylation indicating high thermal stability. In unfolding experiments (Lambeir, A.M., Diaz Pereira, J.F., Chacon, P., Vermeulen, G., Heremans, K., Devreese, B., Van Beeumen, J., De Meester, I. & Scharpe, S. (1997). A prediction of DPP IV/CD26 domain structure from a physicochemical investigation of dipeptidyl peptidase IV (CD26) from human seminal plasma. *Biochim. Biophys. Acta* **1340**, 215-2) with protein purified from human seminal plasma DPP-IV retained its native conformation up to 8 M Urea.

The crystal structure points to several factors that may contribute to this stability. Firstly, the structural organization as a dimer with an extended hydrophobic interface stabilizes the molecule as shown for several other proteins (Thoma, R., Hennig, M., Sterner, R. & Kirschner, K. (2000). Structure and function of mutationally generated monomers of dimeric phosphoribosylanthranilate isomerase from Thermotoga maritima. *Structure Fold. Des.* **8**, 265-276). Secondly, we observe five disulphide bonds and two free sulfhydryl groups by SH titration experiments under denaturing conditions that are now confirmed by the X-ray structure. All disulphide bridges in the β-propeller connect different strands in blades or stabilize loops (Cys444/Cys447; Cys385/Cys394, Cys454/Cys472, Cys328/Cys339). One disulfide bond is observed in the (α/β-hydrolase domain (Cys649/Cys762) and covalently links the C-terminal helix αF to the core of the α/β hydrolase domain.

### Glycosylation

sDPP-IV overexpressed in P. pastoris shows a decreasing molecular weight over the elution peak in the analytical gelfiltration as analyzed online with a multiangle laser light scattering detector. In contrast, sDPP-IV deglycosylated with EndoF glycosidase shows an uniform molecular weight over the whole peak range, because of the specific cleavage of asparagine linked oligomannose after the first N-acetylglucoamines residue (GlcNAc). This leads to a decrease in molecular weight of 20 kDa as estimated by SDS-PAGE. Crystals suitable for X-ray diffraction are only observed for deglycosylated sDPP-IV and structure analysis shows four GlcNAc with interpretable electron density at the positions N85, N150, N229 and N281 in subunit A. In subunit B, again N85, N150 and N229 are visible, but no electron density was found for N281 and an additional site could be identified at N92. The GlcNAc of N85 is involved in a crystal contact in both subunits.

DPP-IV expressed in human has a more complex type of glycosylation compared to P. pastoris (Cremata, J., Montensino, R., Quintero, O. & Garcia, R. (1998). Glycosylation Profiling of Heterologous Proteins. In *Pichia* Protocol (Higgins, D.R. & Cregg, J.M., eds.), vol. 103. pp. 95-106, Humana Press: Totowa, New Jersey) and contains terminal sialic acid, however, this seems not to be a requirement for correct folding as shown here.

### Interaction with ADA

Adenosine deaminase (ADA; EC 3.5.4.4) is a 41 kDa protein expressed in all mammaliantissues that catalyzes the deamidation of adenosine and 2'-deoxyadenosine to inosine and 2'-deoxyinosine, respectively. It is important for the regulation of the extracellular concentration of adenosine and for the regulation of the immune response. ADA is involved in T cell activation in general and the pathogensis of autoimmune disorders (such as rheumatoid arthritis) as well as the mechanism of immunodeficiency disease (such as SCID or AIDS). Binding of the soluble extracellular ADA is a unique property of DPP-IV molecules of higher mammals and is not observed in mouse nor rat DPP-IV (Iwaki-Egawa, S., Watanabe, Y. & Fujimoto, Y. (1997). CD26/dipeptidyl peptidase IV does not work as an adenosine deaminase-binding protein in rat cells. *Cell Immunol.* **178**, 180-186). Using analytical ultra-centrifugation, we observe a 1:1 complex of a ADA molecules with a sDPP-IV subunit giving a molecular weight of 252 kDa. Surface plasmon resonance (Biacore) measurements show a binding constant of 3.15 ± 2 nM to ADA from bovine with a very low dissociation rate (k_{off}=8.75*10⁻⁵ s⁻¹, kₒₙ=2.98*10⁴ M⁻¹s⁻¹) indicating a strong interaction.

Mutagenesis studies (Abbott, C.A., McCaughan, G.W., Levy, M.T., Church, W.B. & Gorrell, M.D. (1999). Binding to human dipeptidyl peptidase IV by adenosine deaminase and antibodies that inhibit ligand binding involves overlapping, discontinuous sites on a predicted beta propeller domain. *Eur. J. Biochem.* **266**, 798-810; Dong, R.P., Tachibana, K., Hegen, M., Munakata, Y., Cho, D., Schlossman, S.F. & Morimoto, C. (1997). Determination of adenosine deaminase binding domain on CD26 and its immunoregulatory effect on T cell activation. J. *Immunol.* **159**, 6070-6076) identified two important regions in DPP-IV LeU₃₄₀-Val₃₄₁-Ala₃₄₂-Arg₃₄₃ (at the beginning of β5/4) and Leu294 (α4, at the end of blade 4) and a less important region Glu₃₃₂-Ser₃₃₃-Ser₃₃₄-Gly₃₃₅-Arg₃₃₆ (loop region, at the end of β5/3) that are all located at the surface of the β-propeller domain (Figure 1). Mutation to amino acids found in rat DPP-IV reduces binding affinity to ADA. These residues form a binding site that is located far away from the active site (Figure 2) confirming the independence of DPP-IV activity on ADA binding (Table 1; De Meester, I., Vanham, G., Kestens, L., Vanhoof, G., Bosmans, E., Gigase, P. & Scharpe, S. (1994). Binding of adenosine deaminase to the lymphocyte surface via CD26. Eur. J. *Immunol.* **24**, 566-570). It is concluded that the function of DPP-IV is the localization and orientation of ADA for proper catalysis. The structure gives an indication for the orientation and localization at the cell surface, because the N-terminus must be close to the membrane and the ADA binding would be on the opposite site of the molecule - pointing away from the cell surface (Figure 2). Further, there would be sufficient space enabling interaction of ADA to the A1-adenosine receptor (Ciruela, F., Saura, C., Canela, E.I., Mallol, J., Lluis, C. & Franco, R. (1996). Adenosine deaminase affects ligand-induced signaling by interacting with cell surface adenosine receptors. *FEBS* Left. **380**, 219-223) which probably plays an important role in the ontogenesis of immune tissues. This view would also support the hypothesis proposing a link for cell-cell interaction via the binding of DPP-IV, ADA and A1-adenosine.

### Biological Implications

The crystal structure of DPP-IV at 2.1 Å resolution reveals a V-shaped dimeric molecule with an extended dimer interface fostering the conformation of the overall molecule. The membrane association and stability of DPP-IV is used for binding of other proteins like ADA in order to achieve localization without disturbance of the enzymatic functionality.

Analysis of the complex with Diprotin A shows key structural features for proline specific exopeptidase specificity and activity. The negative charge of the double Glu motif guides the N-terminus of the peptide to the active site and fixes the substrate in the correct position for cleavage. The distance between this motif and the catalytic Ser630 limits the cleavage to dipeptides and the S1 pocket can just adopt proline or with less affinity alanine as side chains.

The low turnover rate of Diprotin A may be explained by the hydrophobic interaction of the two Ile-residues in the P2 and P1' positions as well as an extensive salt bridge cluster that involves the negatively charged C-terminus of Diprotin A. This structural information will aid the design of new specific inhibitors.

The active site is very accessible to the solvent by two entrances explaining that peptides can be cleaved by DPP-IV with almost no size limitation. A second access to the active site by the tunnel of the β-propeller domain is large enough to enable the release of the cleaved dipeptides. This structural arrangement certainly improves the catalytic turnover and is in great contrast to the crystal structure of POP that shows a much more narrow tunnel and no further access to the active site.

For most of the special features of DPP-IV namely dimerization, regulation of substrate access via two entrances, recognition of the substrate (double Glu-motif) and interaction with other proteins like ADA the β-propeller domain plays a key role. Thus, DPP-IV is an excellent example that the β-propeller fold can be tailored to adapt to different functionality.

**Table 1.**

| Enzyme Kinetic Constants of DPP-IV | | | |
|---|---|---|---|
| proteins | k_{cat}* | K_{M}* | k_{cat}/K_{M} |
| | (s⁻¹) | µM) | µM⁻¹s⁻¹) |
| sDPP-IV_{deglycos} | 43.1 | 17.2 | 2.51 |
| sDPP-IV_{glycos} | 37.3 | 15.5 | 2.41 |
| sDPP-IV_{deglycos}./ADA | 39.6 | 14.8 | 2.68 |

| | | | |
|---|---|---|---|
| * analyzed using Lineweaver-Burk plots; buffer: 50 mM Tris/HCl pH 7.8, containing 100 mM NaCl, 0.1 mg/ml BSA and 0.5% Dimethyl-formamid; temperature: 25°C | | | |

**Table 2.**

| K_{I} and K_{D} Values of DPP-IV Inhibitors | | | | |
|---|---|---|---|---|
| | K_{I} | K_{D} | kₒₙ | k_{off} |
| | µM) | µM) | M⁻¹s⁻¹ | s⁻¹ |
| Ile-Pro-Ile | 4.63^{‡} | 3.8^{†} | - | - |
| NVP-DPP728 | 0.006^{‡} | 0.002^{†} | 1.36*10⁶^{†} | 2.48*10⁻³^{†} |
| NVP-DPP728_{(Lit.)} | 0.011 | 0.010 | 1.3*10⁵ | 1.3*10⁻³ |

| | | | | |
|---|---|---|---|---|
| ^{†}measured with biacore; buffer: 0.01 M Hepes, pH 7.4, containing 0.15 M NaCl, 3 mM EDTA, 0.005% polysorbate 20 (v/v) | | | | |
| ^{‡} temperature: 25°C; in assay buffer (see Table 1); glycosylated sDPP-IV | | | | |
| * Hughes, T.E., Mone, M.D., Russell, M.E., Weldon, S.C. & Villhauer, E.B. (1999). NVP-DPP728 (1-[[[2-[(5-cyanopyridin-2-yl)amino]ethyl]amino]acetyl]-2-cyano-(S)-pyrrolidine), a slow-binding inhibitor of dipeptidyl peptidase IV. *Biochemistry* 38, 11597-11603 | | | | |

**Table 3.**

| Crystallographic Data and Refinement Statistics | | | | | |
|---|---|---|---|---|---|
| Data set | MAD Remote | MAD Peak | MAD Inflection | Apo | Diprotin-A complex |
| Wavelength | 0.992 | 1.0065 | 1.009 | 0.9765 | 0.92 |
| X-ray source | SLS | SLS | SLS | ID14,ESRF | SLS |
| Detector | MAR IP^{a} | MAR IP^{a} | MAR IP^{a} | Quantum CCD | MAR CCD |
| Exposure time/frame (s) | 10 | 10 | 10 | 2 | 4 |
| angular increment per frame (°) | 2.0 | 2.0 | 2.0 | 0.25 | 0.25 |
| total rotation range (°) | 110 | 136 | 140 | 130 | 130 |
| crystal to detector distance (mm: | 410 | 410 | 410 | 240 | 260 |
| unit cell parameters a,b,c (Å) | 65.2; 68.7; 420.1 | 65.2; 68.7; 420.1 | 65.2; 68.7;420.1 | 65.5; 68.2; 419.3 | 65.1; 67.1; 419.6 |

| data reduction | | | | | |
|---|---|---|---|---|---|
| Maximum Resolution (Å) | 2.6 | 2.6 | 2.6 | 2.1 | 2.5 |
| No. of measurements | 212619 | 263910 | 276921 | 234528 | 171090 |
| No. of unique reflections | 58627 | 59544 | 59939 | 87 113 | 64208, |
| completeness (%)* | 97.5 (99.4) | 99.9 (100.0) | 99.9 (99.9) | 82.9 (72.3) | 97.5 (99.4) |
| Rsym *,^{b}* | 9.1 (15.9) | 9.0 (18.1) | 8.6 (14.2) | 8.4 (26.8) | 9.1(15.9) |

| heavy-atom refinement paramet | | | | | |
|---|---|---|---|---|---|
| f(e)/f'(e) | -7.0/9.5 | -8.0/9.8 | -12.1/5.0 | | |
| Phasing power^{c} (anomalous) | 0.95 | 1.0 | 0.7 | | |

| Refinement statistics | | | | | |
|---|---|---|---|---|---|
| resolution range (A) | | | | 20-2.1 | 30-2.5 |
| R_{cryst} (R_{free})^{d} (%) | | | | 21.5(26.5) | 22.5(28.2) |
| No. of protein atoms^{e} (mean B | | | | 11 962 | 11 962 (27.1) |
| in Å²) | | | | (34.6) | |
| No. of water molecules | | | | 322 (33.4) | 268(25.0) |
| No. of ligand/heavy atoms (mean B in Å²) | | | | 6 (77.3) | 24 (28.3) |
| No. of NAG atoms (mean B in Å²) | | | | 112 (59.0) | 98 (51.4) |
| rmsd^{f} bonds (Å²) | | | | 0.018 | 0.019 |
| Rmsd^{f} angles (°) | | | | 1.86 | 2.07 |

| | | | | | |
|---|---|---|---|---|---|
| ^{a} Marresearch image plate detector, diameter 345mm, 100µm pixel size | | | | | |
| * Values in parentheses are statistics for highest resolution bin. | | | | | |
| ^{b} R_{sym} = ΣₕΣᵢ\|Iᵢ(h)-<I(h)>\|/_{ΣhΣi}(h), where Iᵢ(h) und <I(h)> are the ith and mean measurement of the intensity of reflection h. | | | | | |
| ^{c} Phasing power =ΣhF_{H}(h)Σₕ\|F_{D}(h)- \|F_{N}(h) + F_{H}(h)\|\|. | | | | | |
| ^{d} Σₕ \|\|F_{obs}\| - \|F_{calc}\|\|Σₕ\|F_{obs}\|, where \|F_{obs}\| and \|F_{calc}\| are the observed and calculated structure factor amplitudes for the reflection h, applied to the working (R_{cryst}) and test (R_{free})sets, respectively. | | | | | |
| ^{e} Non-hydrogen atoms, only. | | | | | |
| ^{f} rmsd: root mean square deviation from mean. | | | | | |

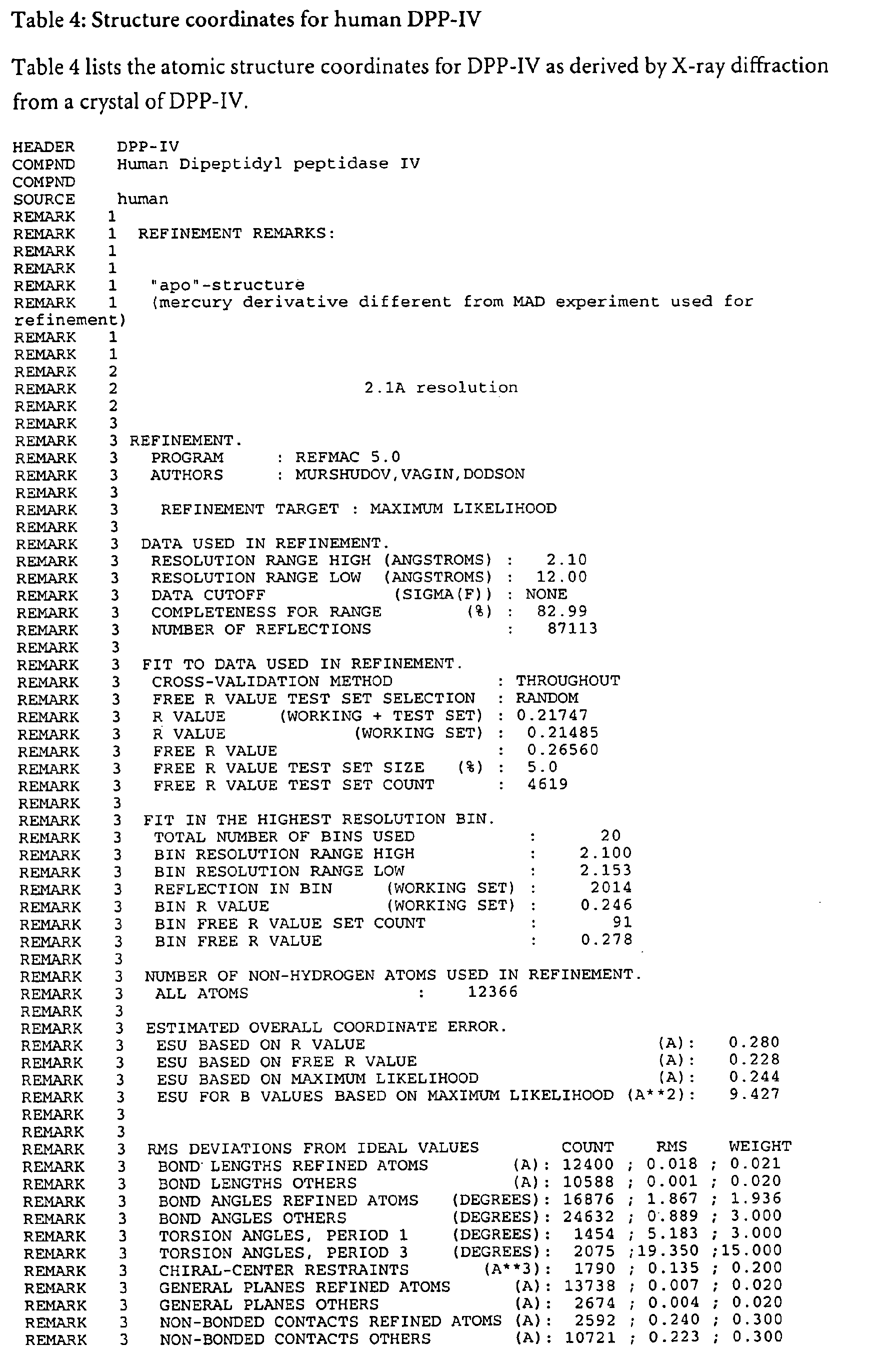

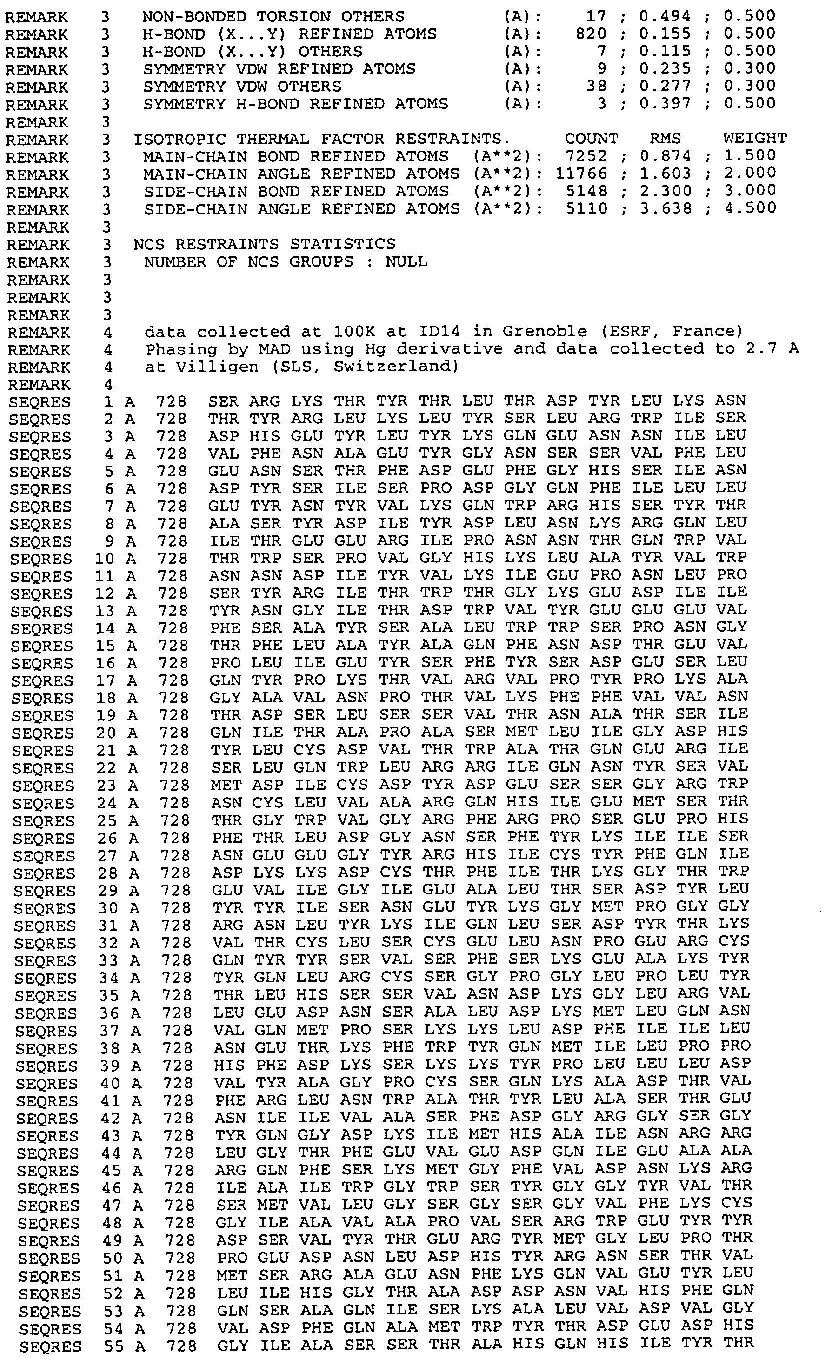

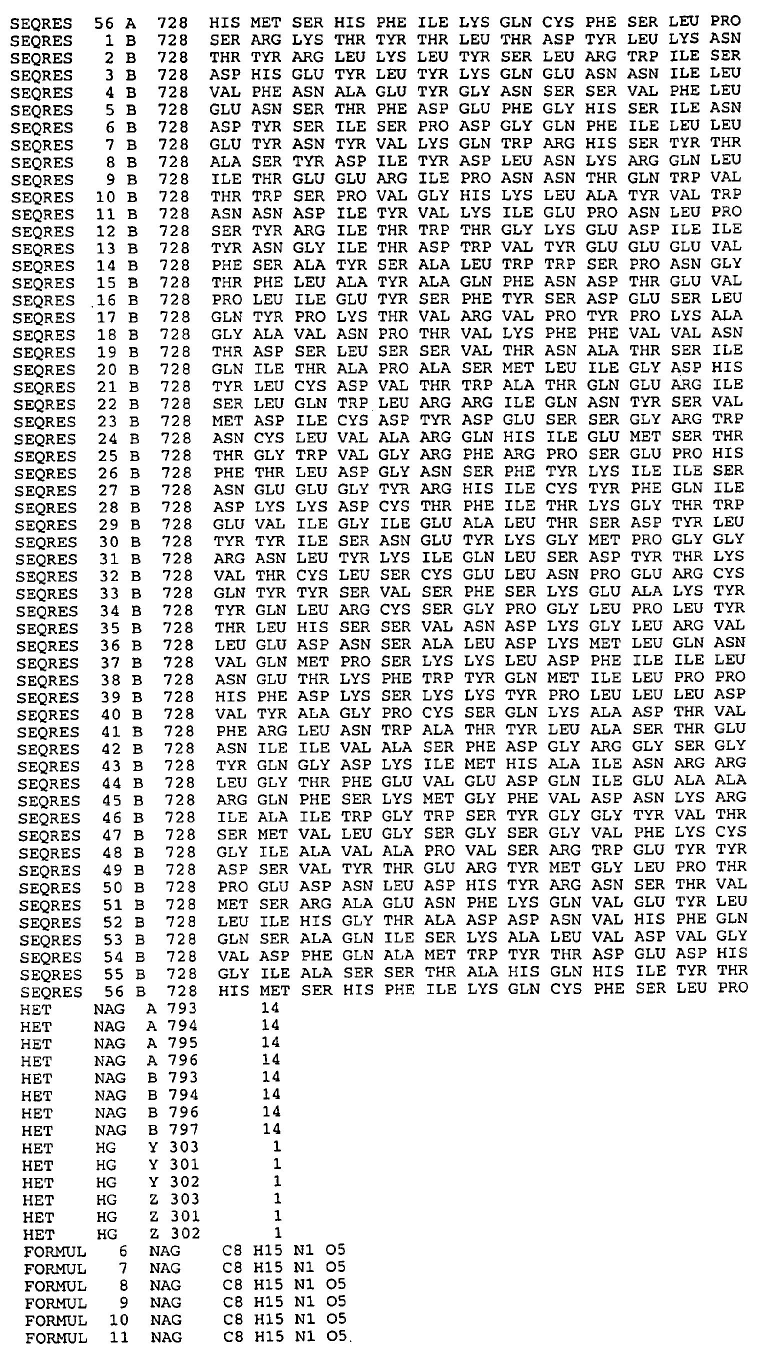

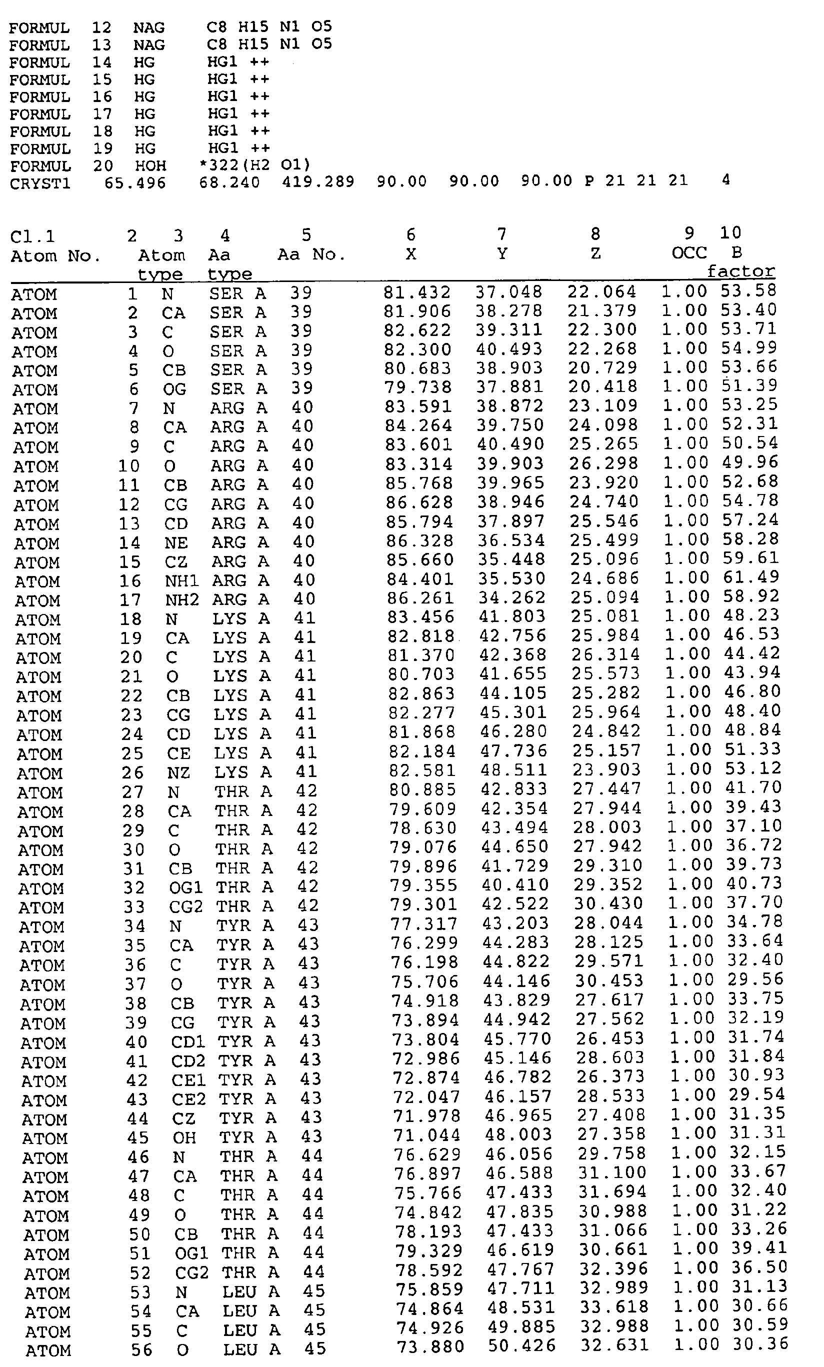

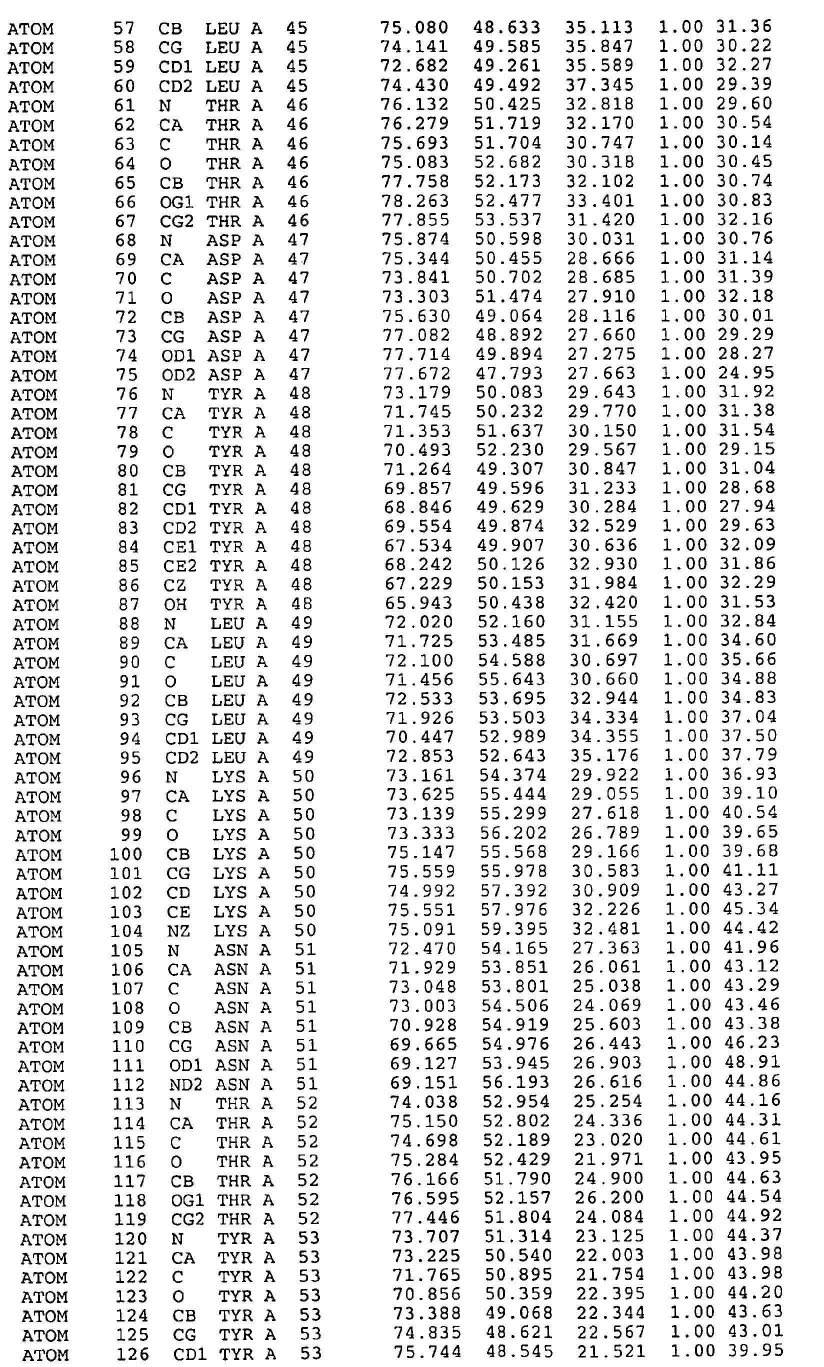

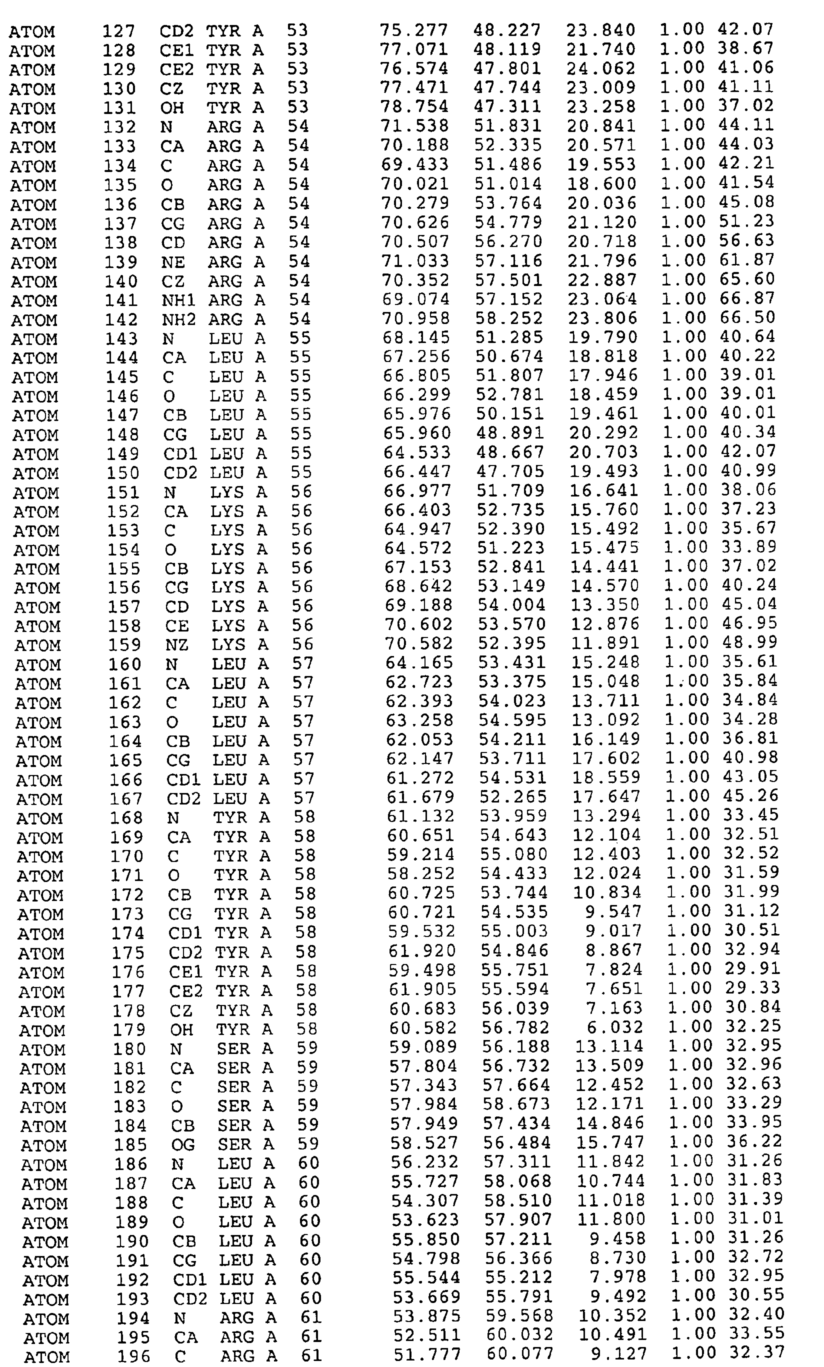

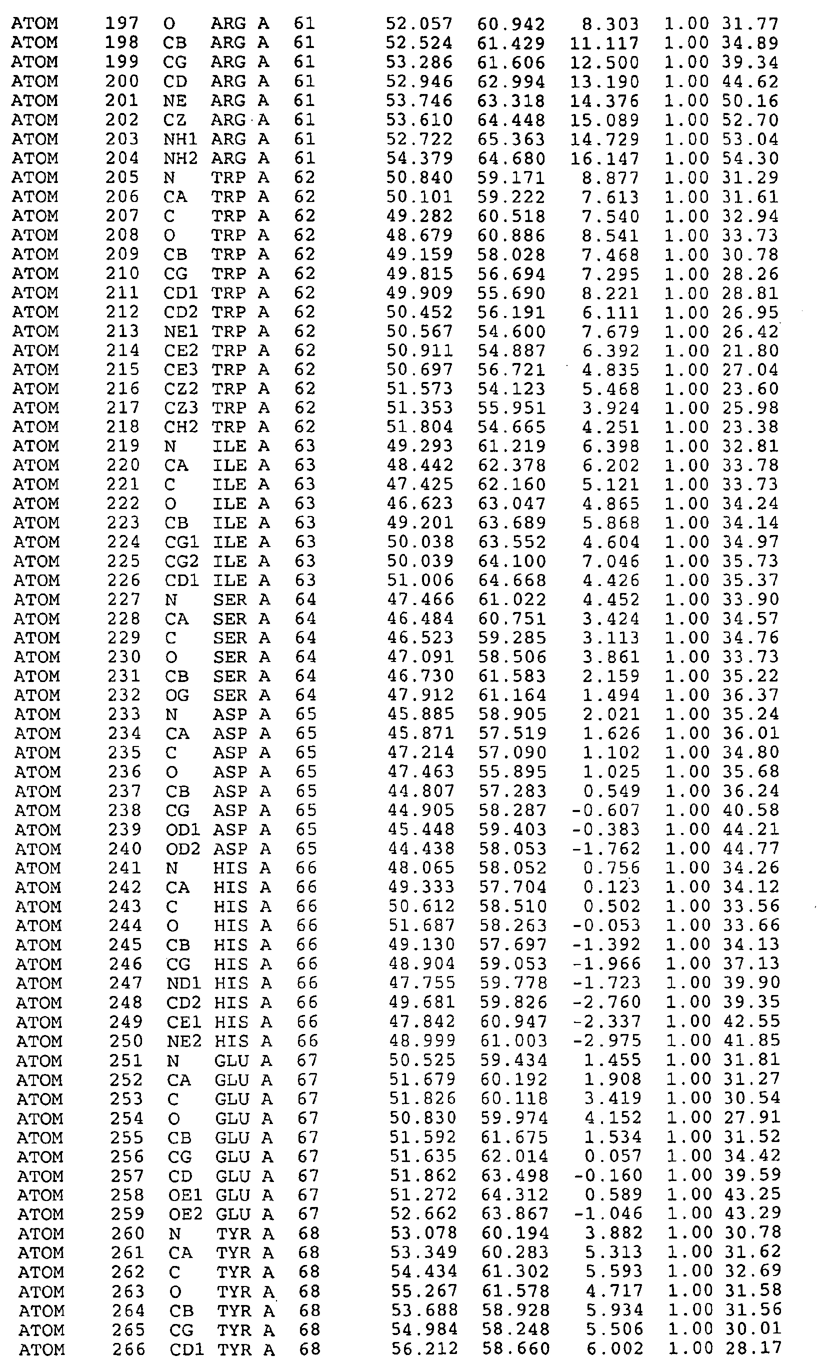

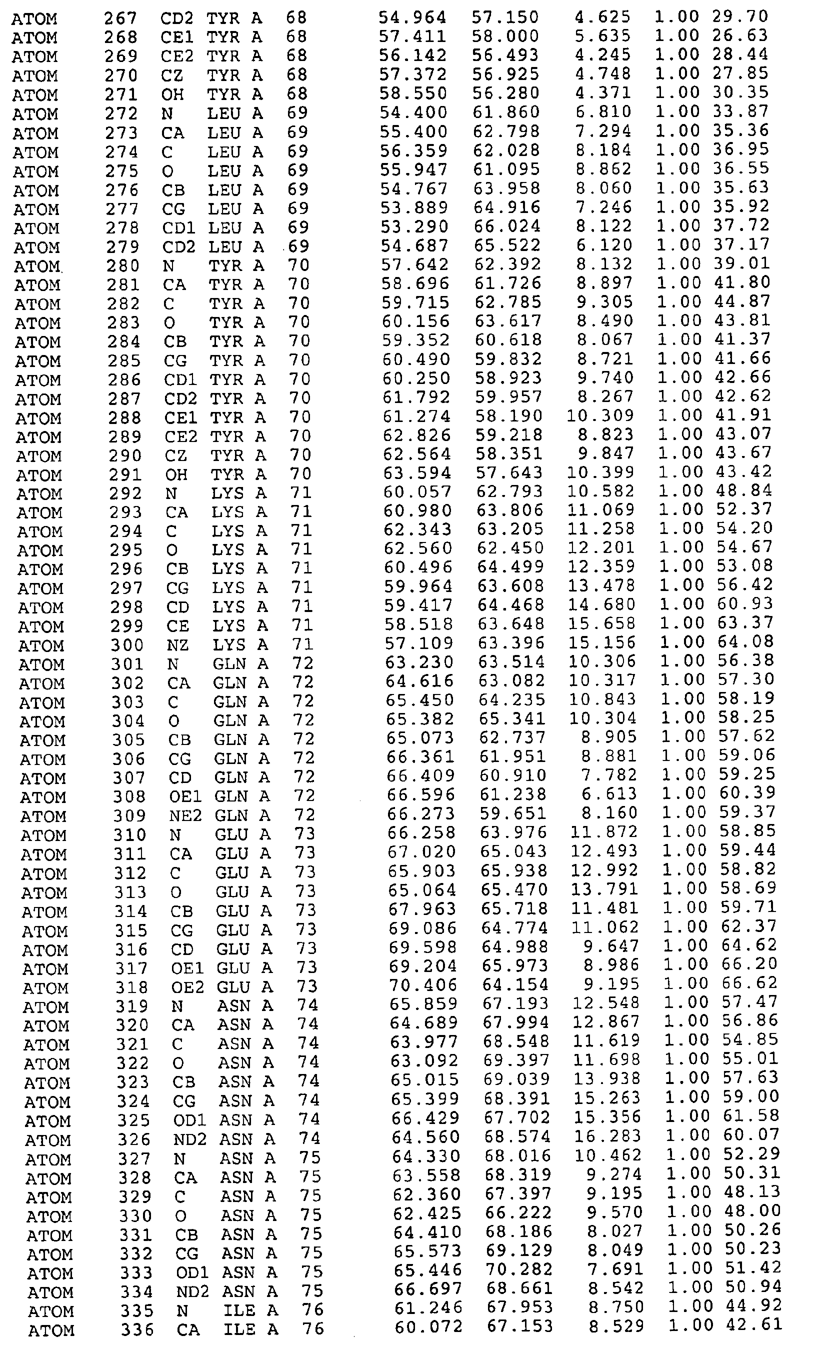

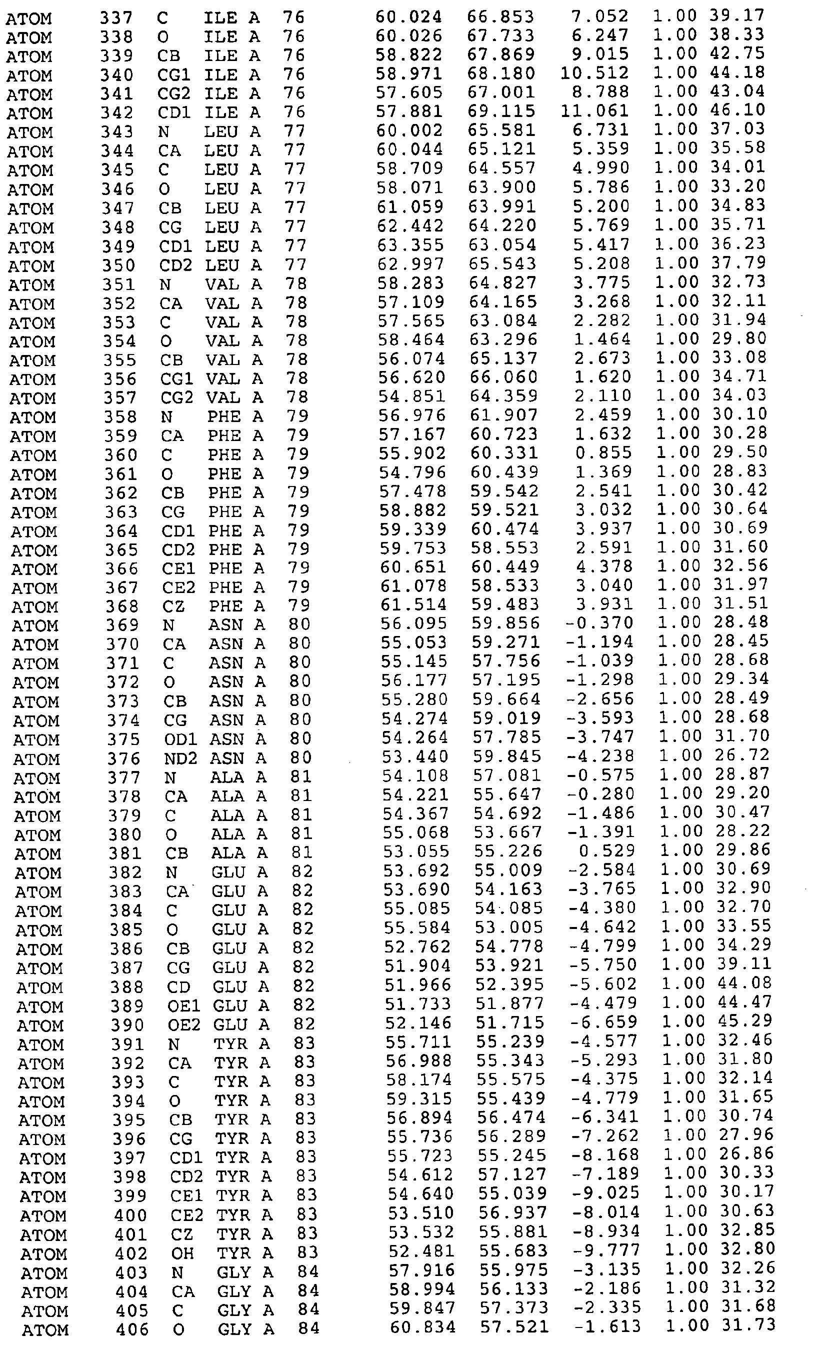

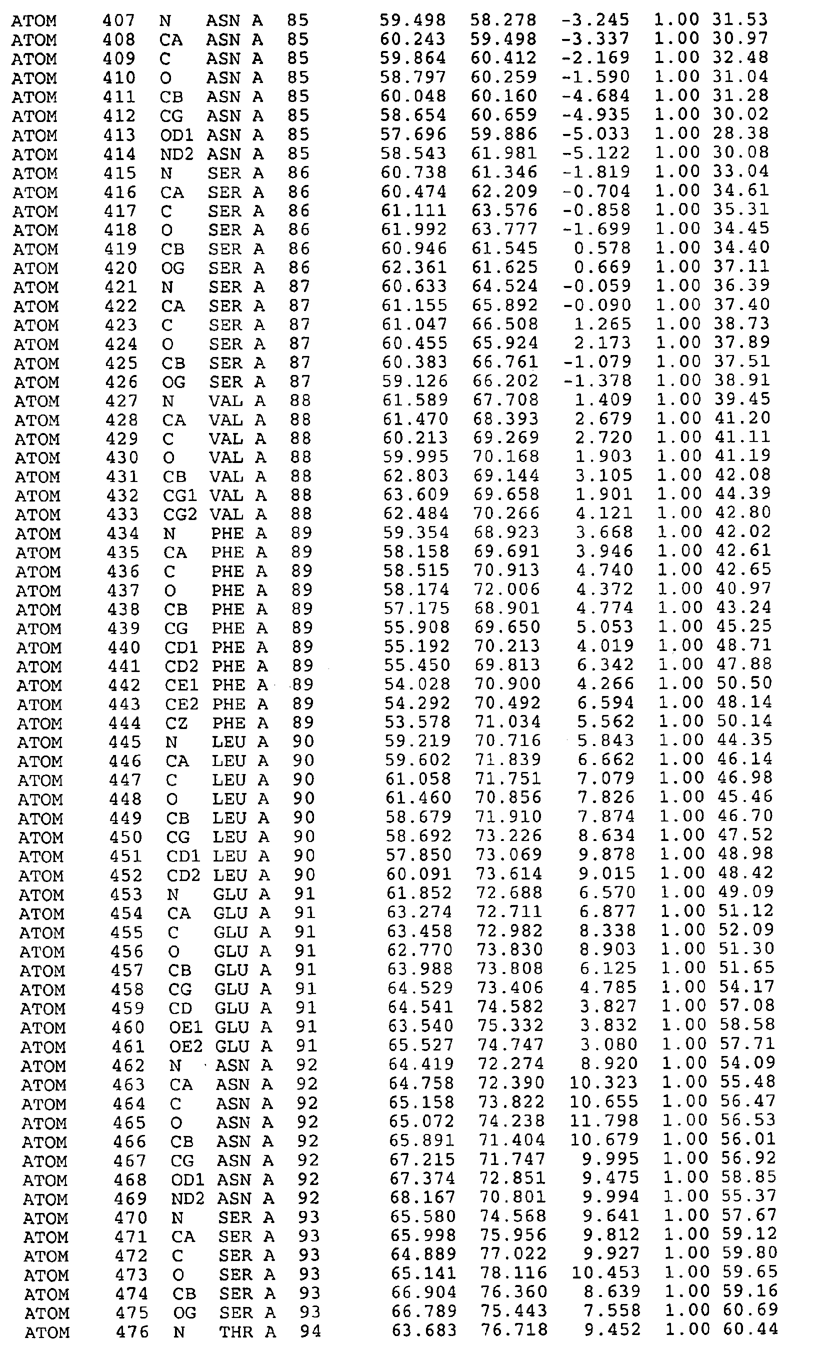

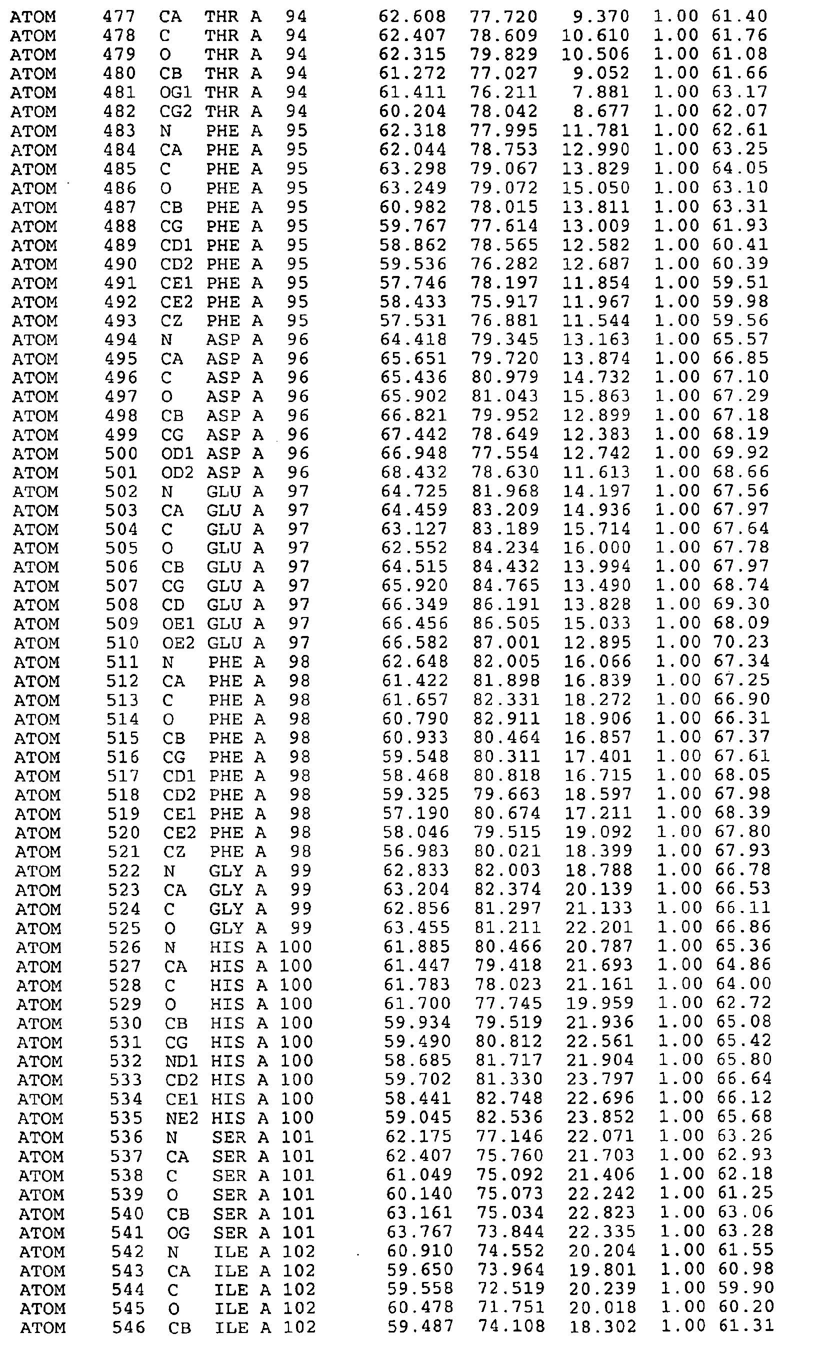

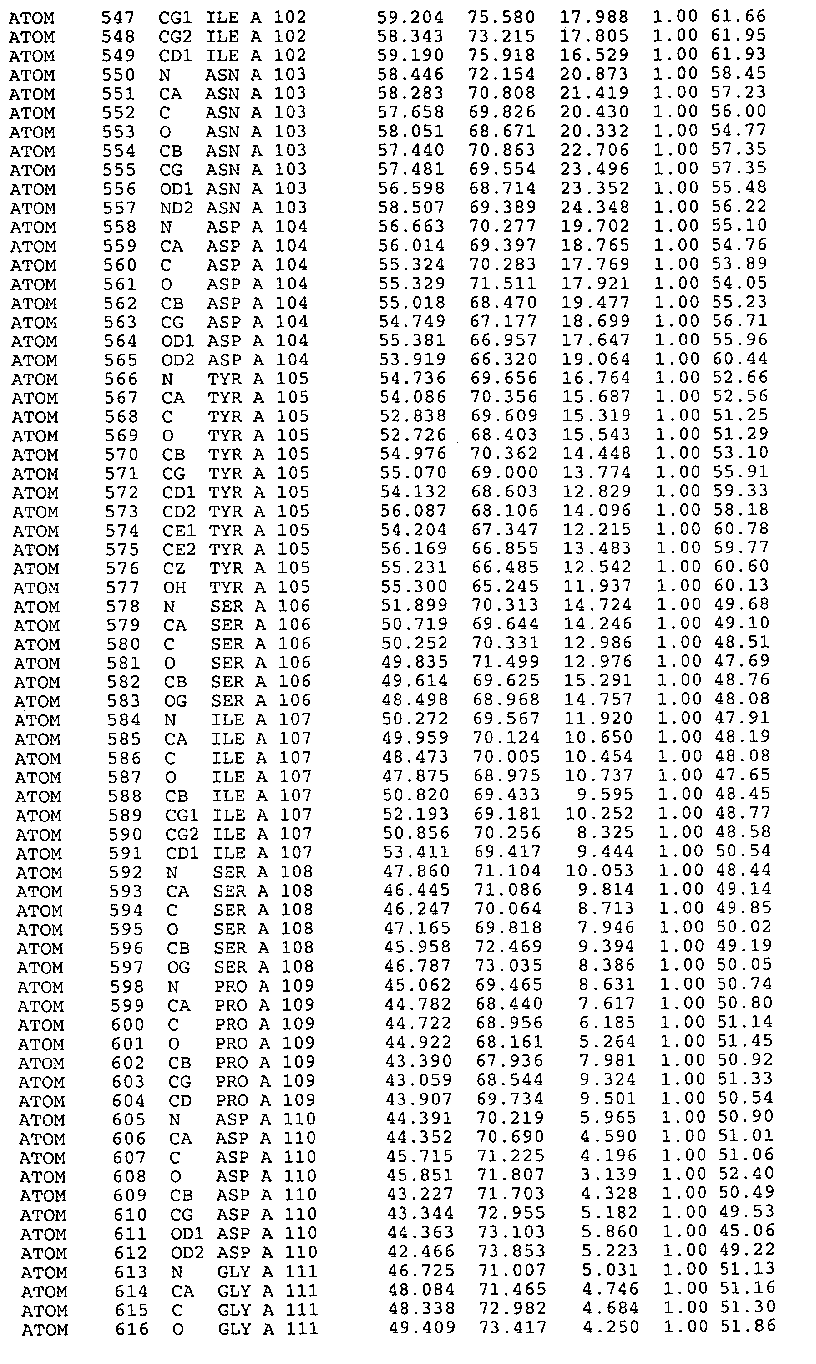

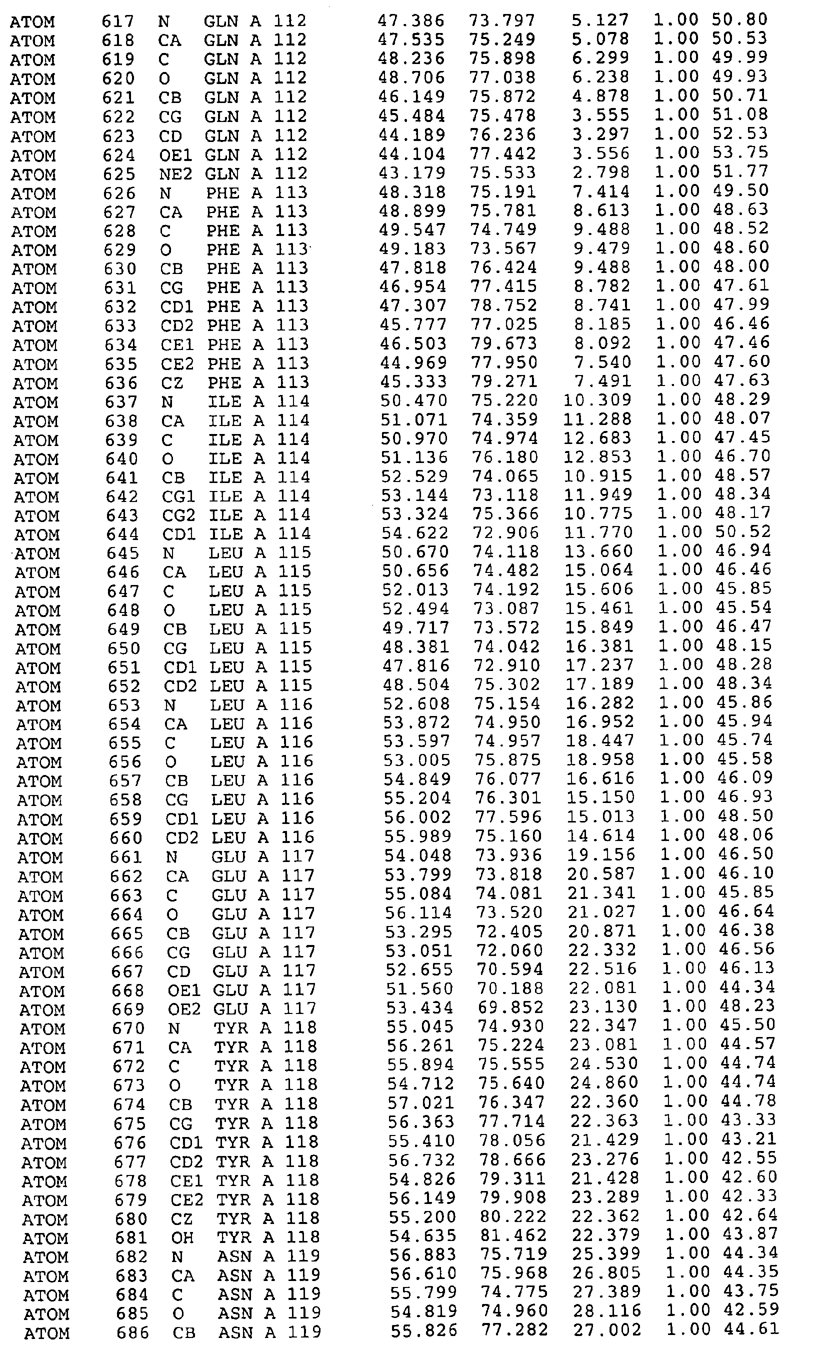

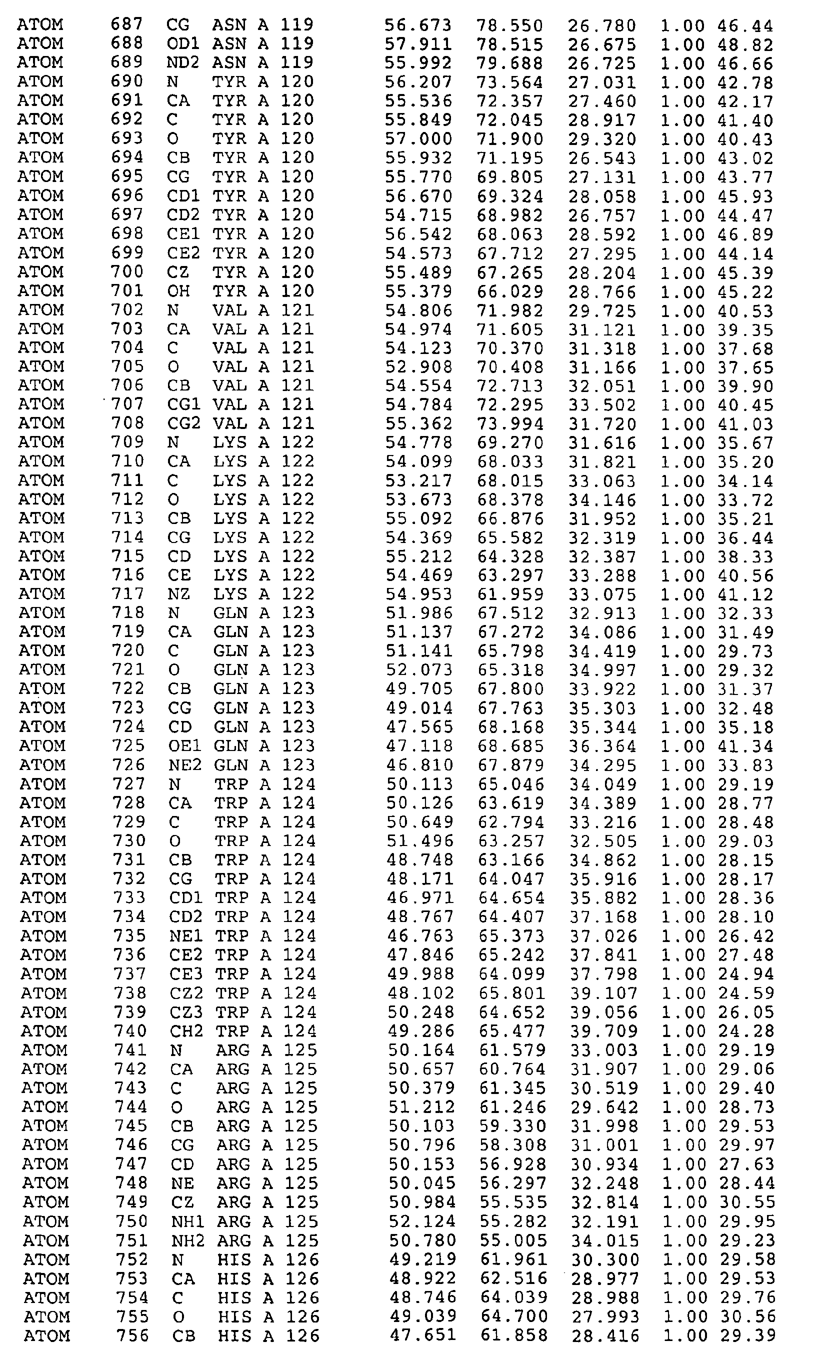

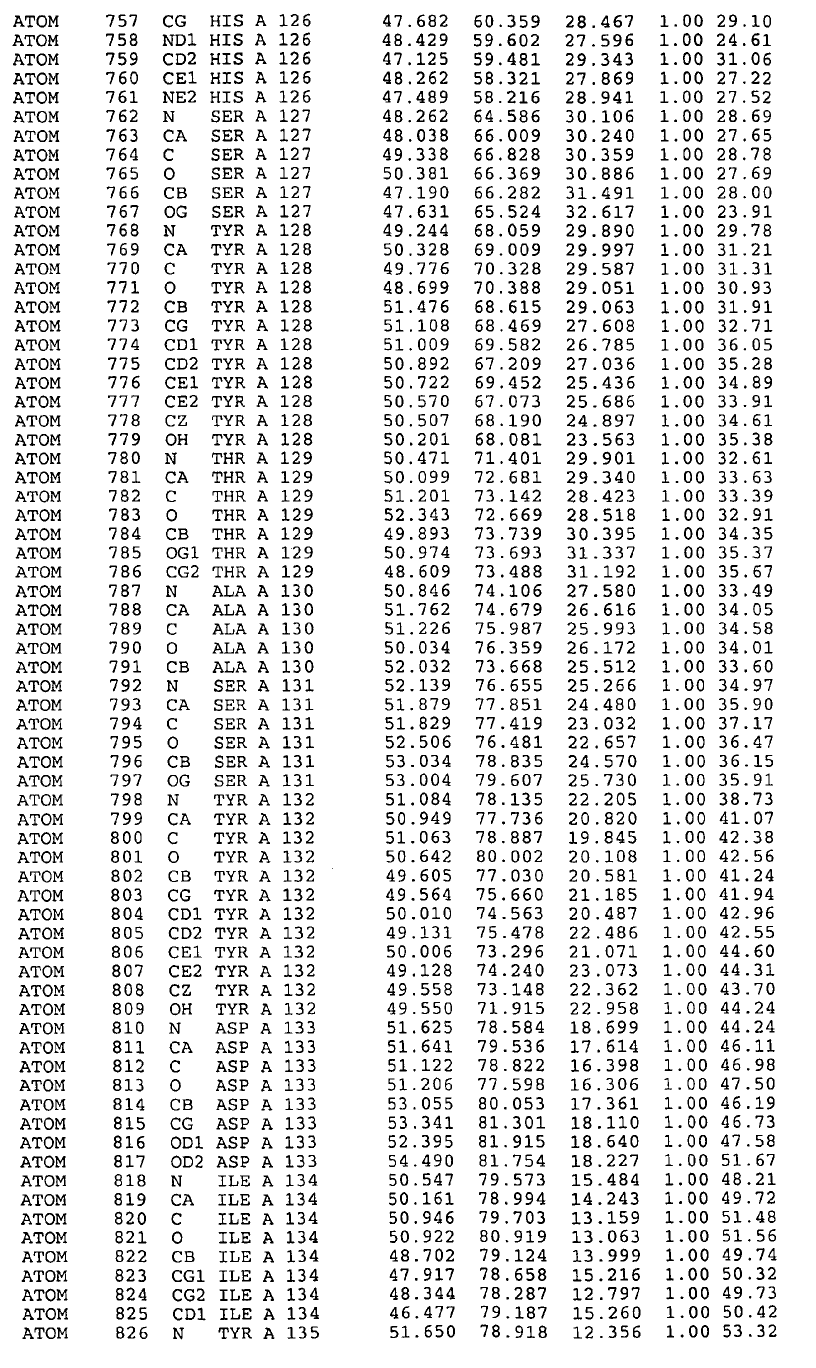

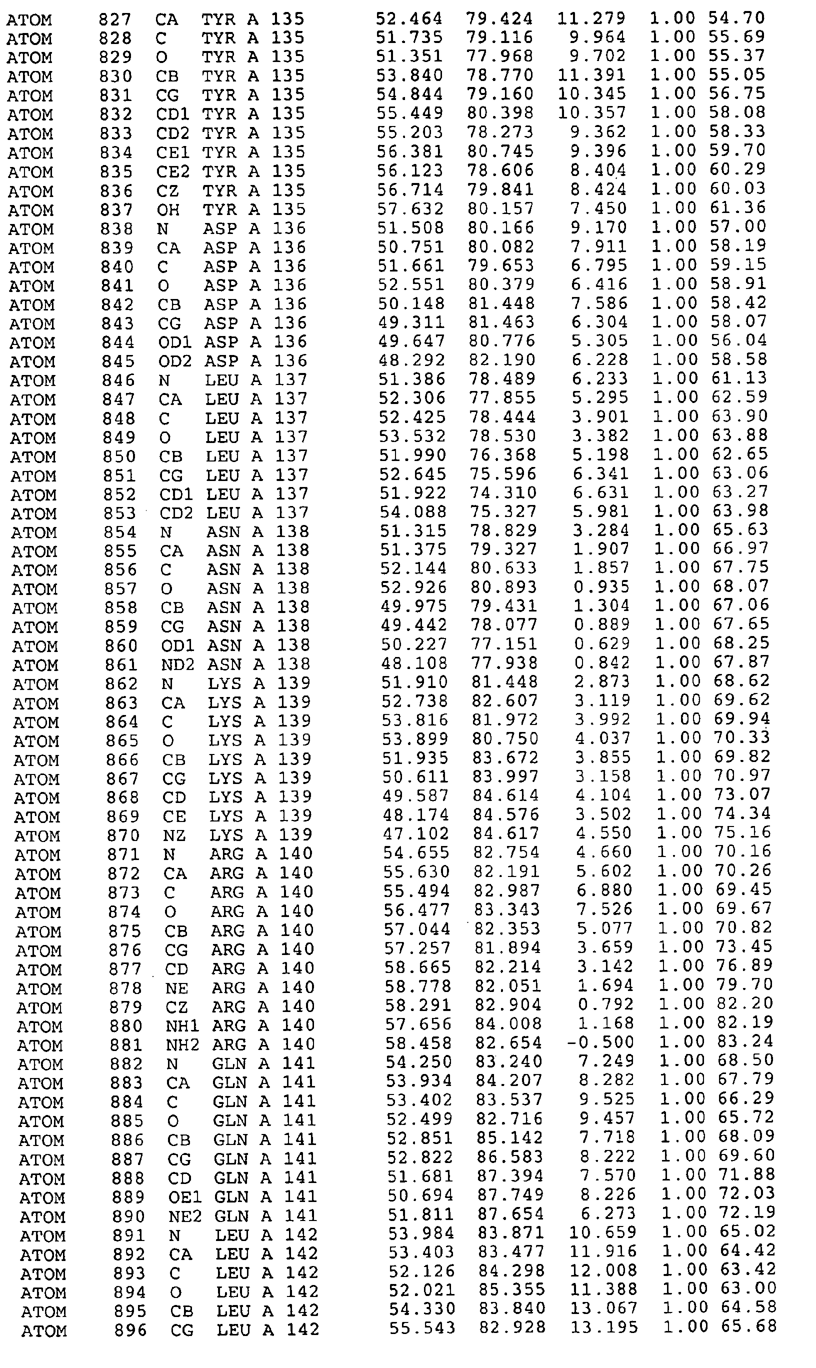

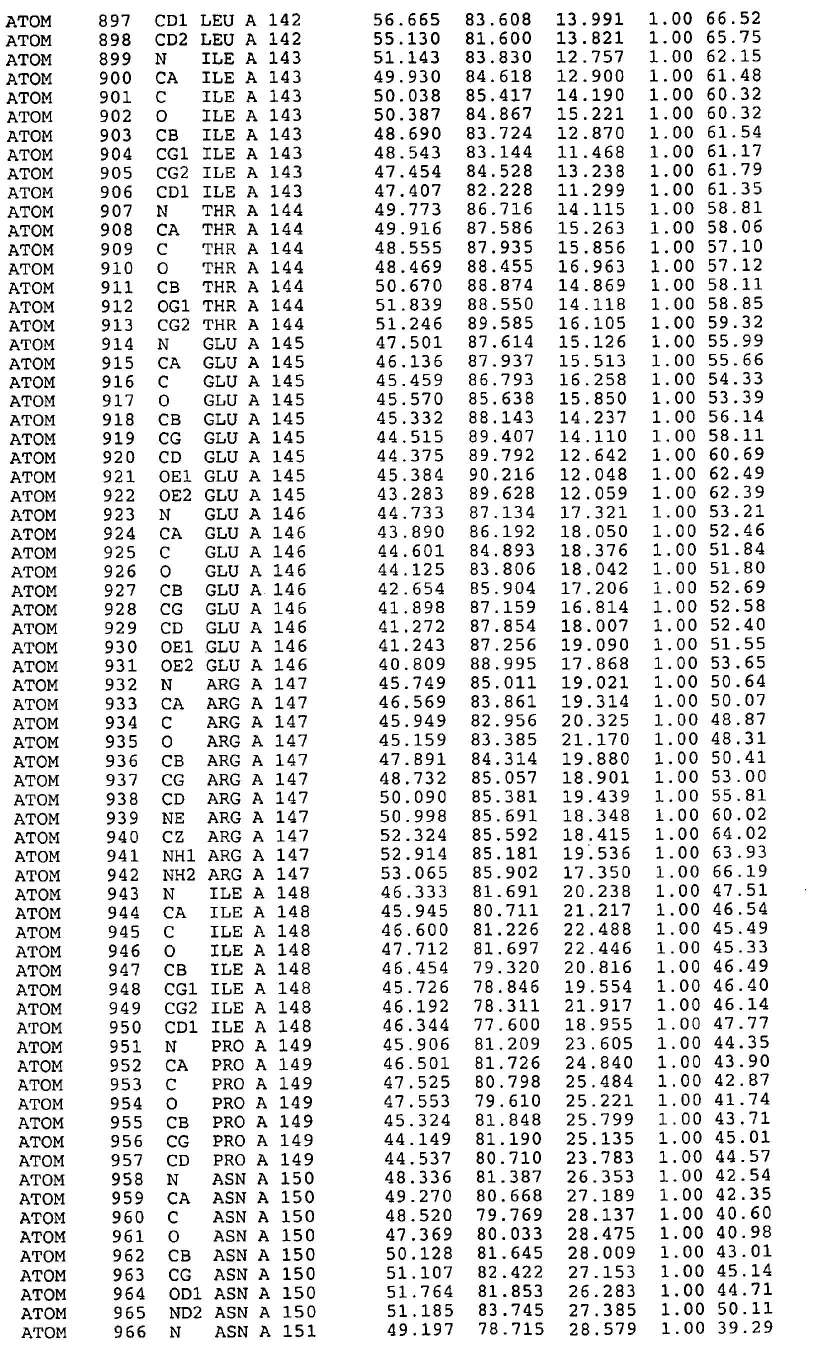

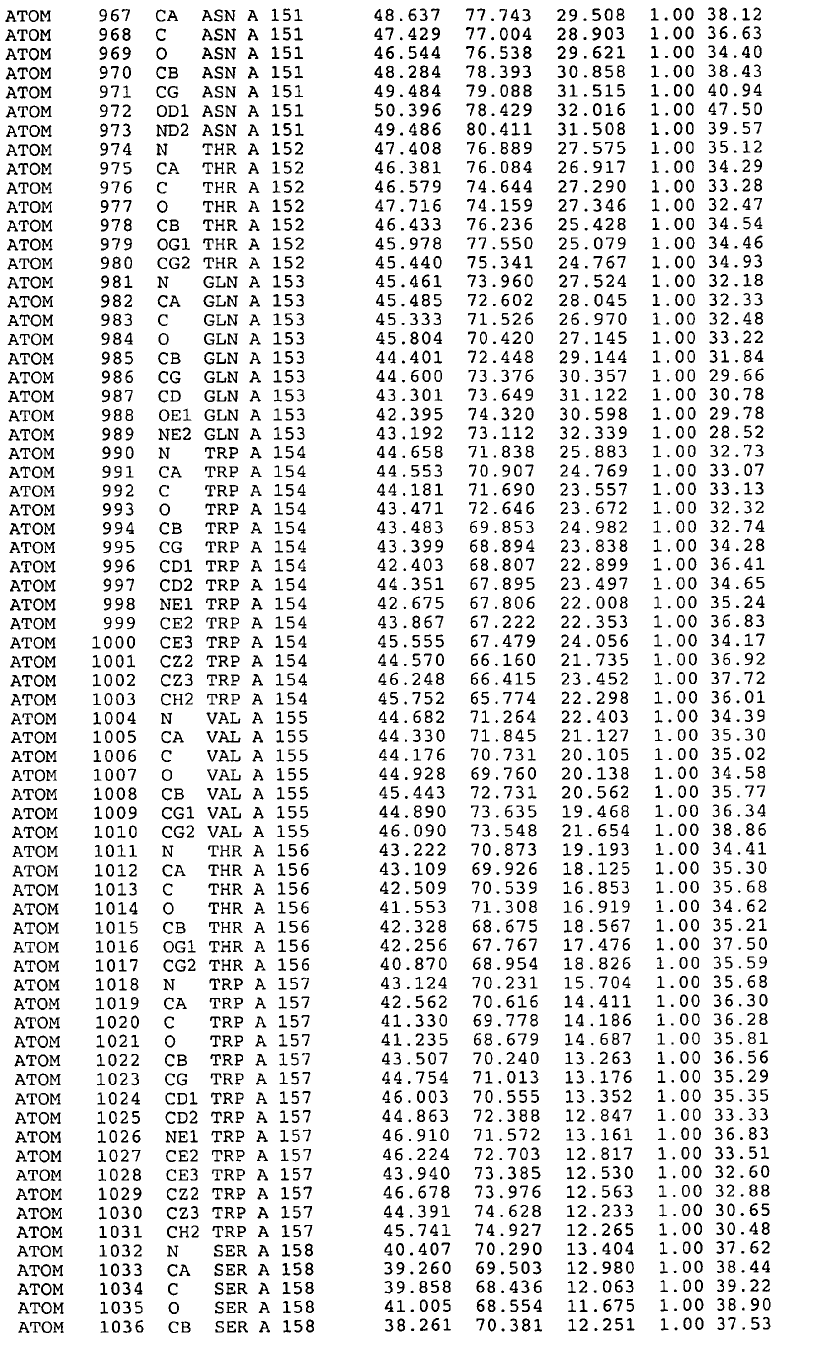

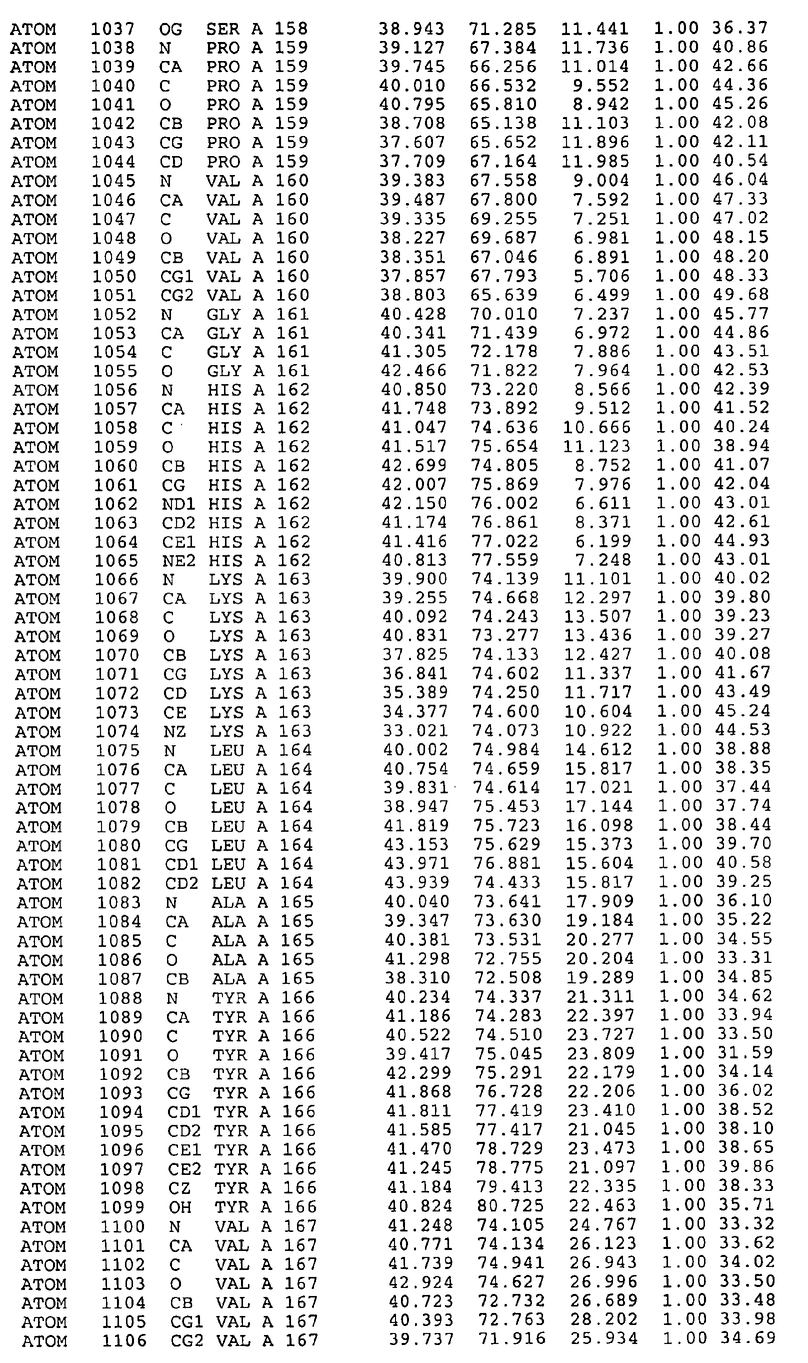

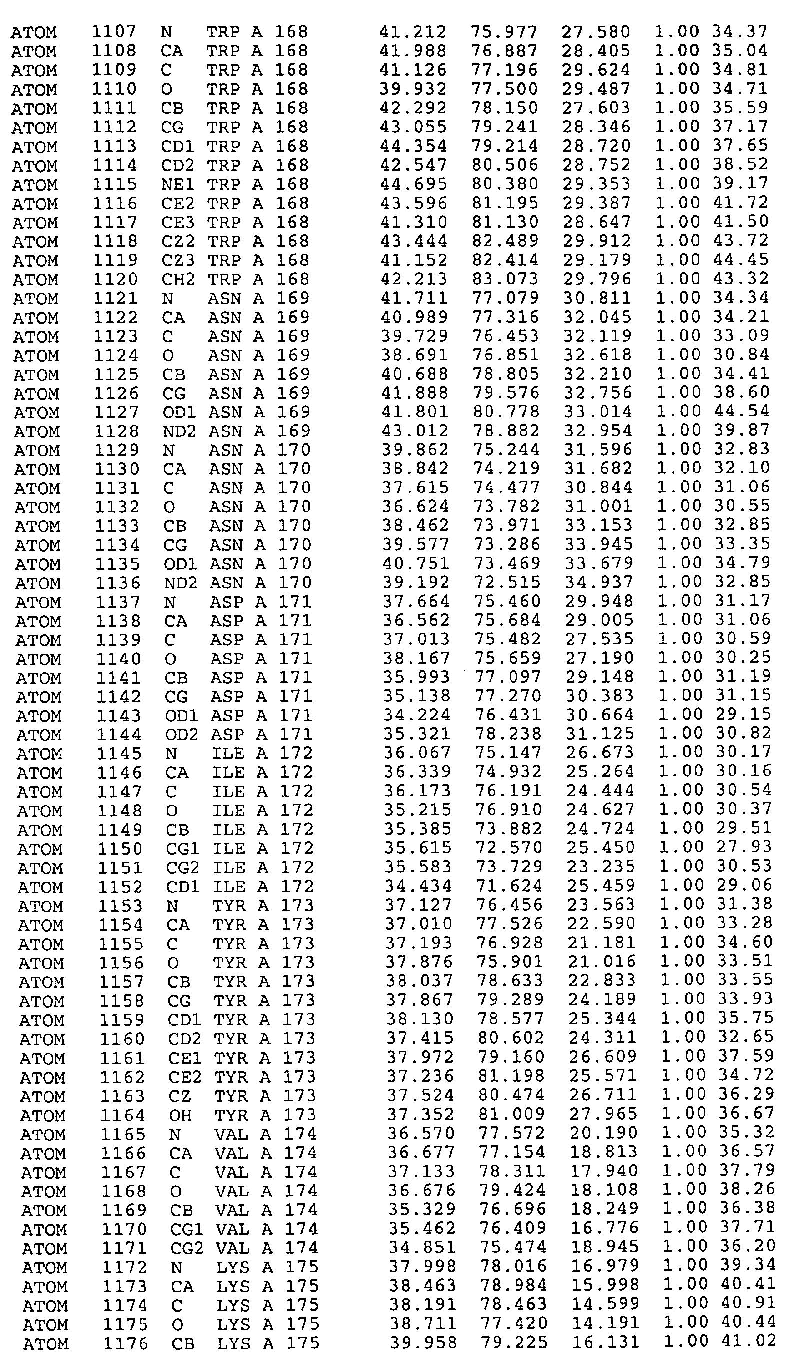

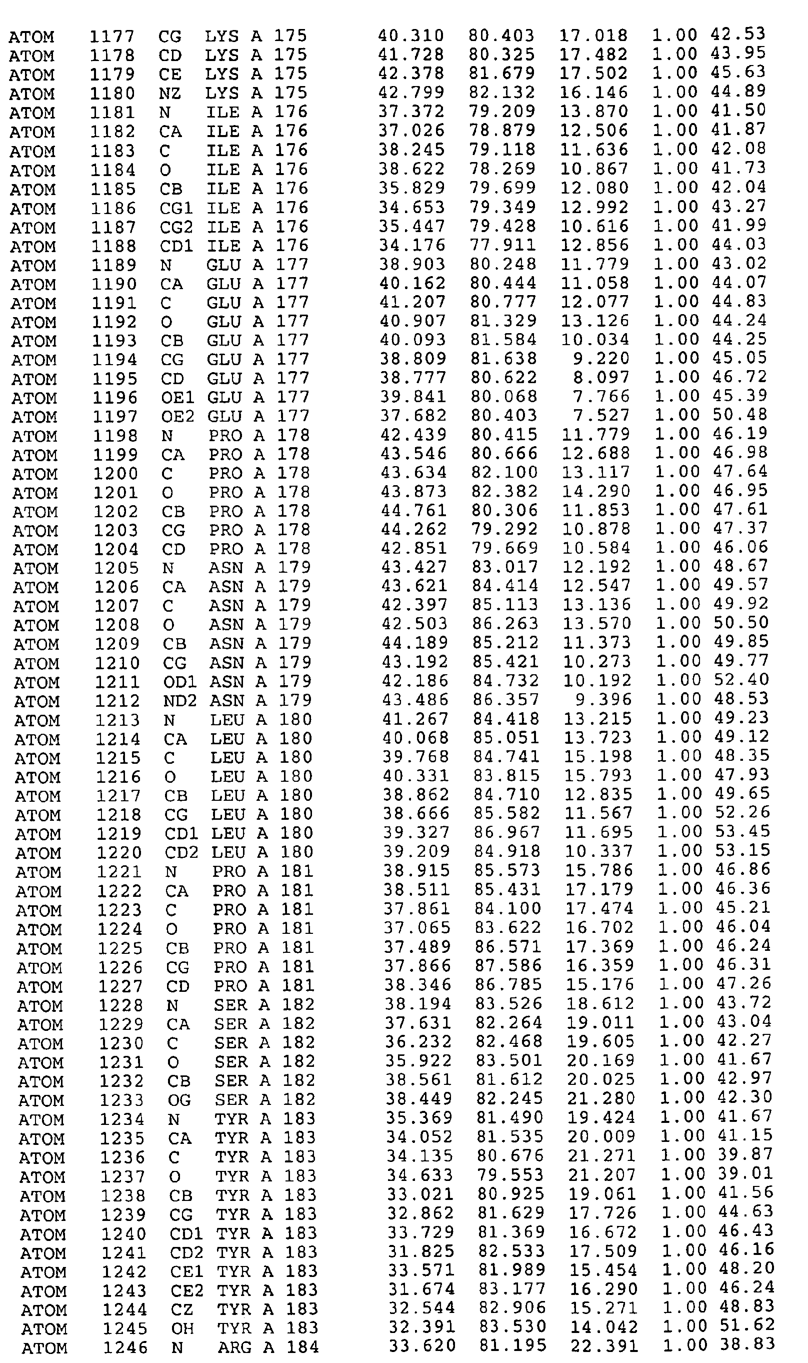

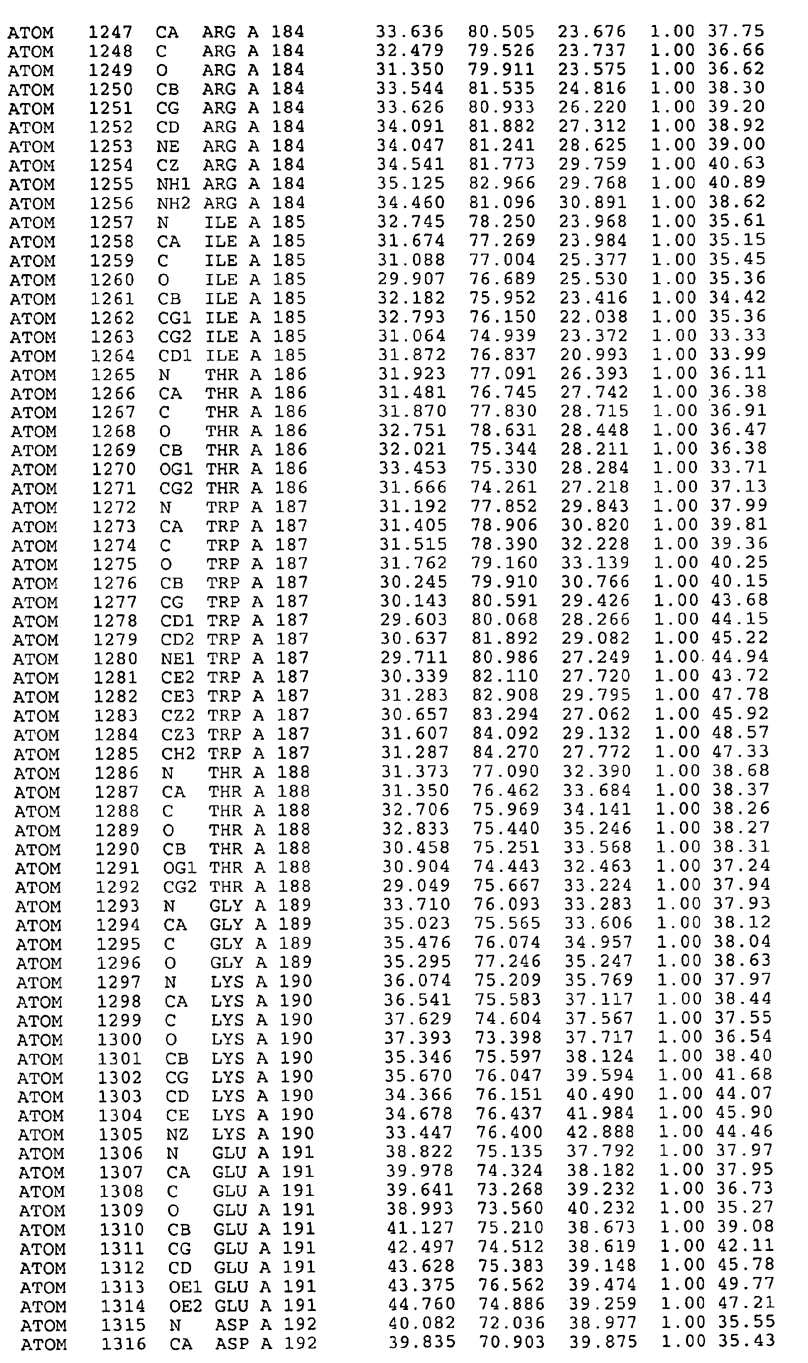

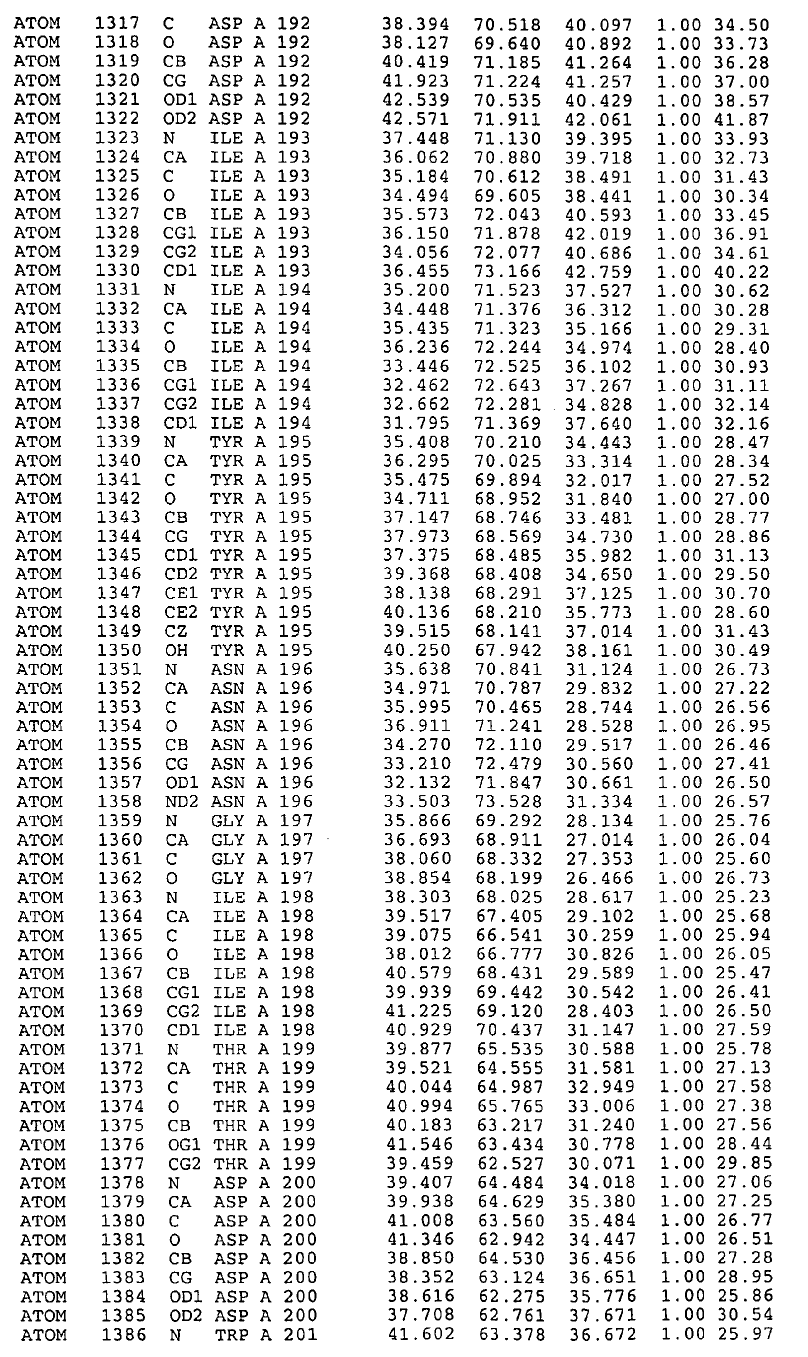

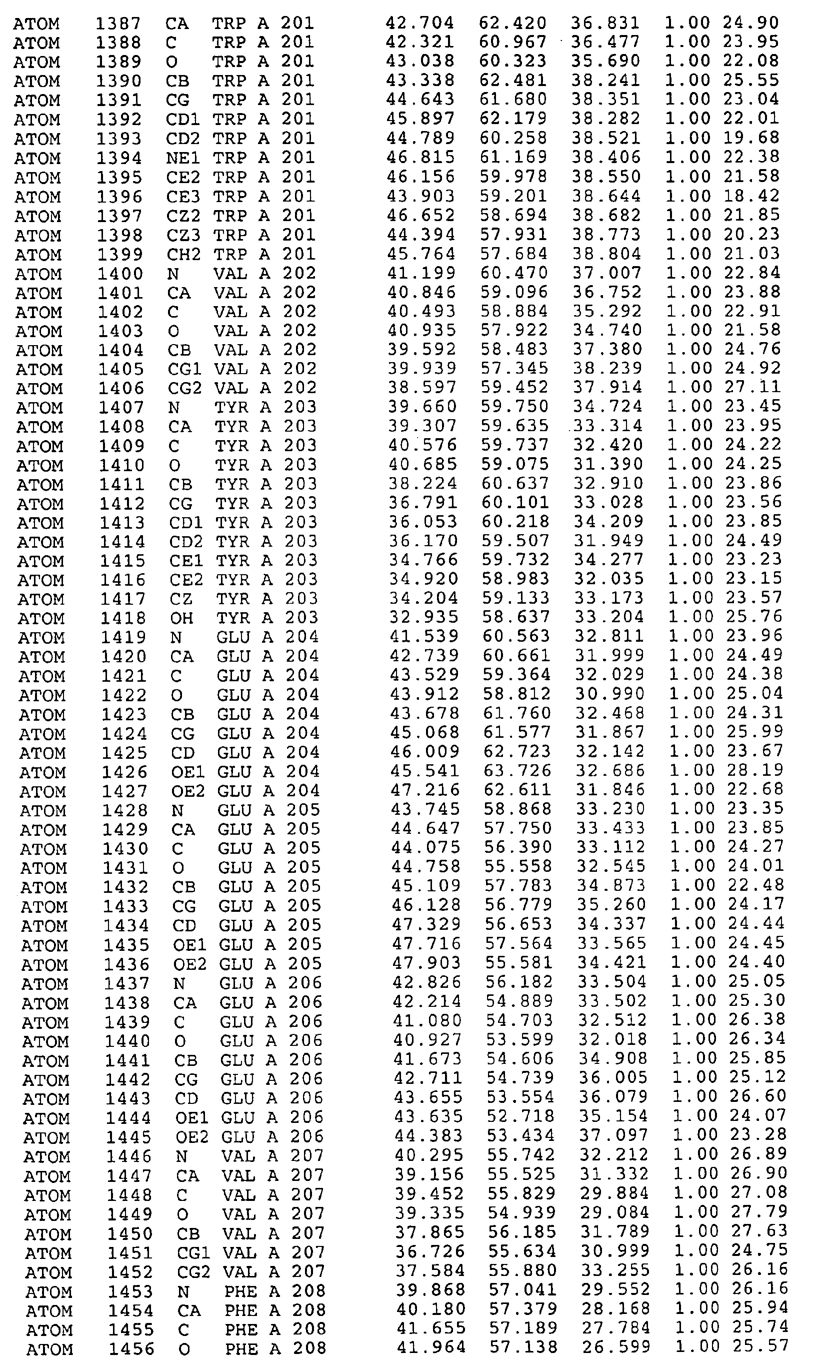

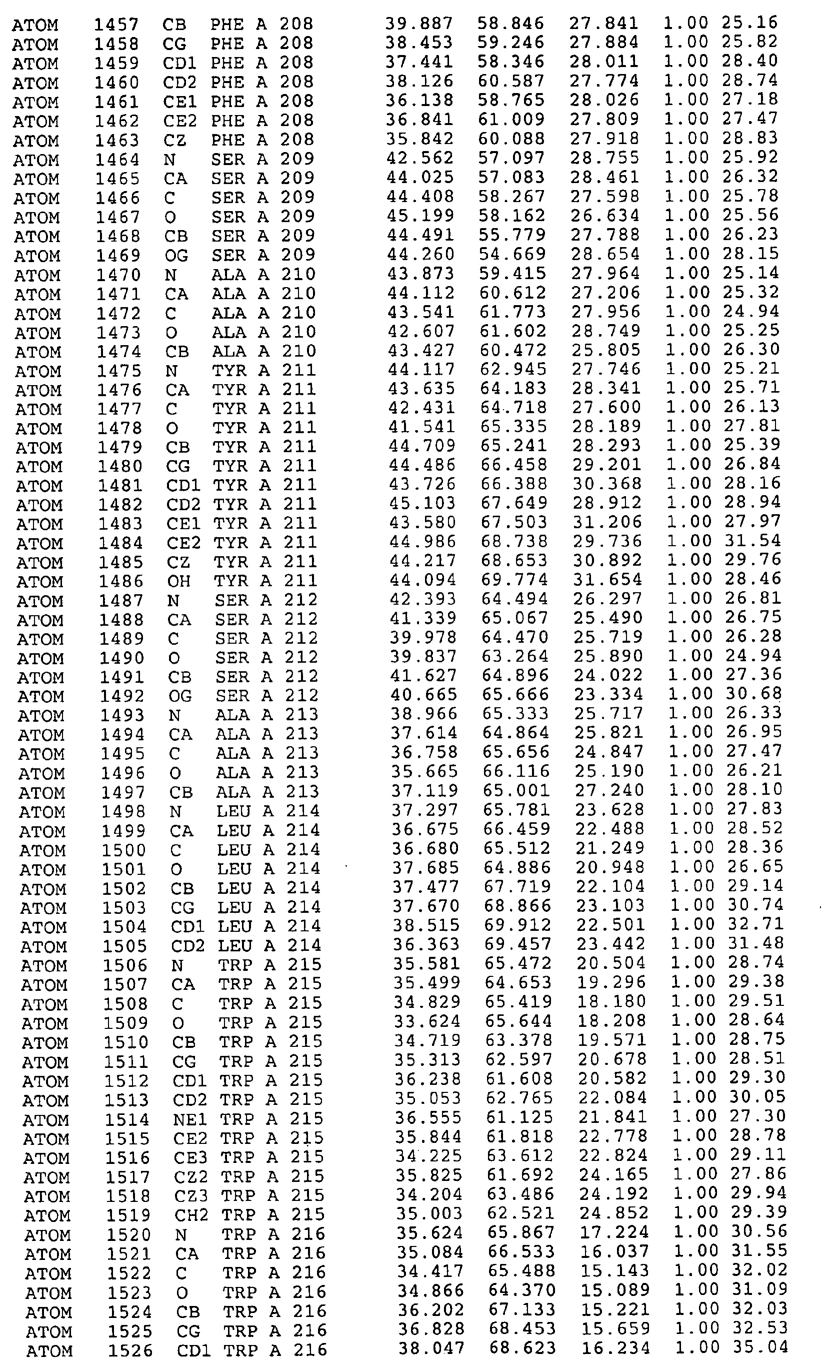

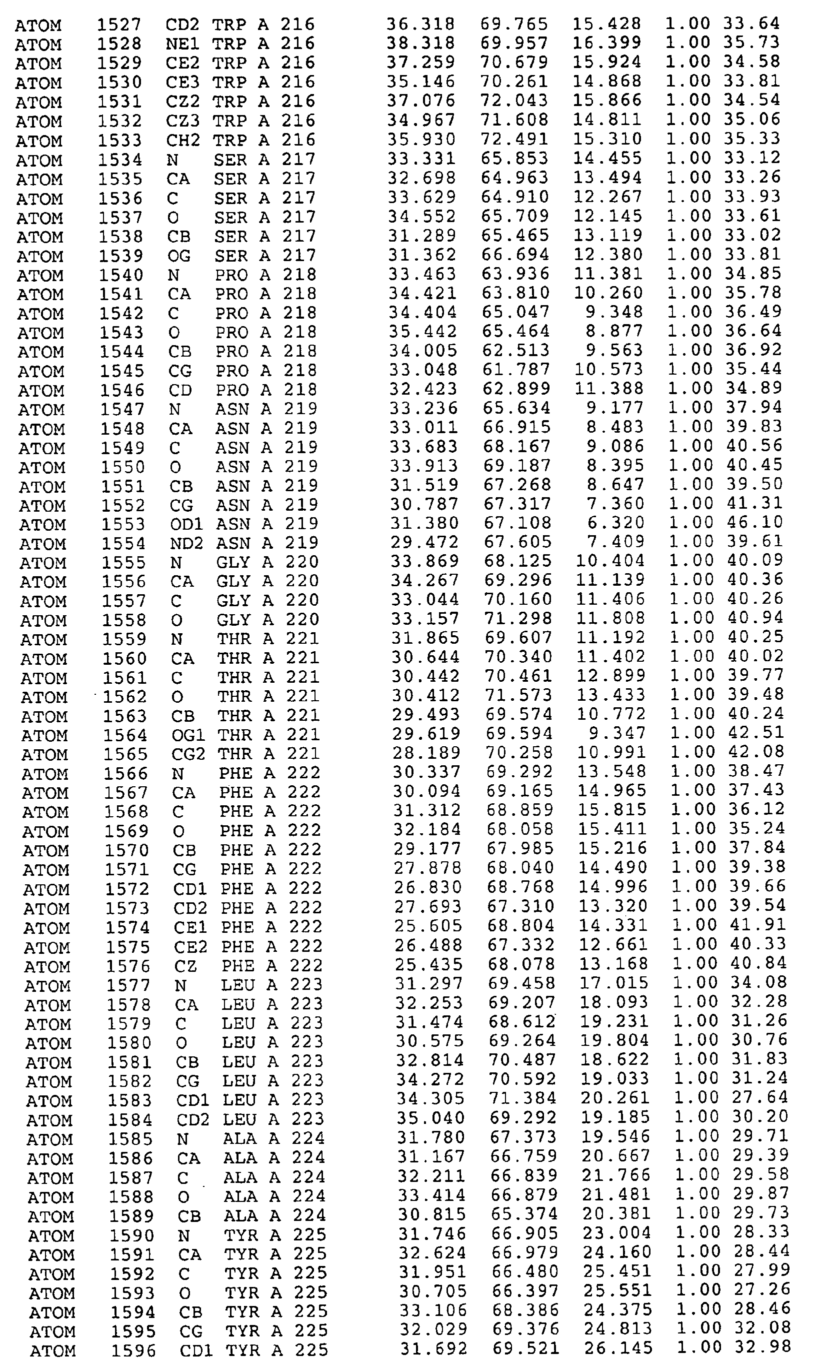

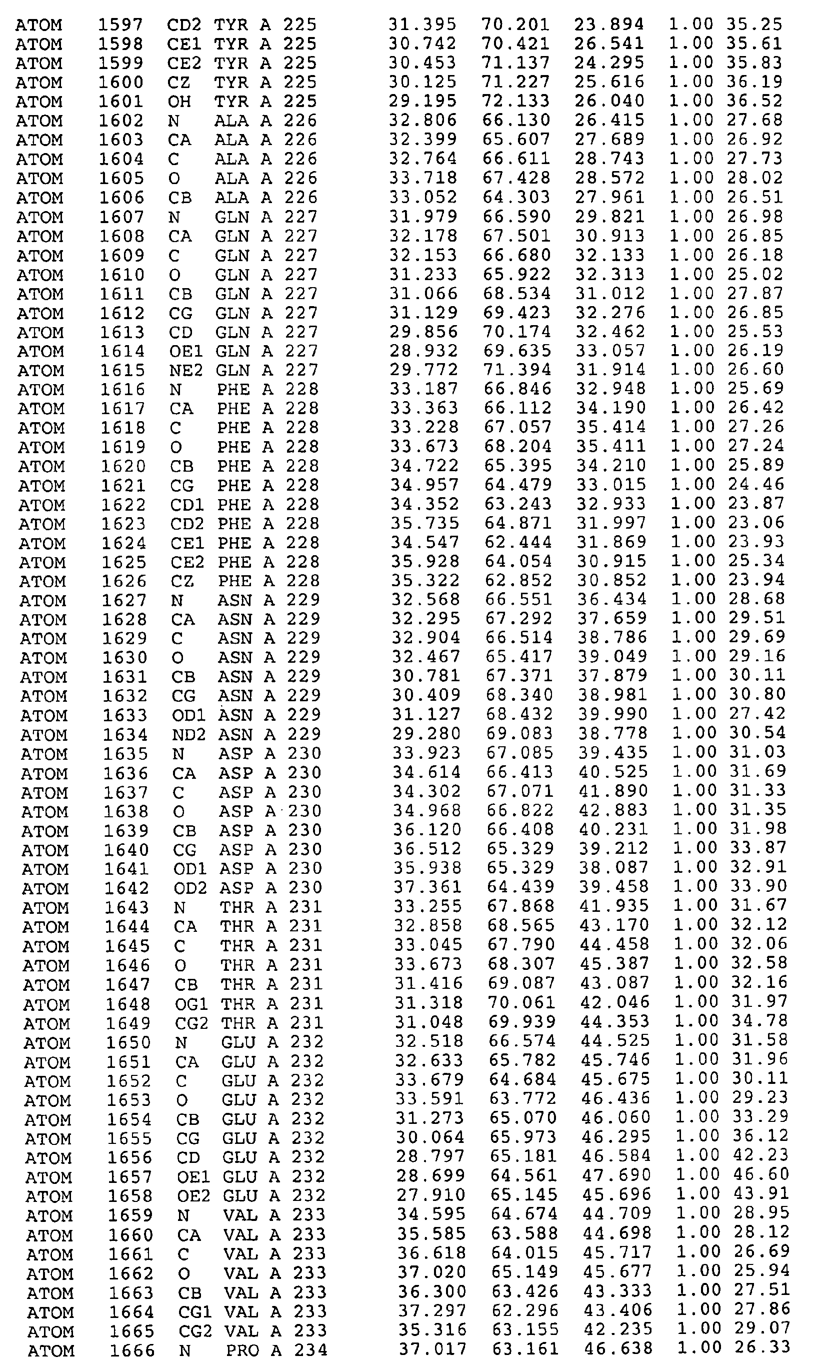

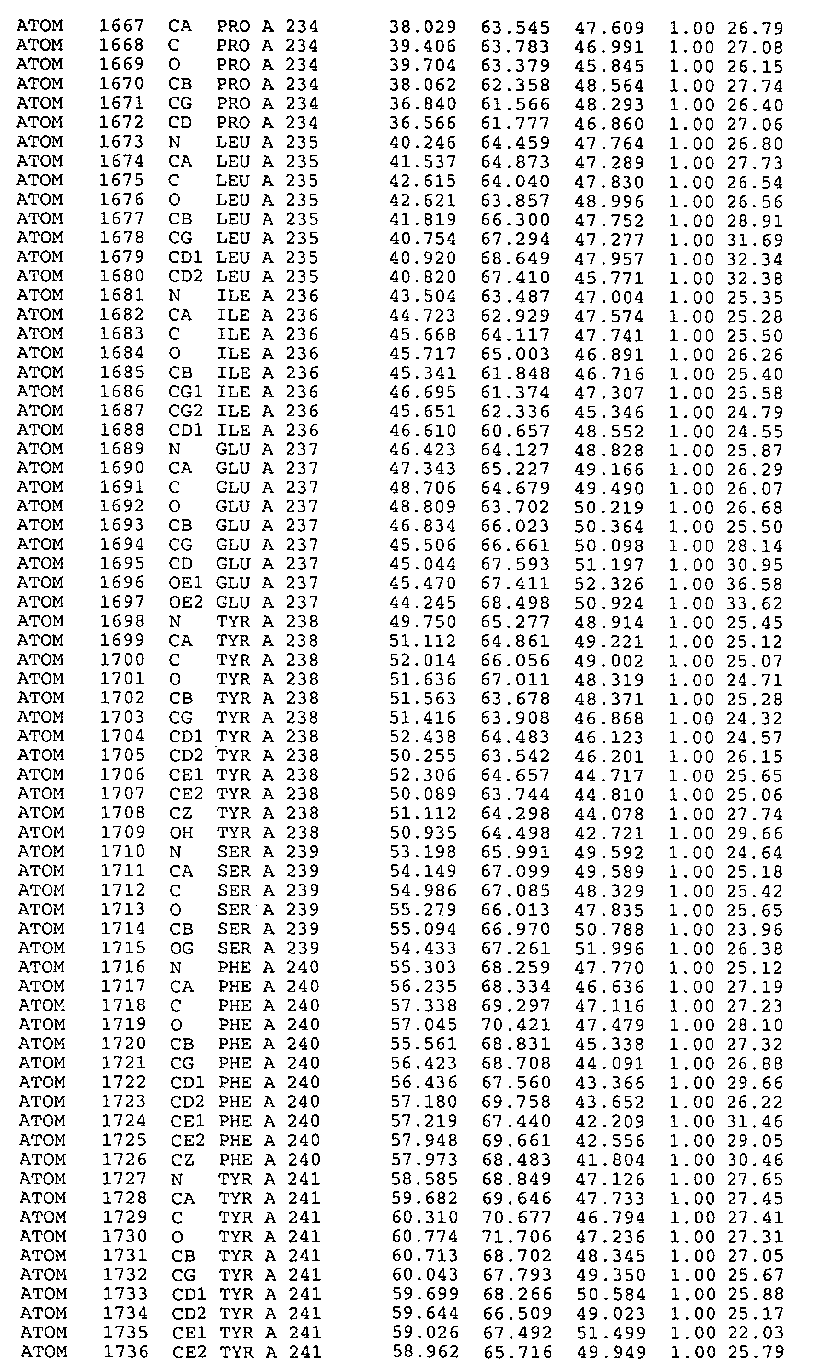

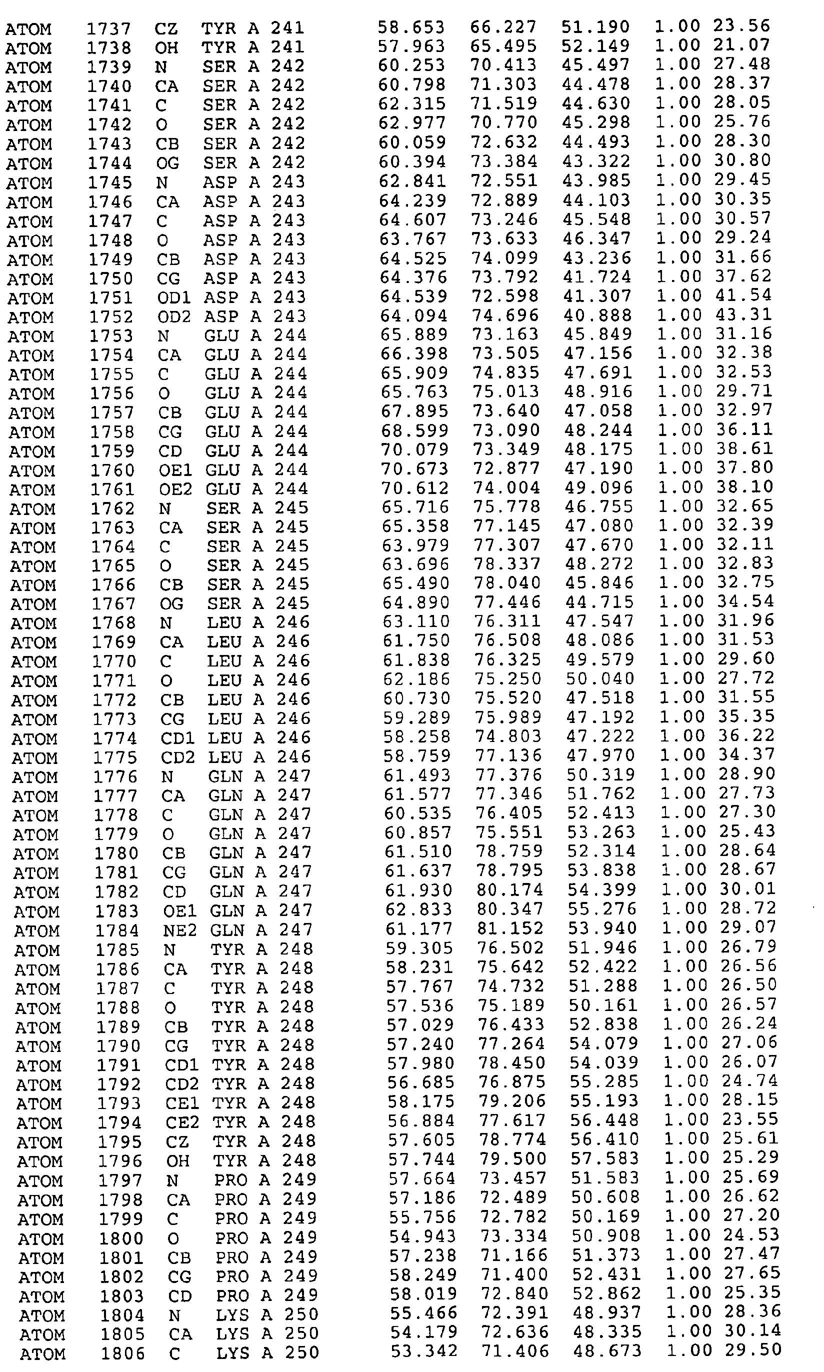

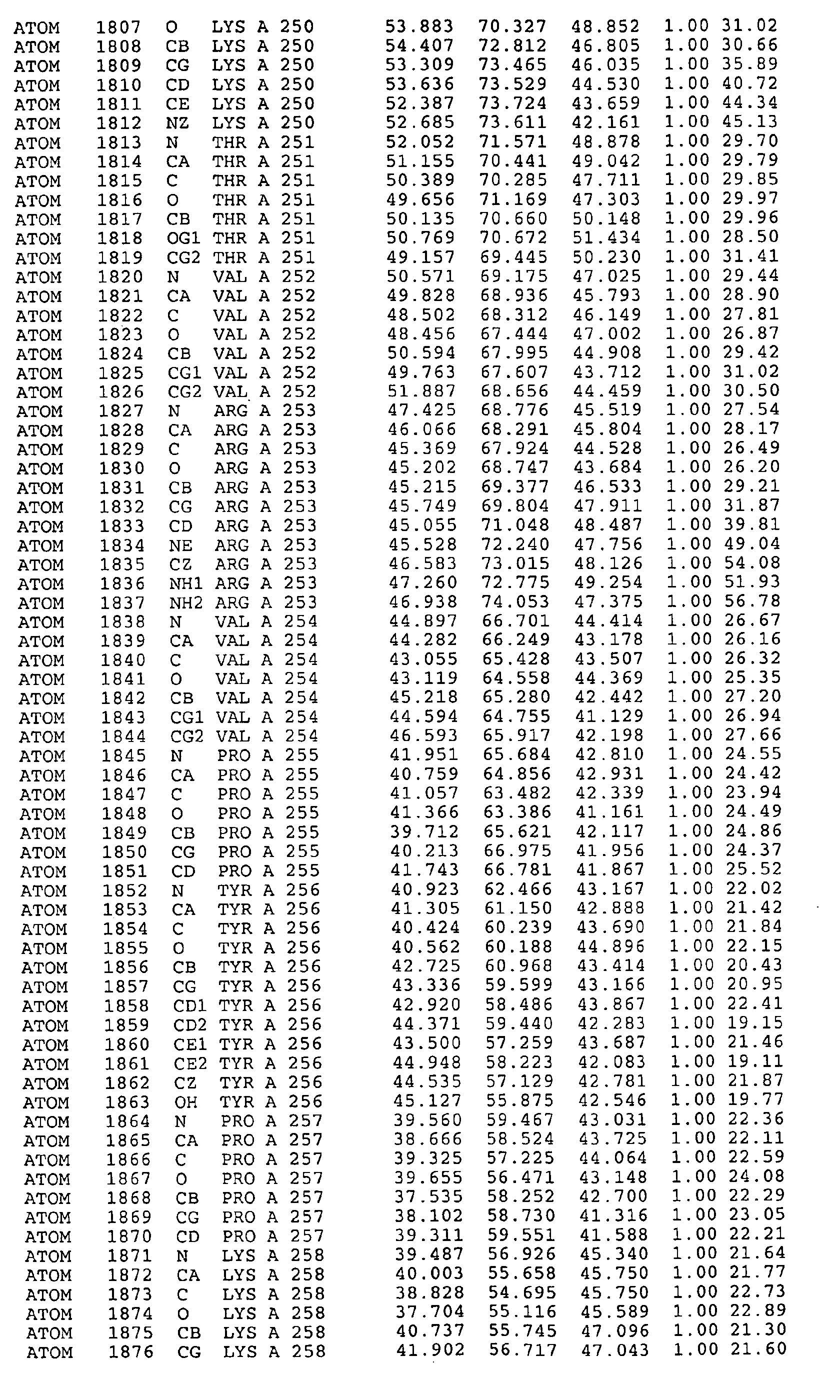

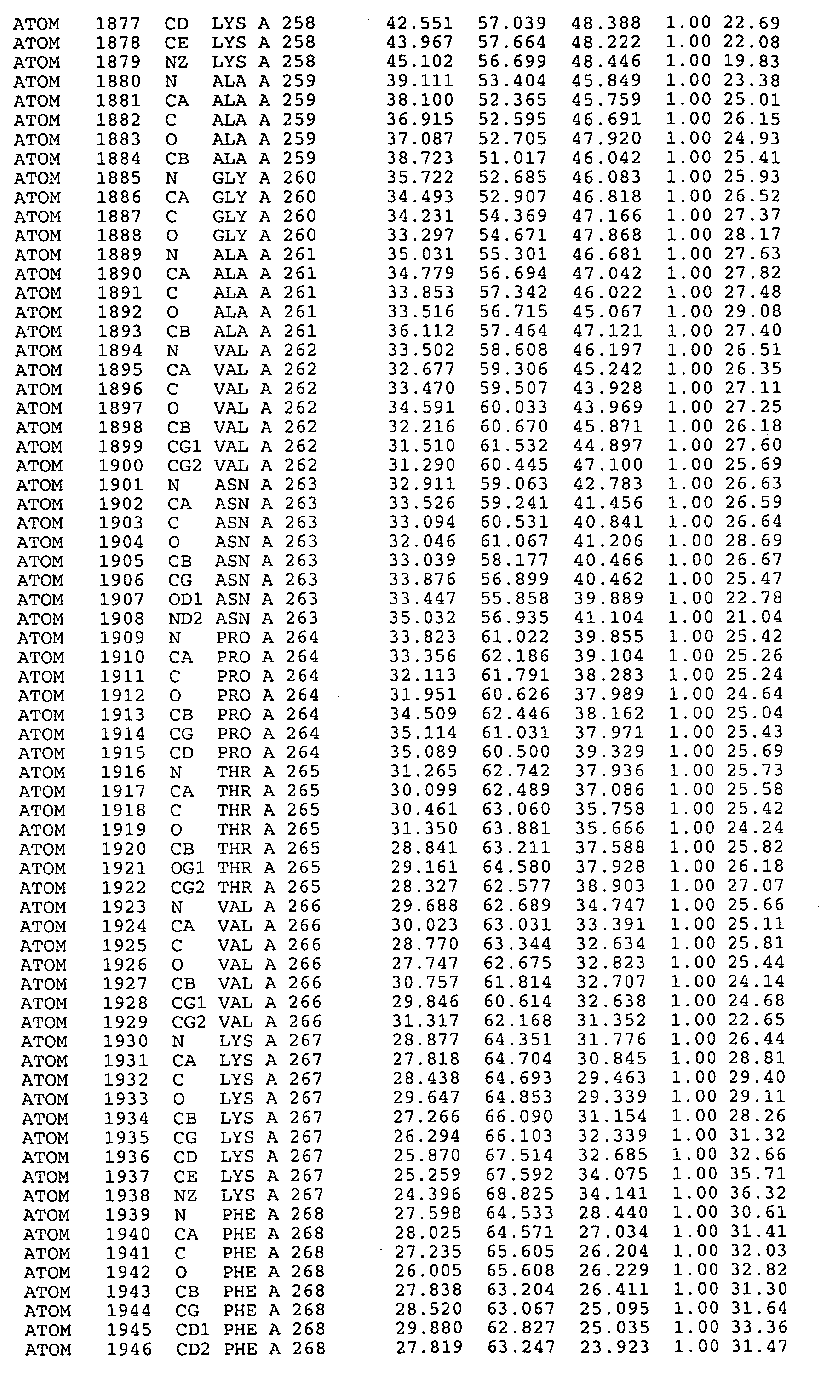

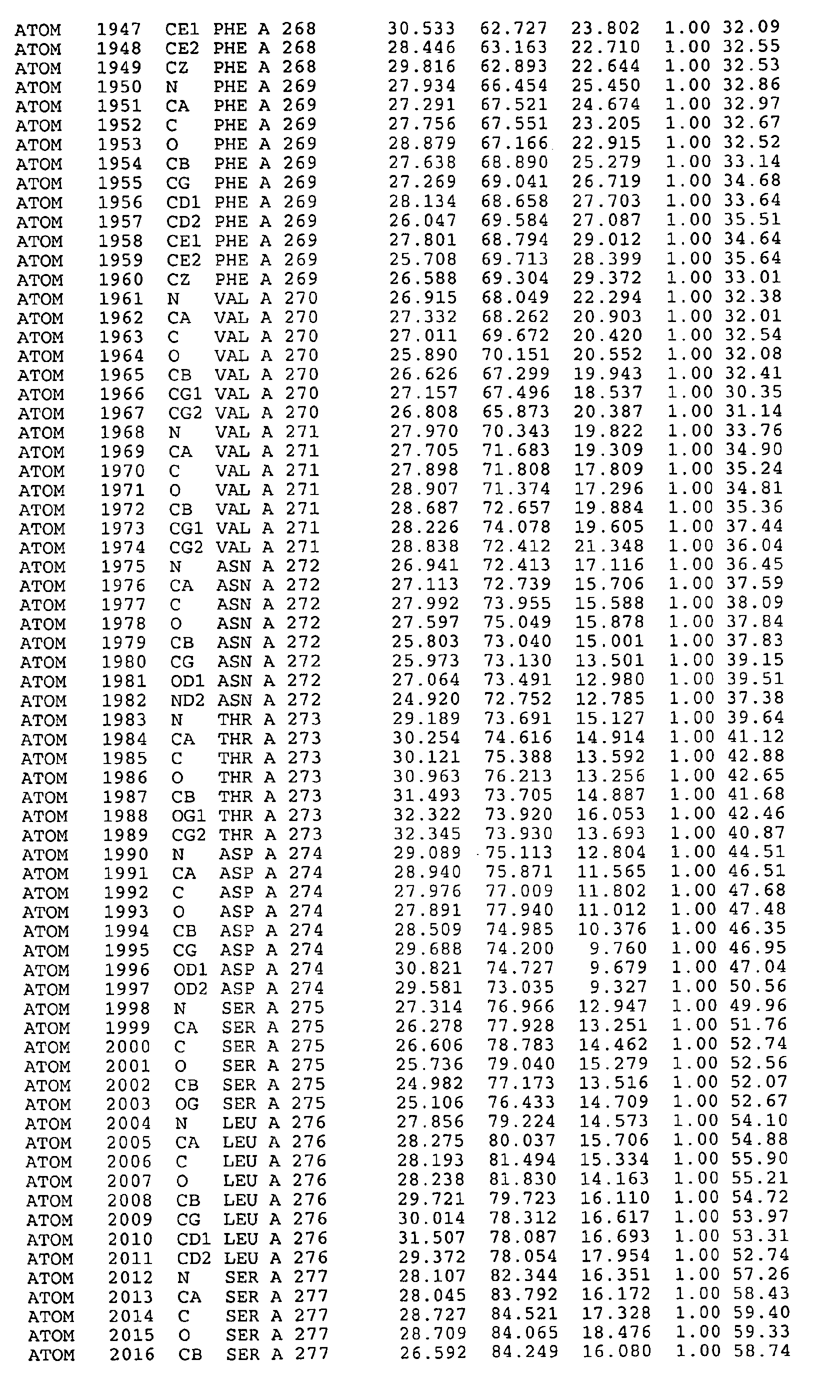

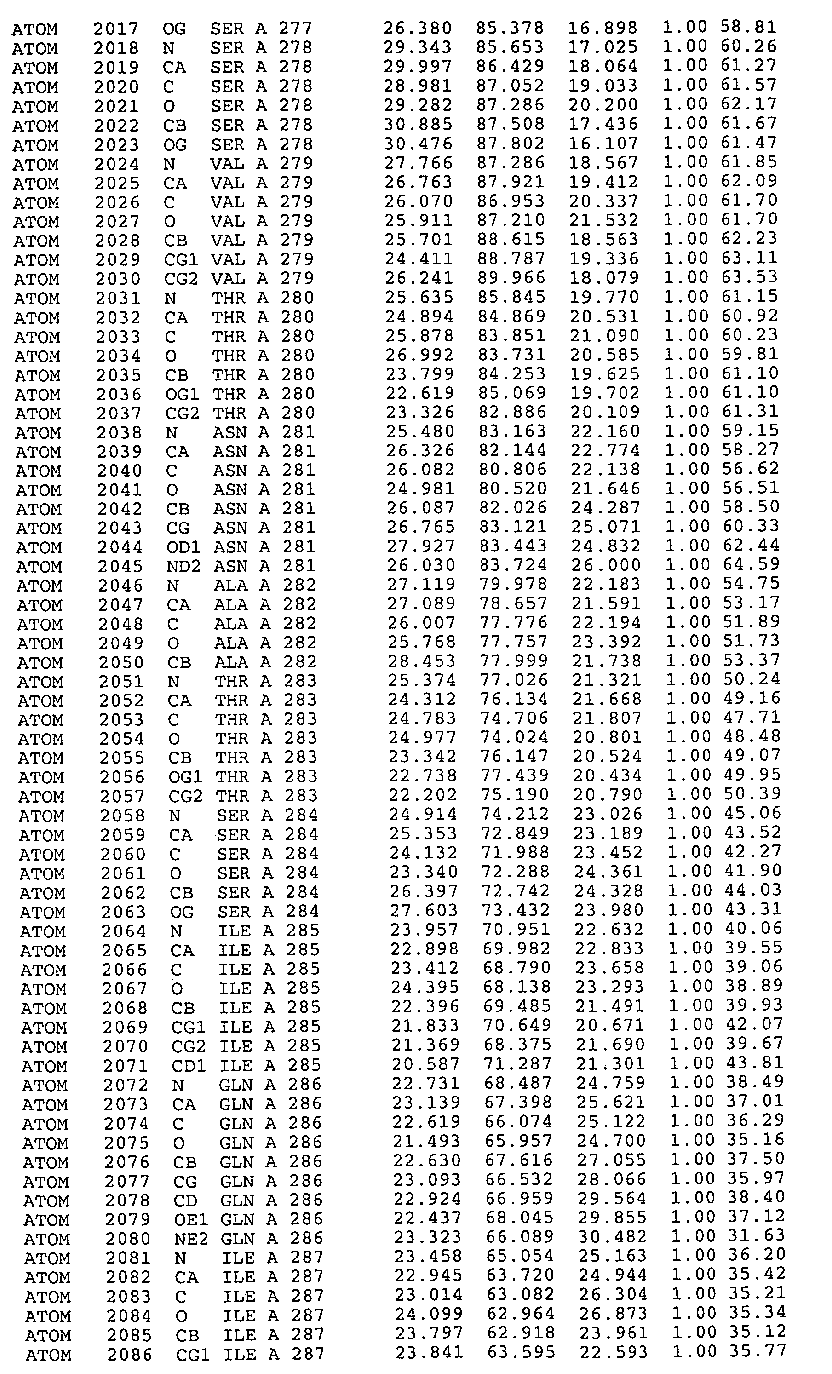

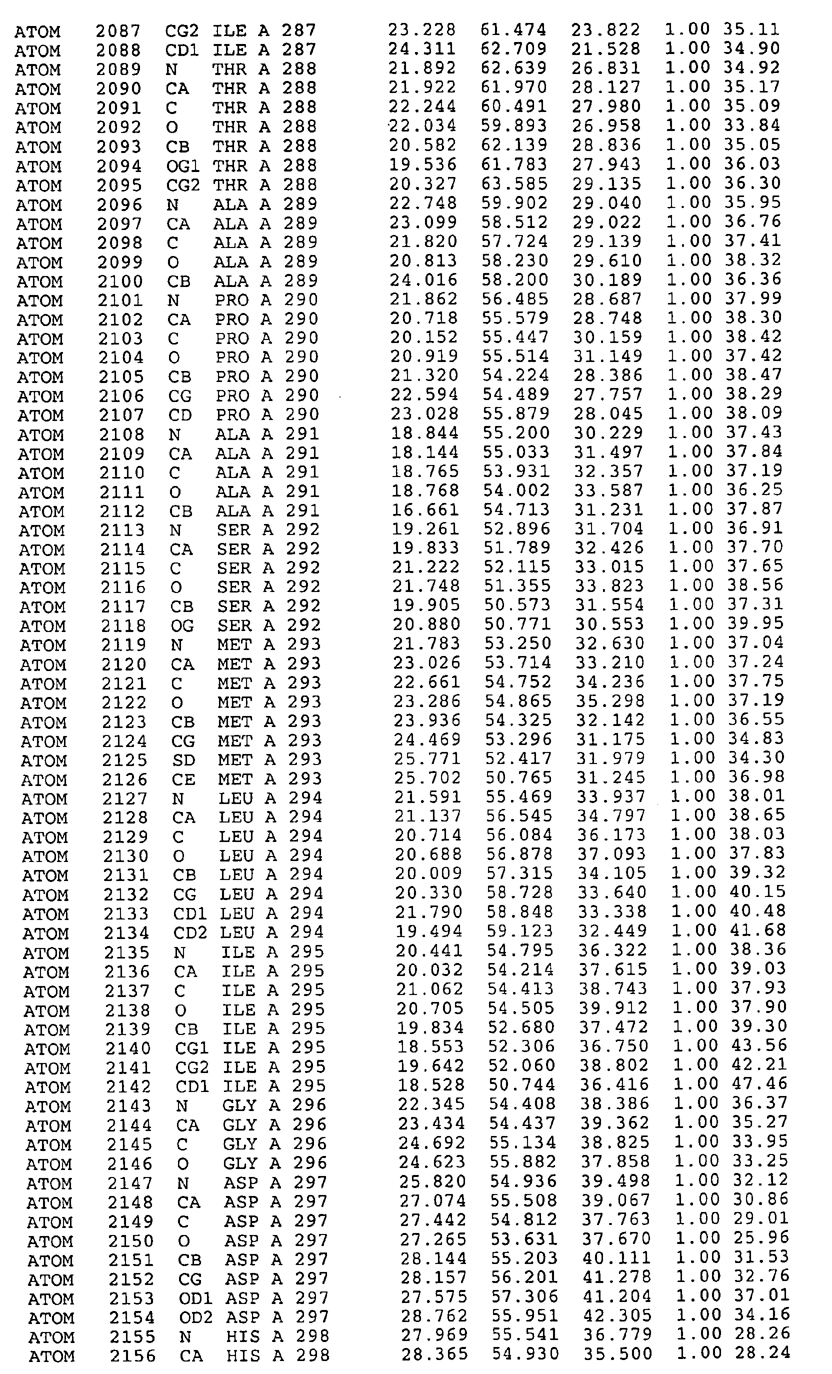

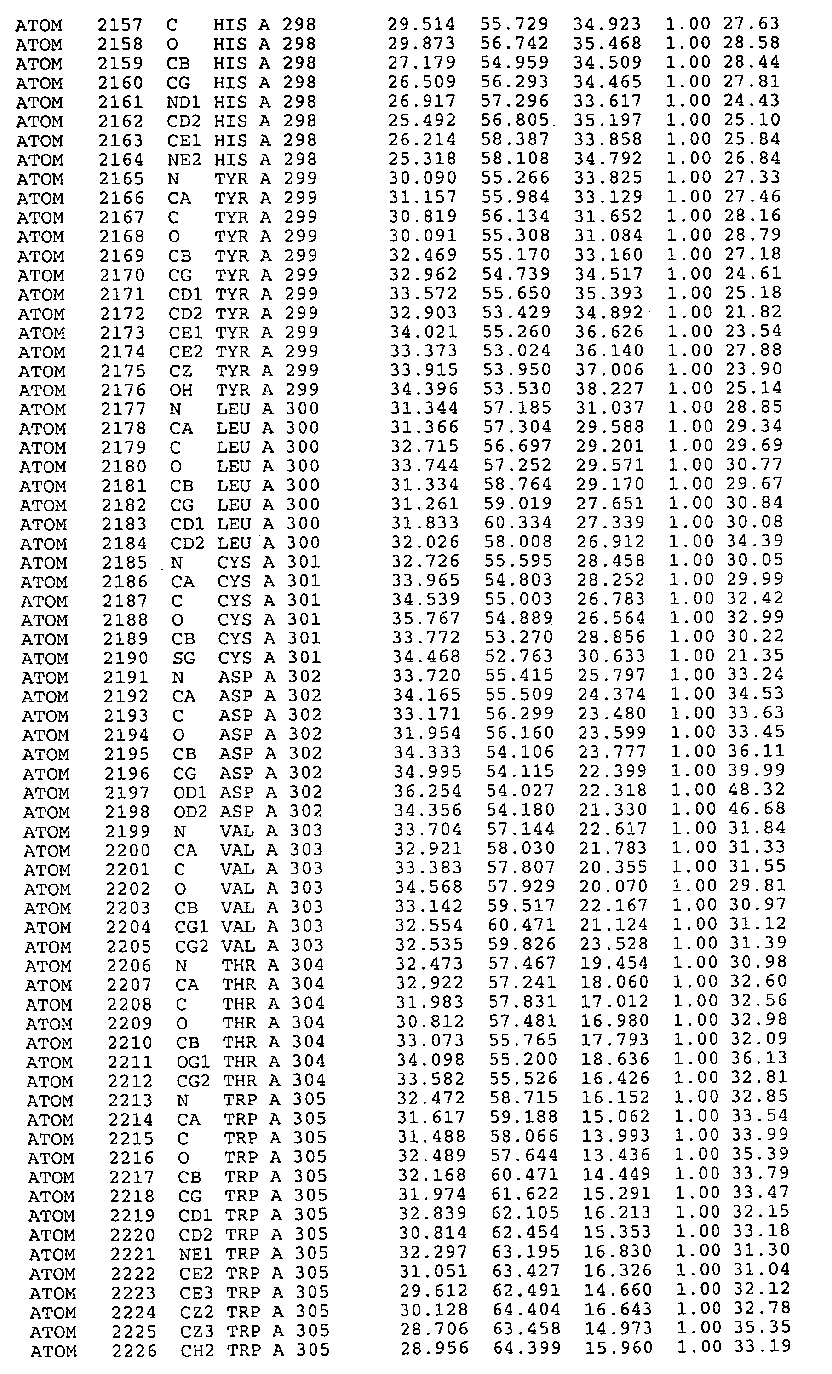

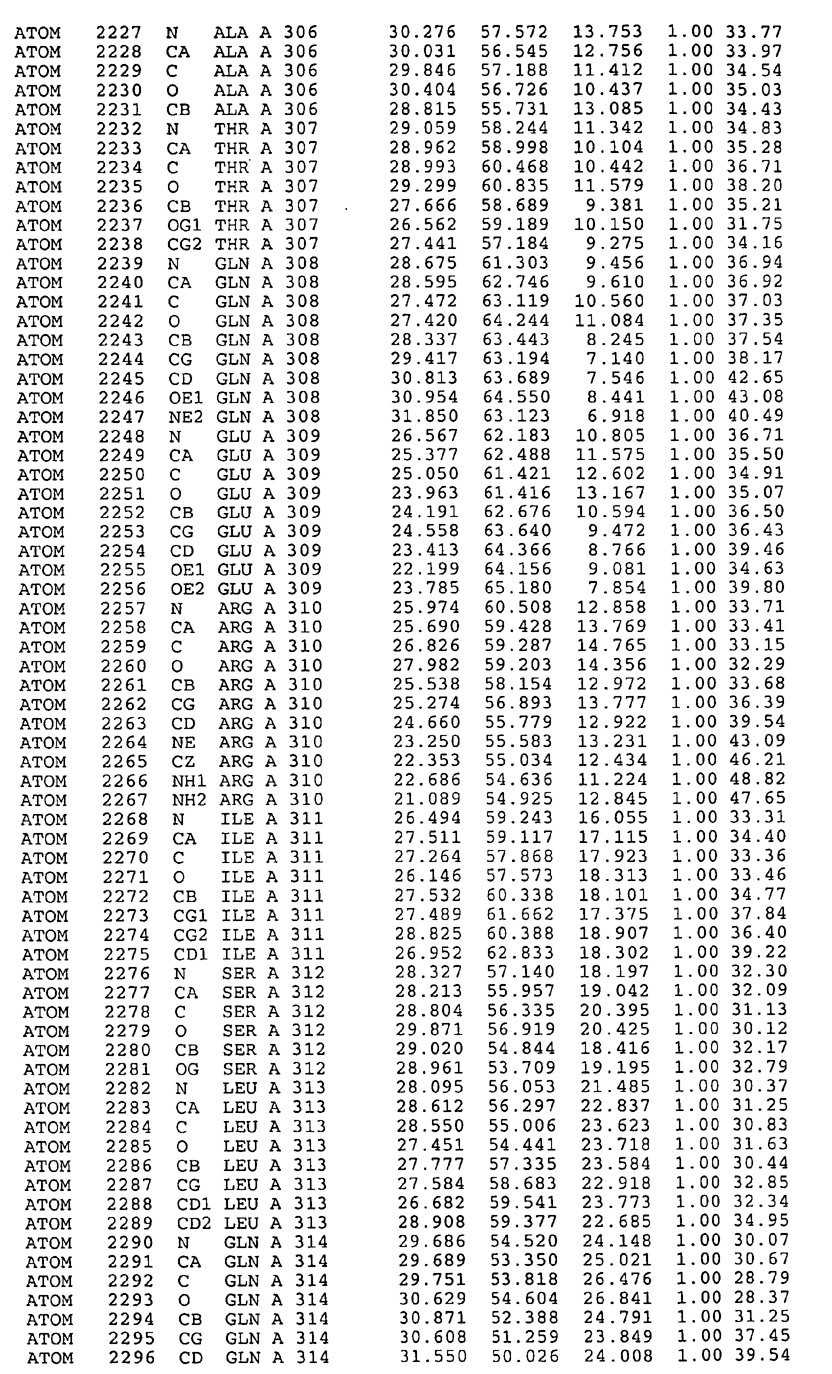

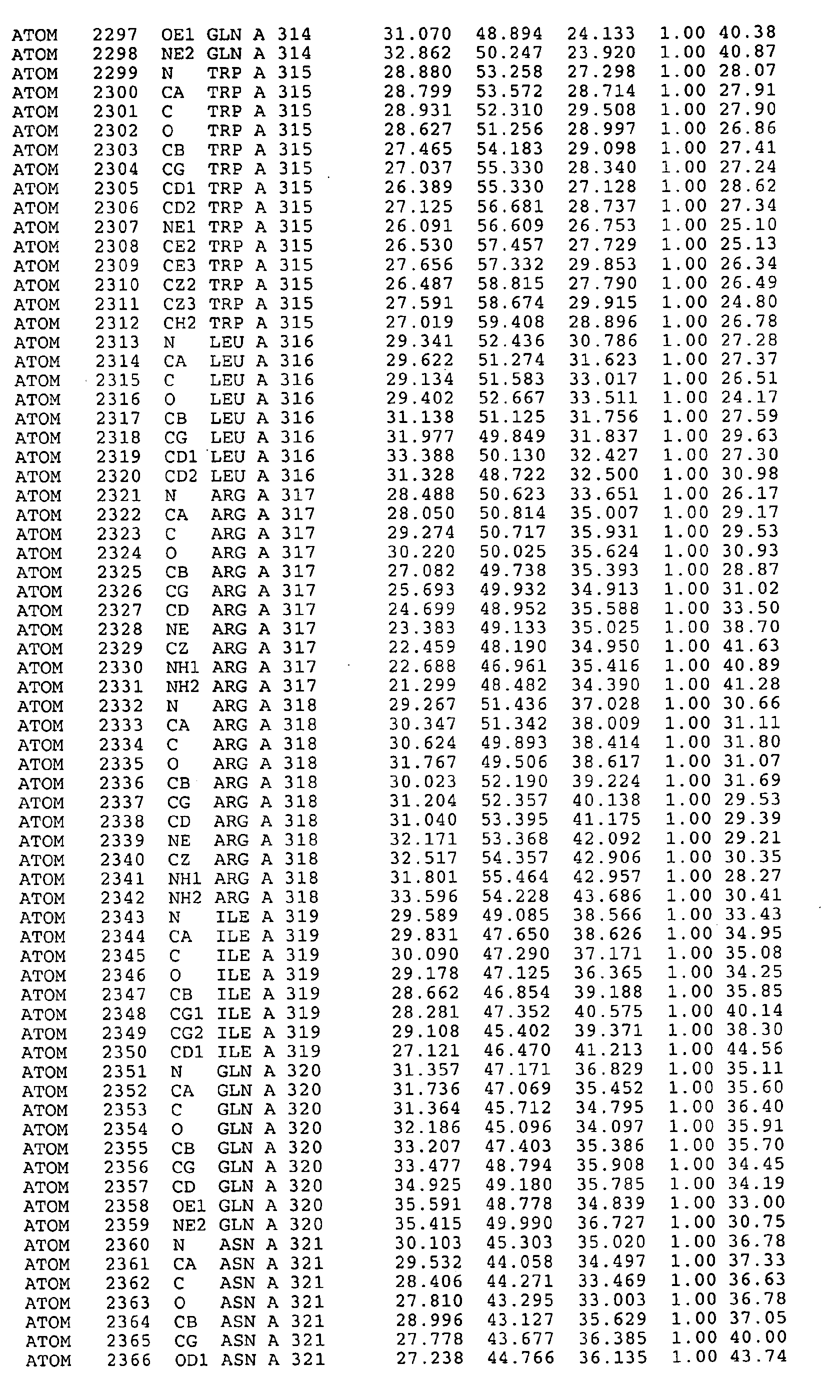

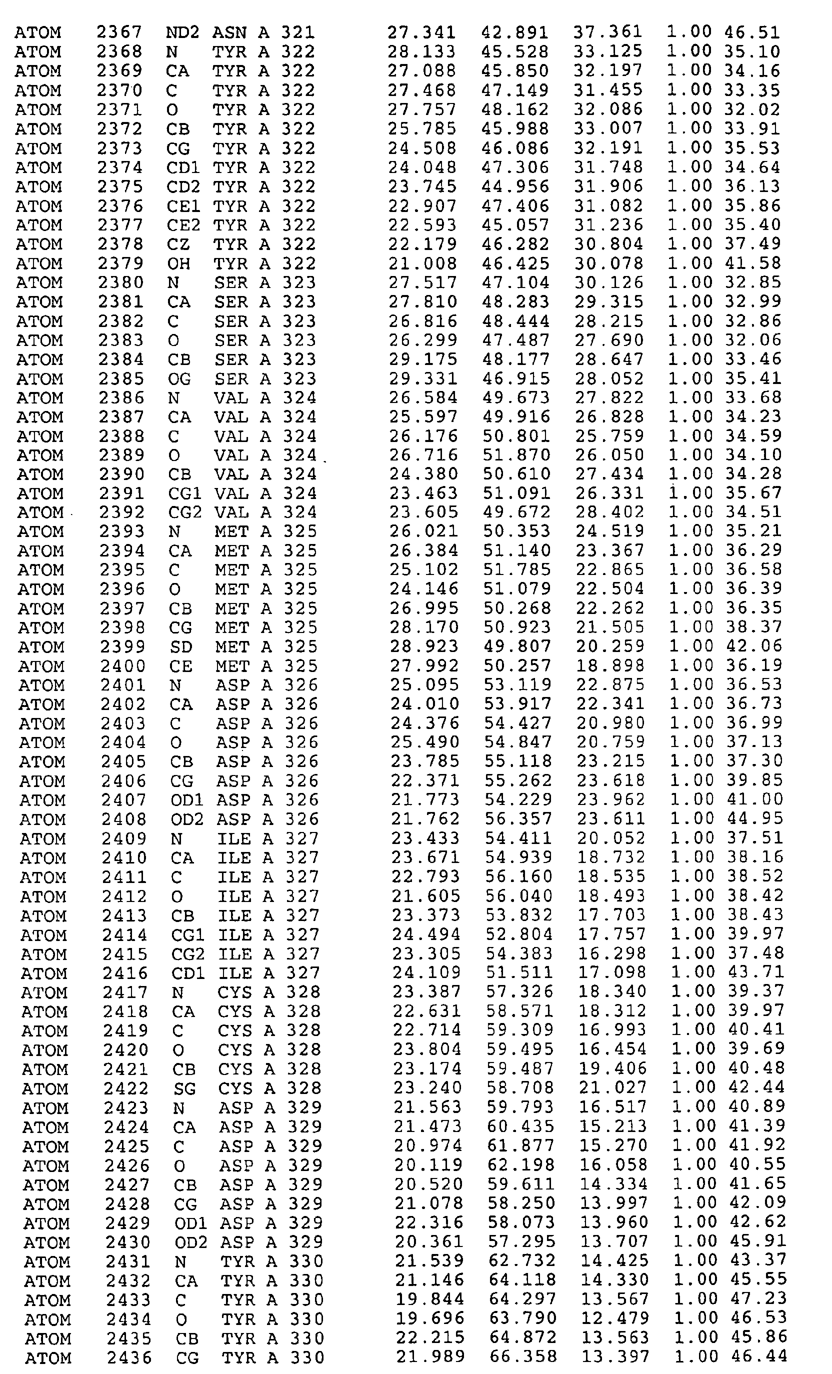

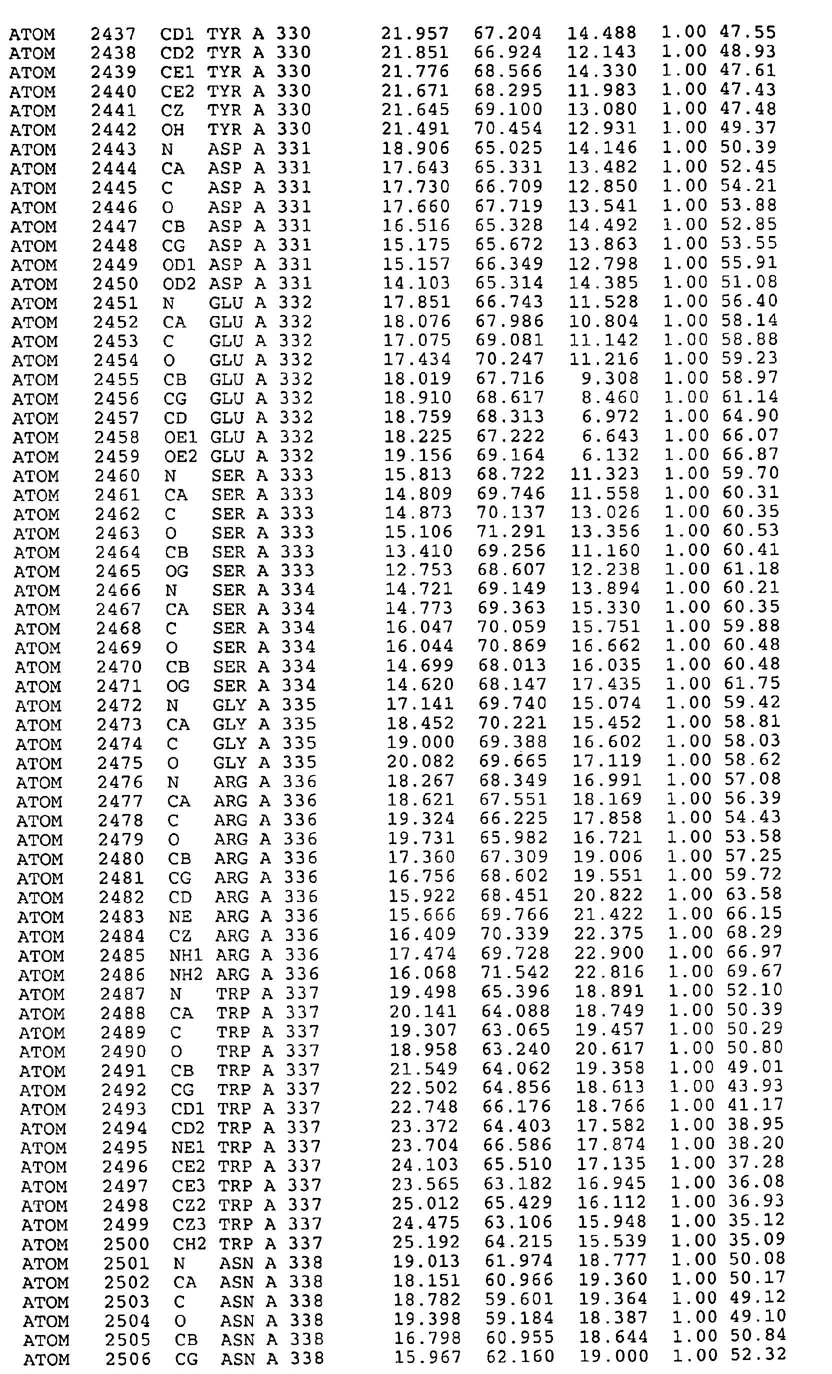

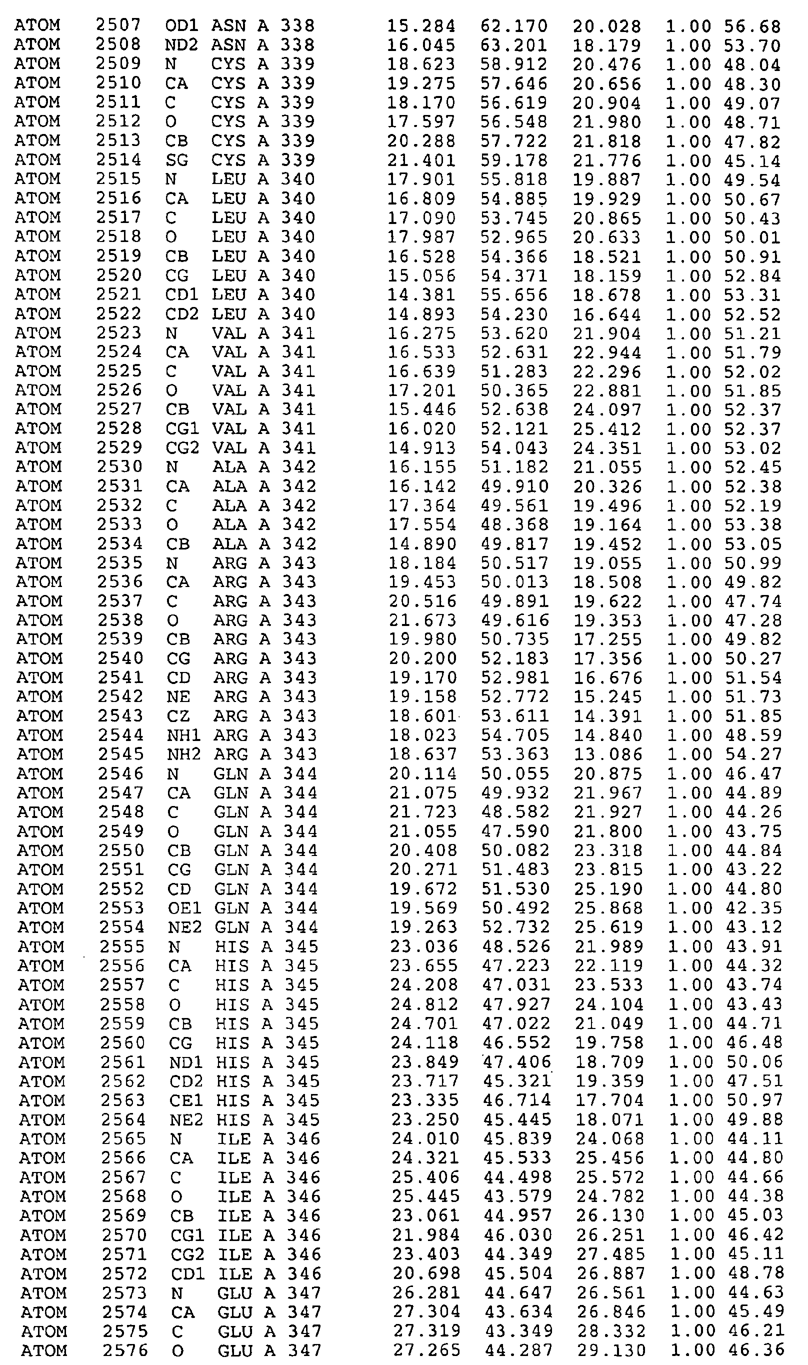

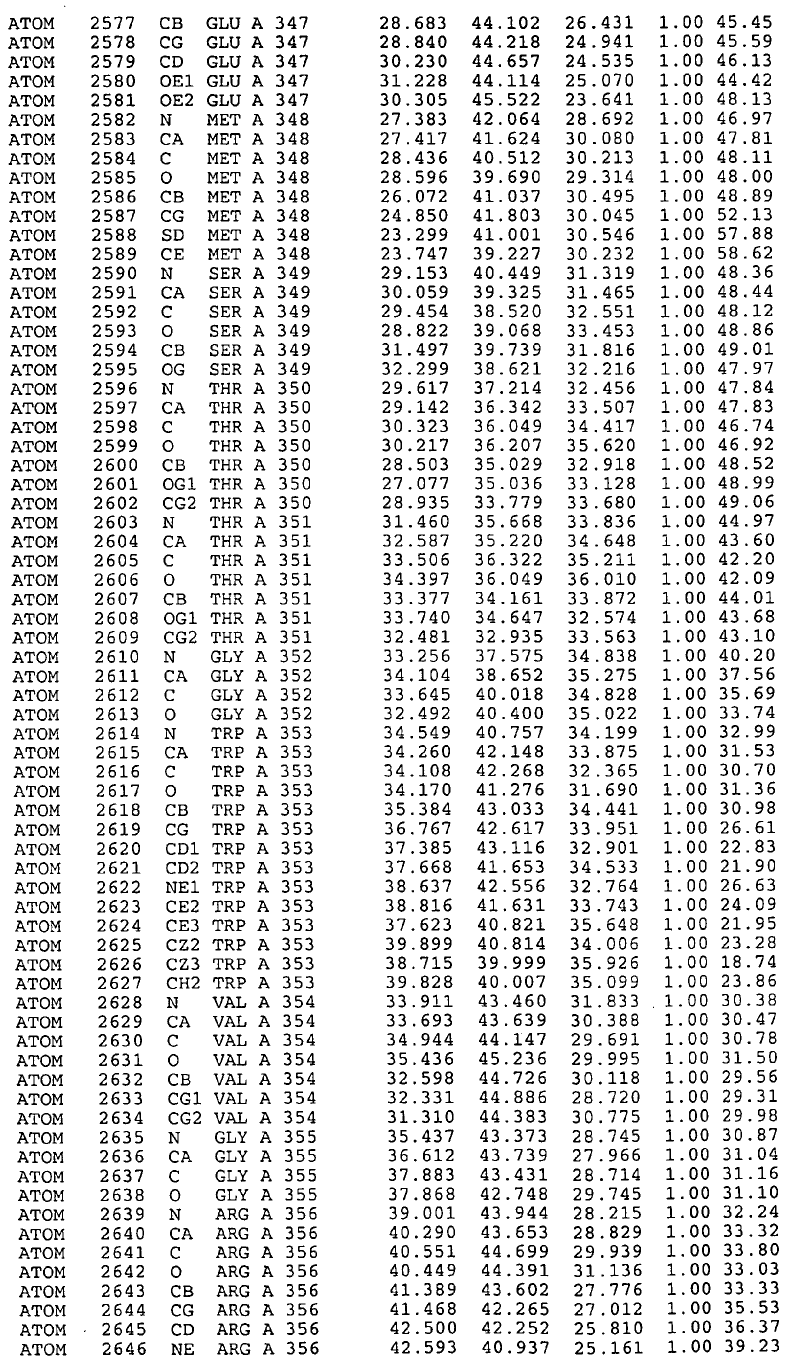

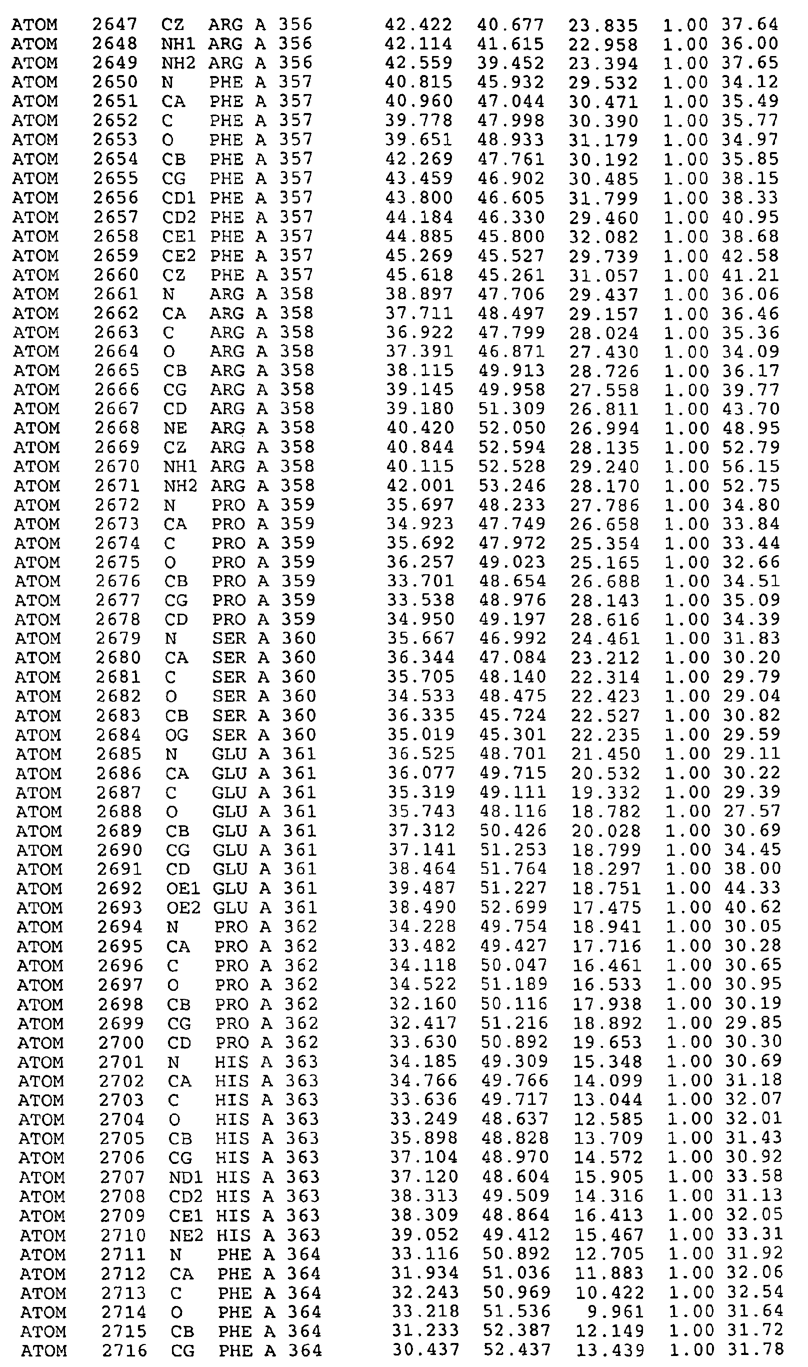

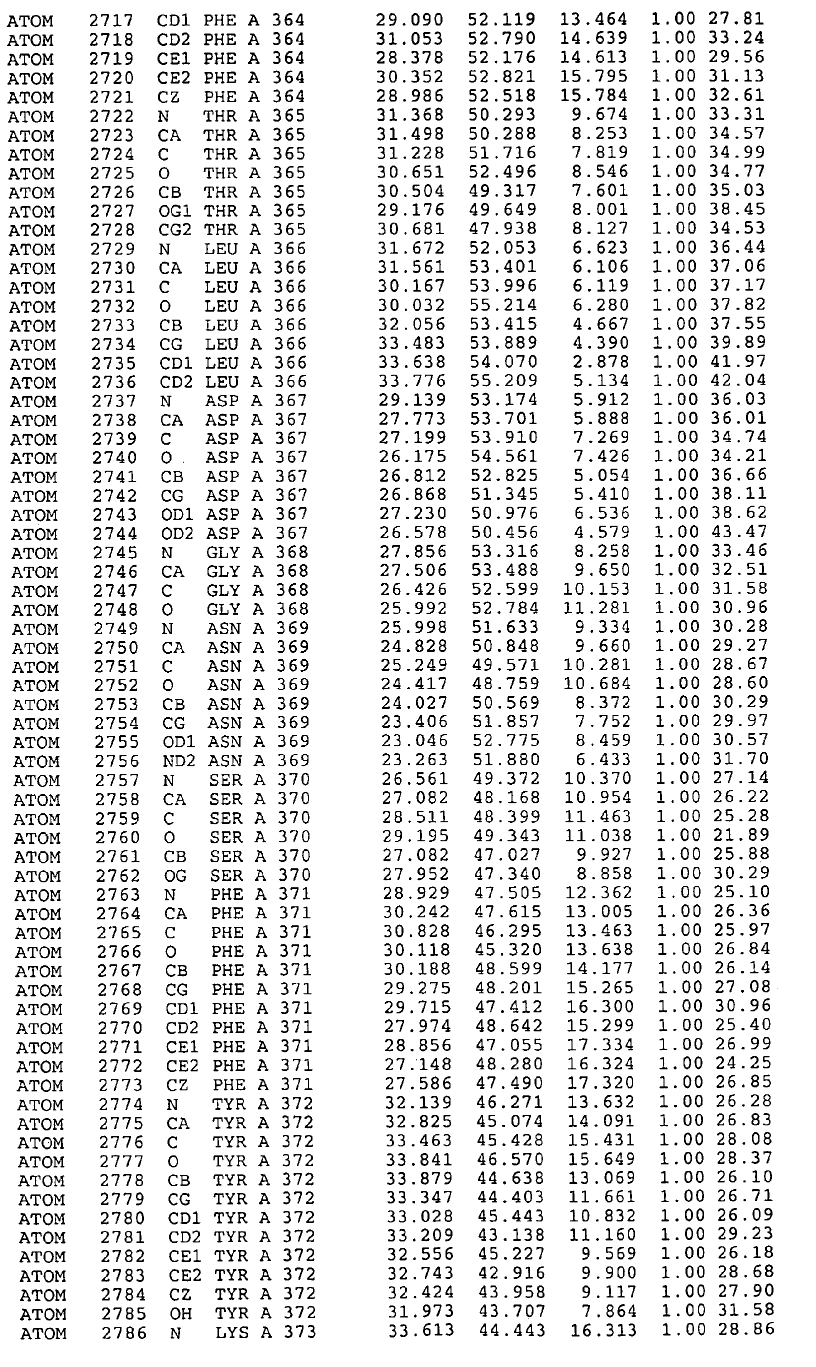

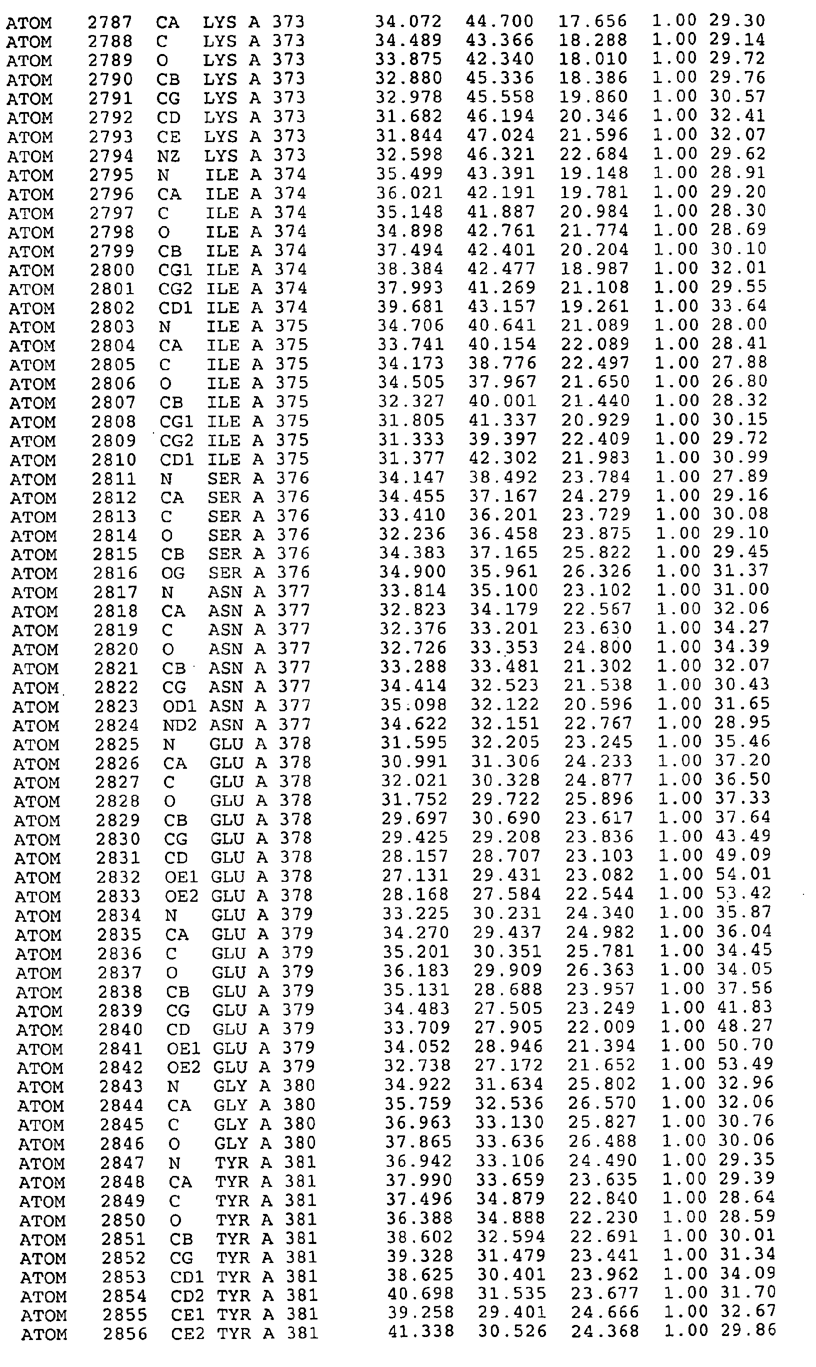

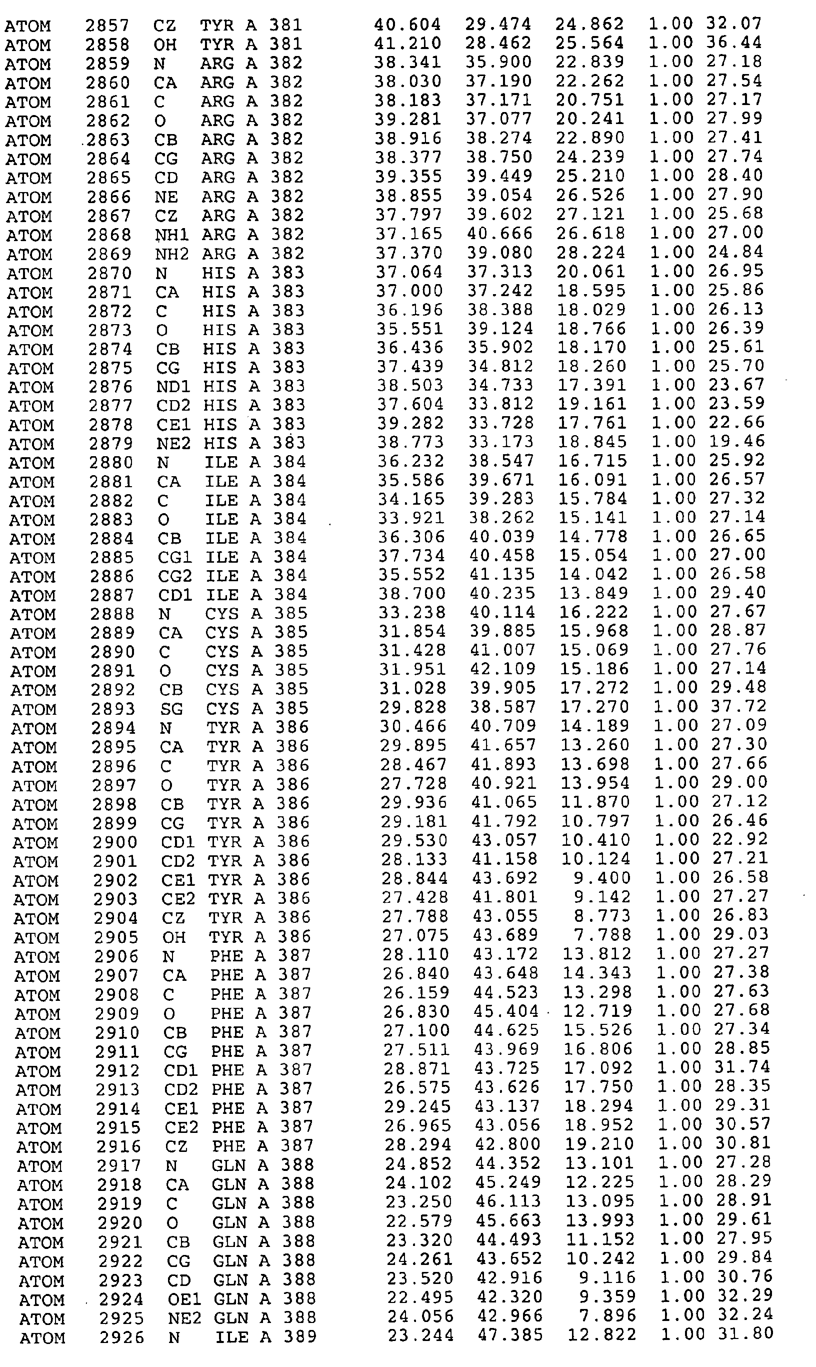

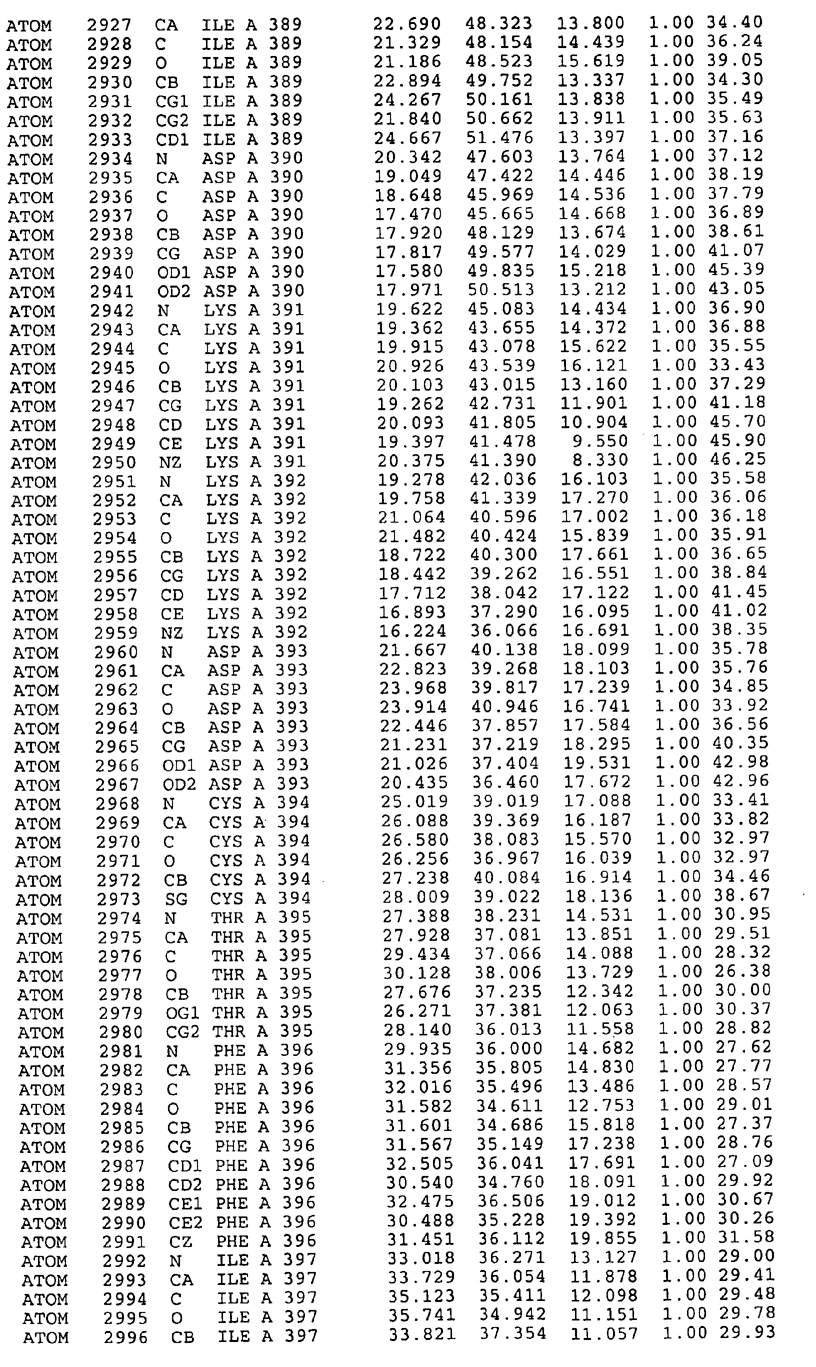

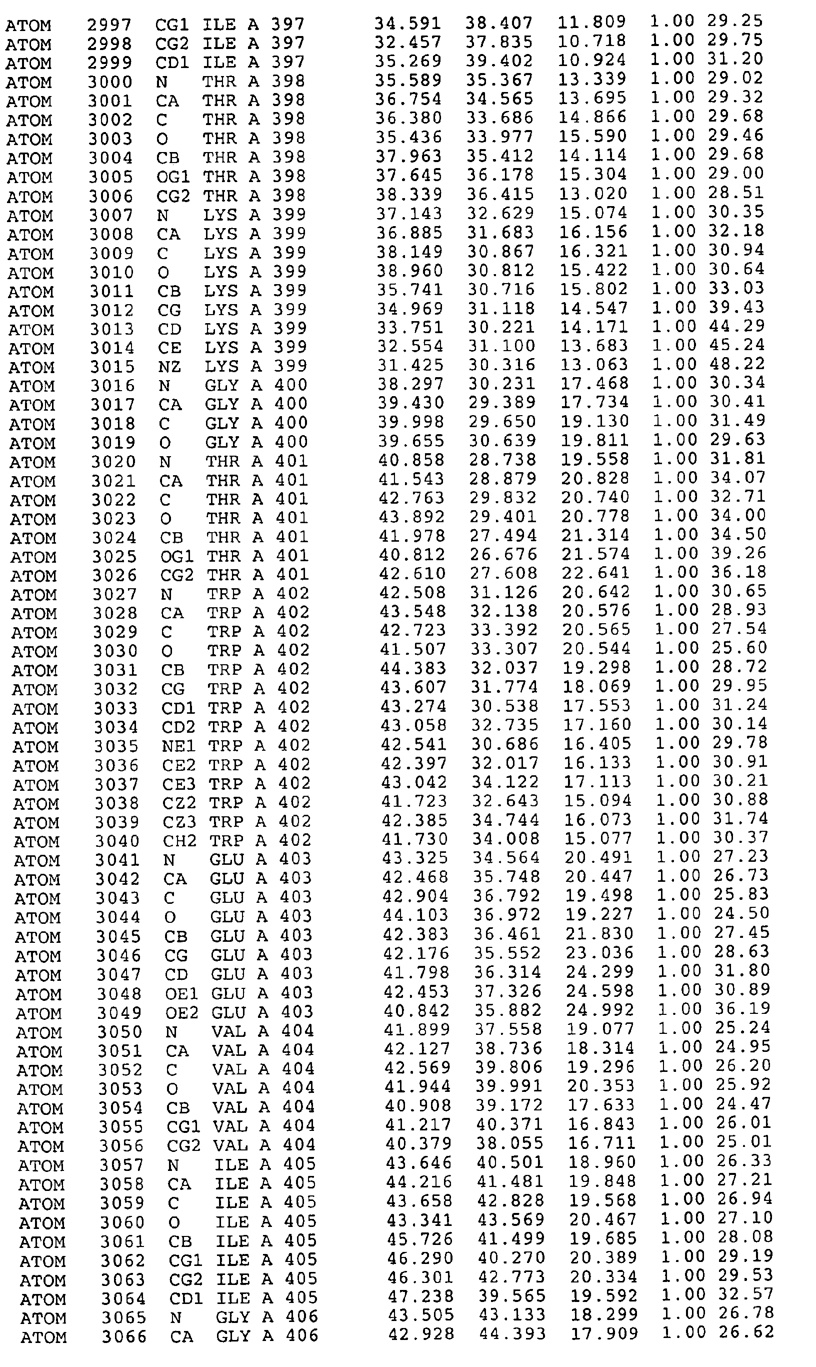

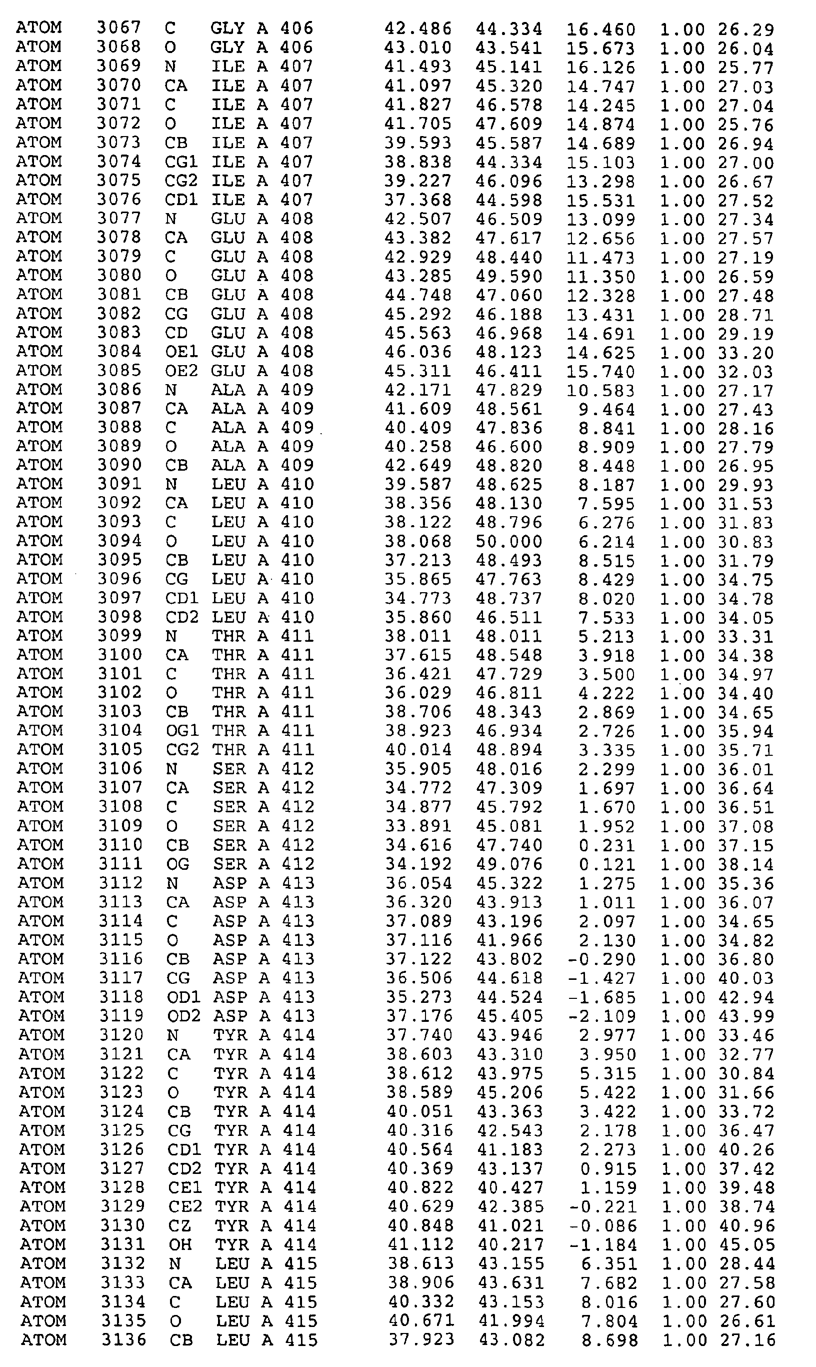

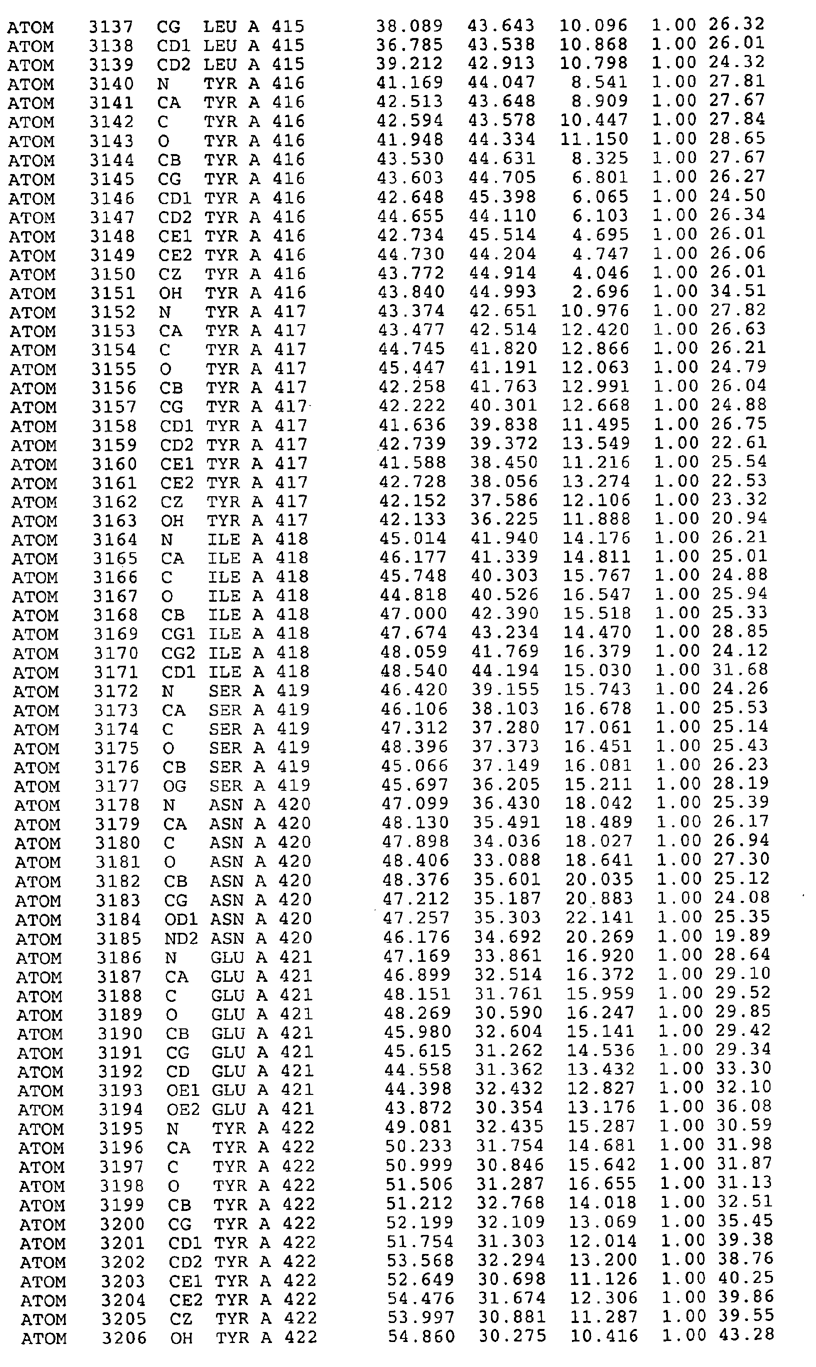

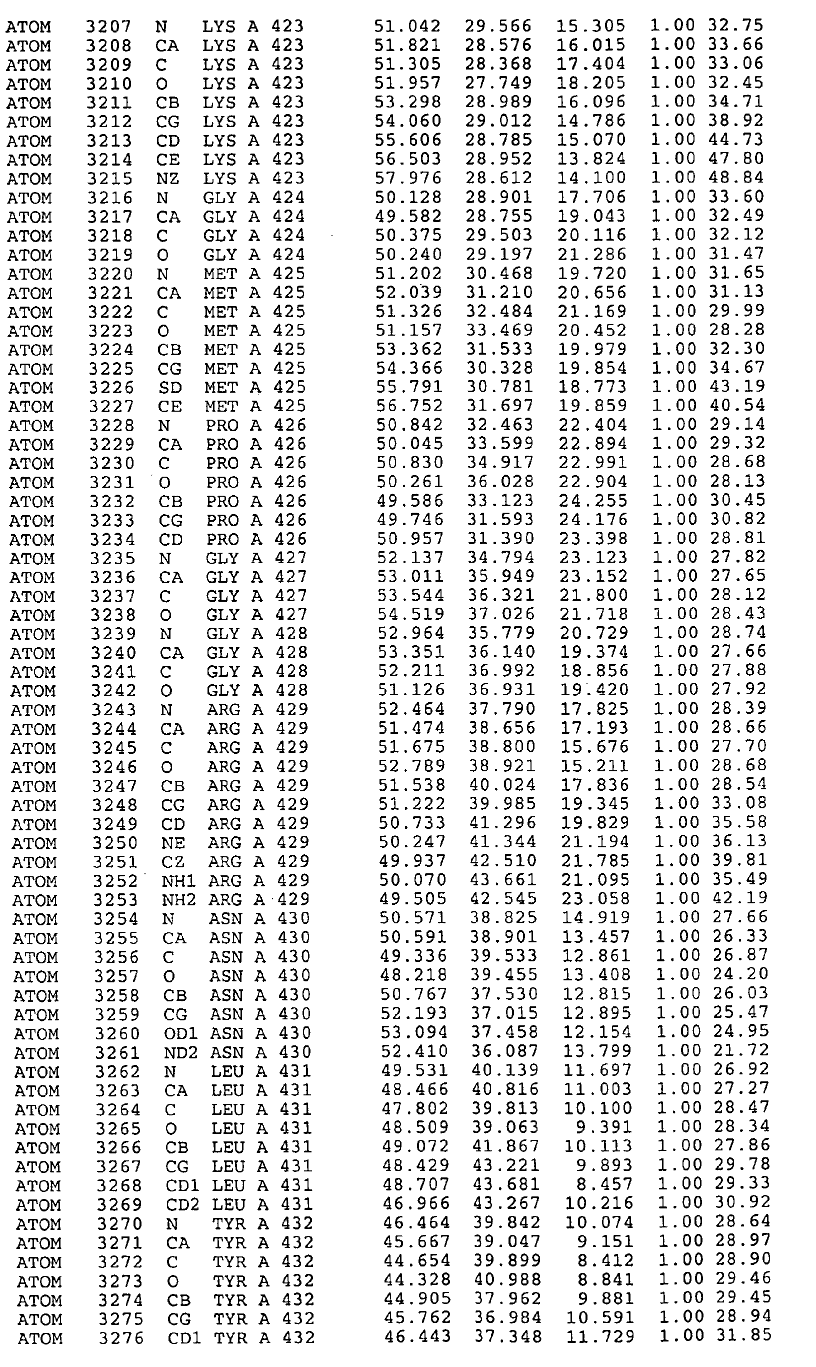

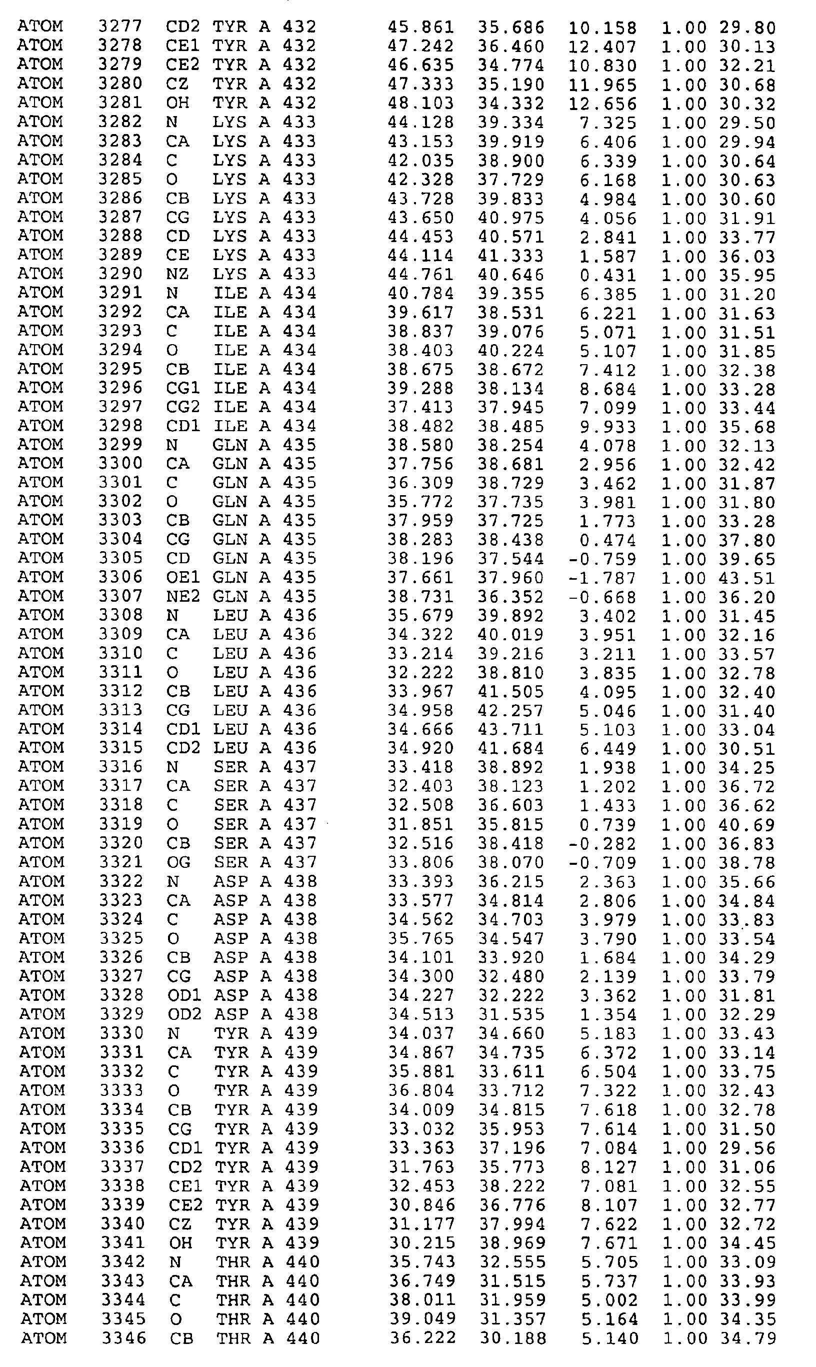

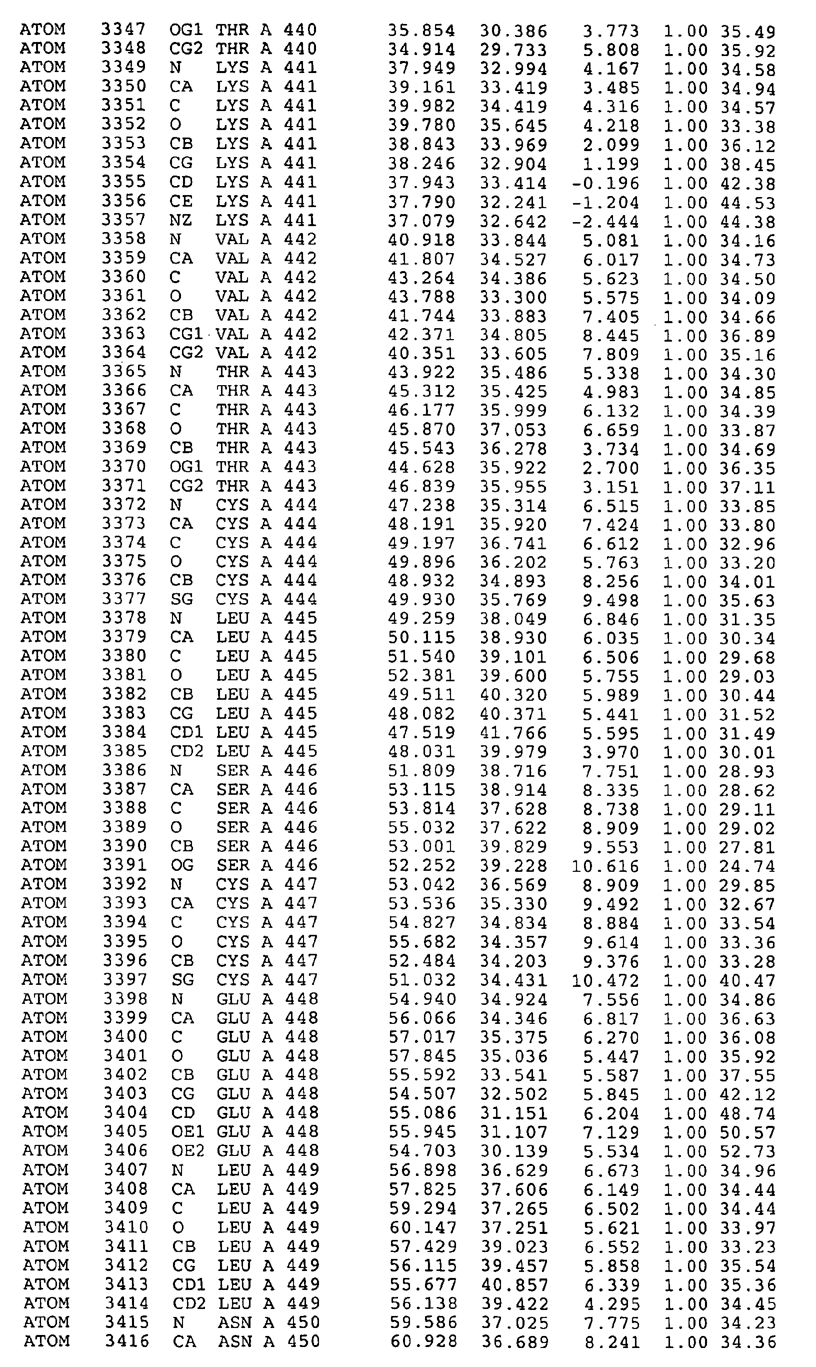

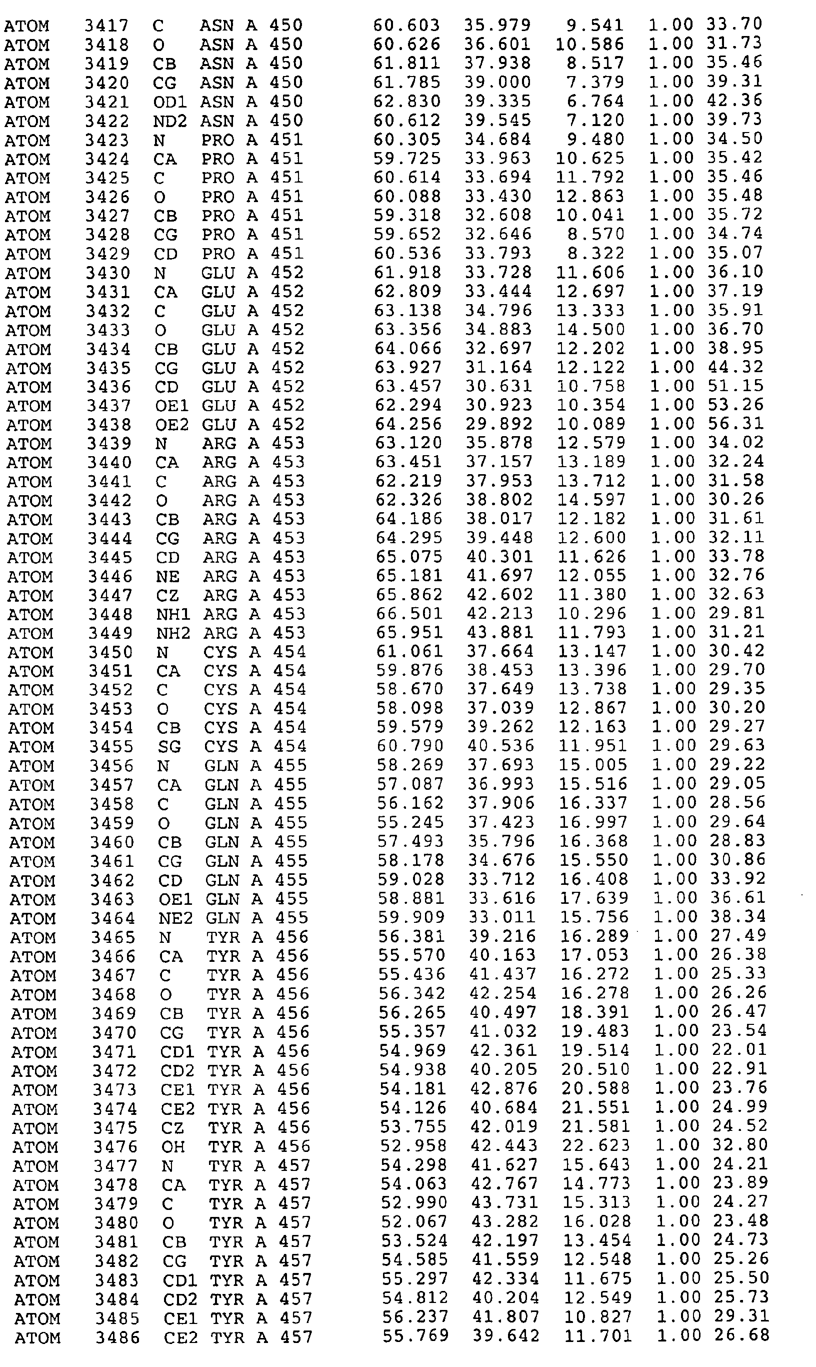

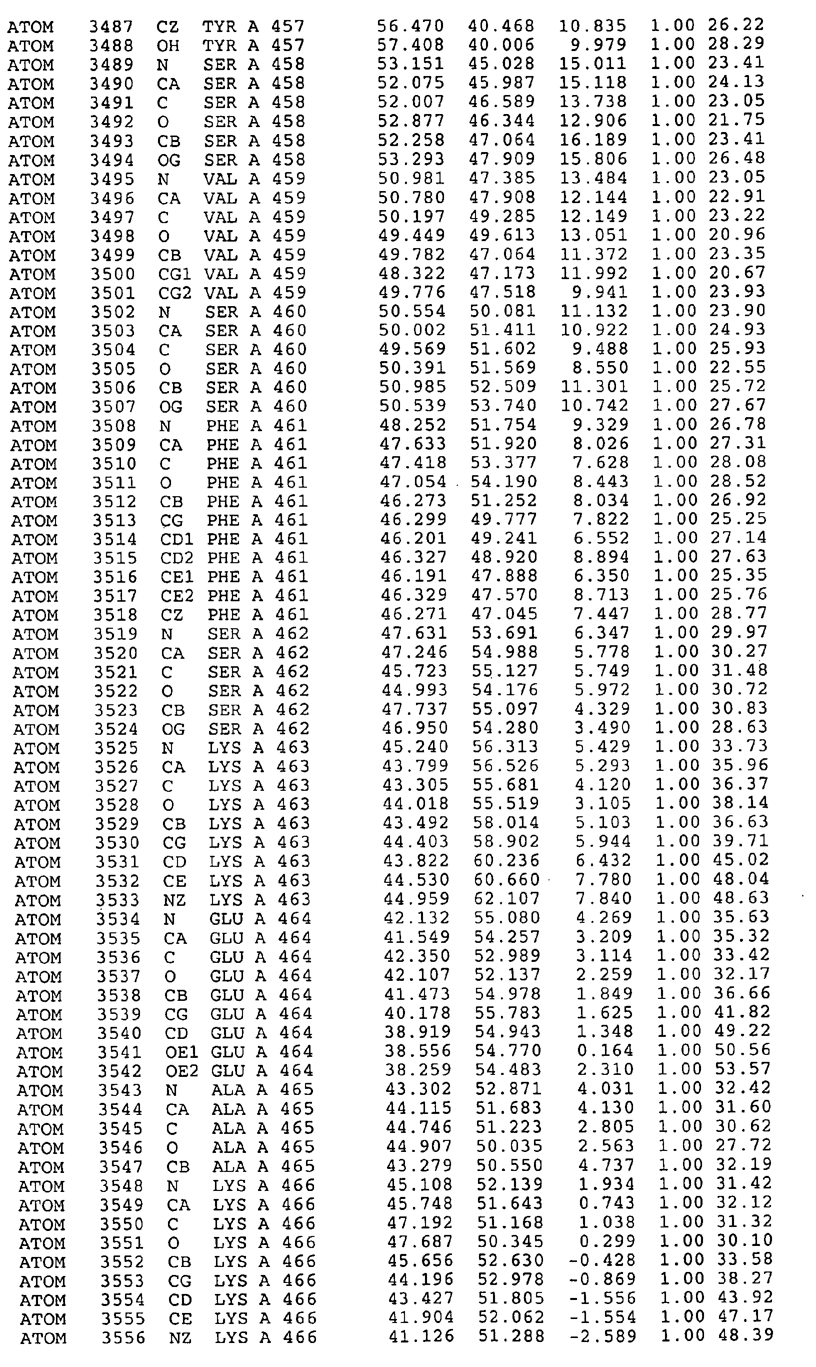

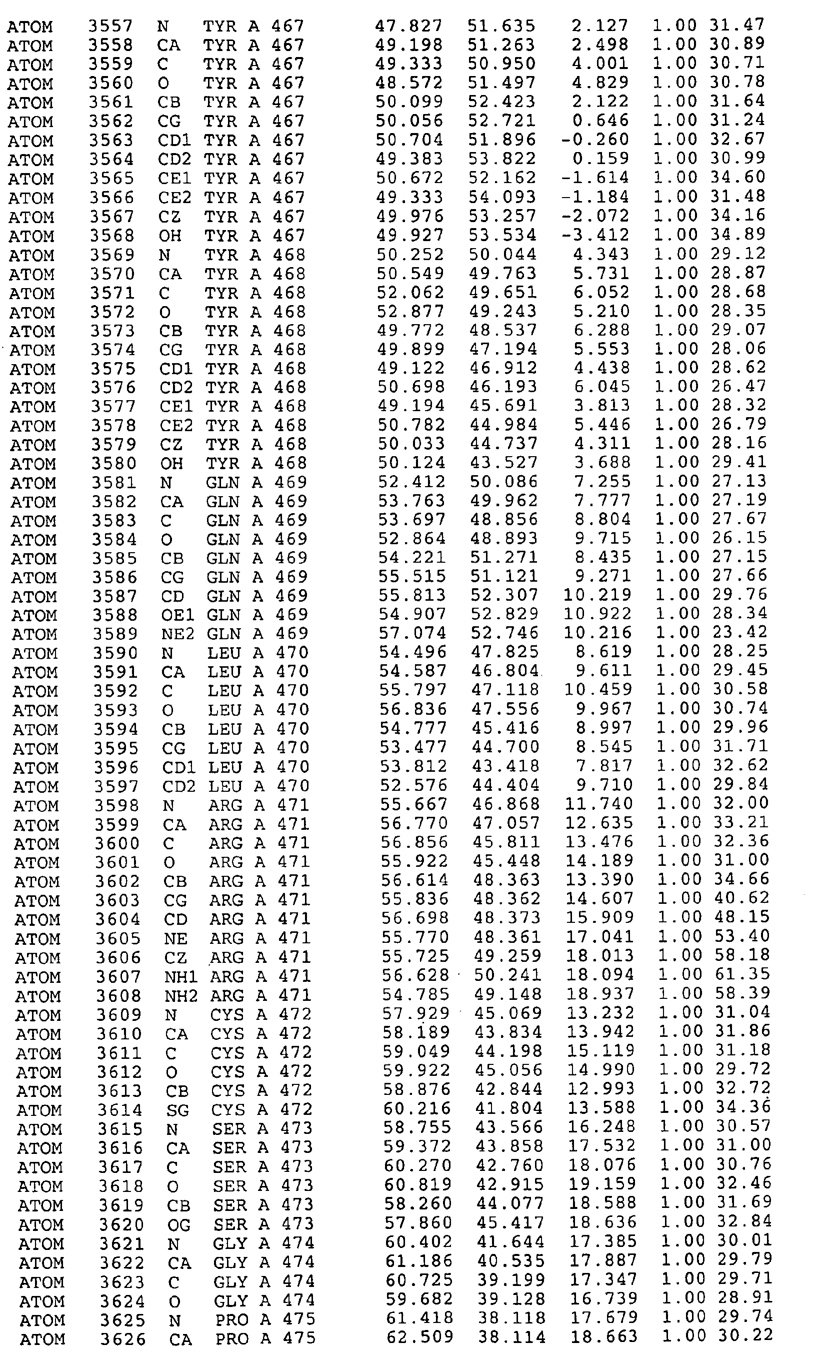

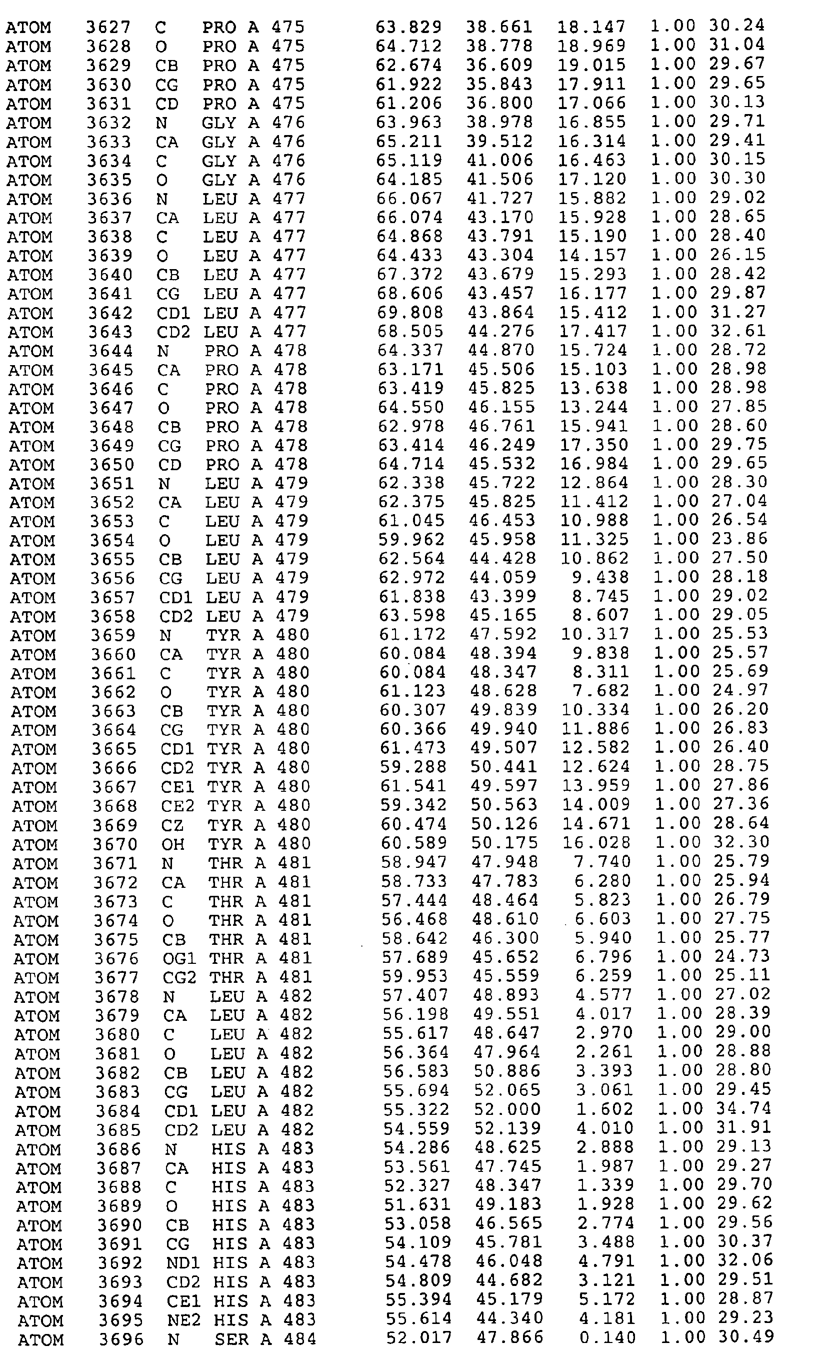

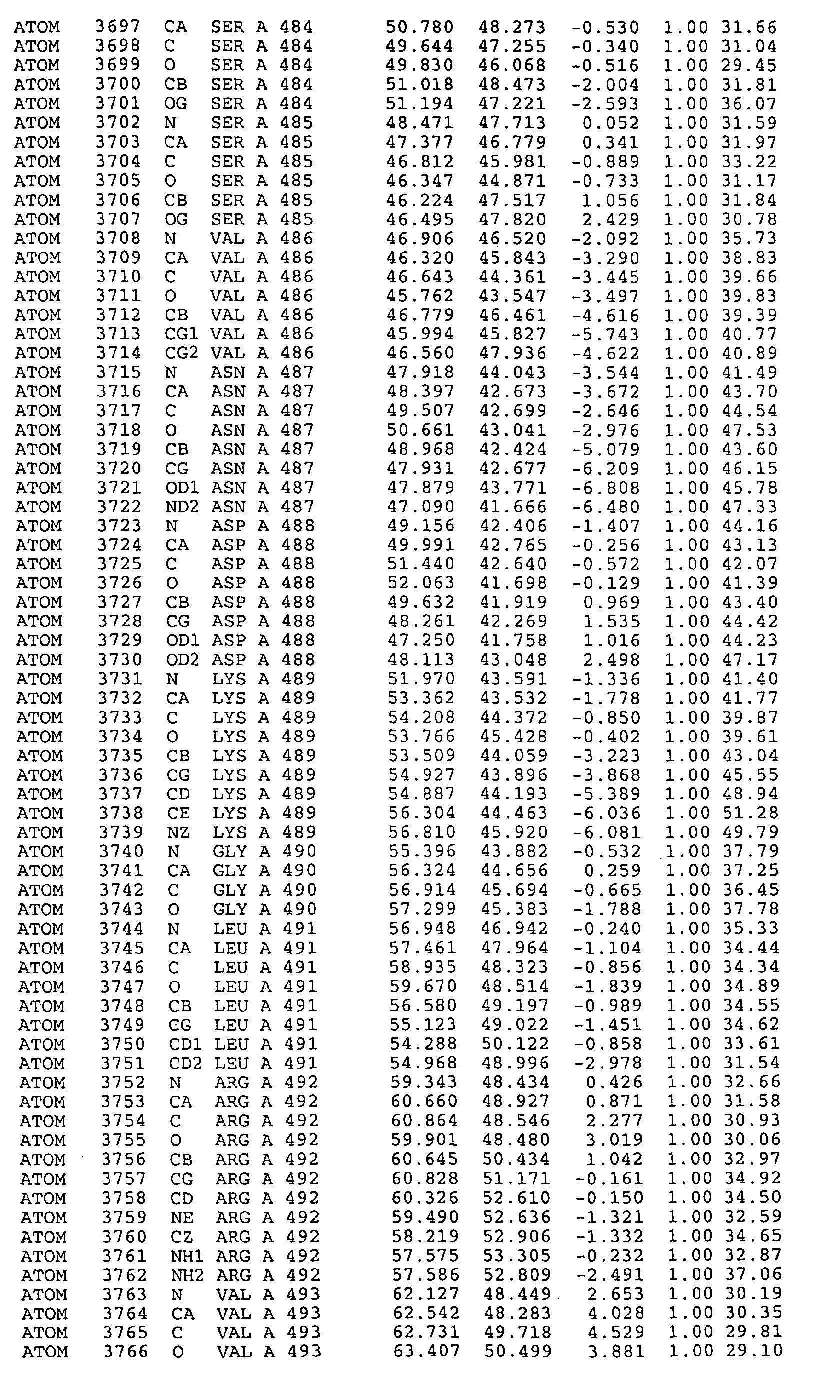

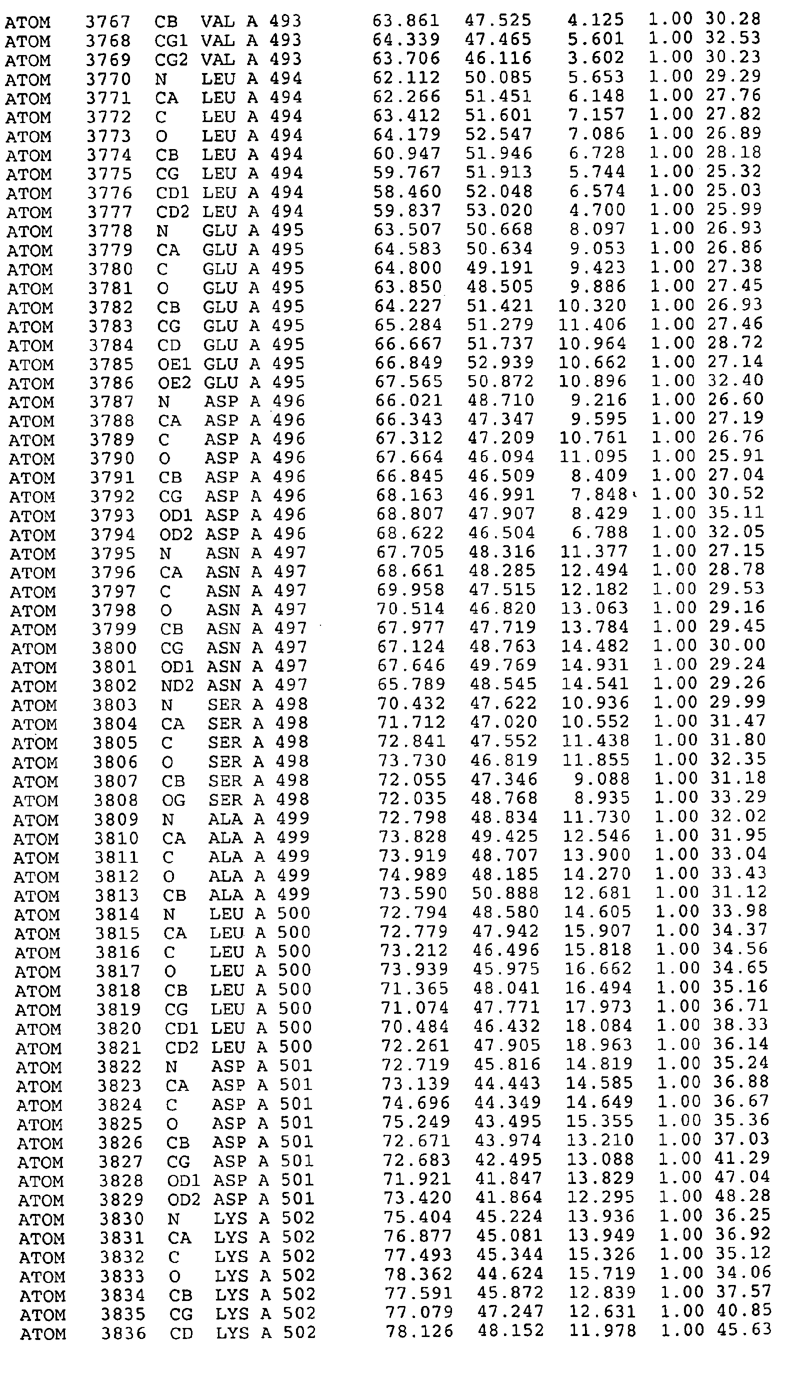

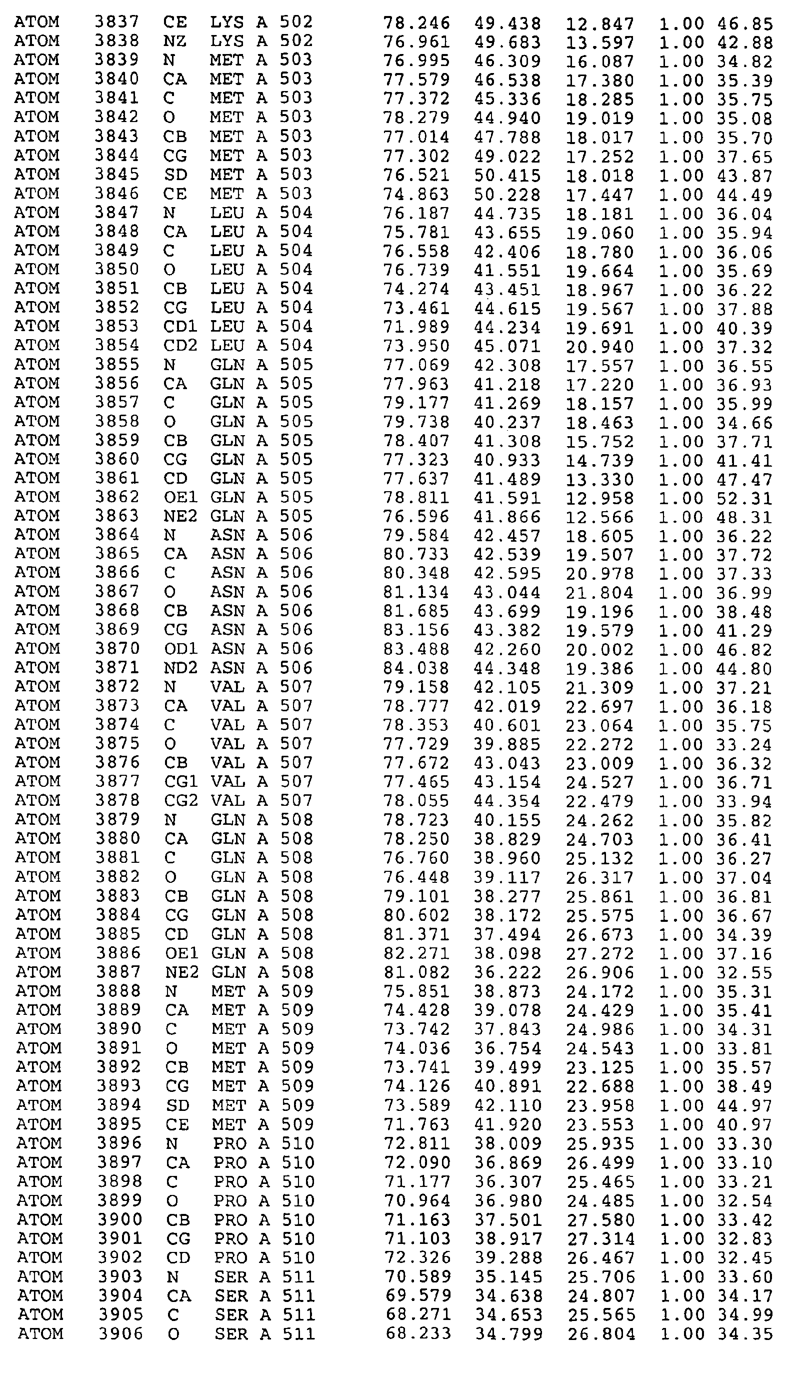

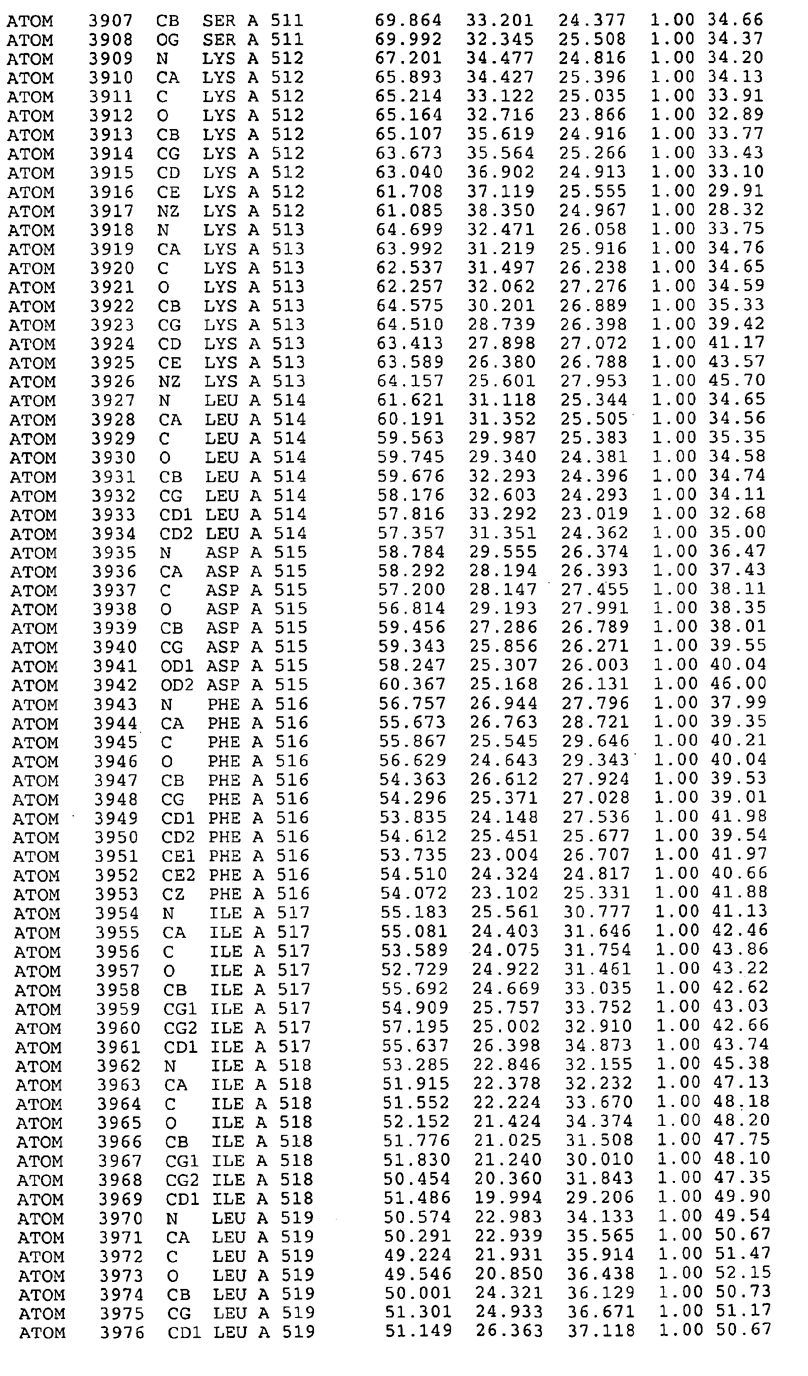

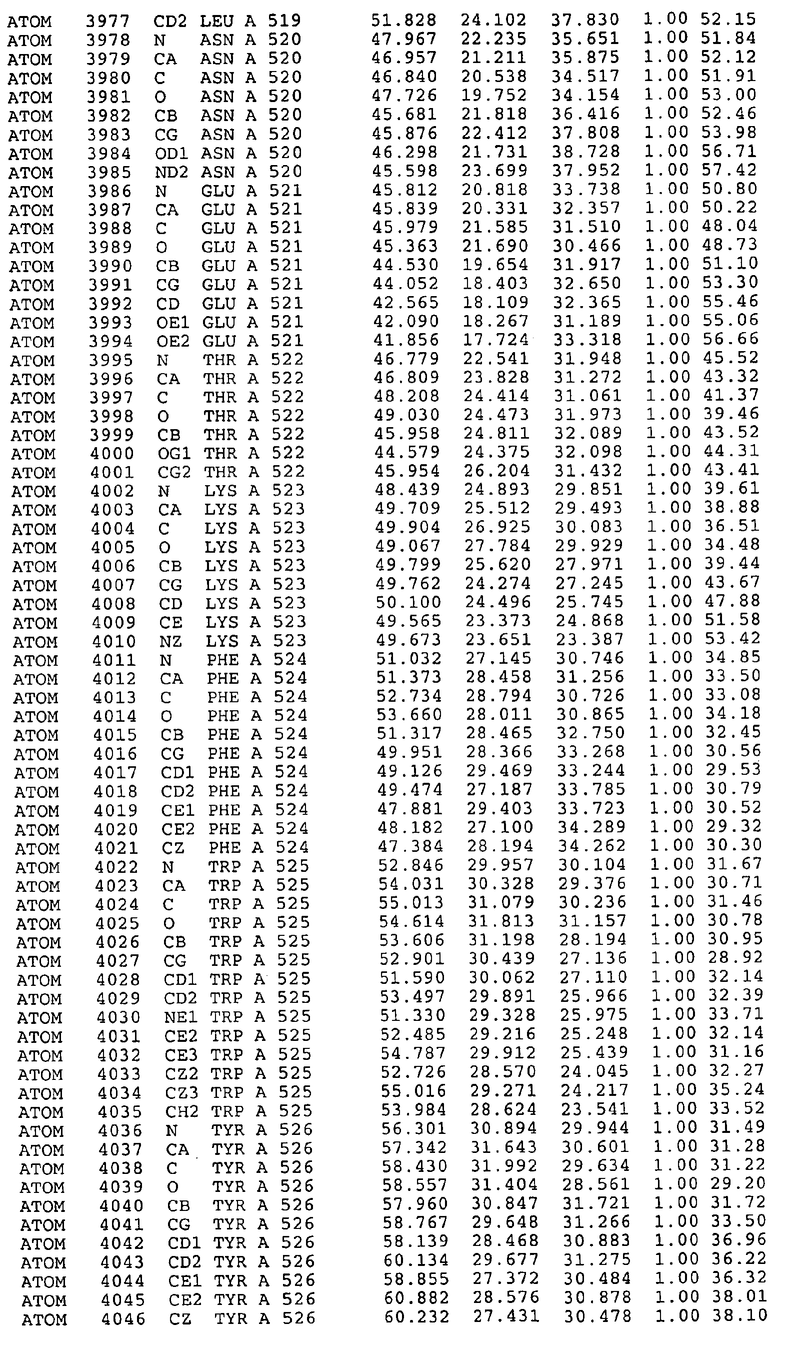

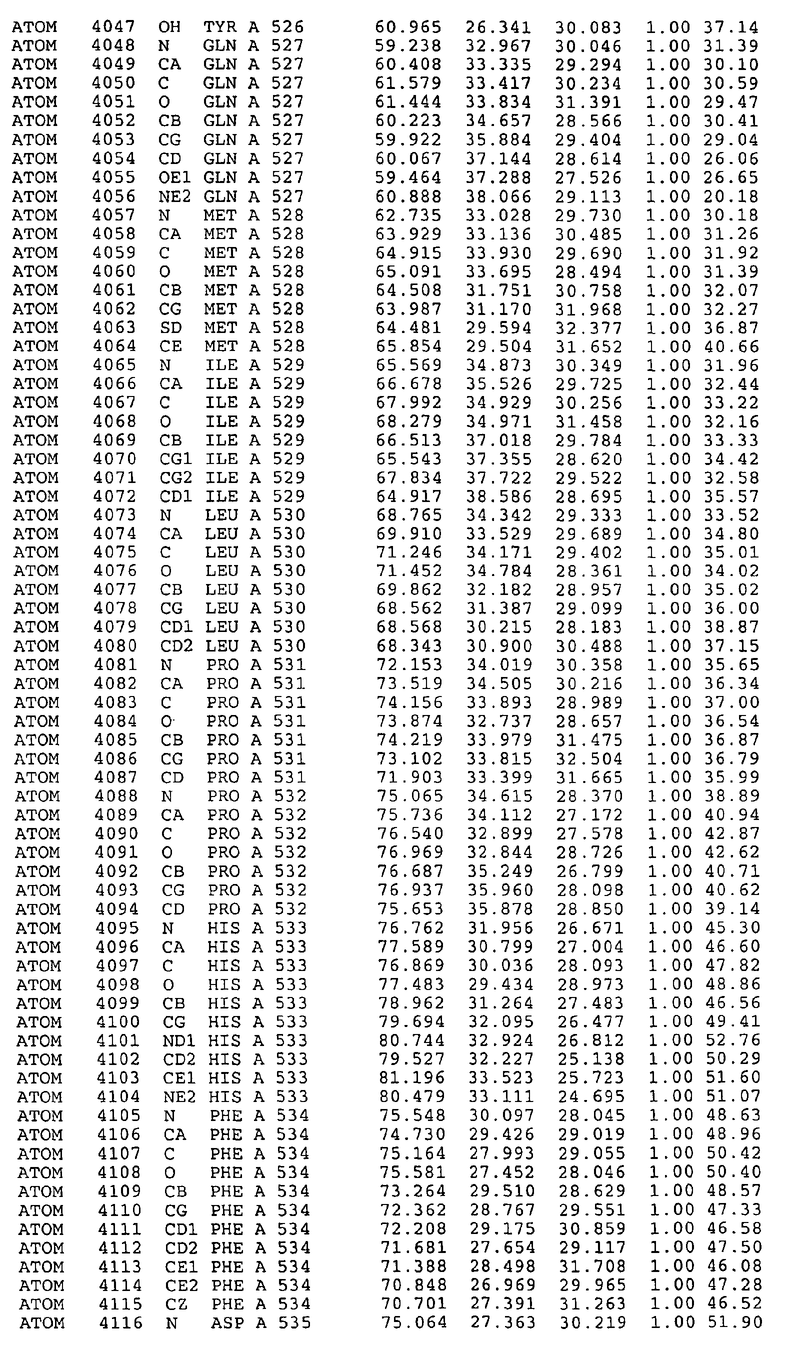

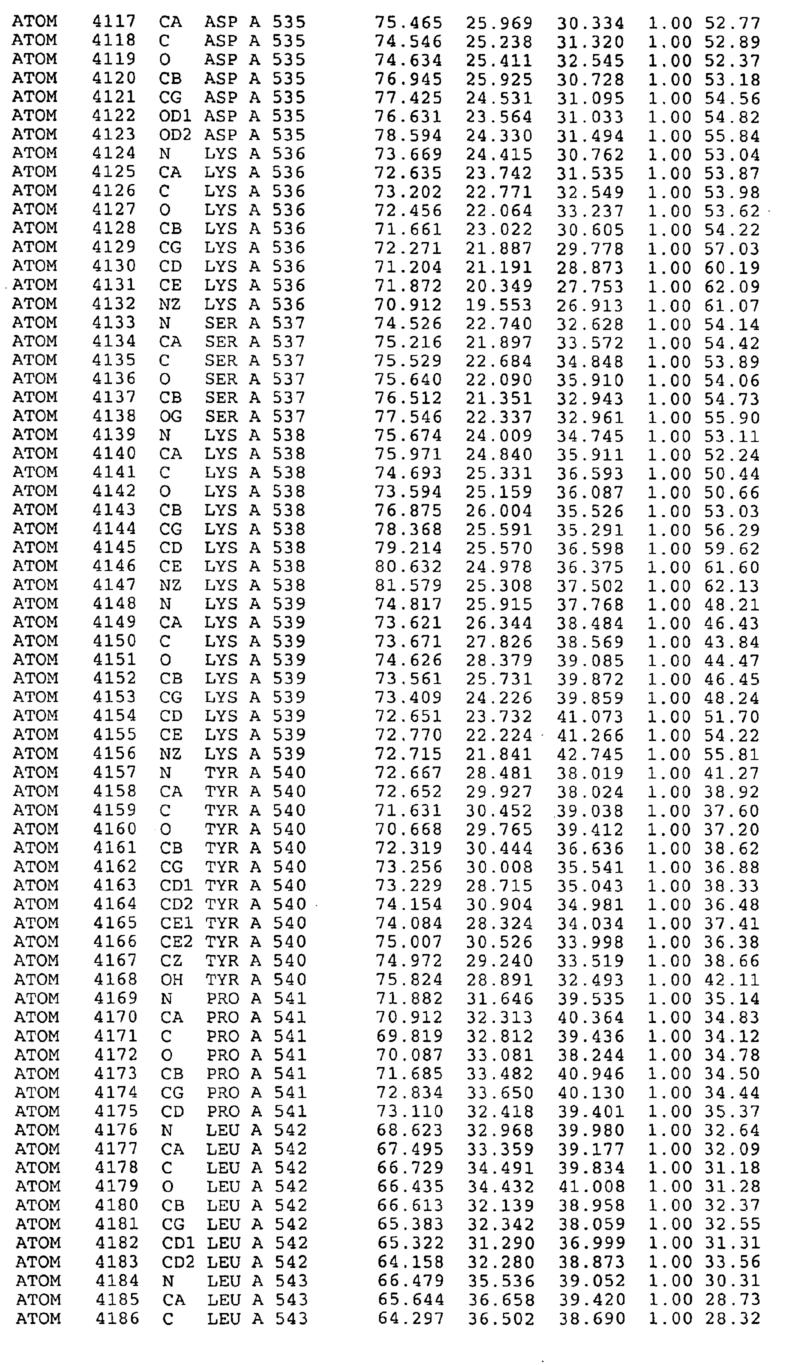

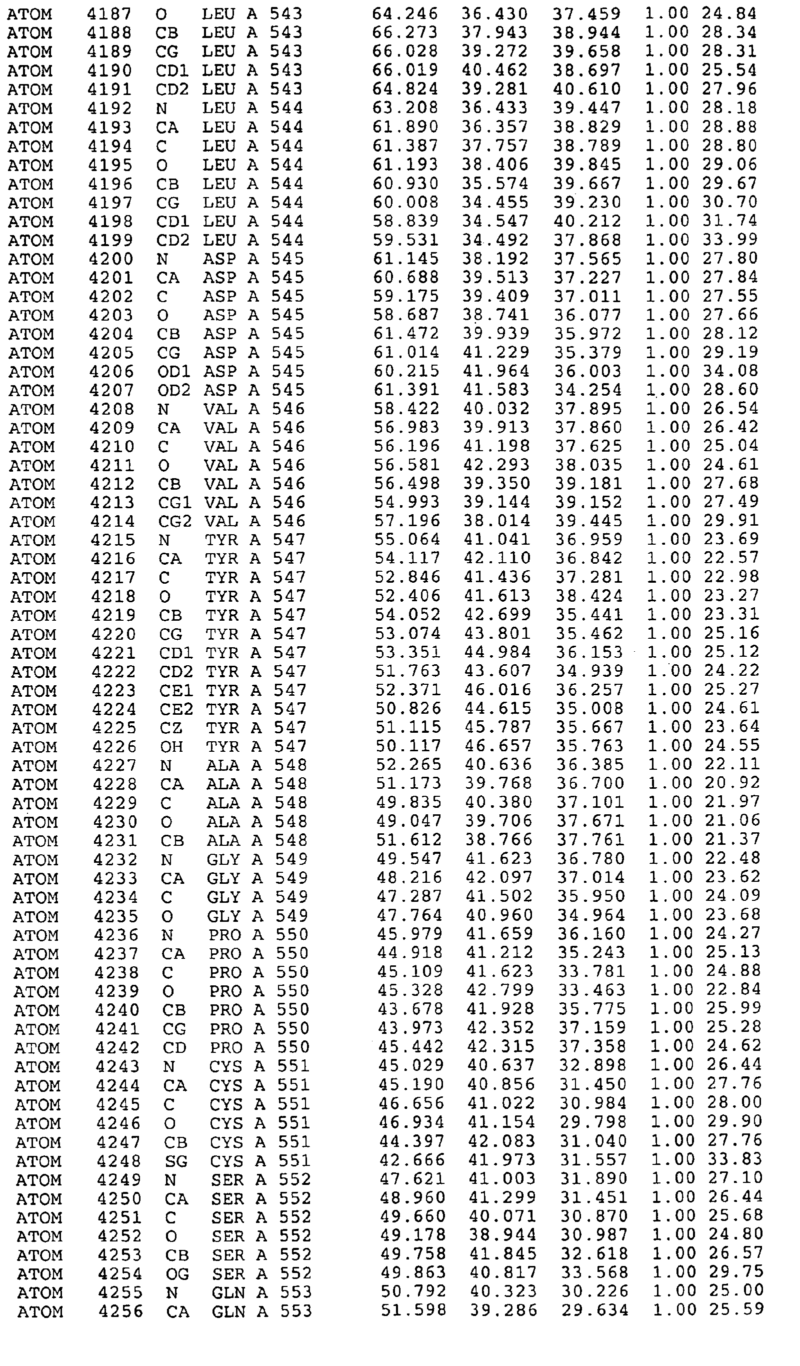

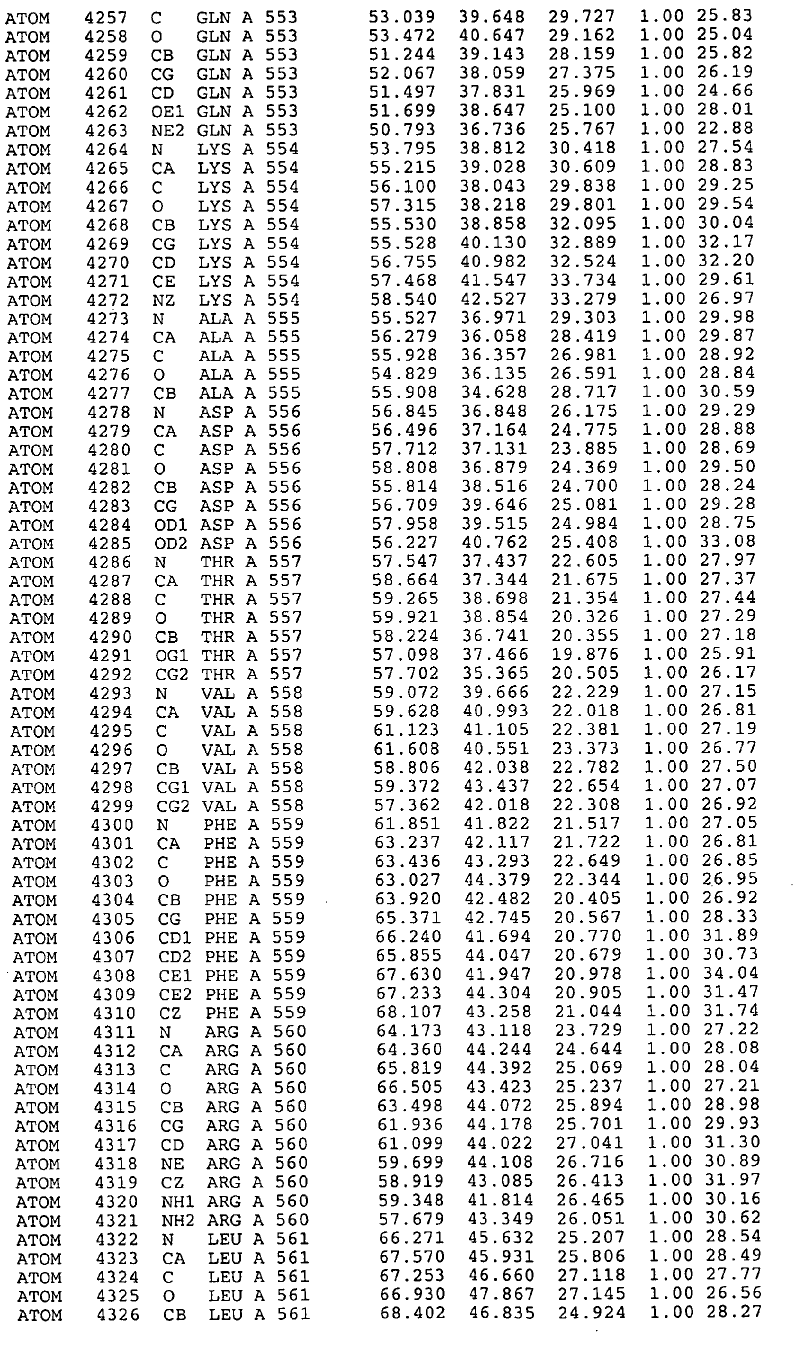

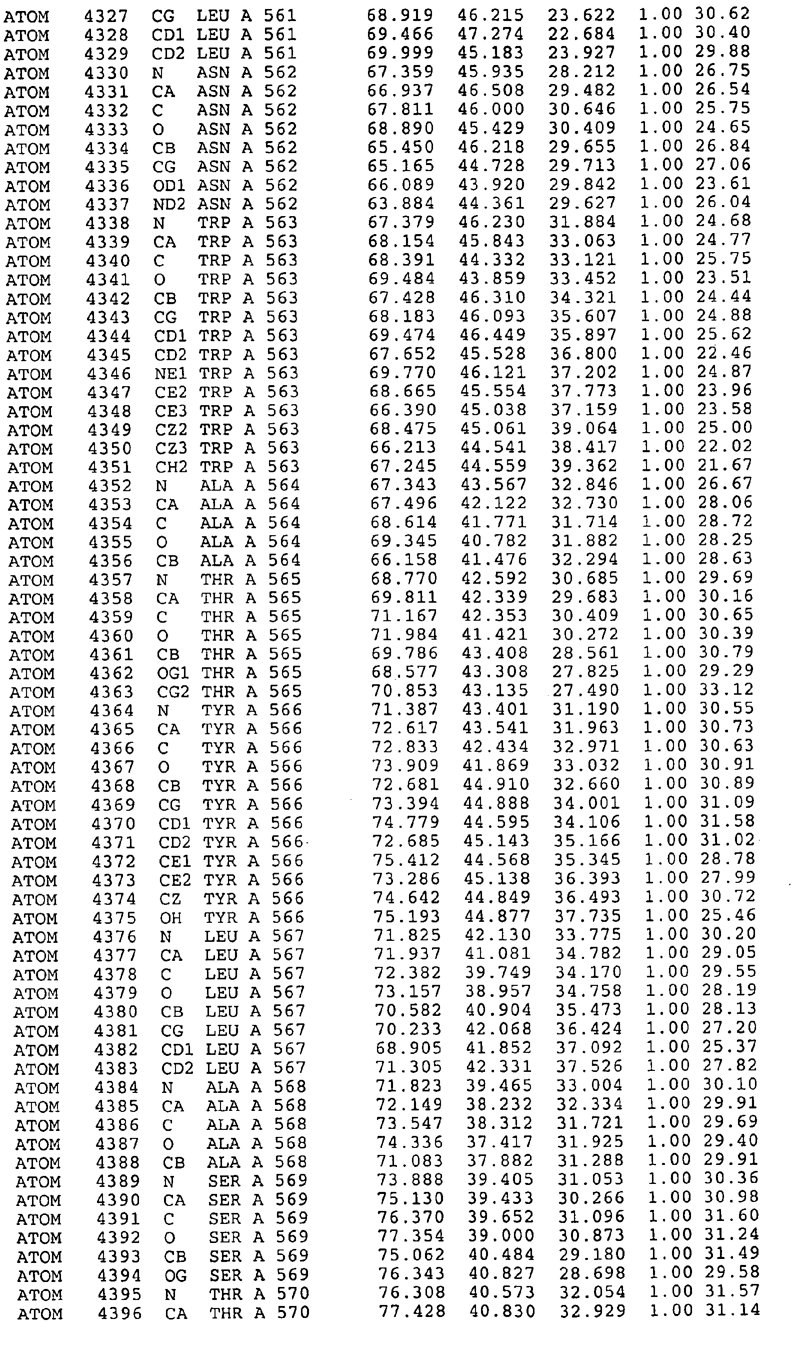

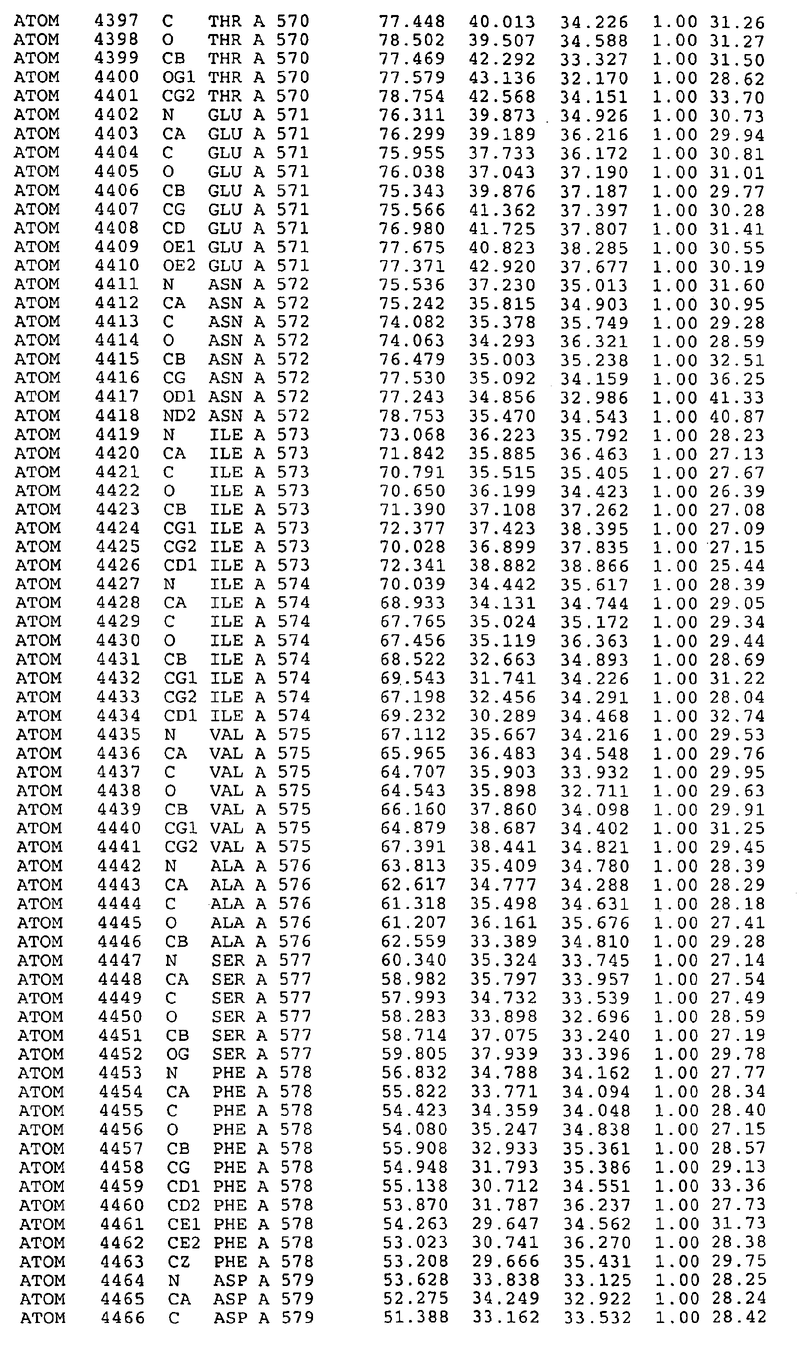

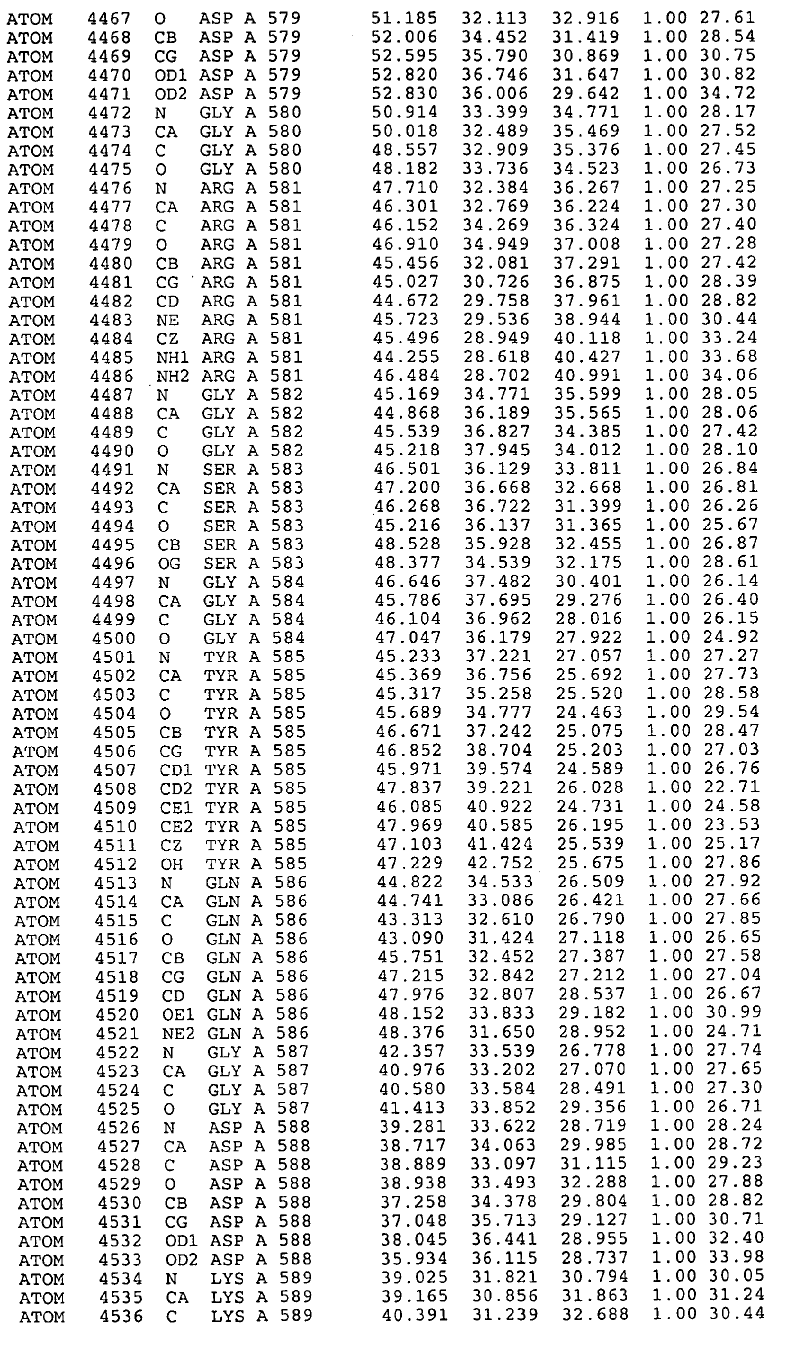

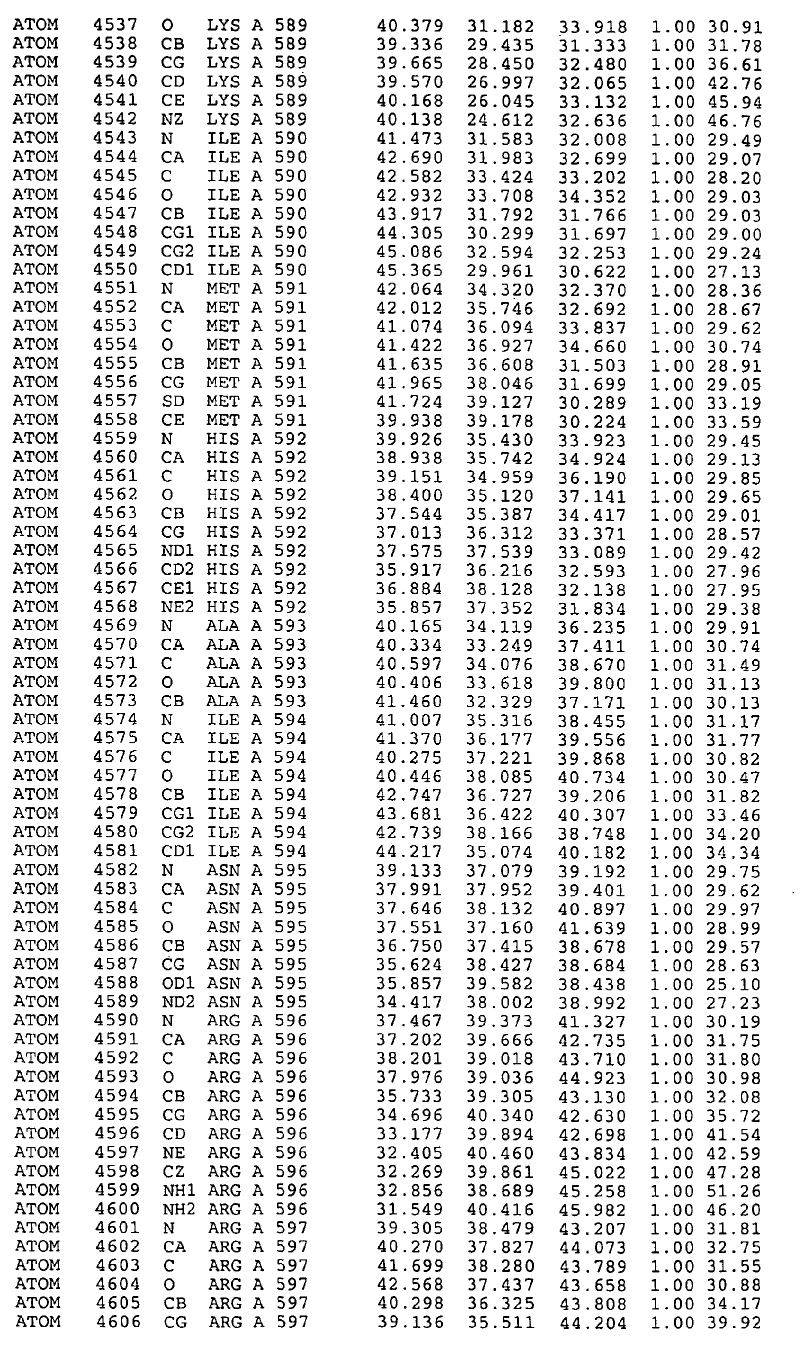

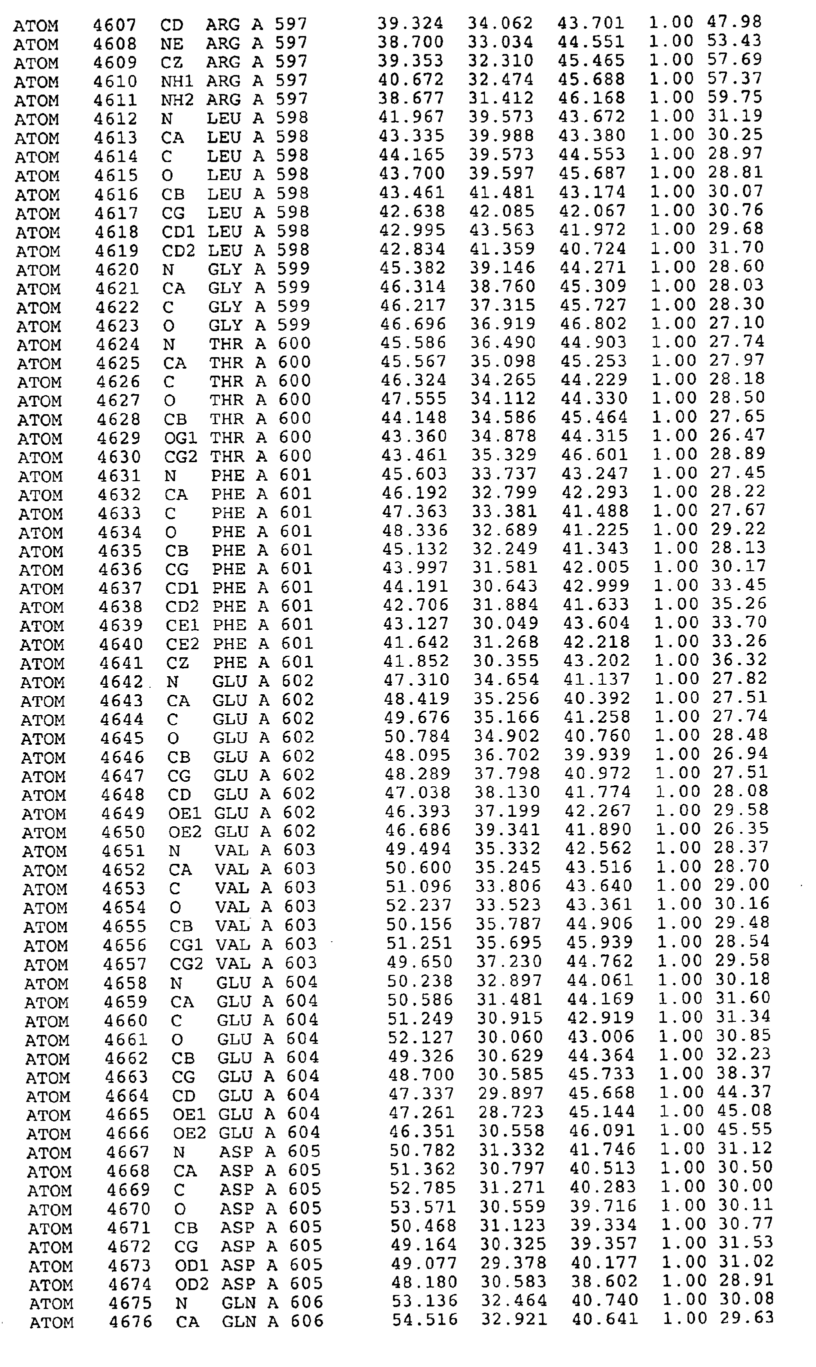

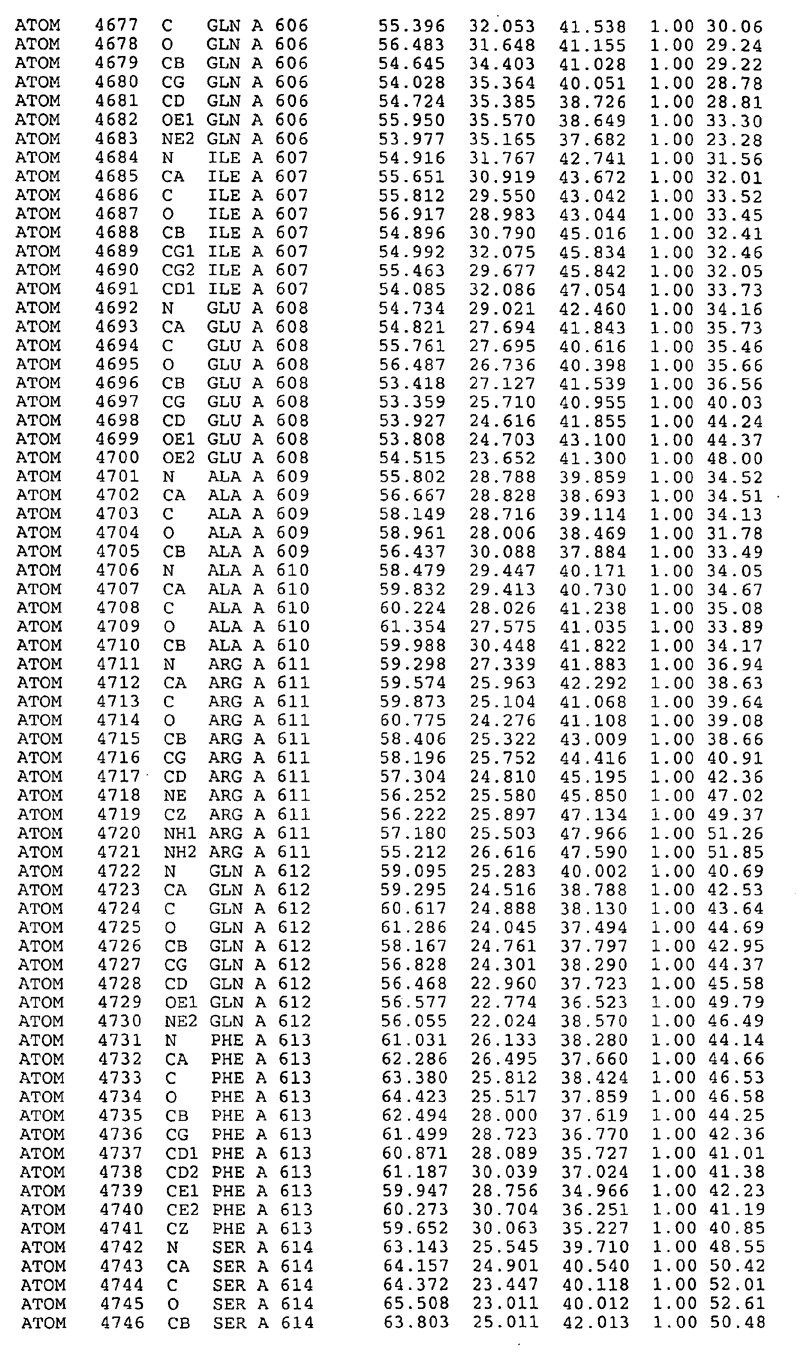

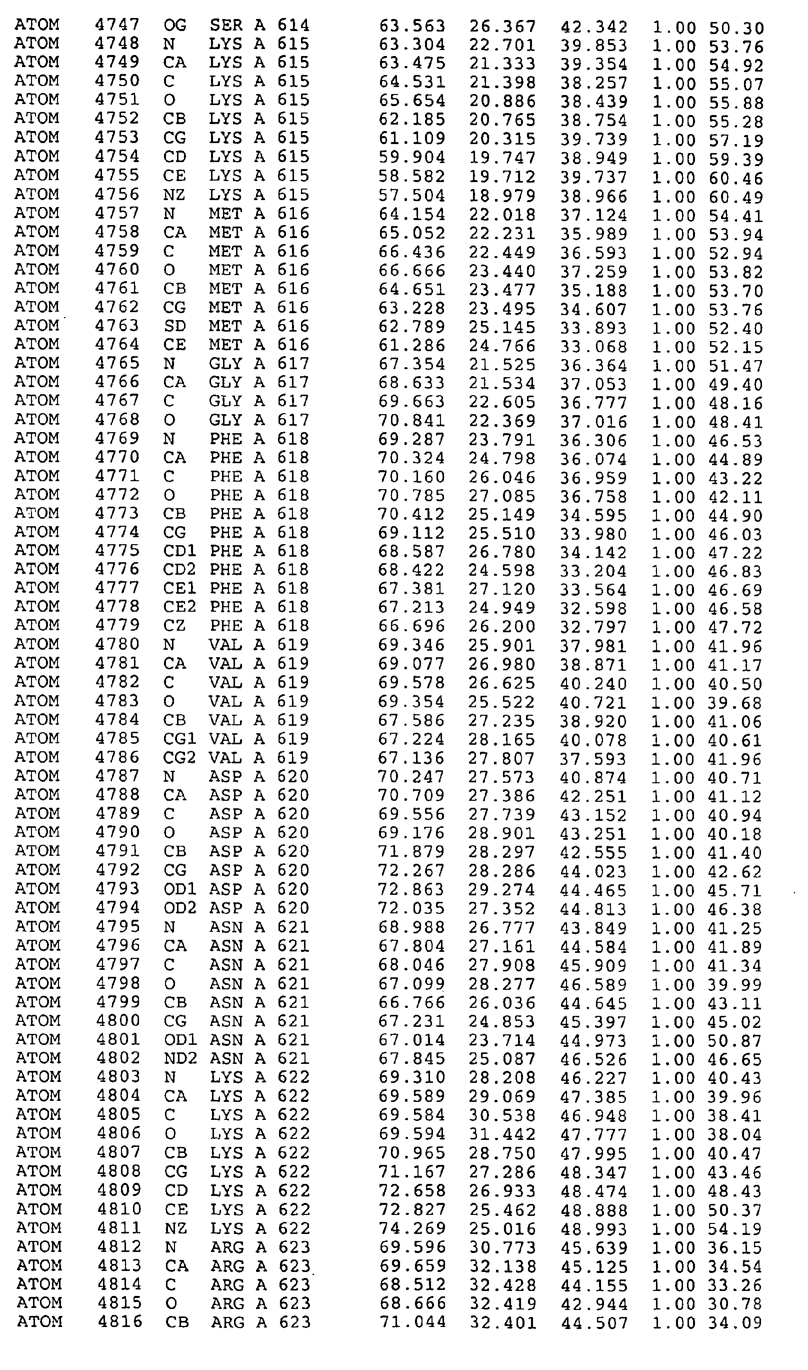

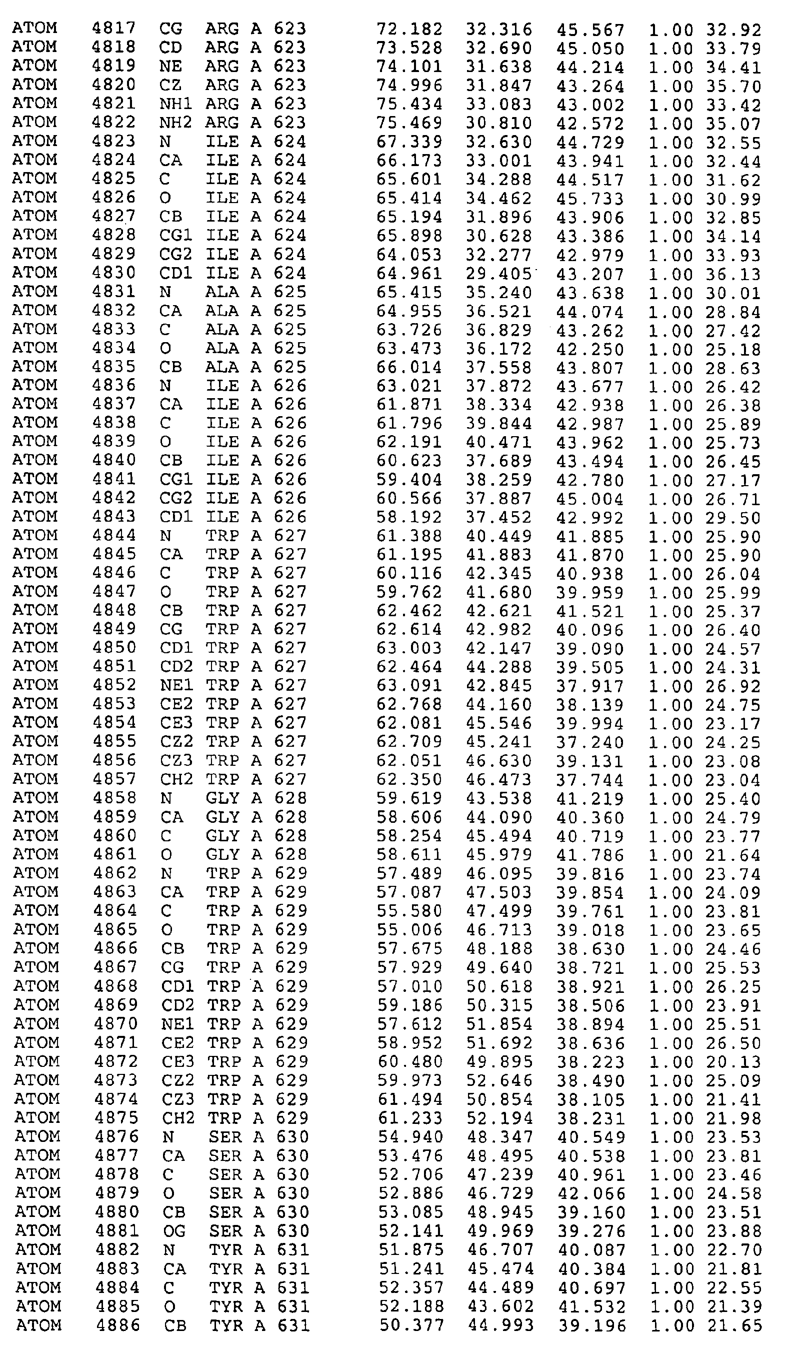

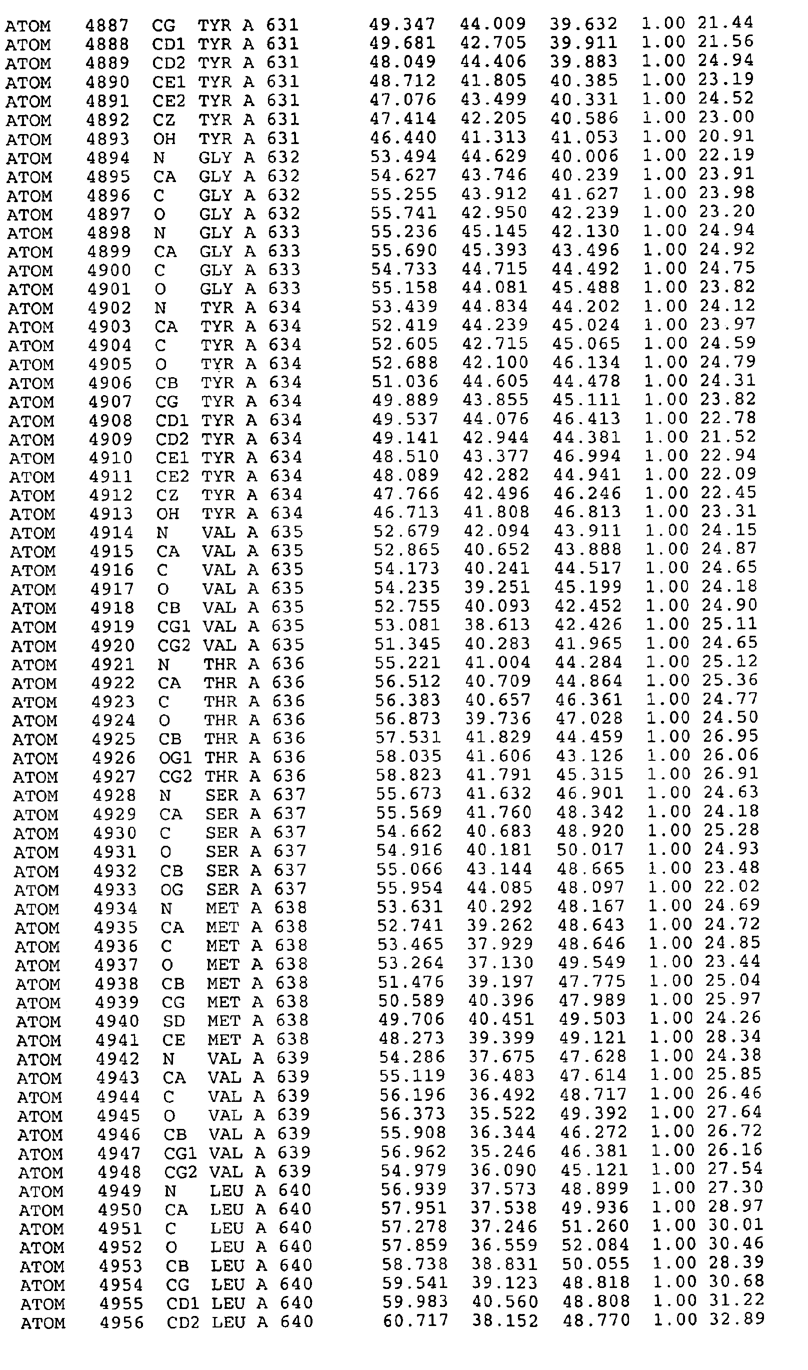

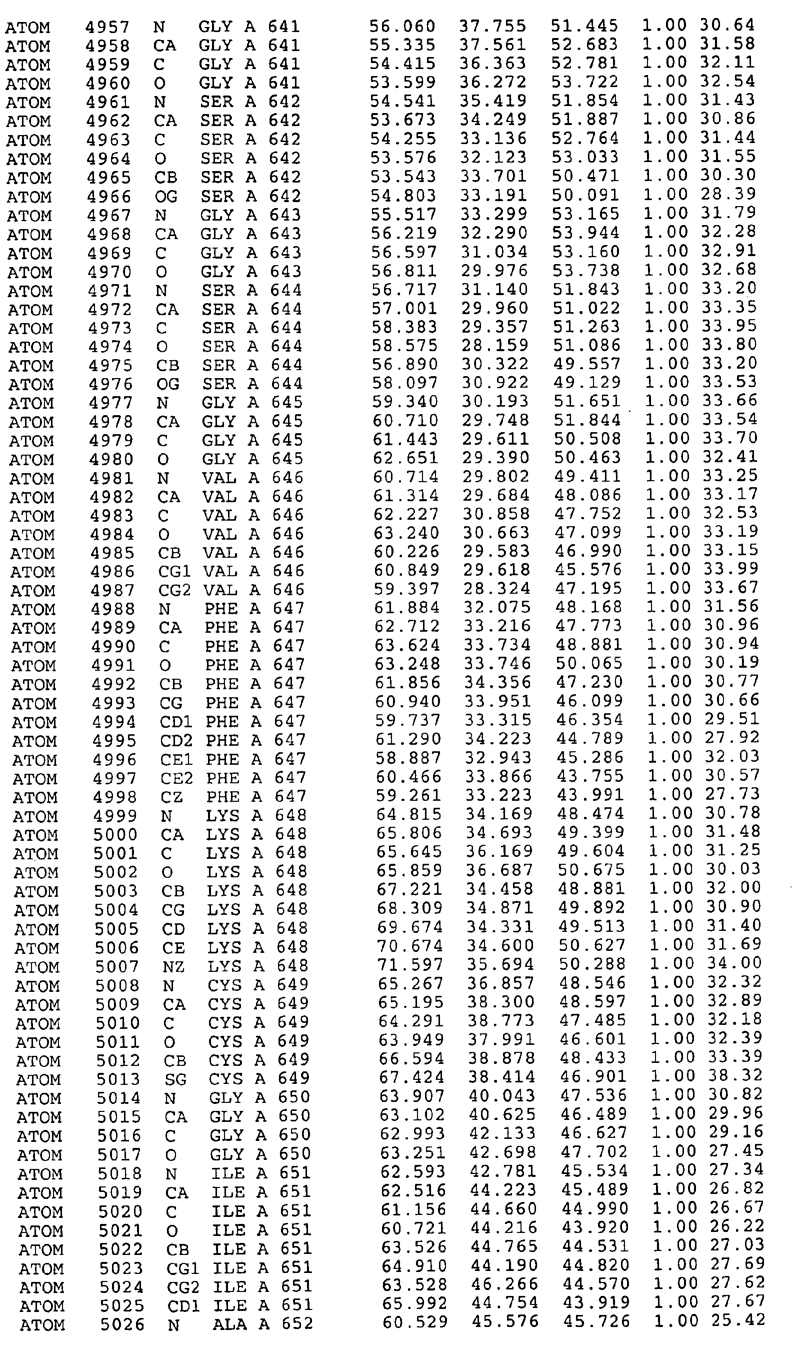

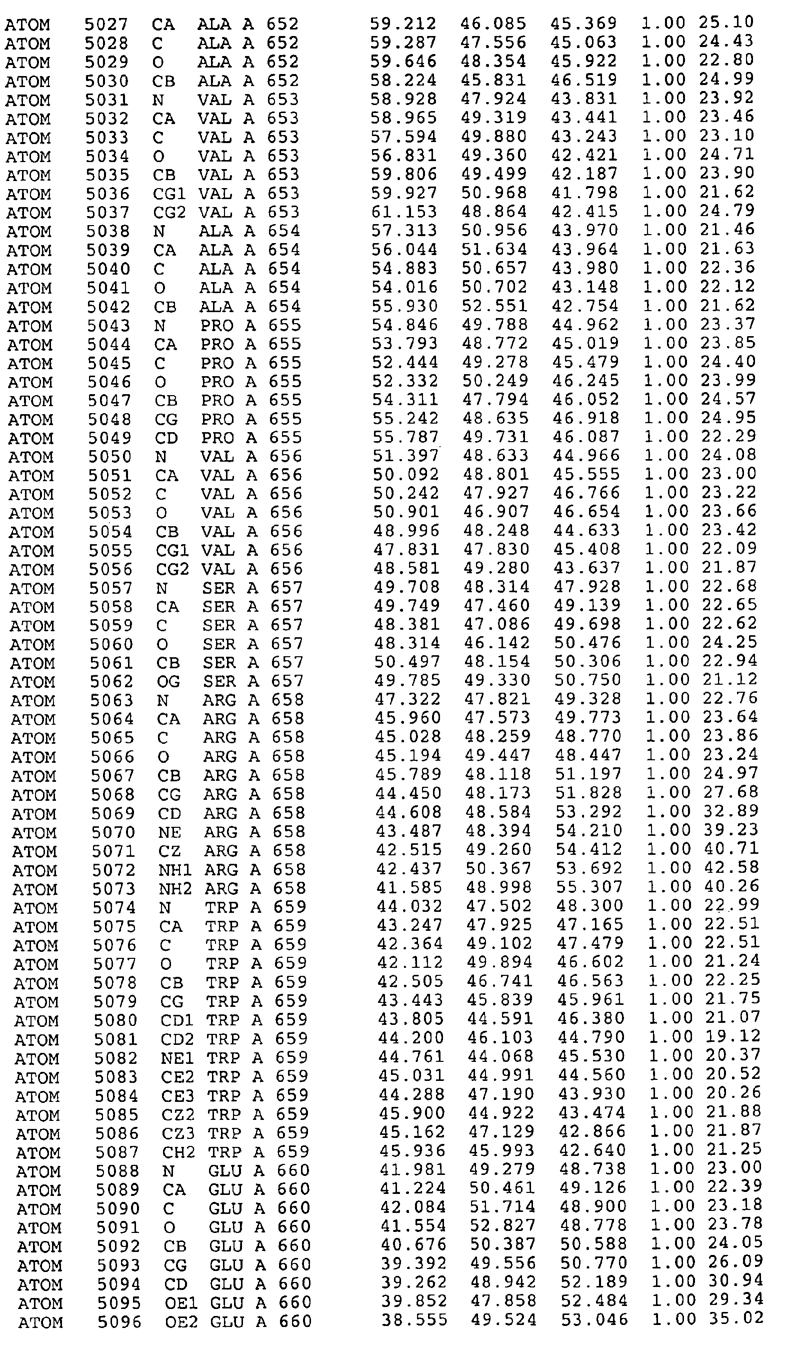

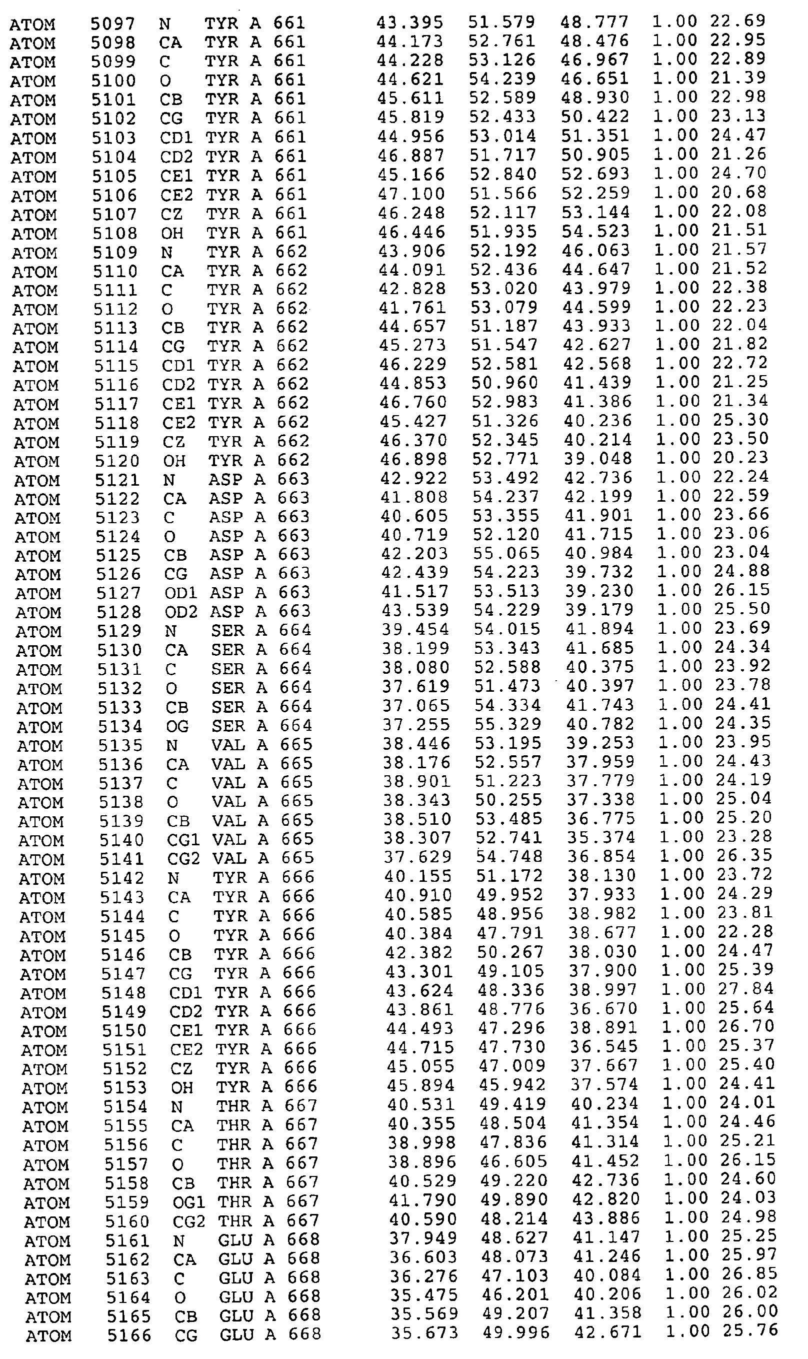

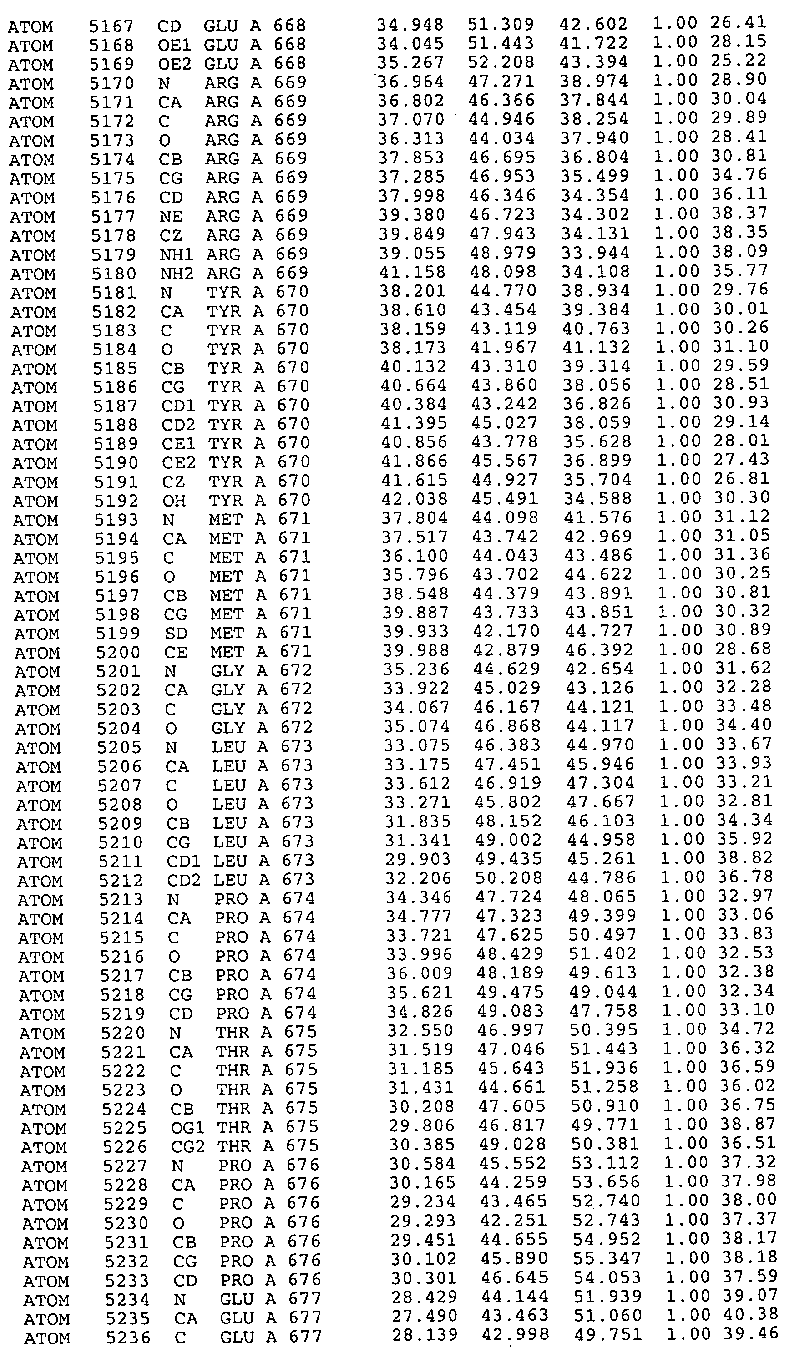

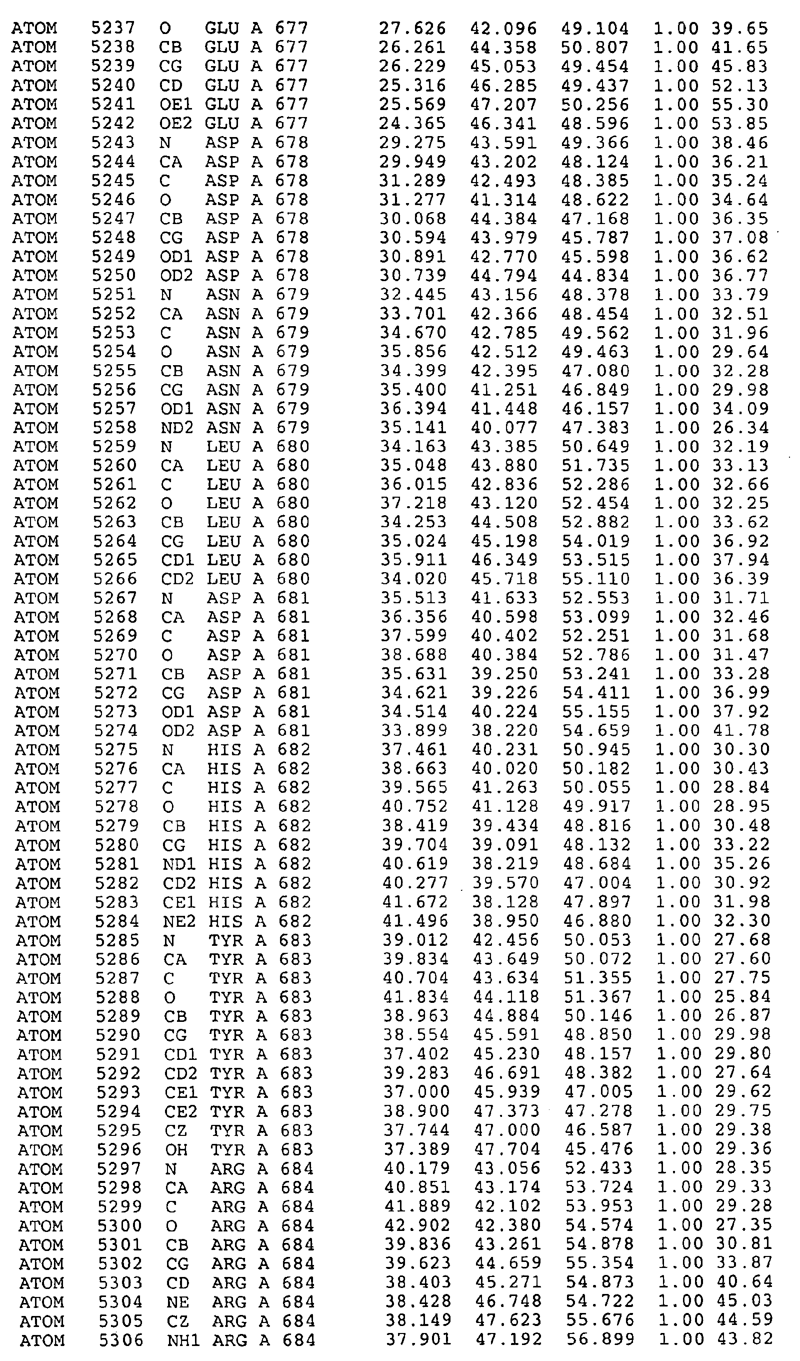

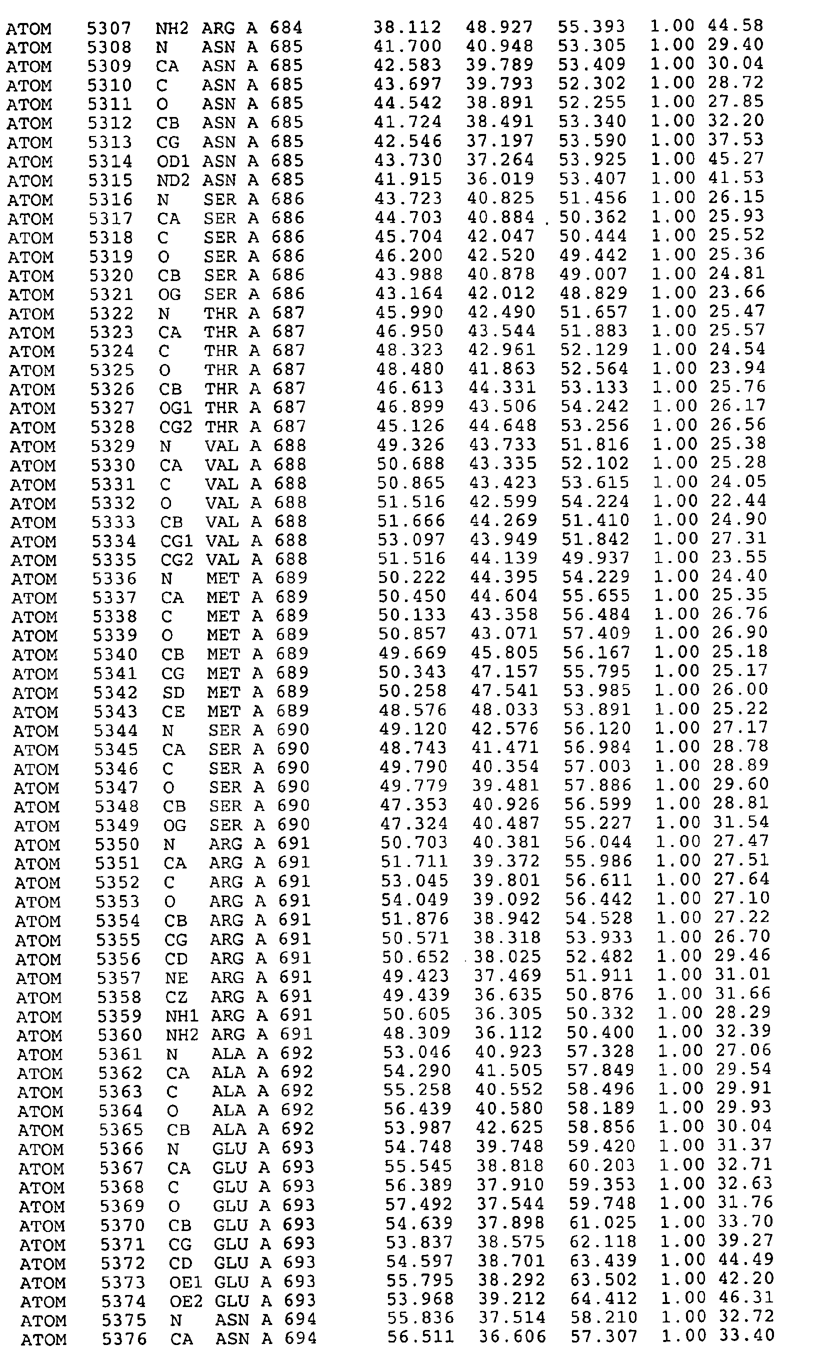

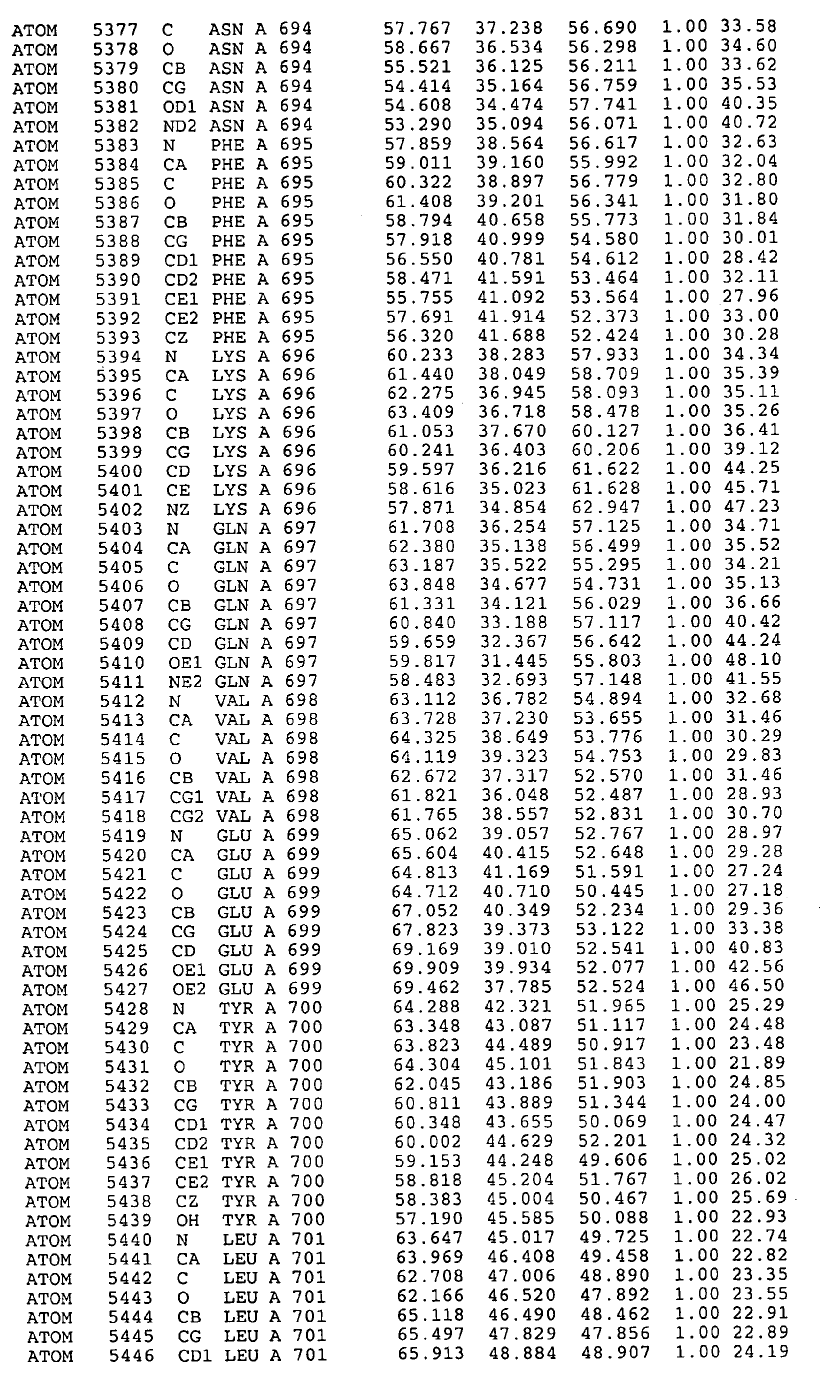

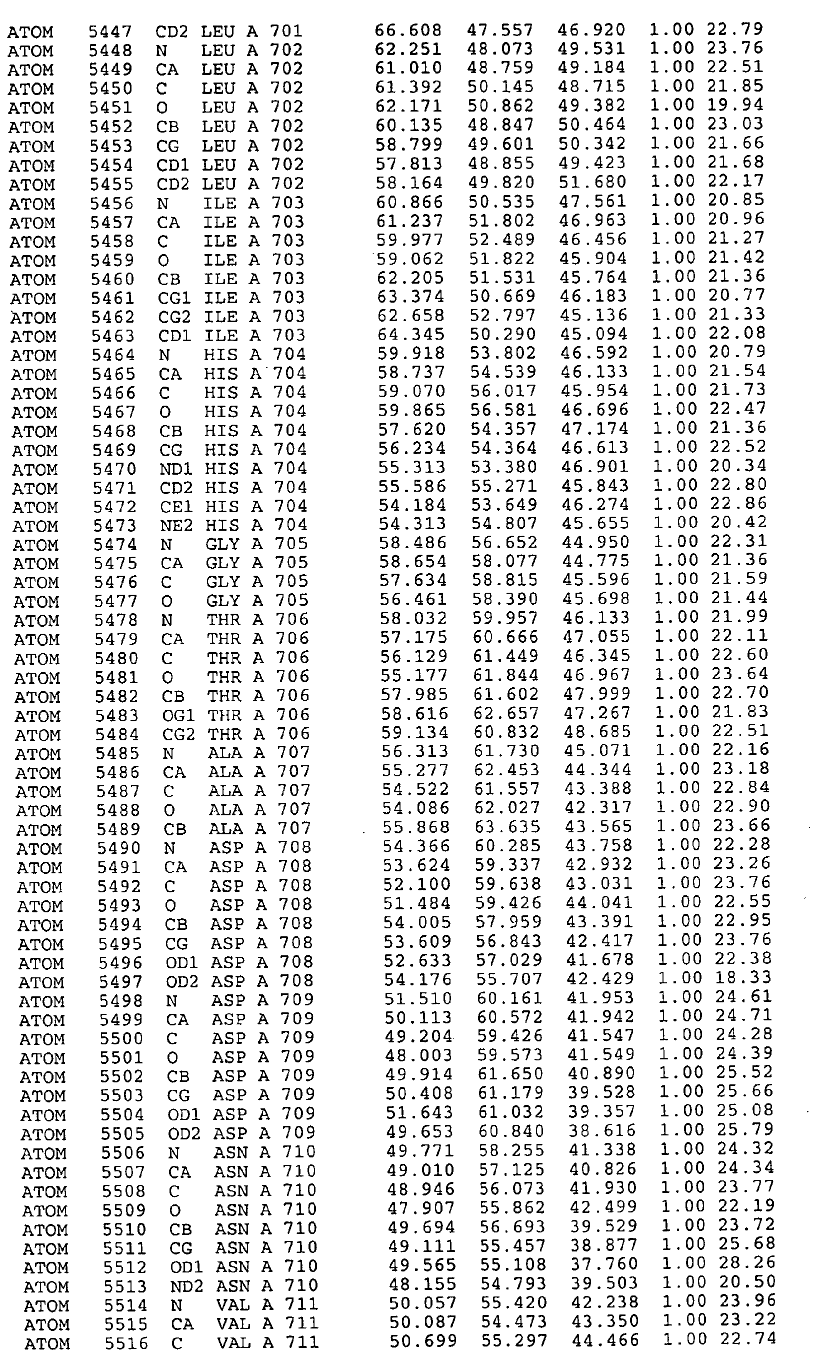

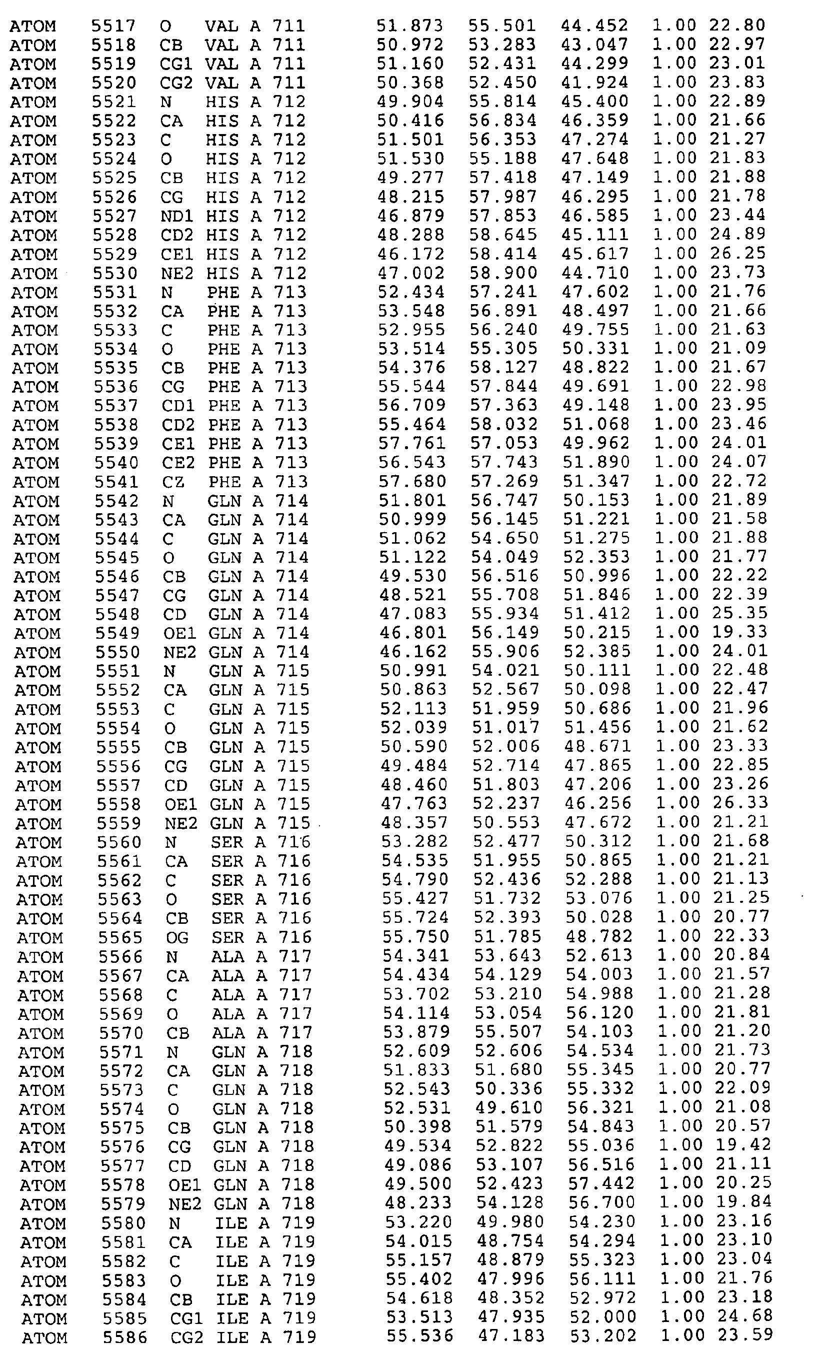

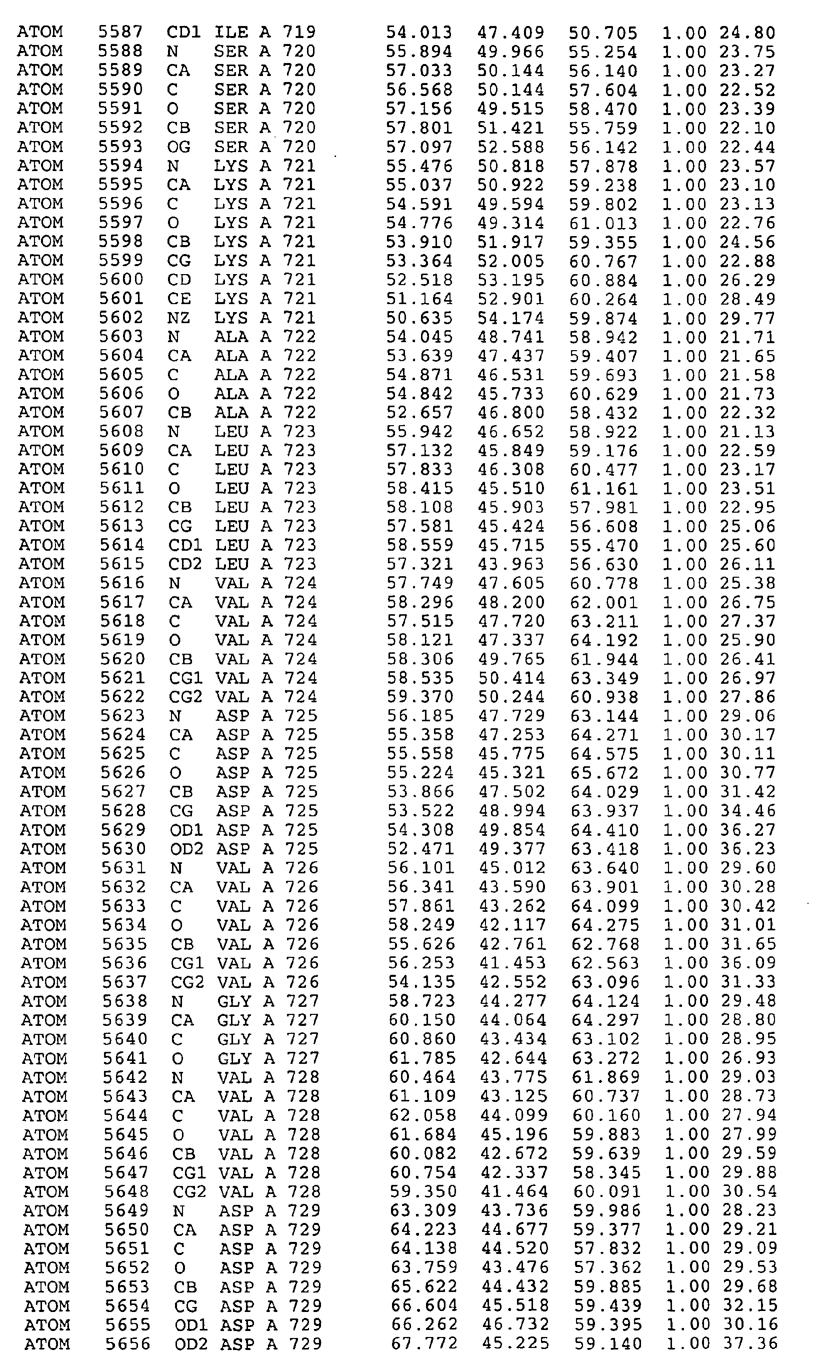

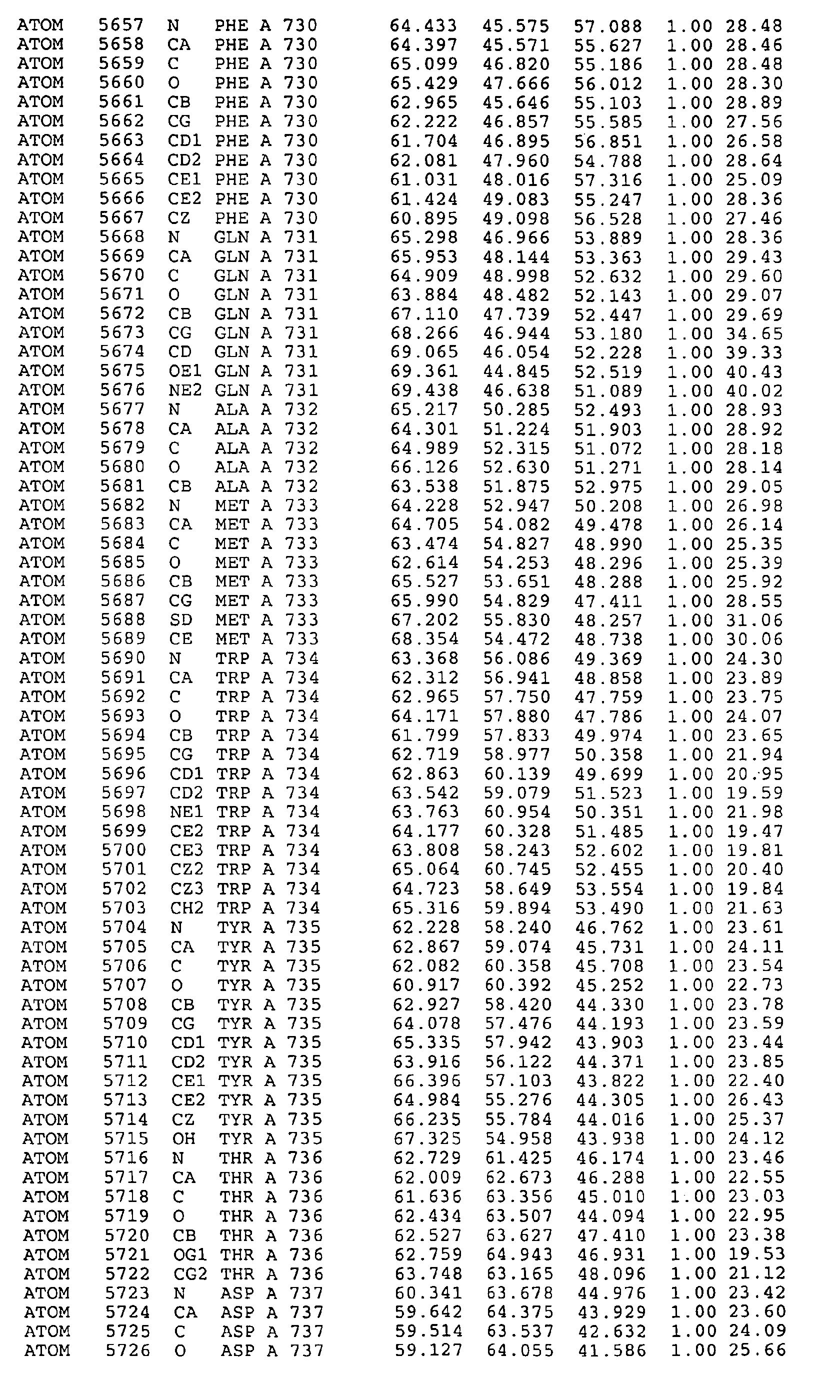

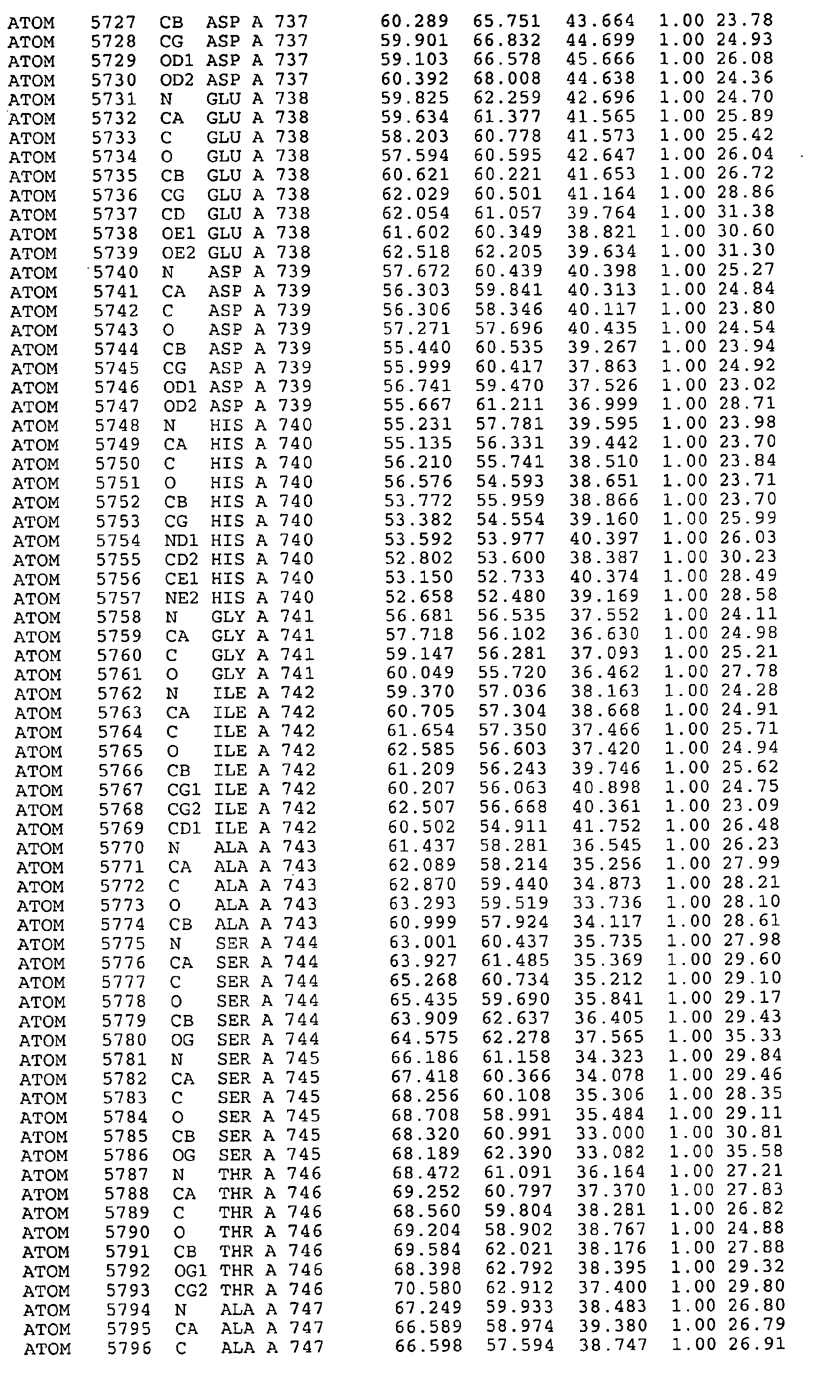

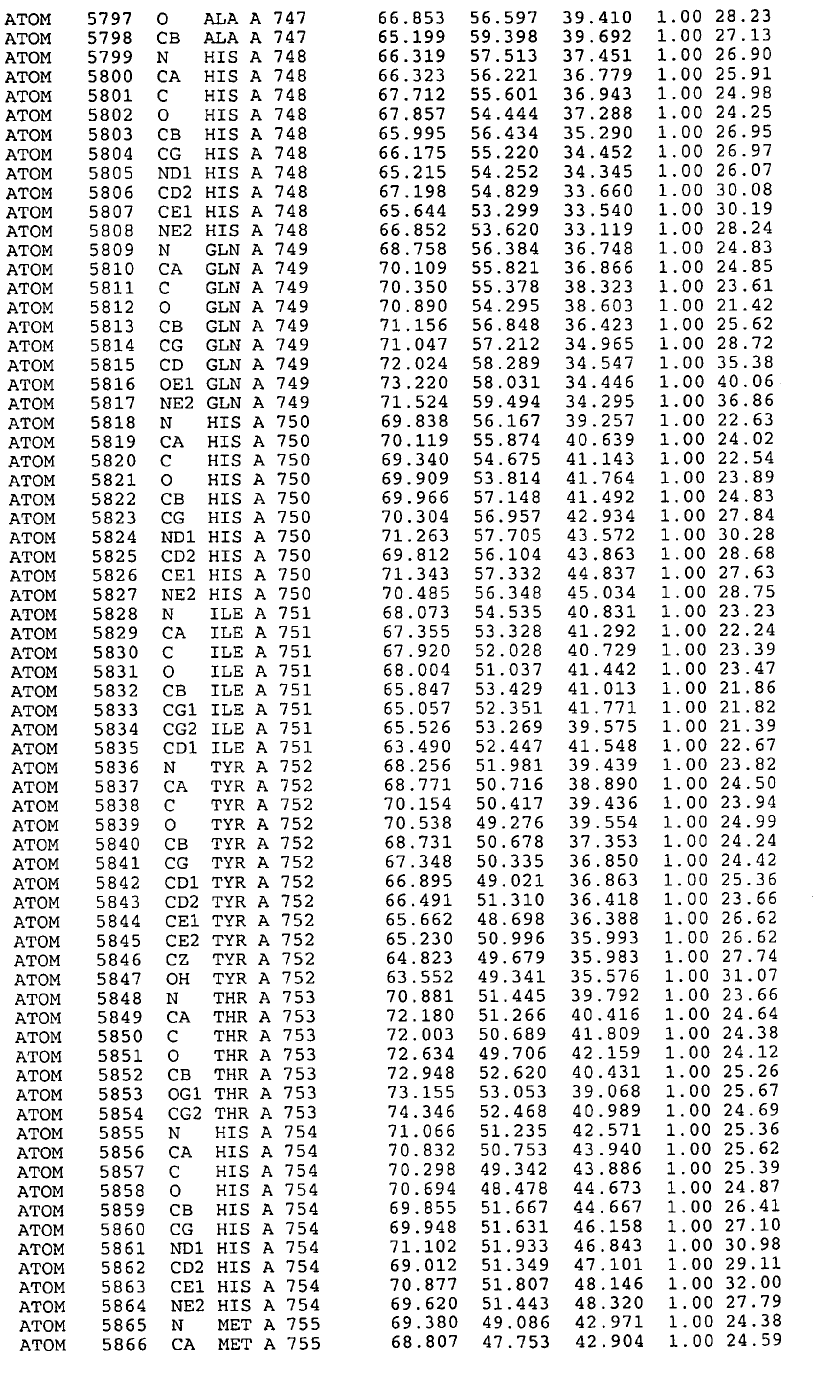

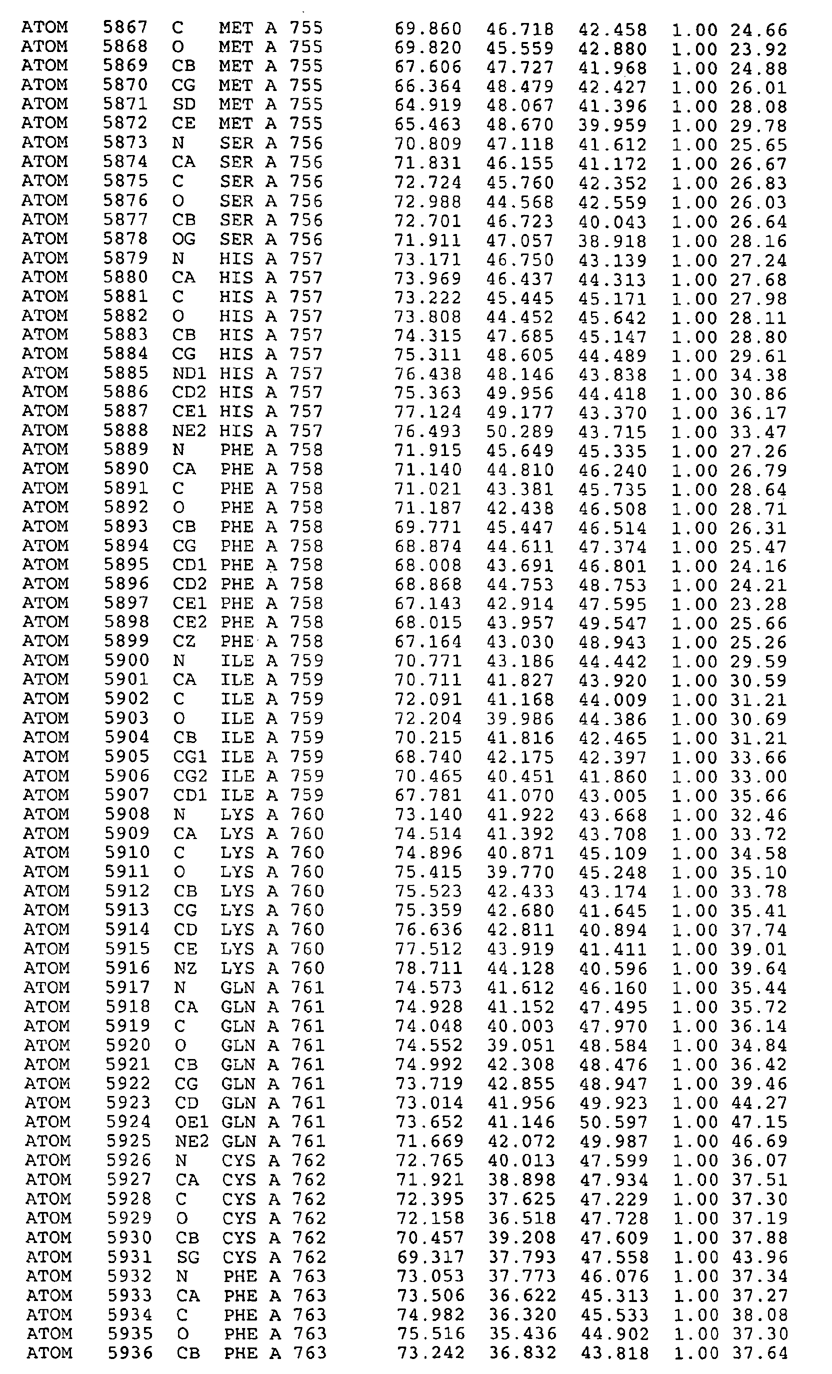

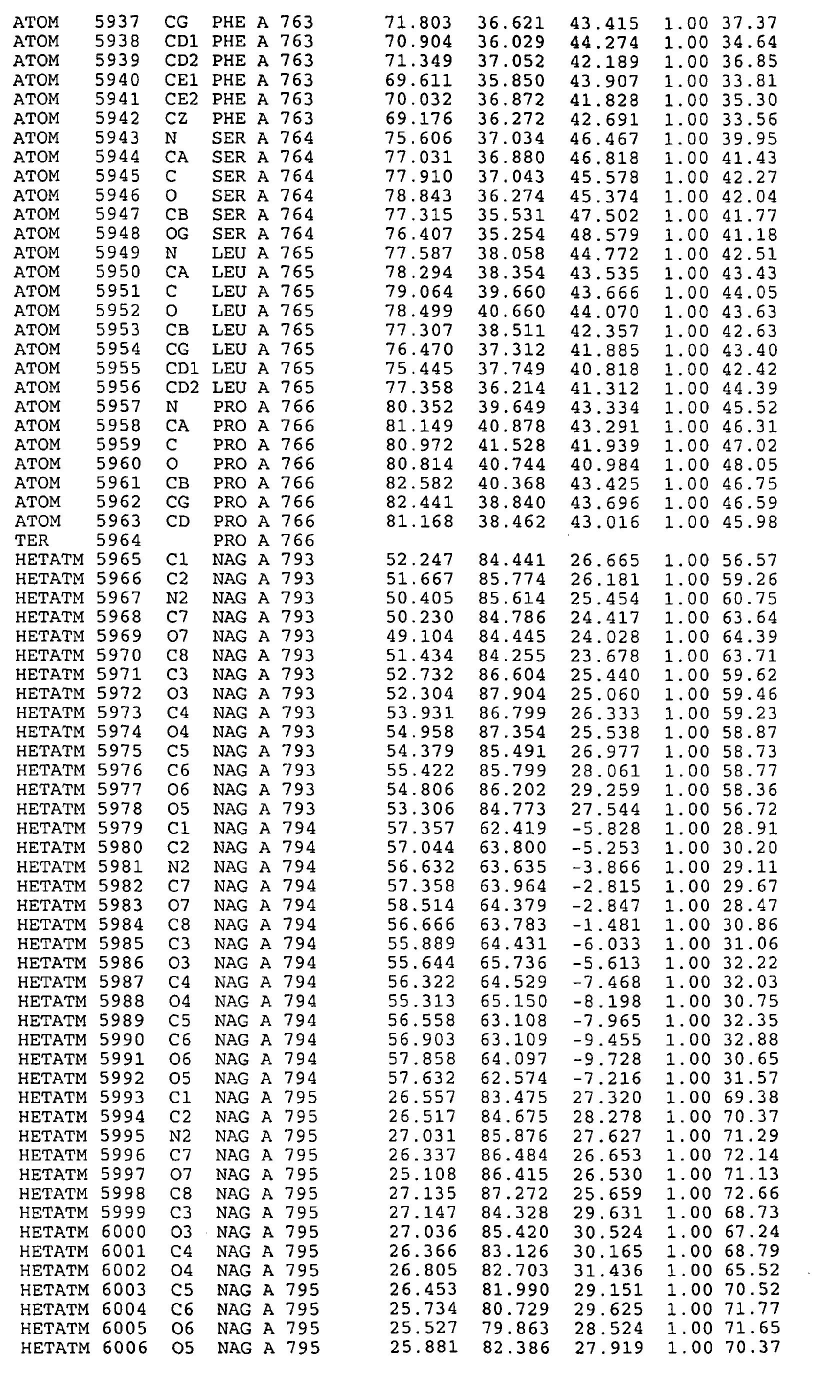

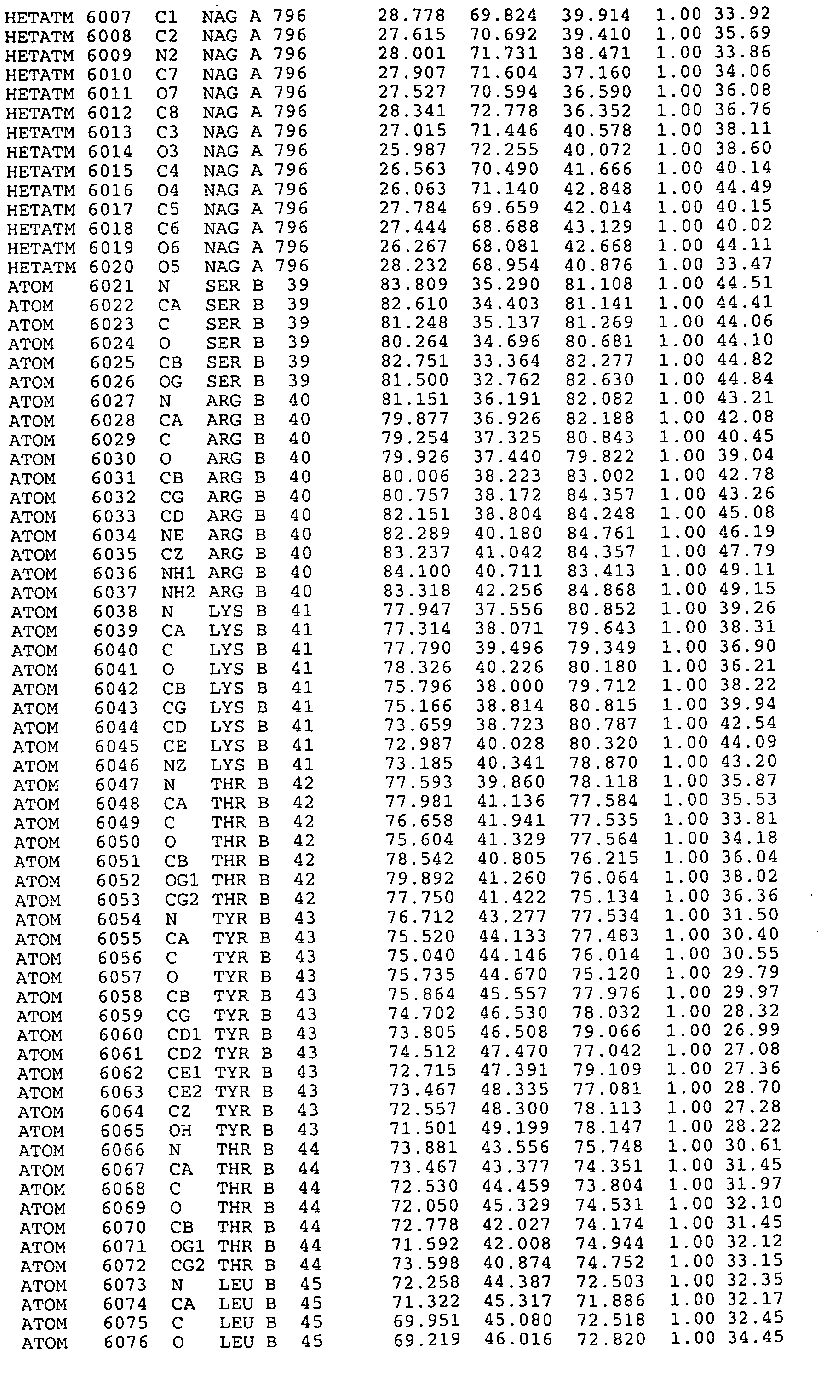

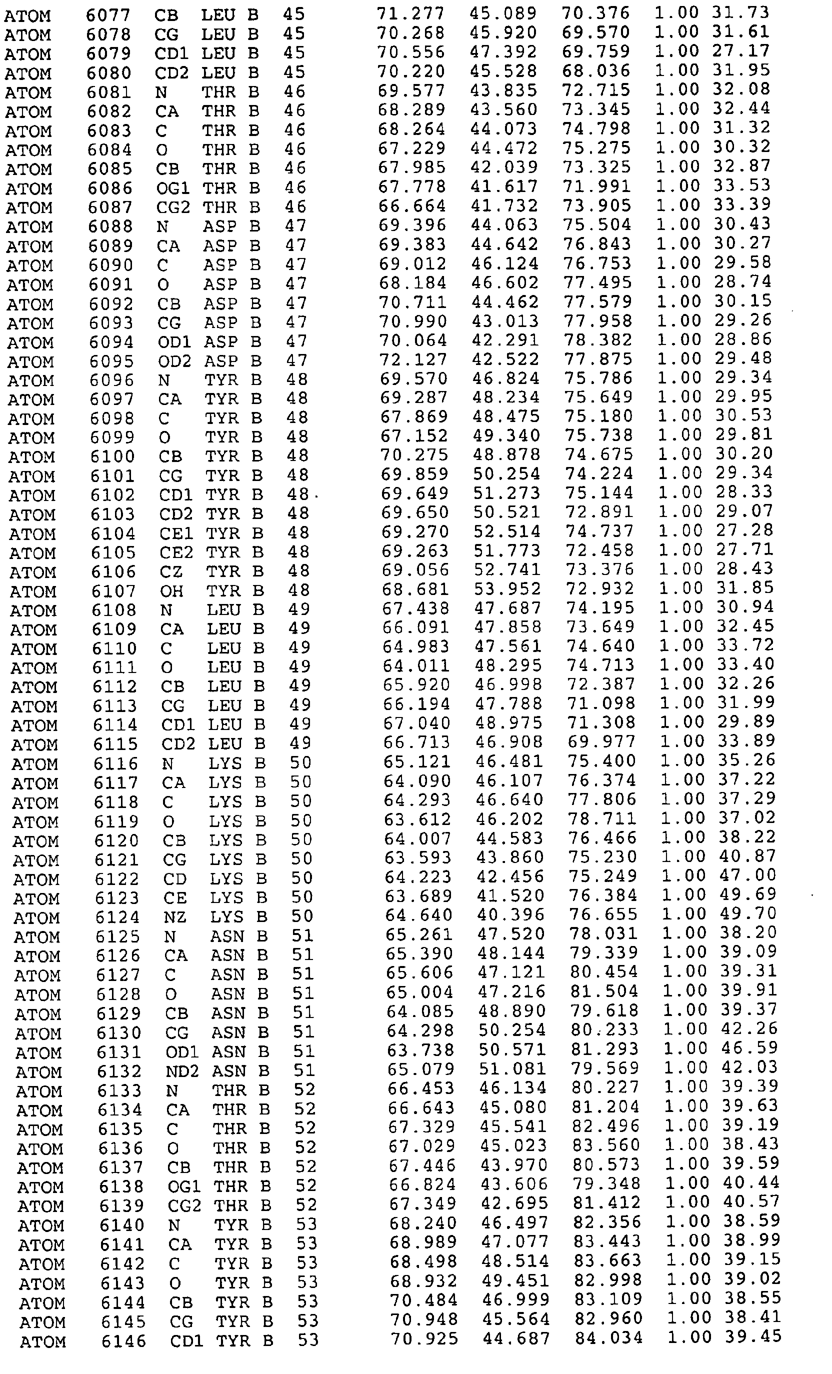

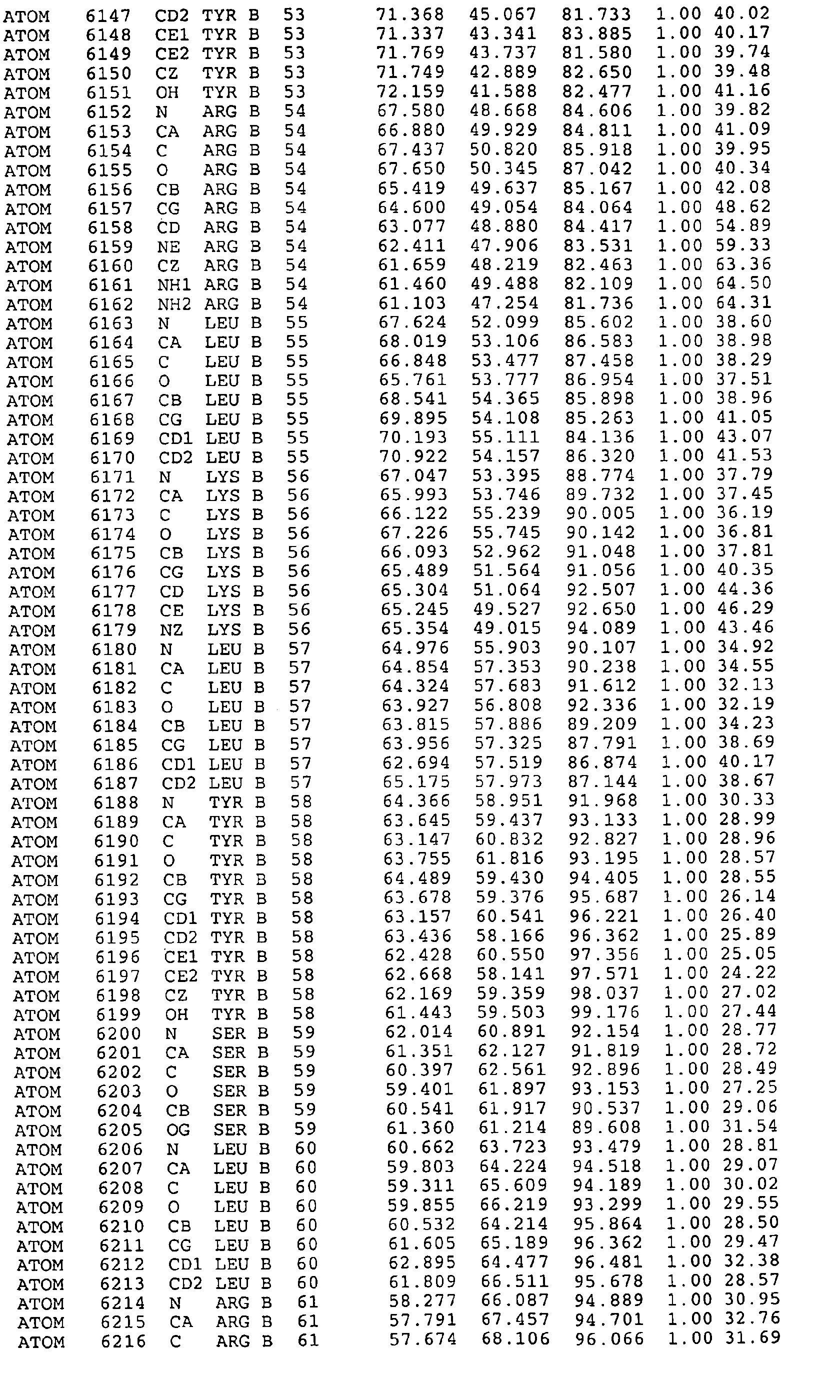

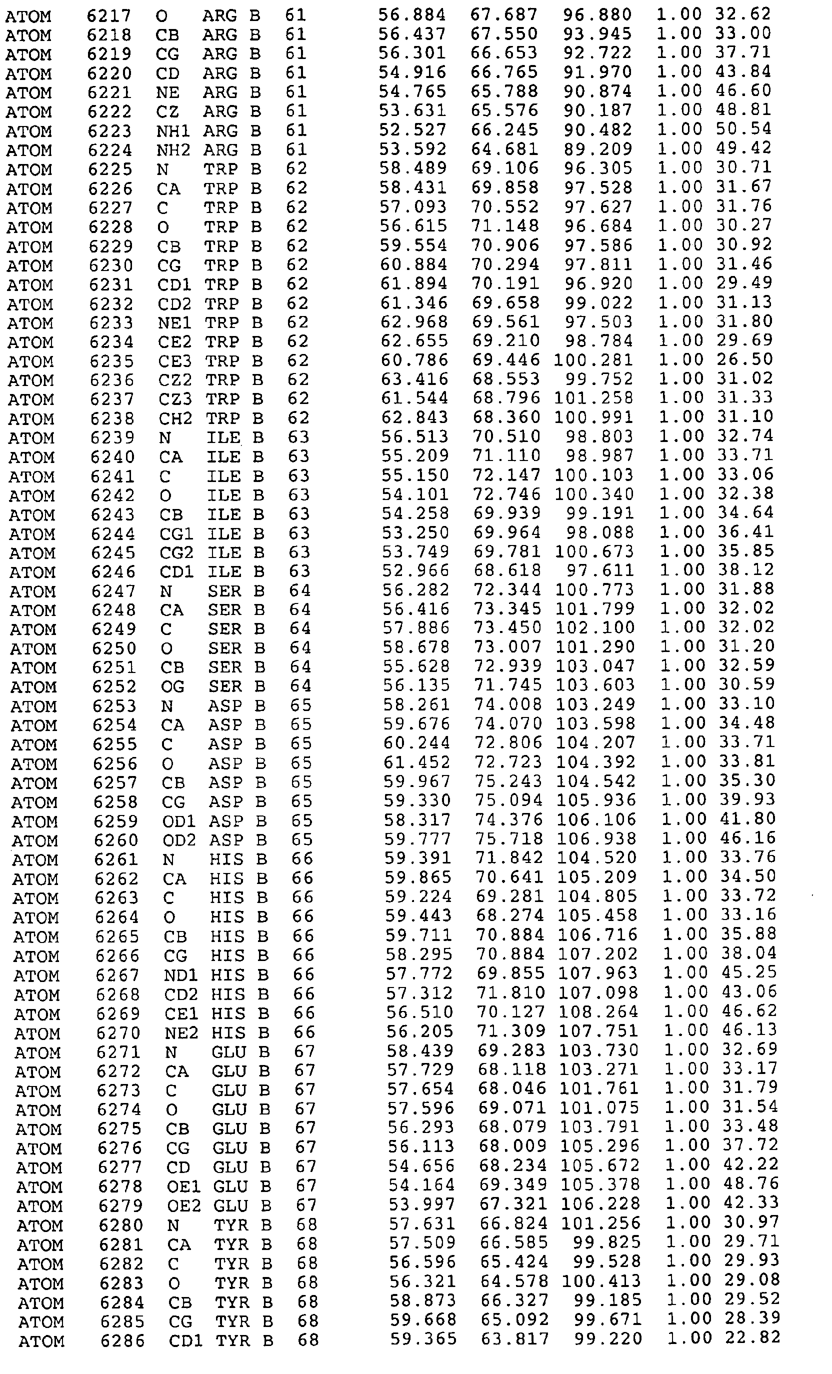

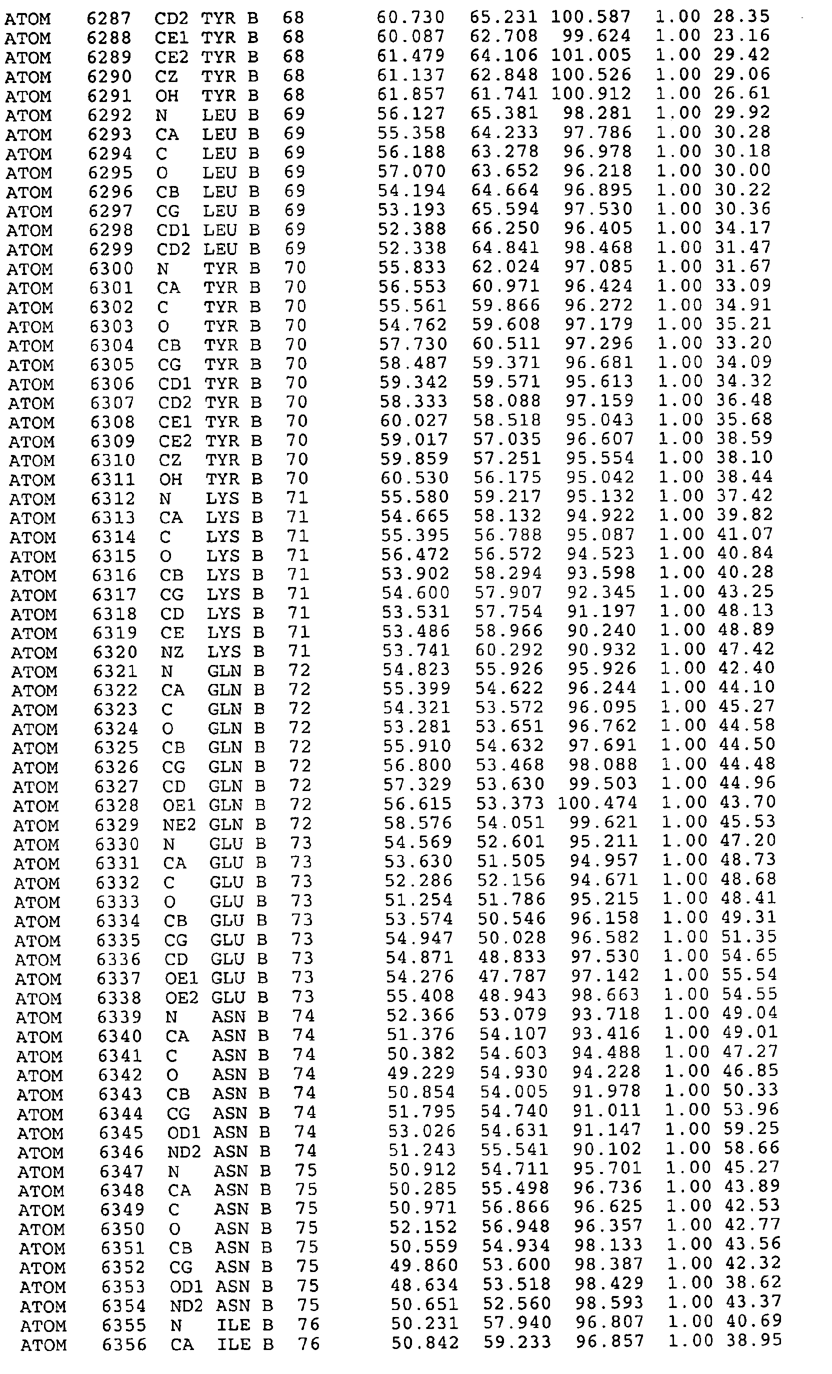

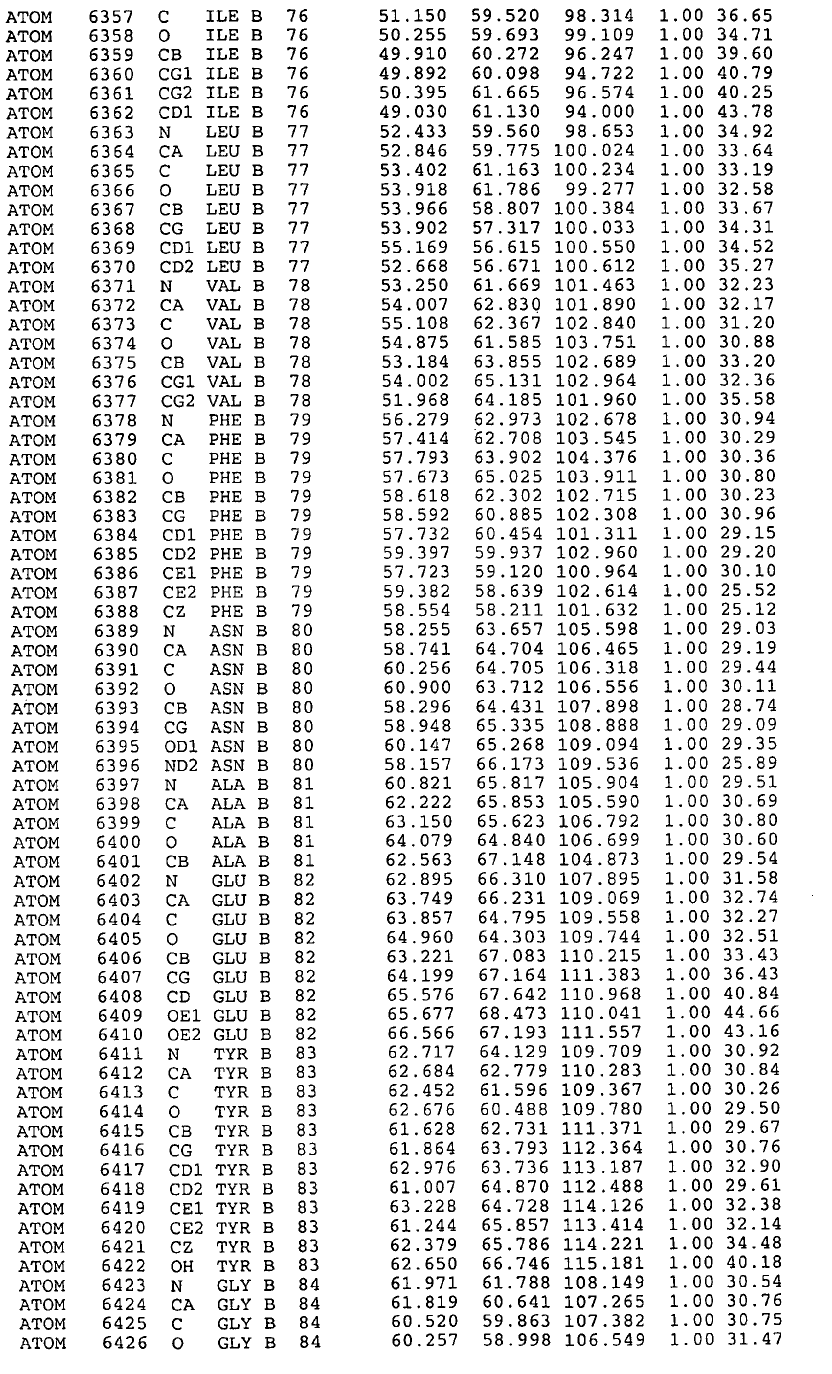

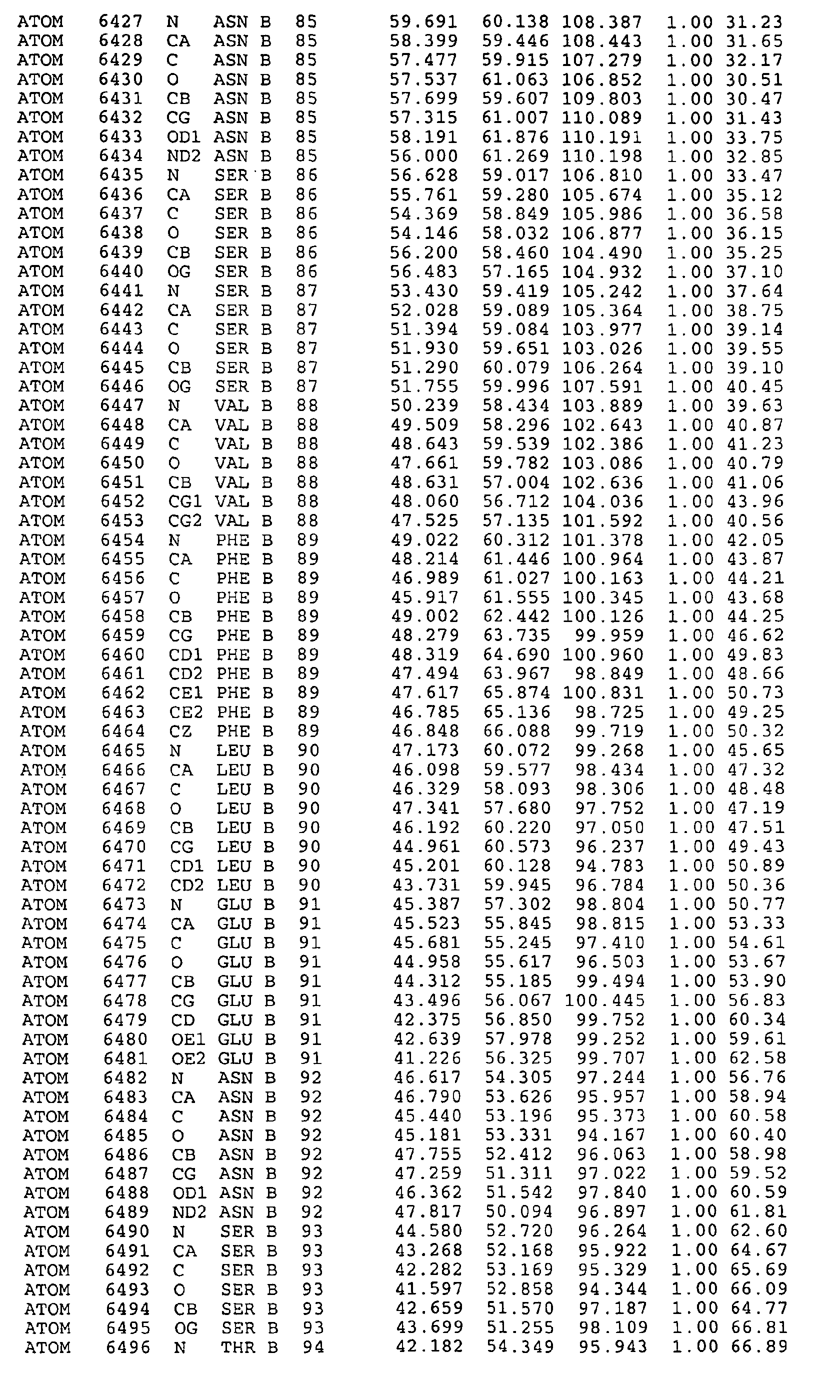

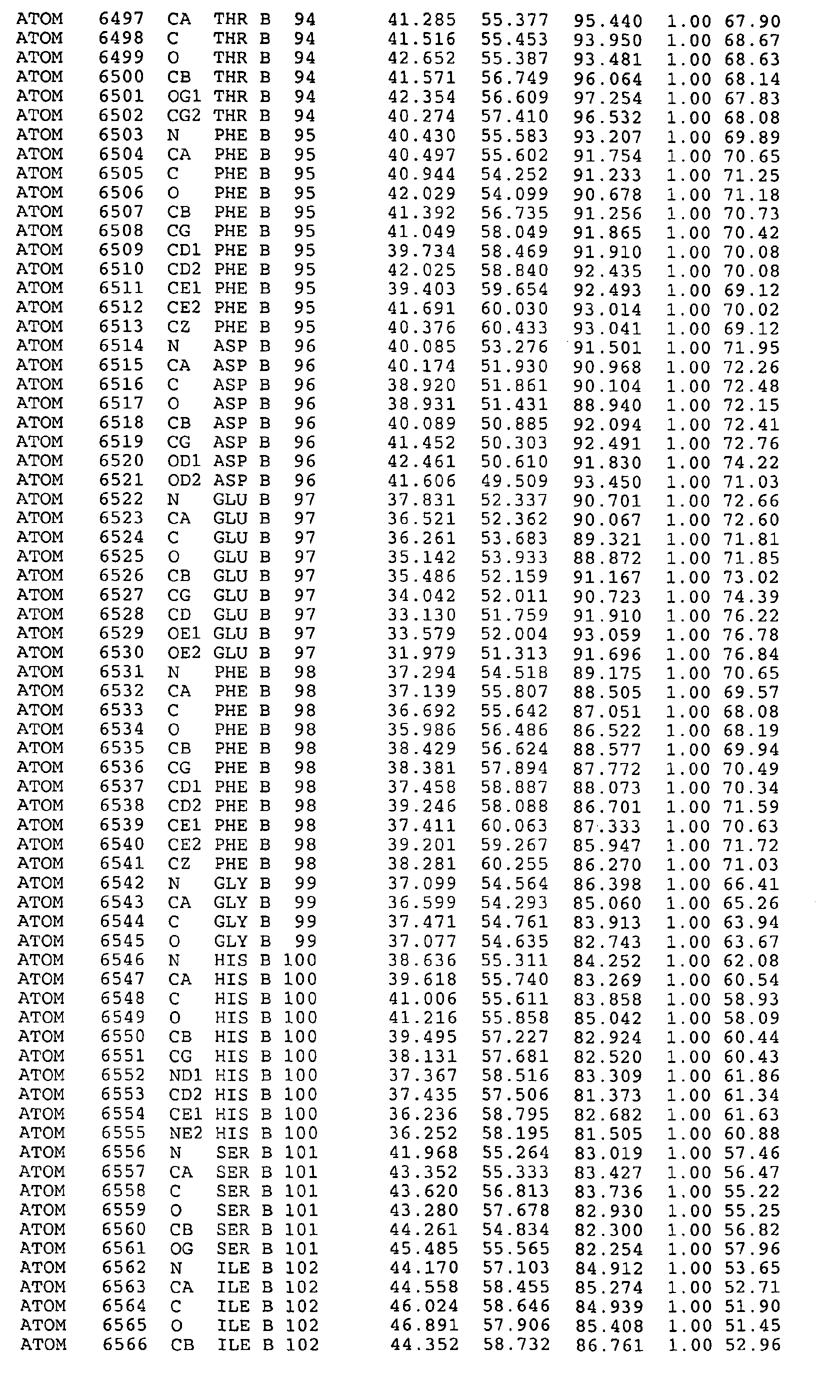

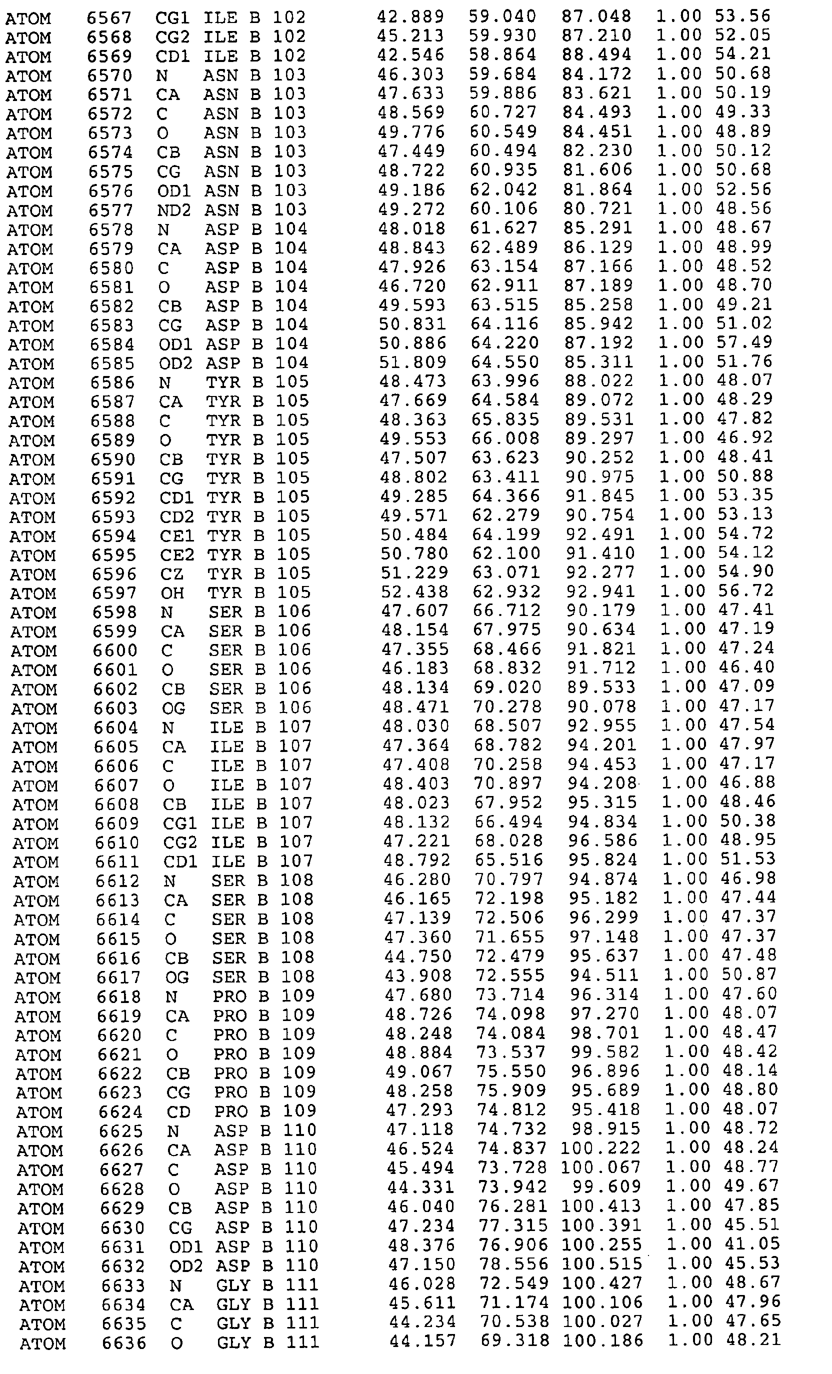

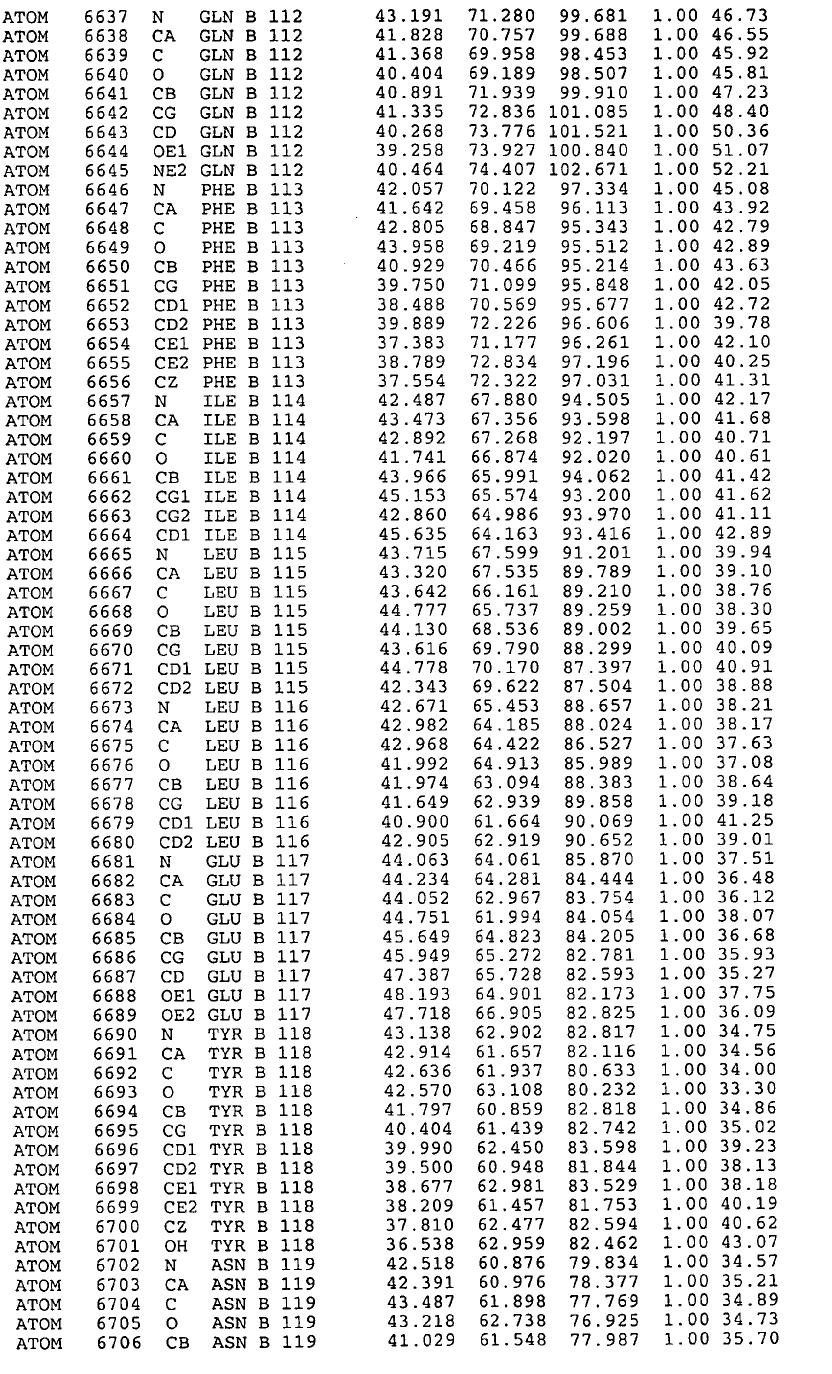

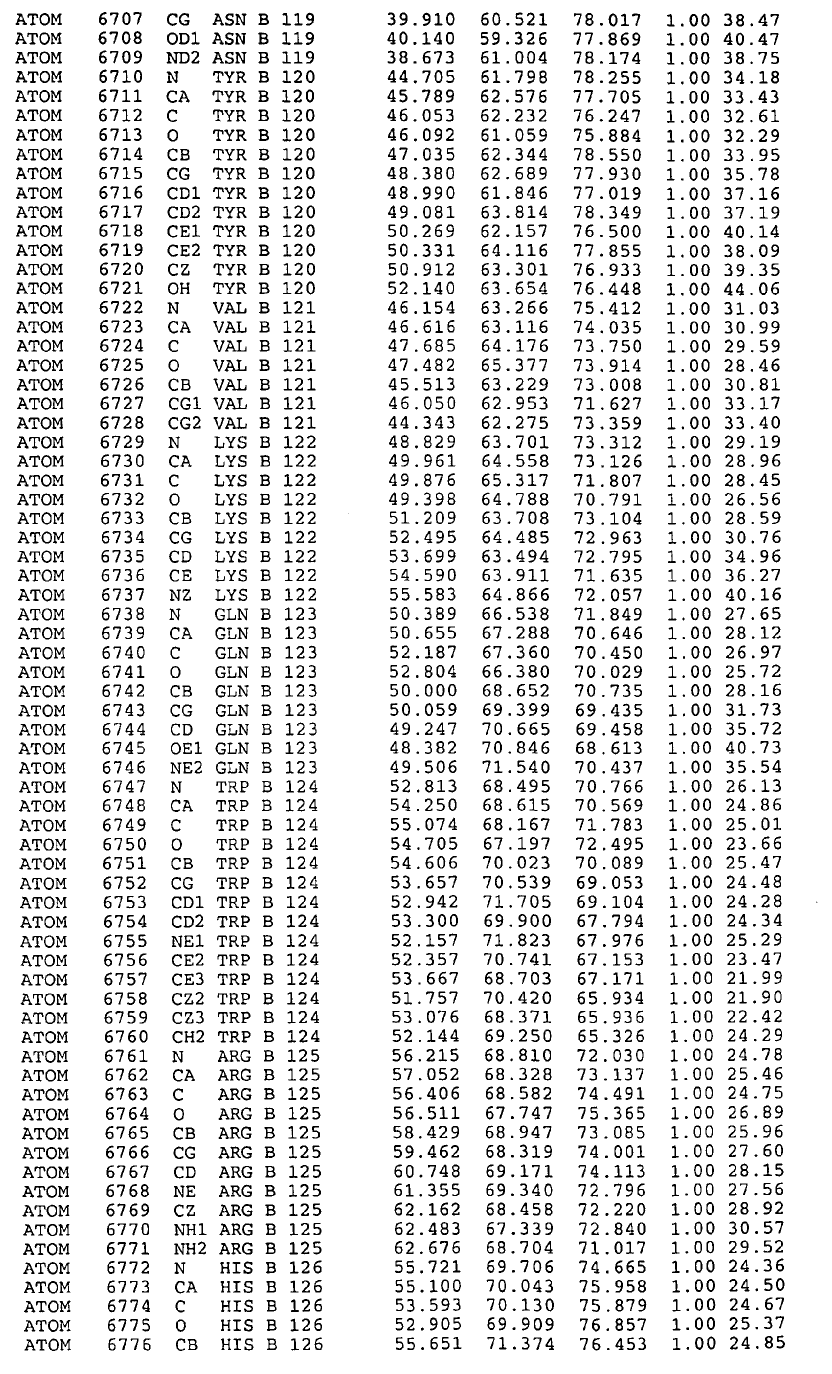

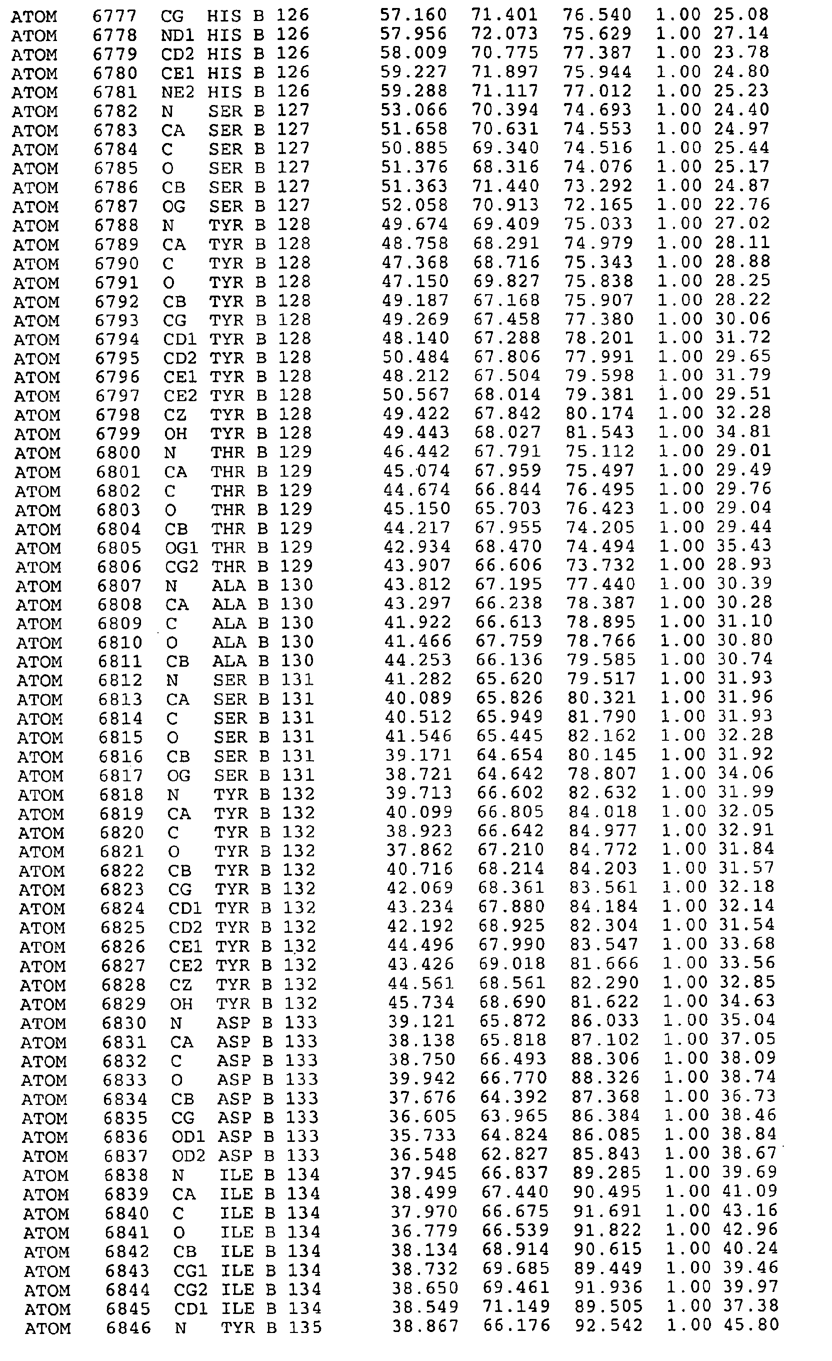

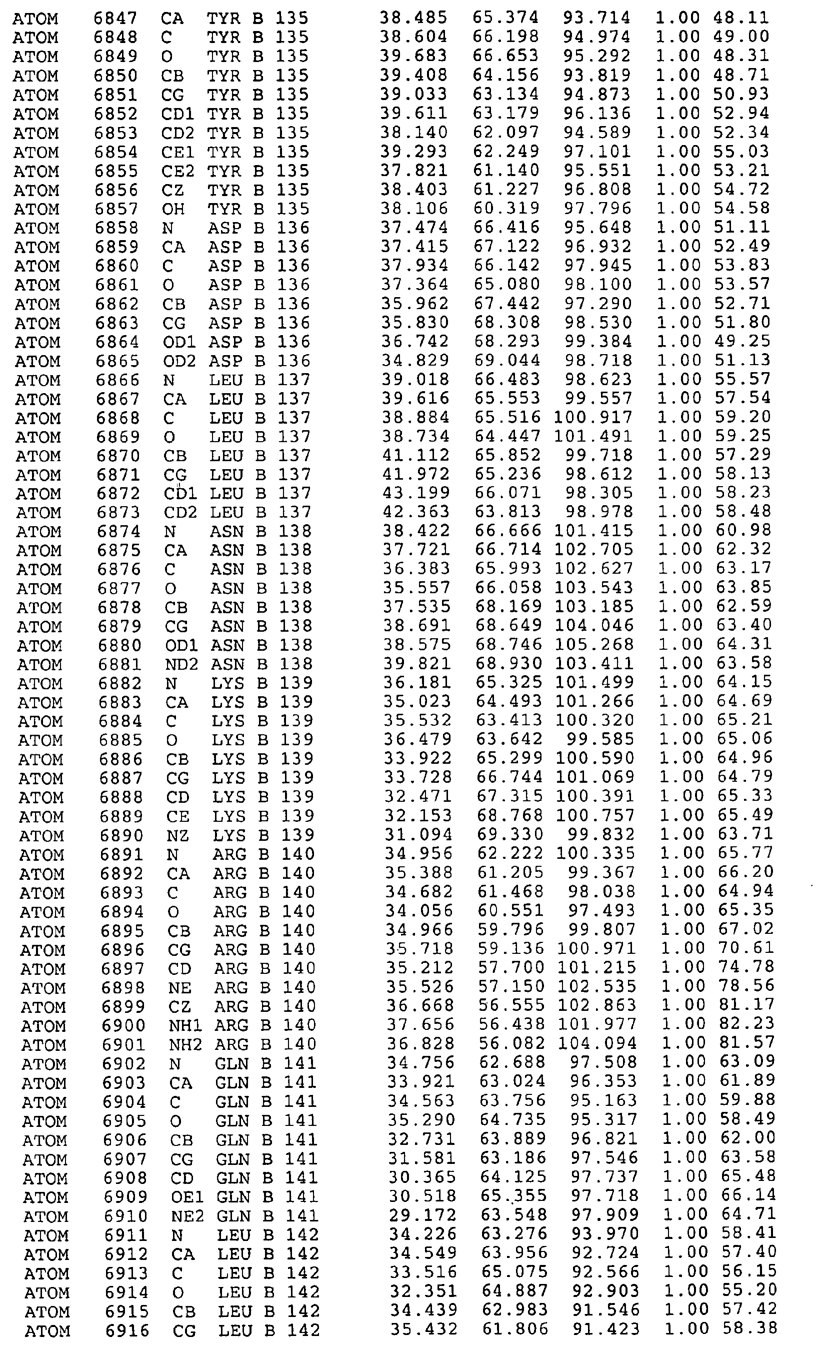

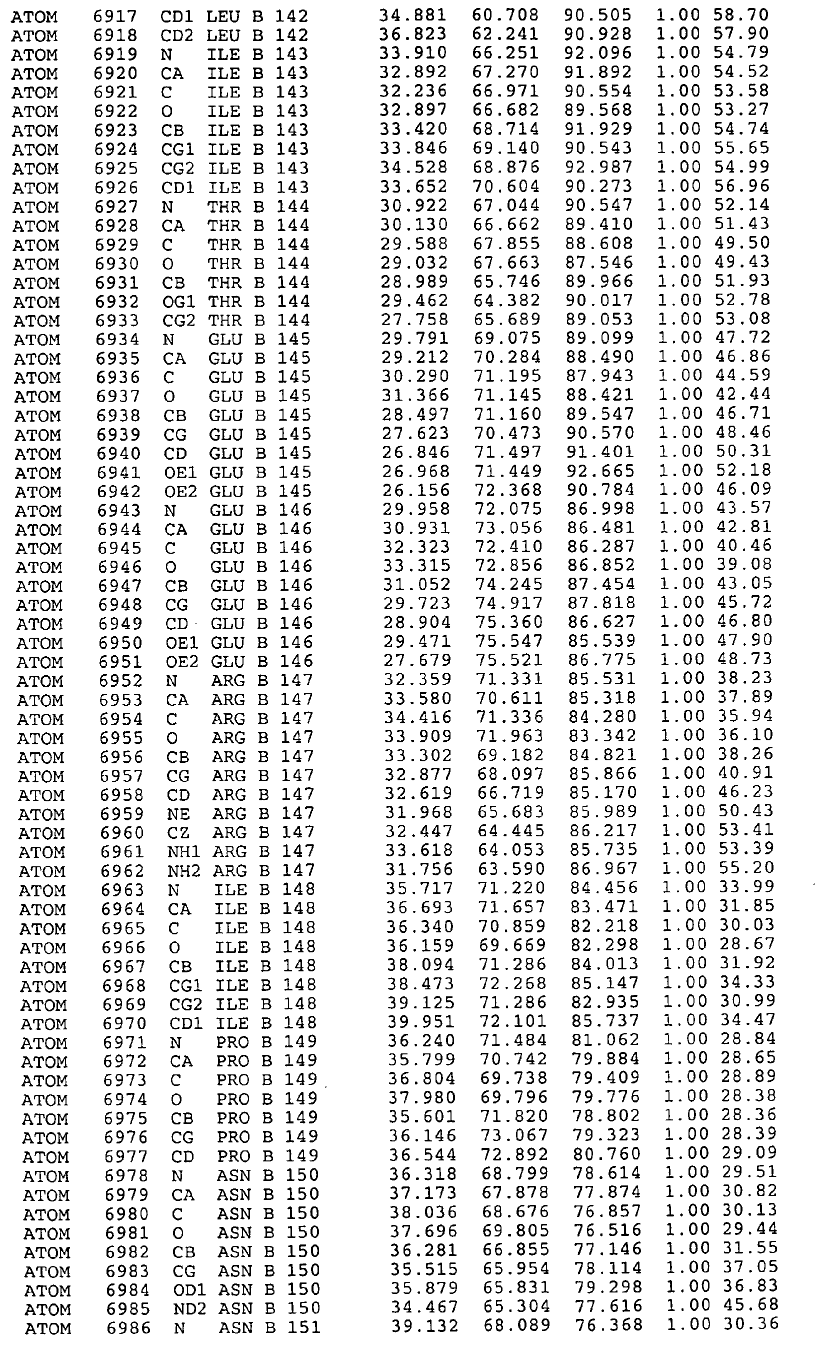

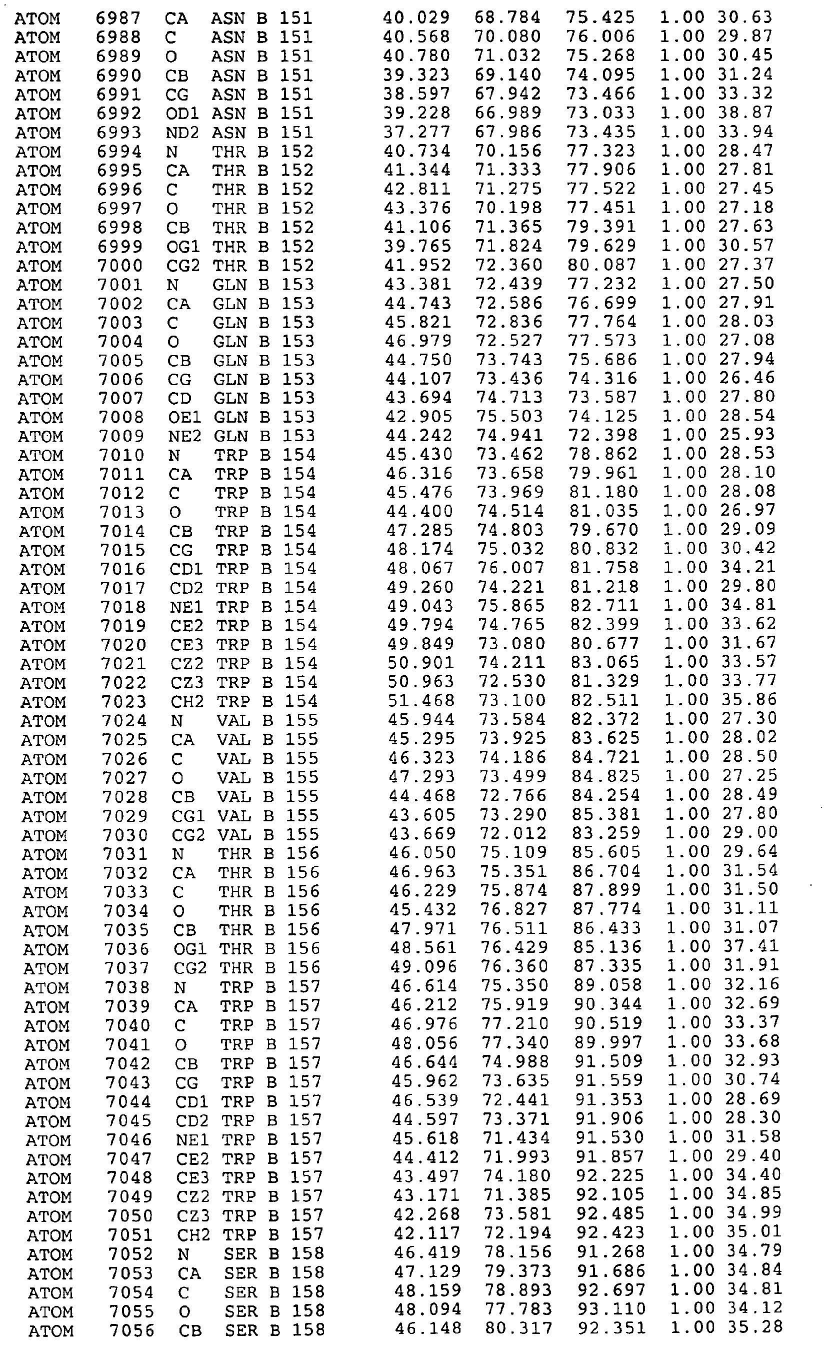

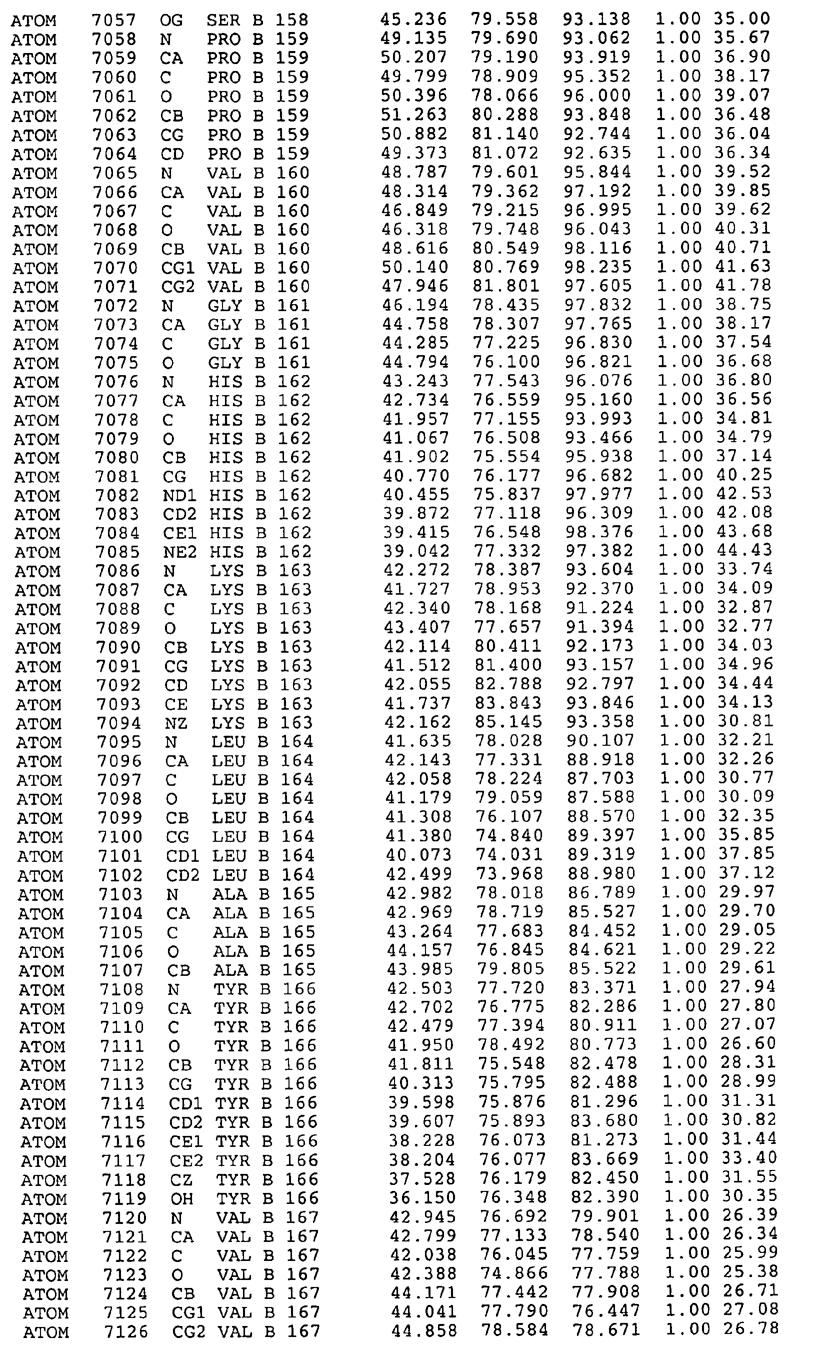

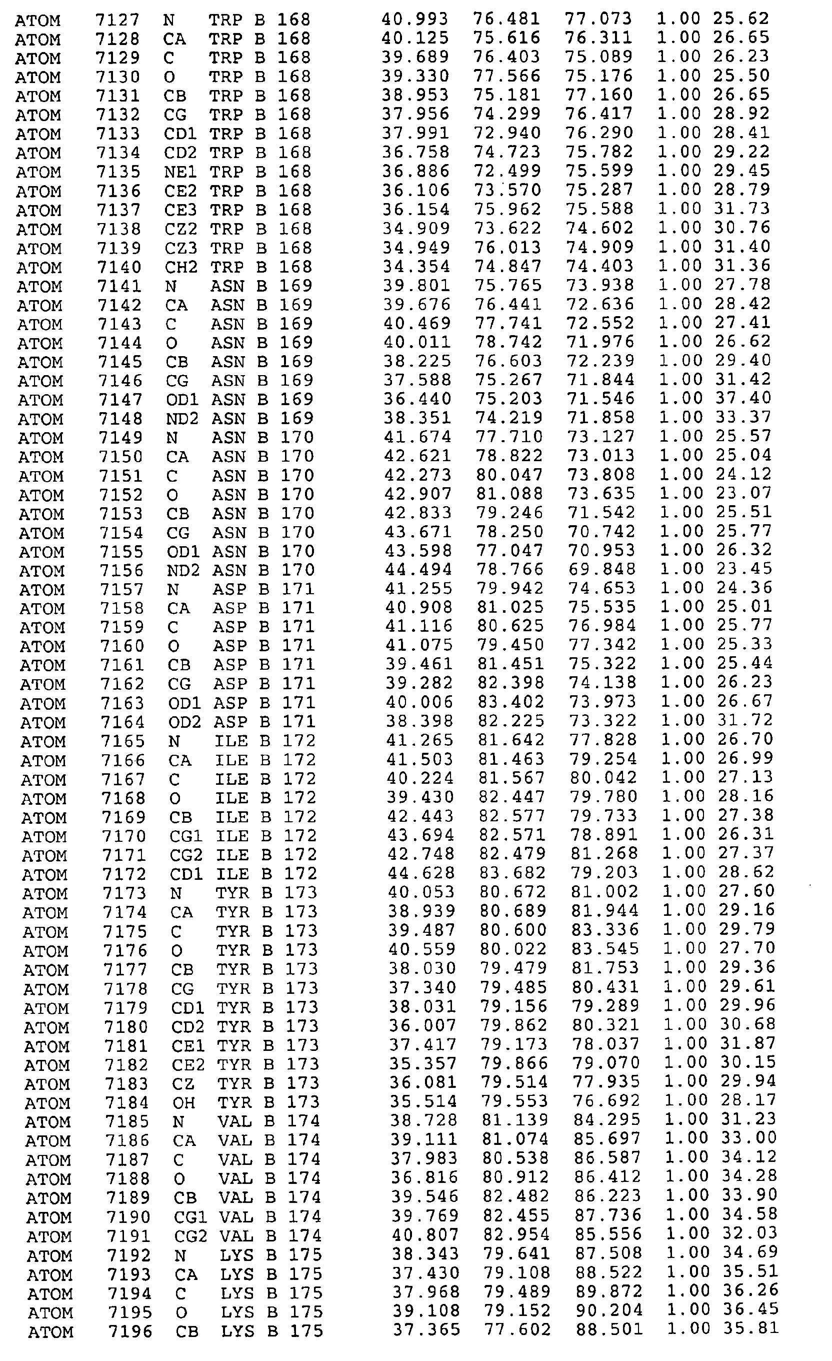

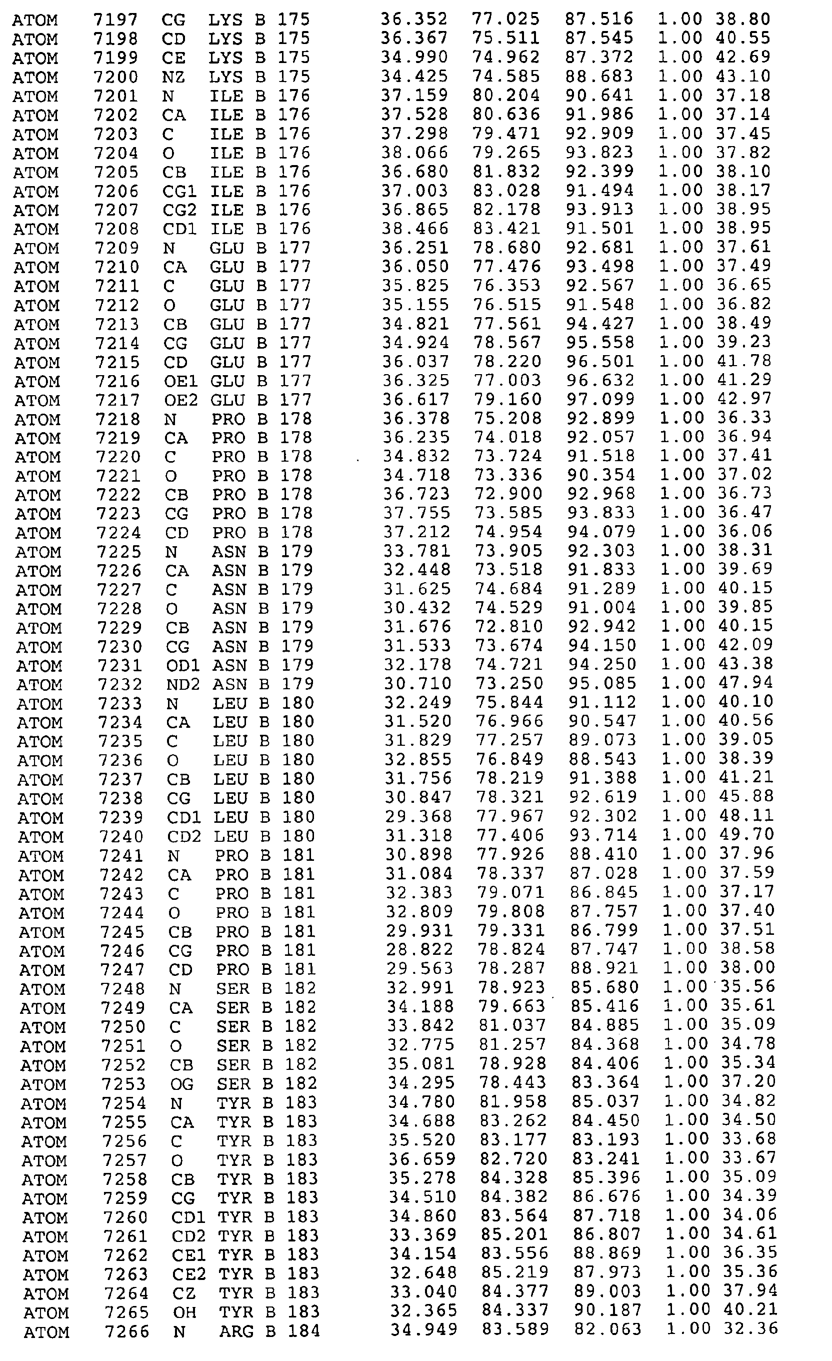

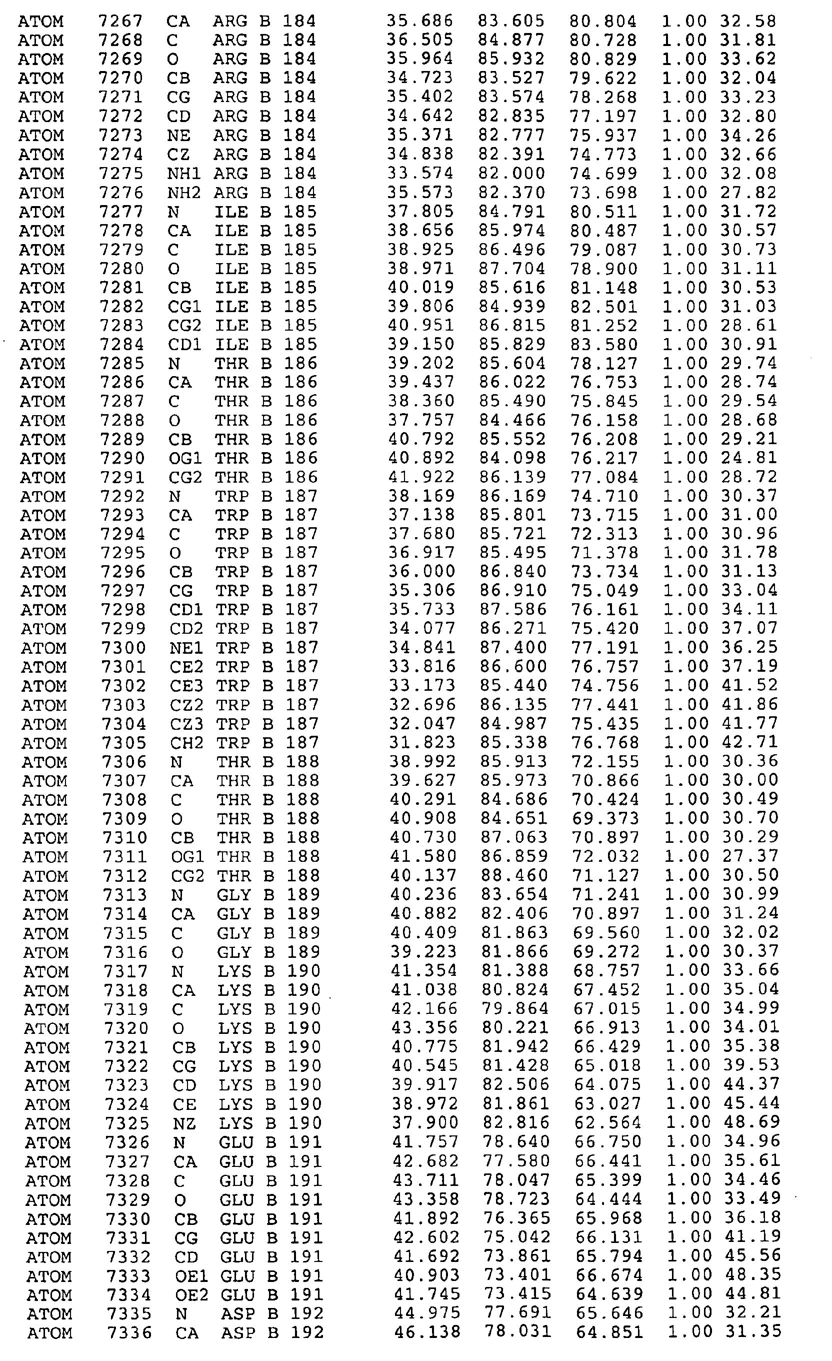

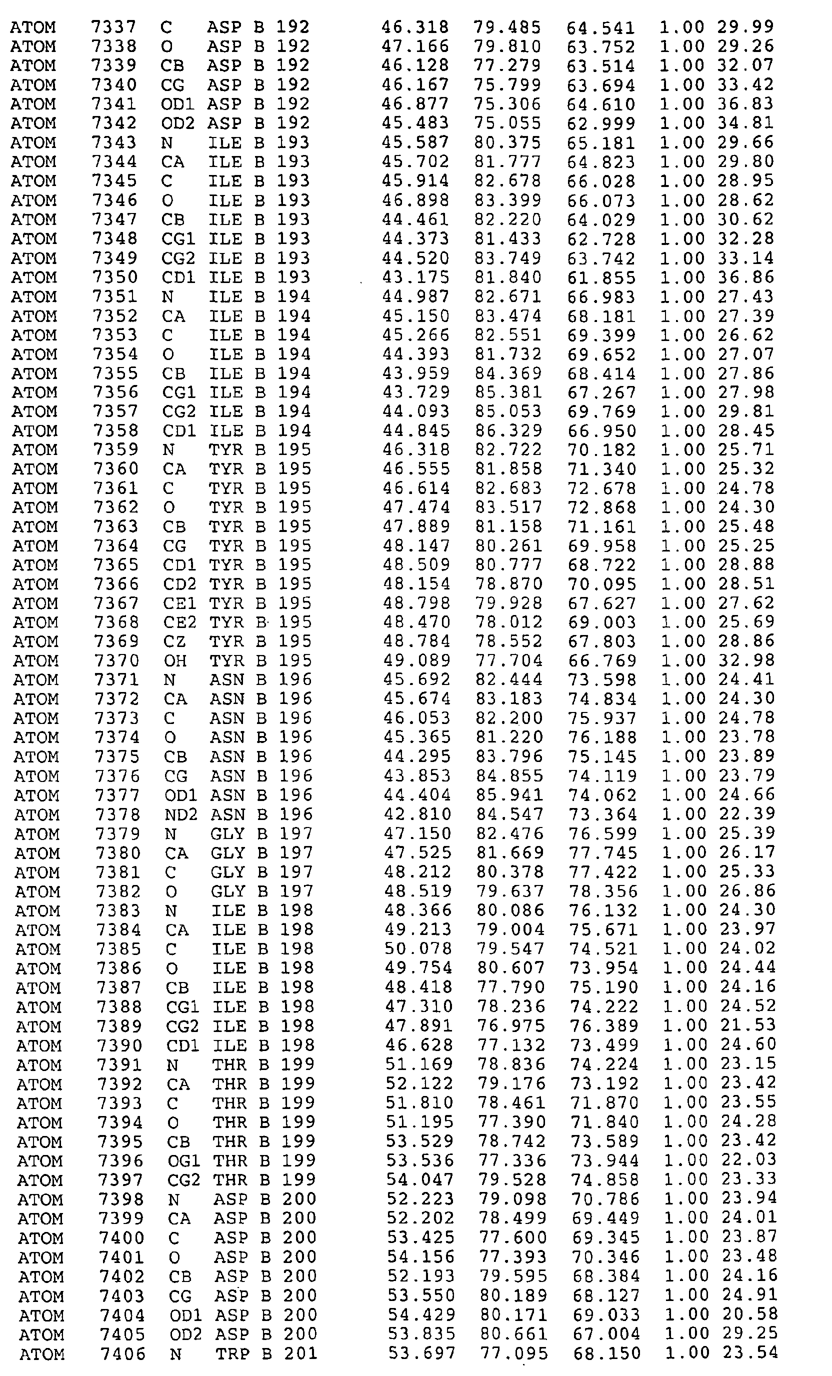

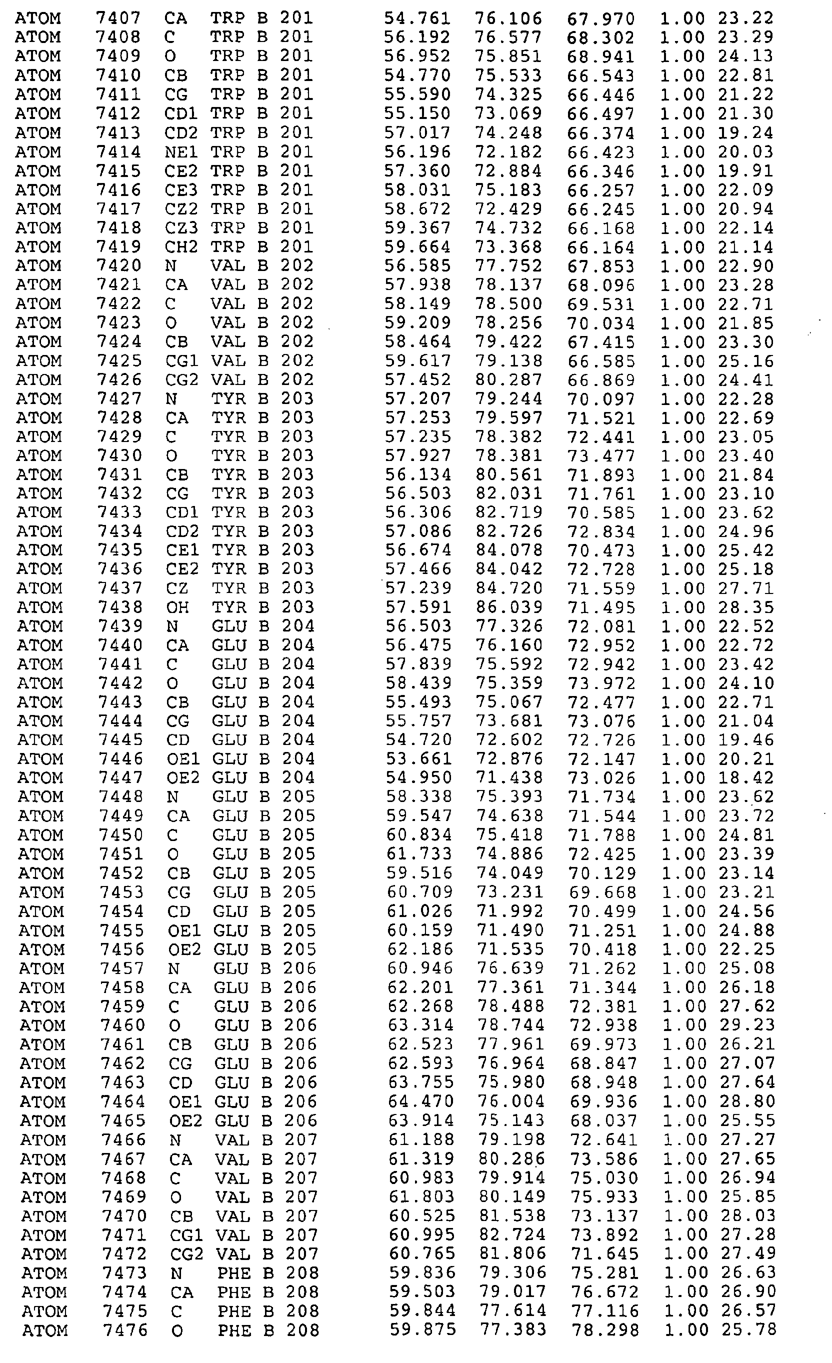

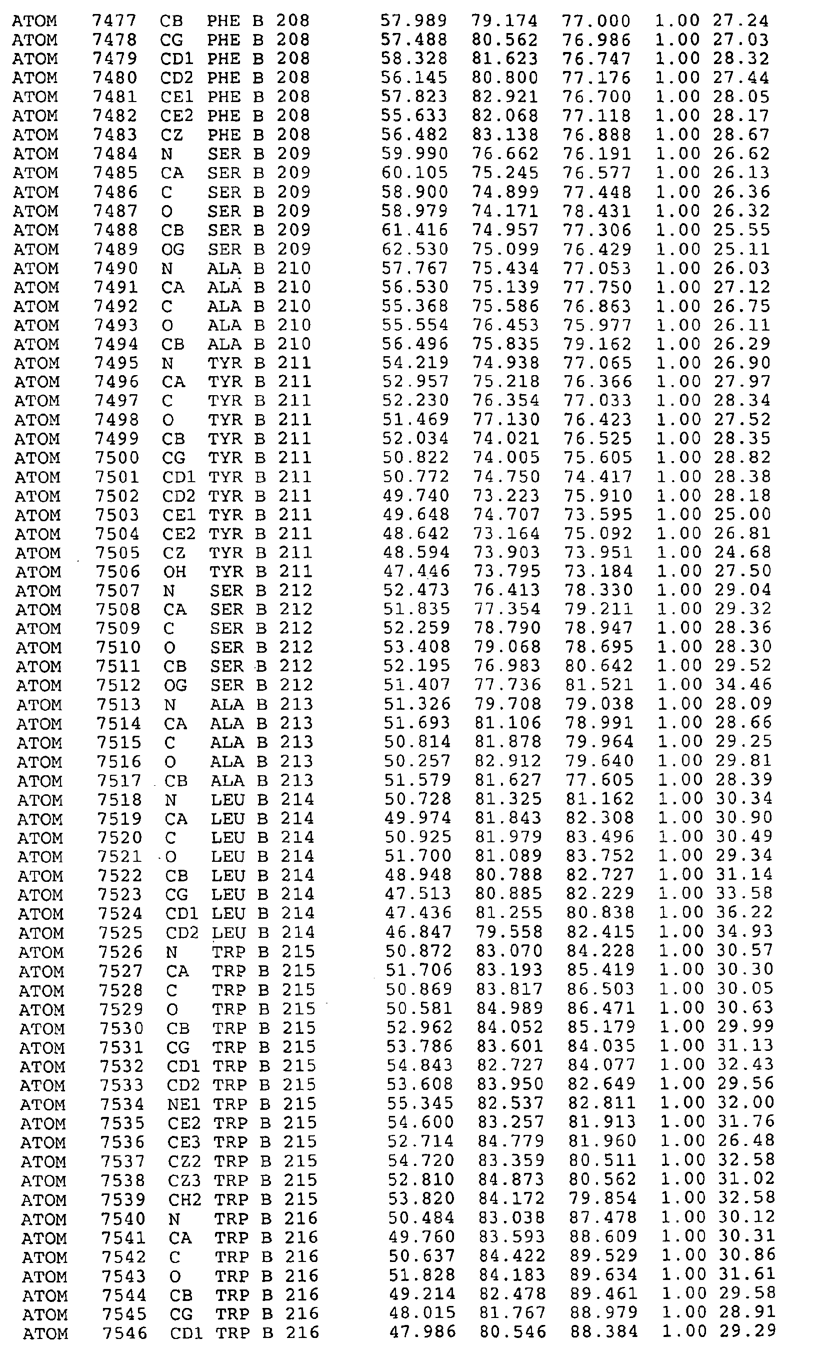

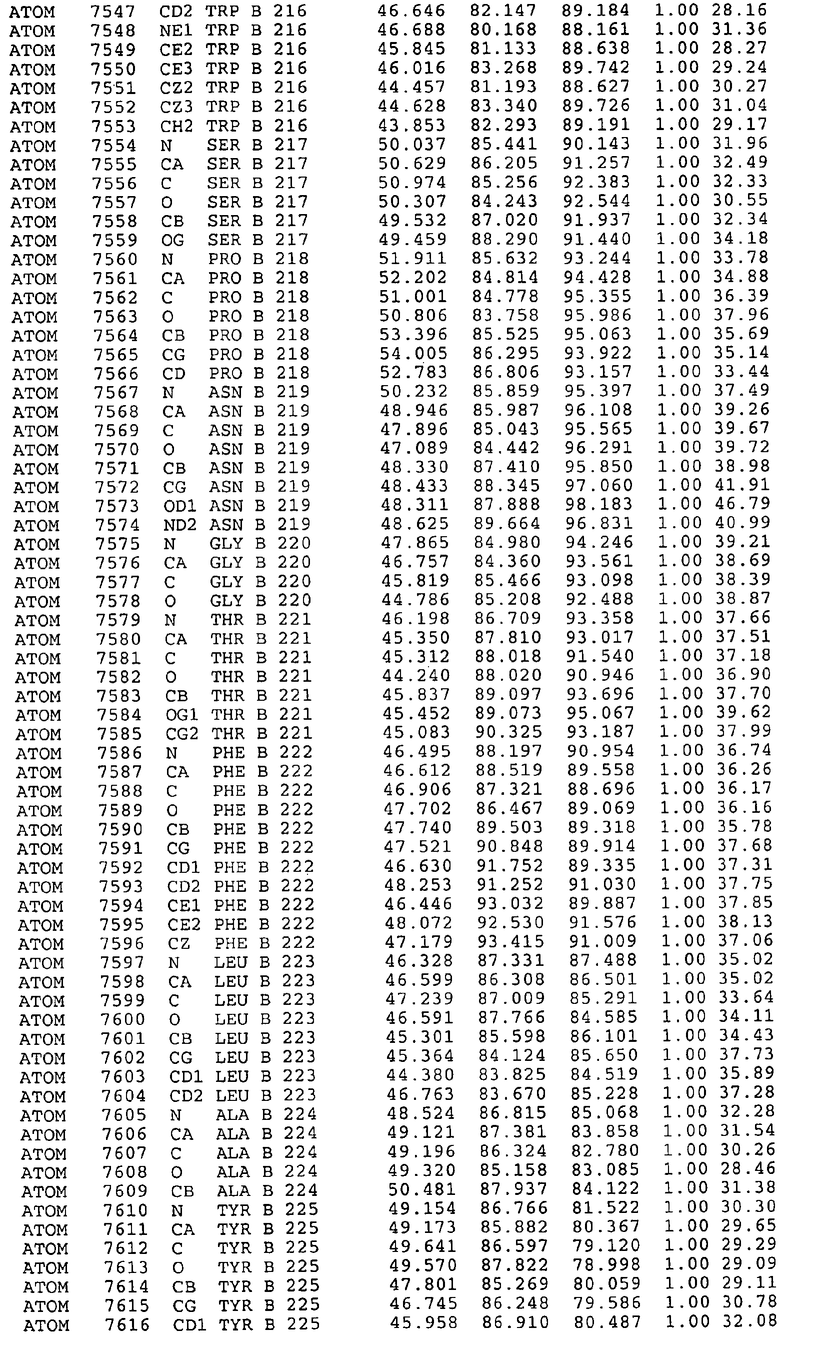

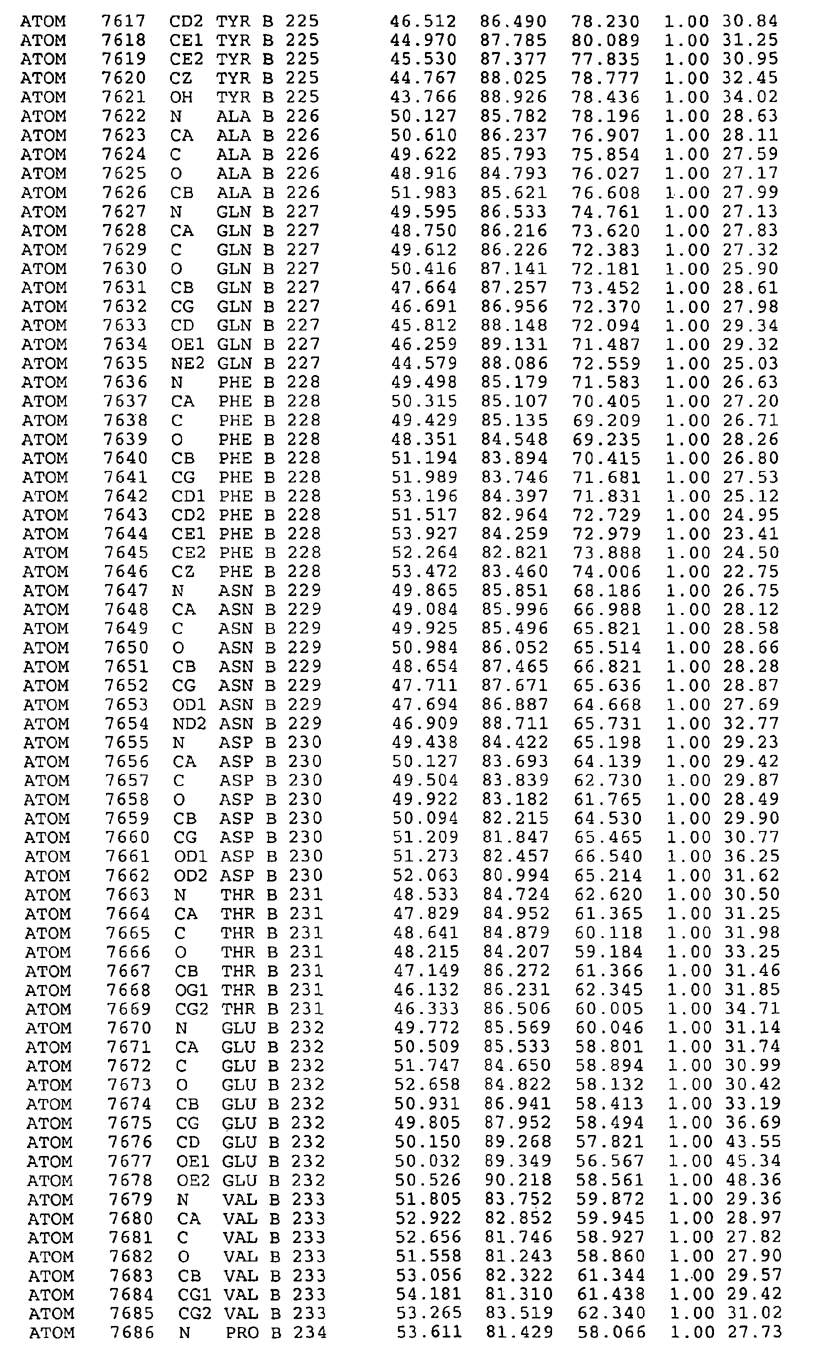

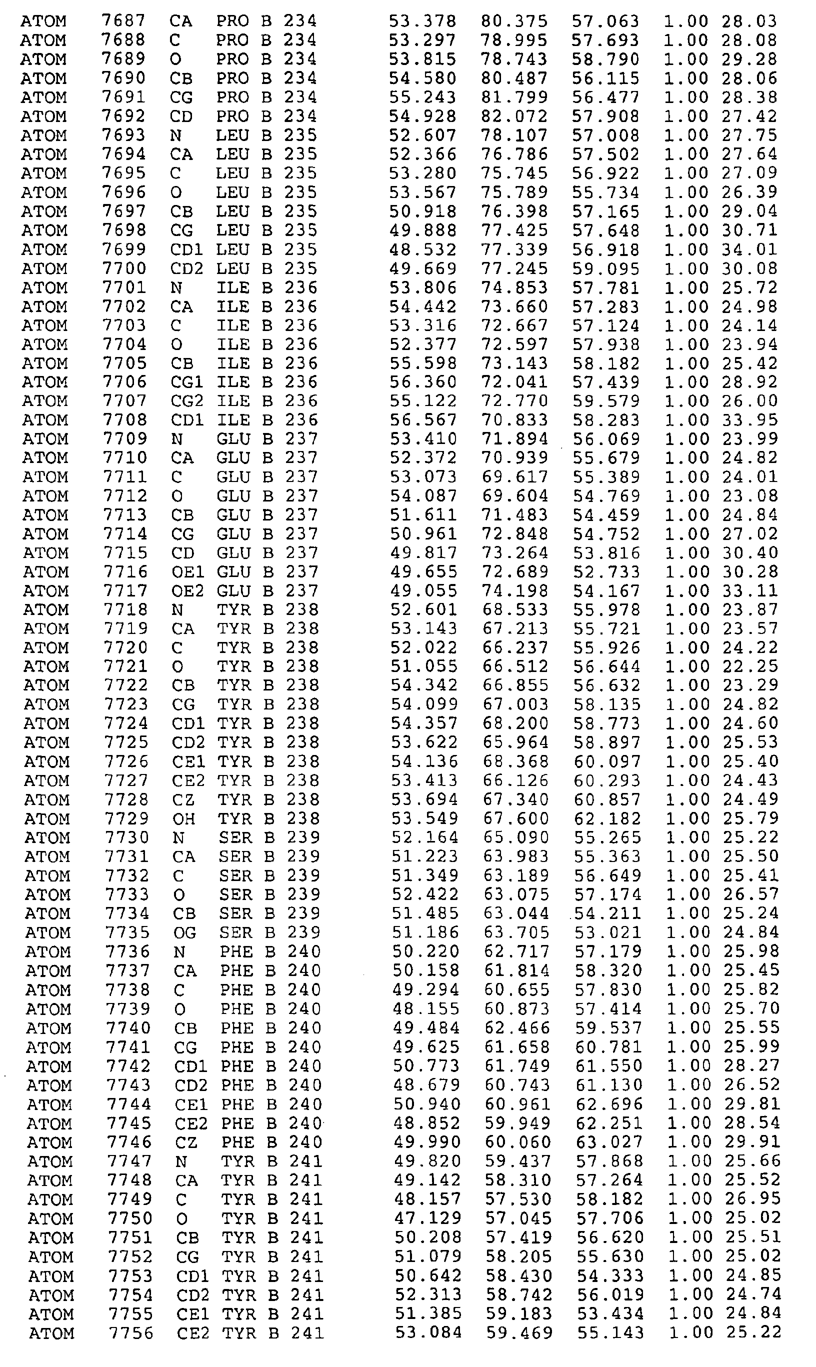

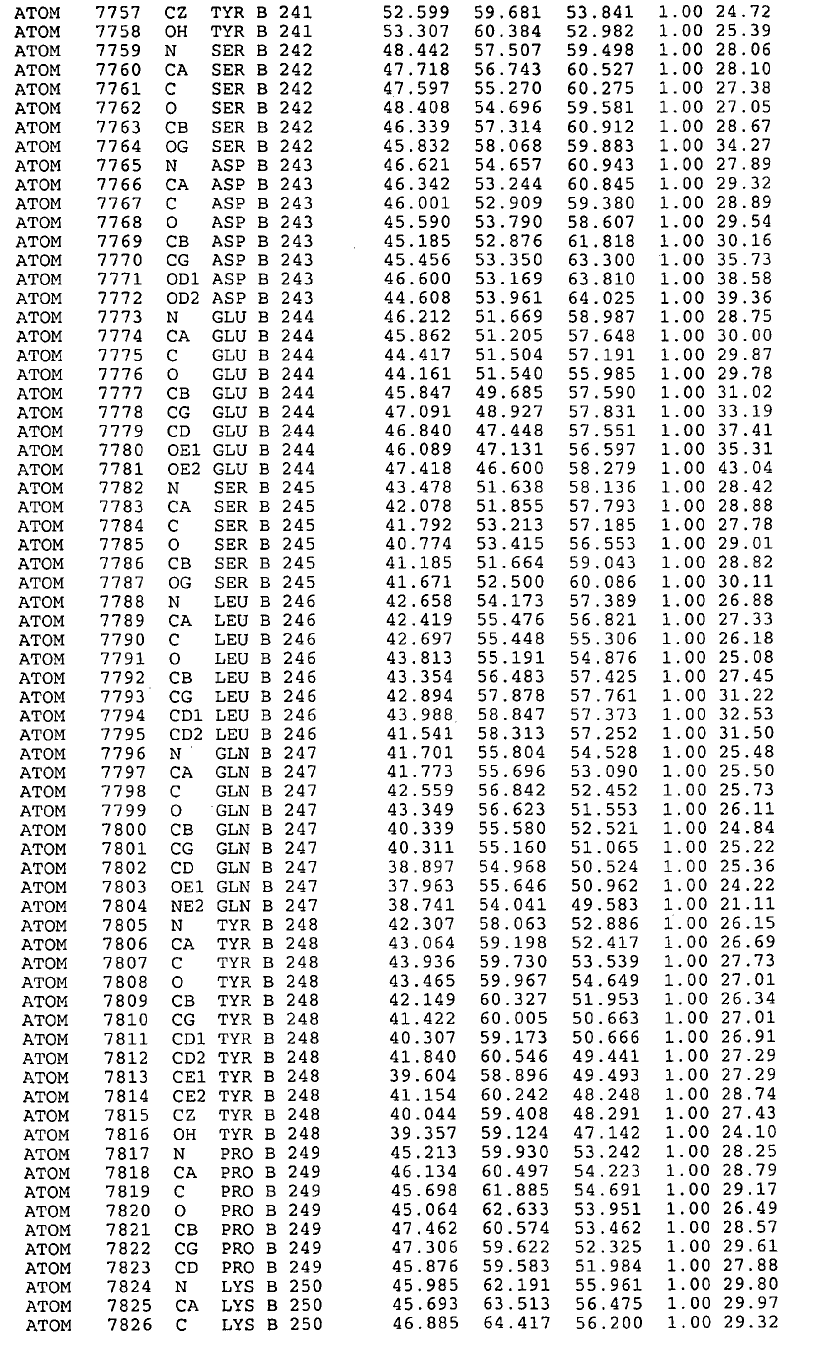

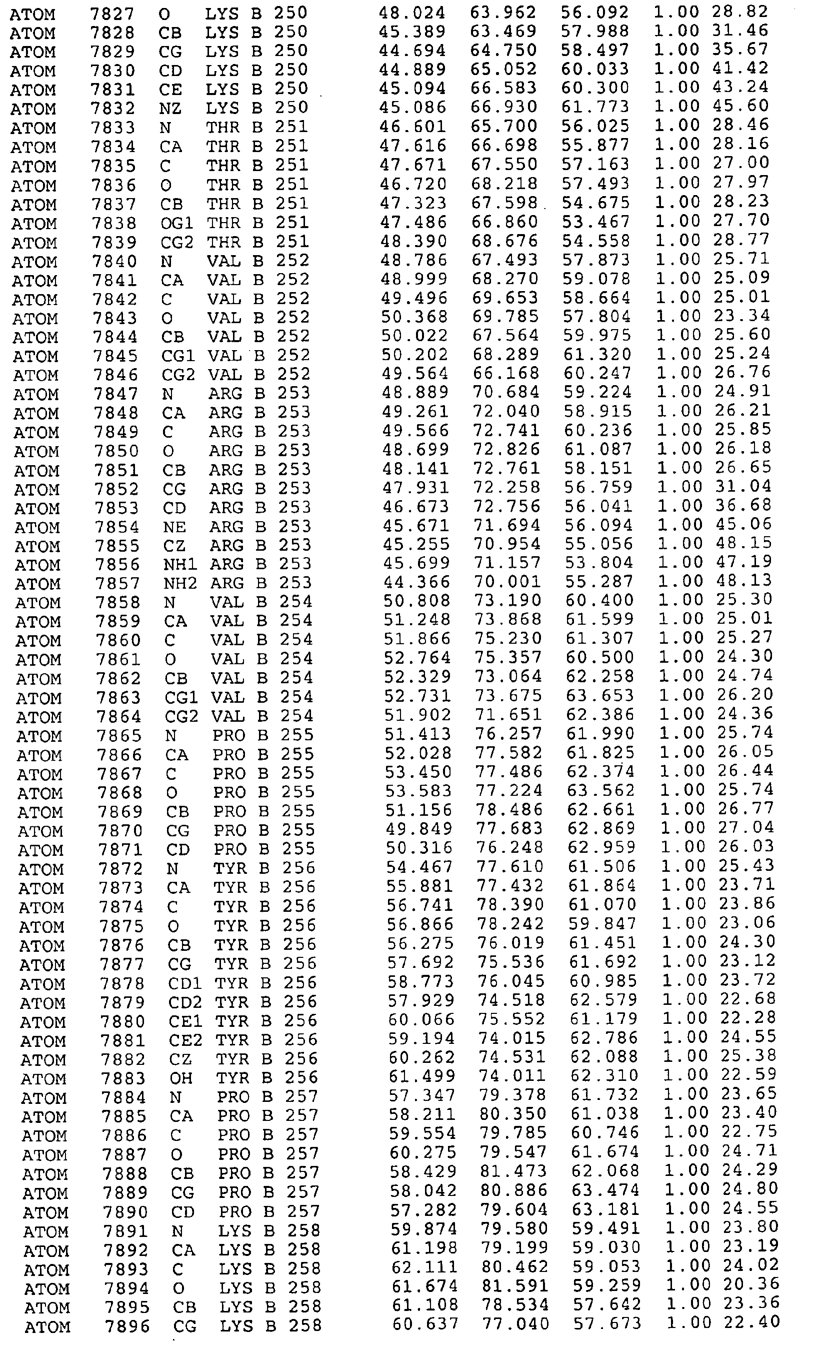

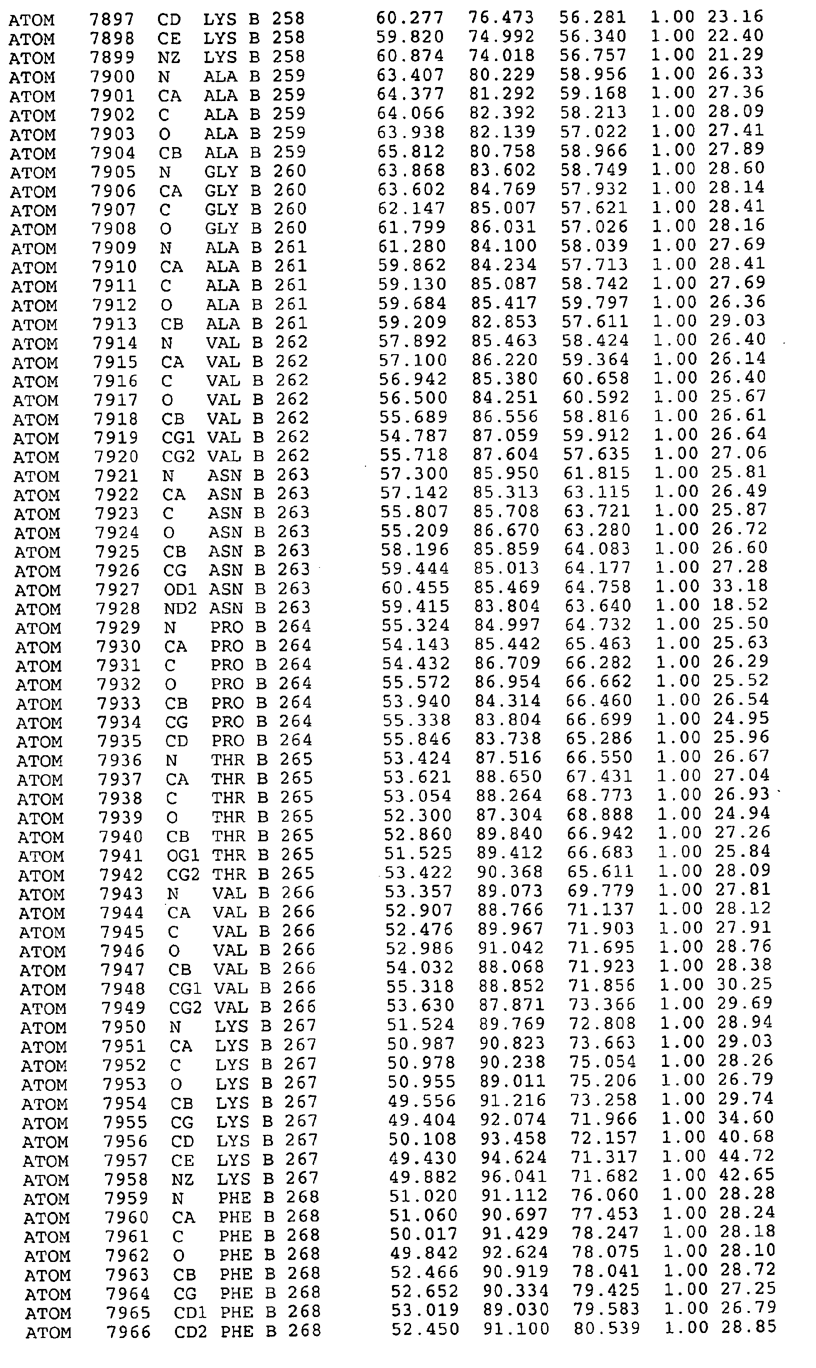

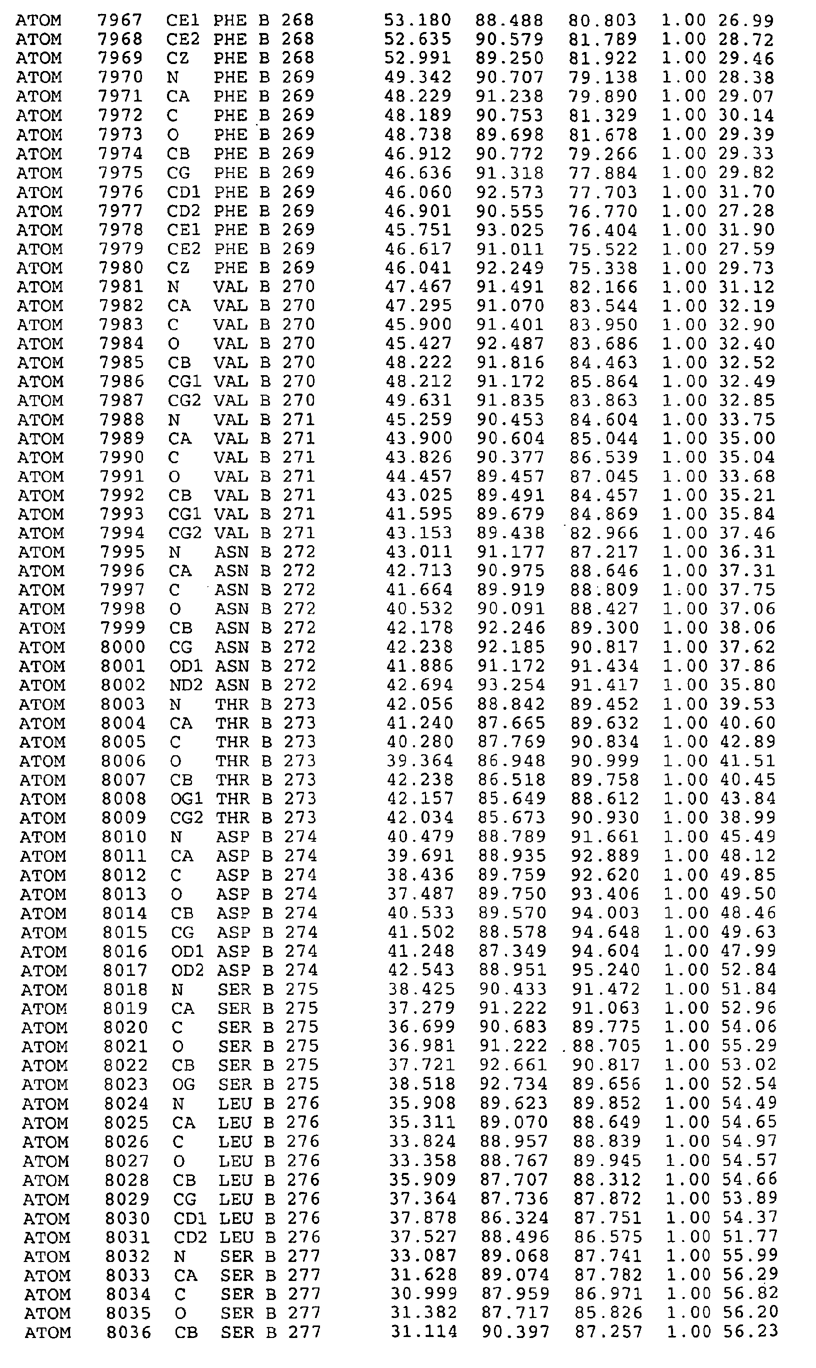

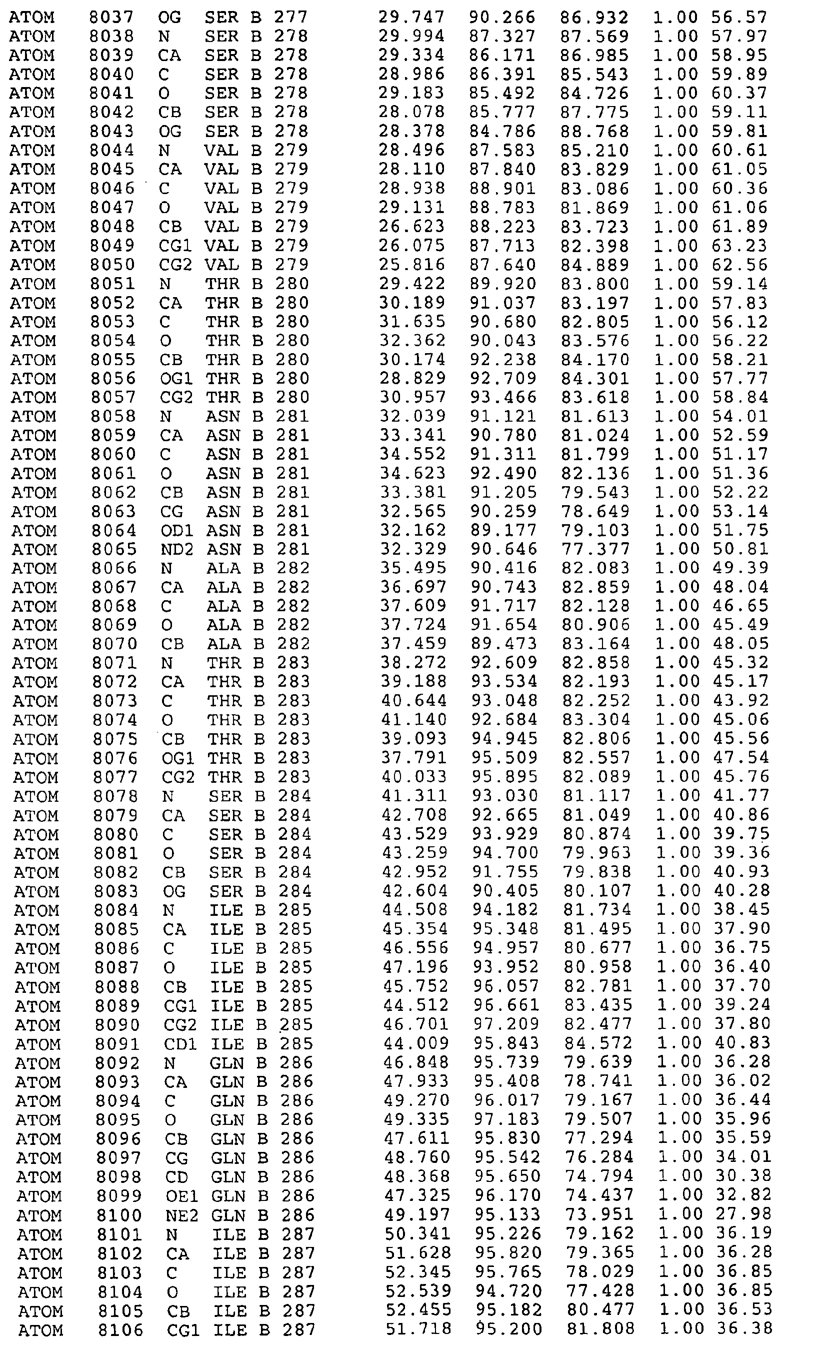

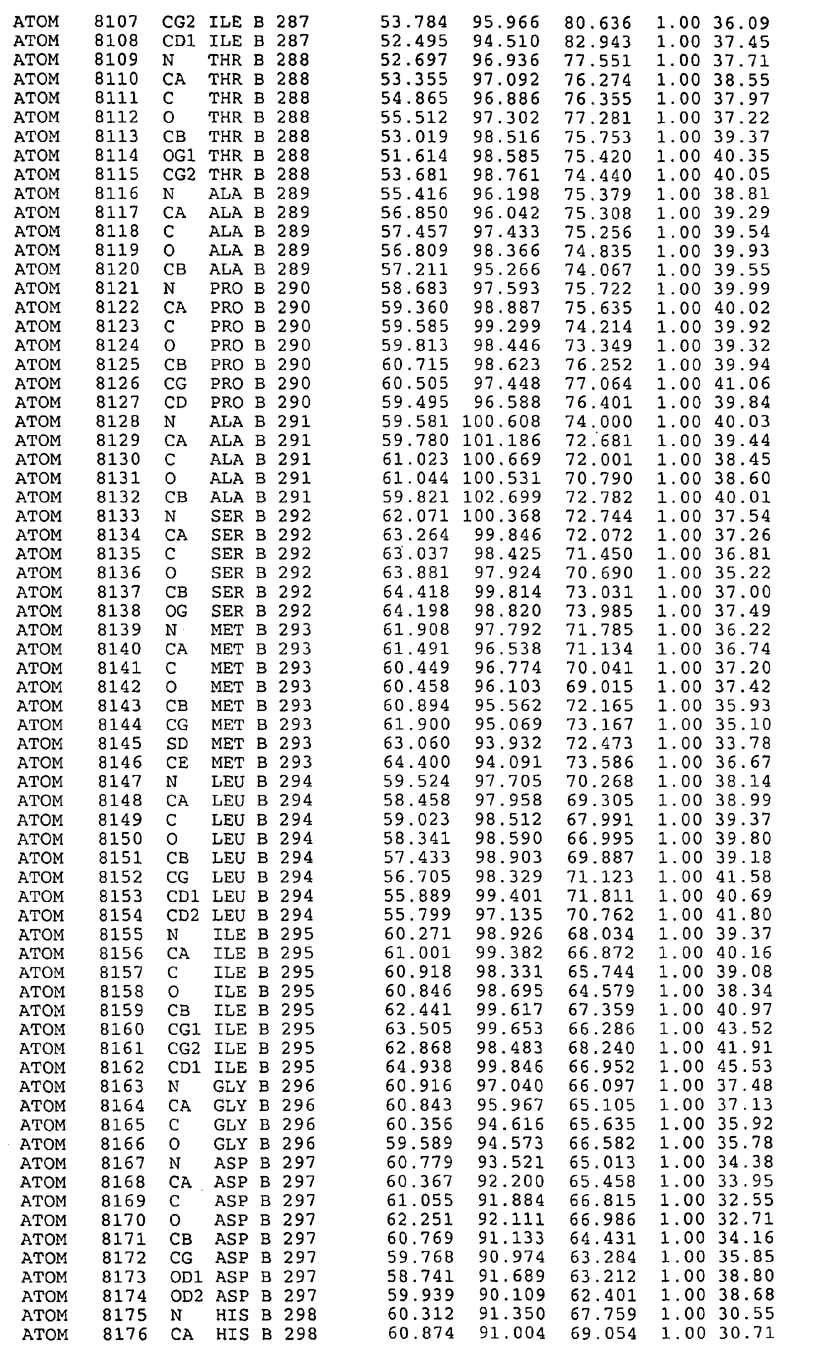

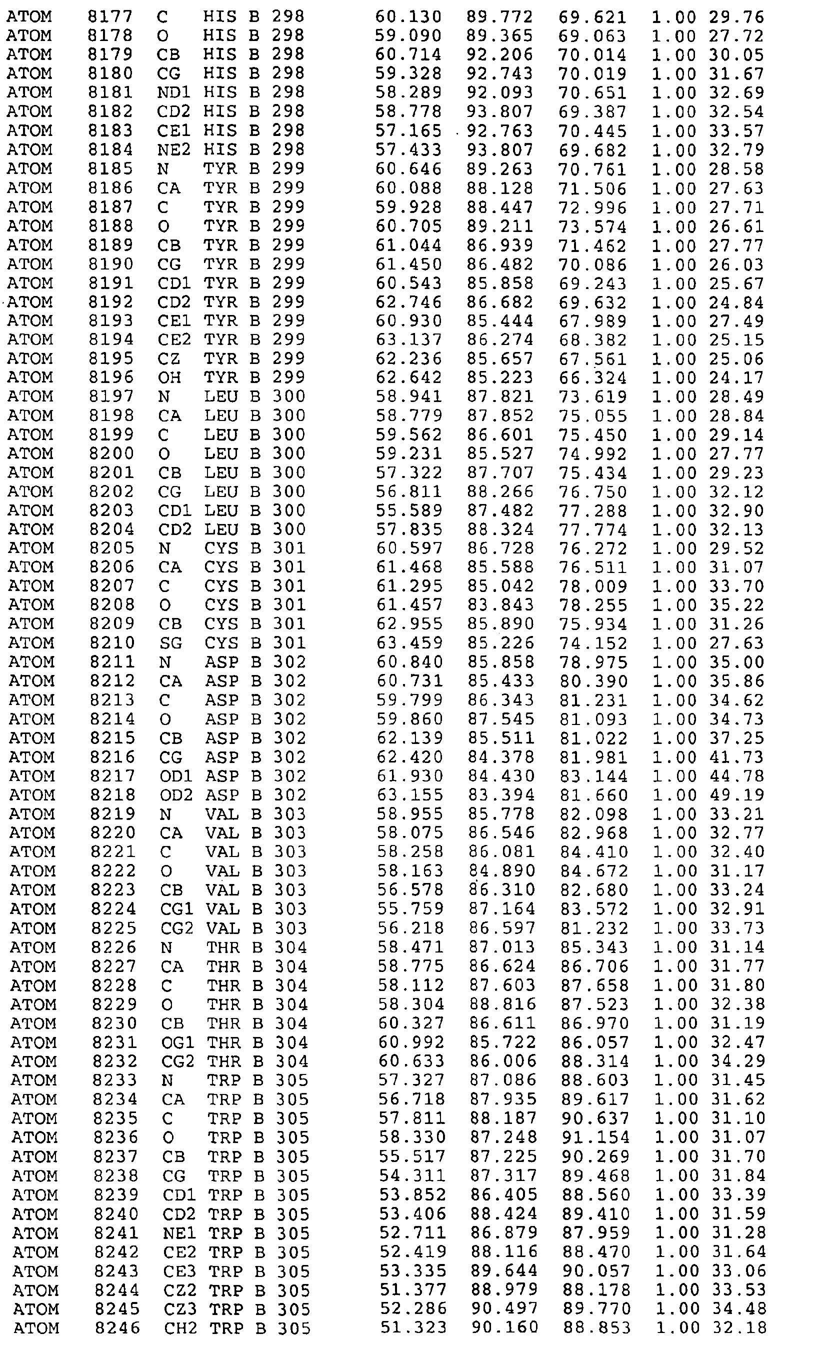

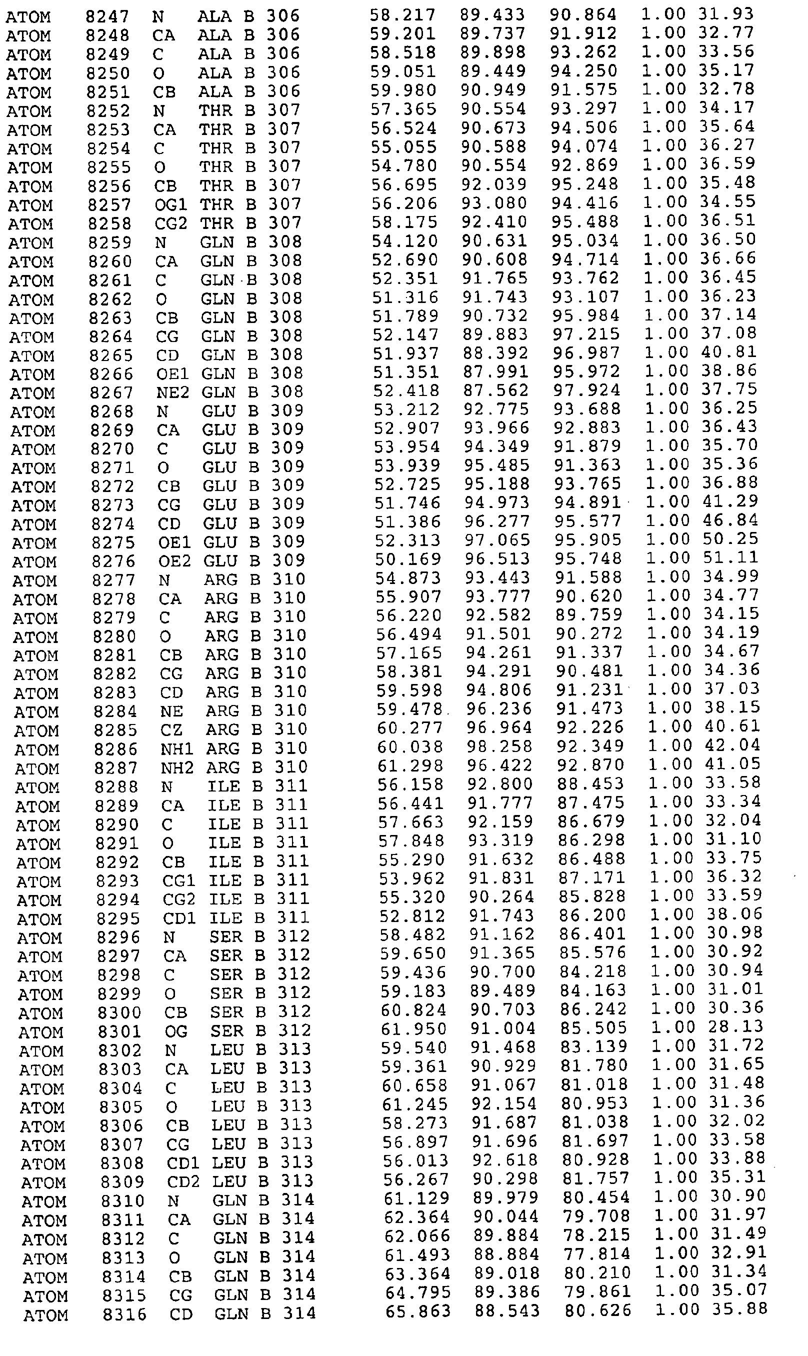

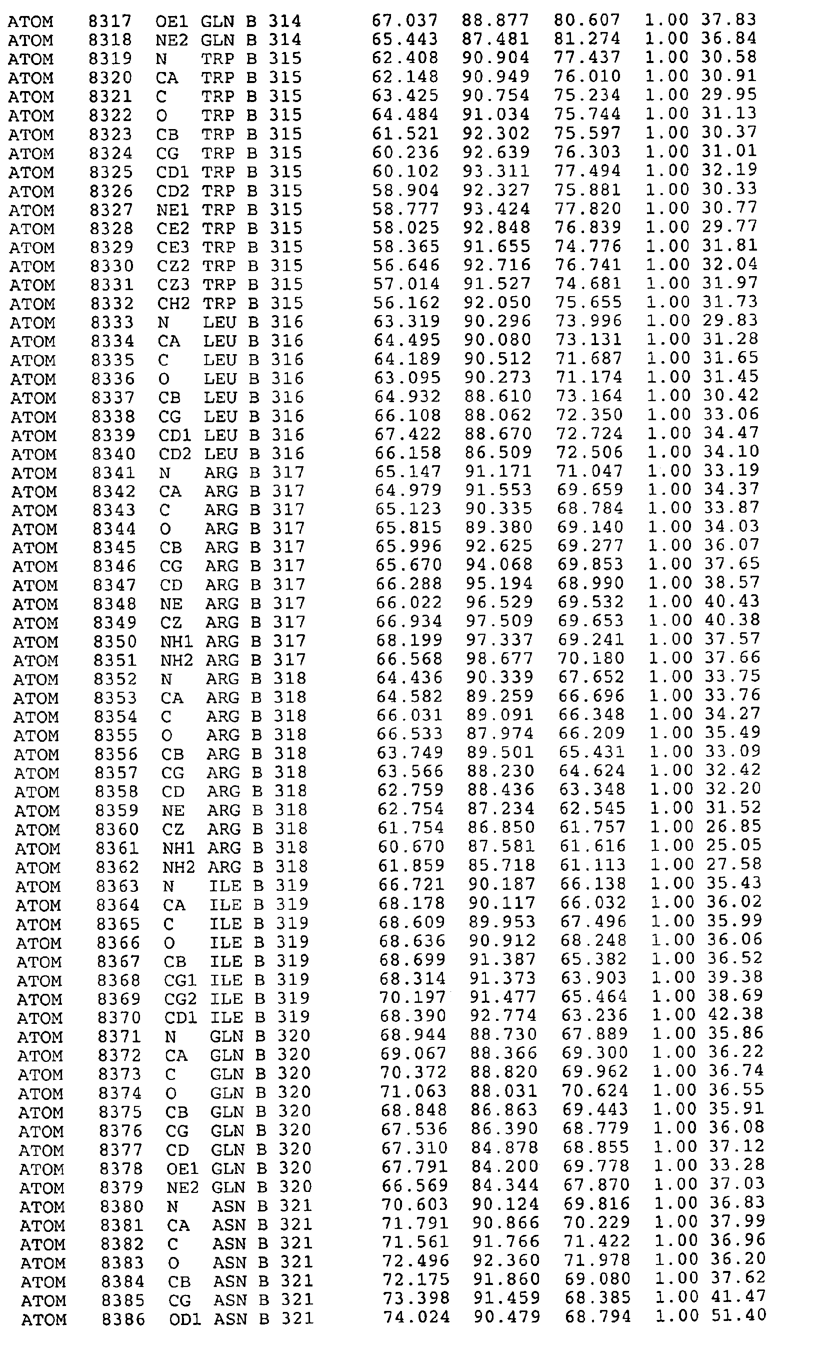

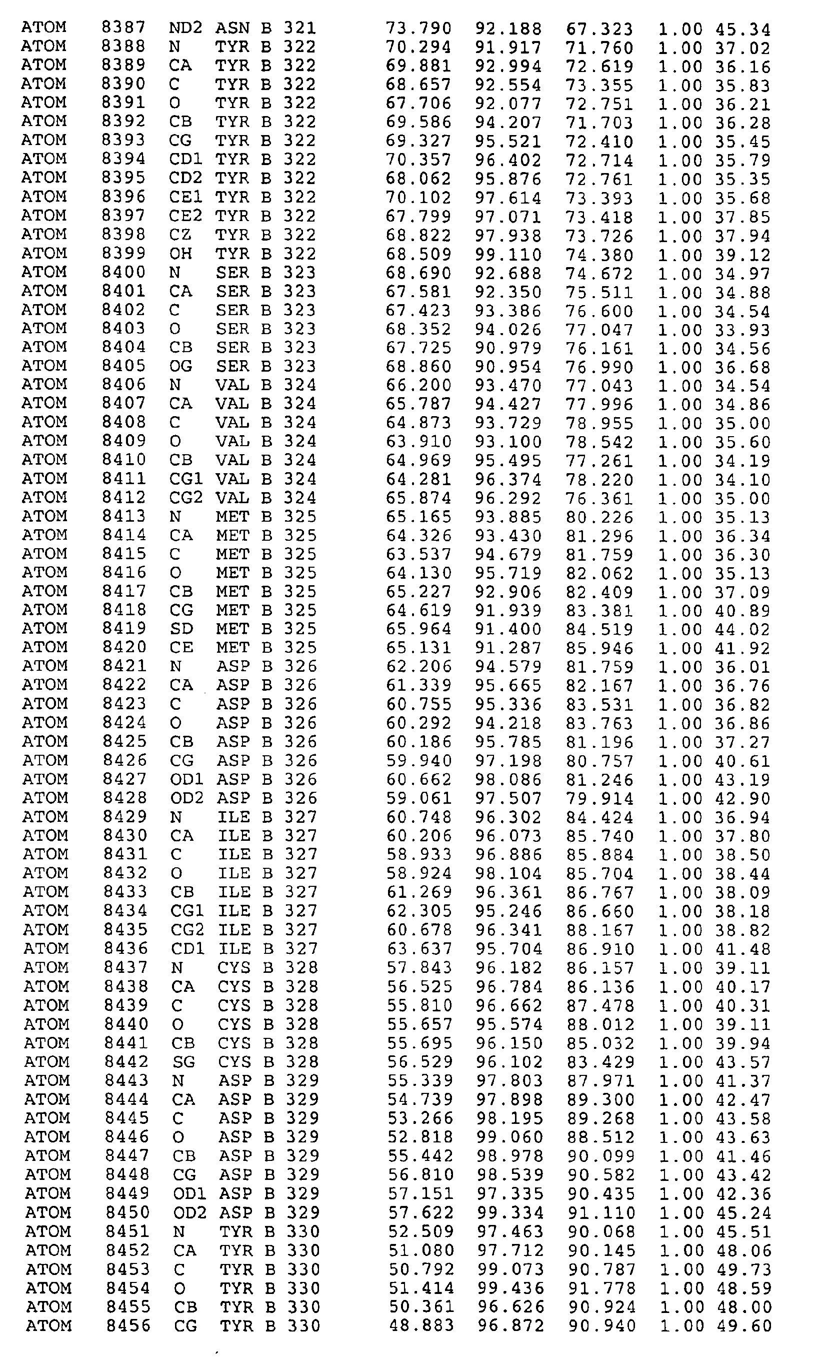

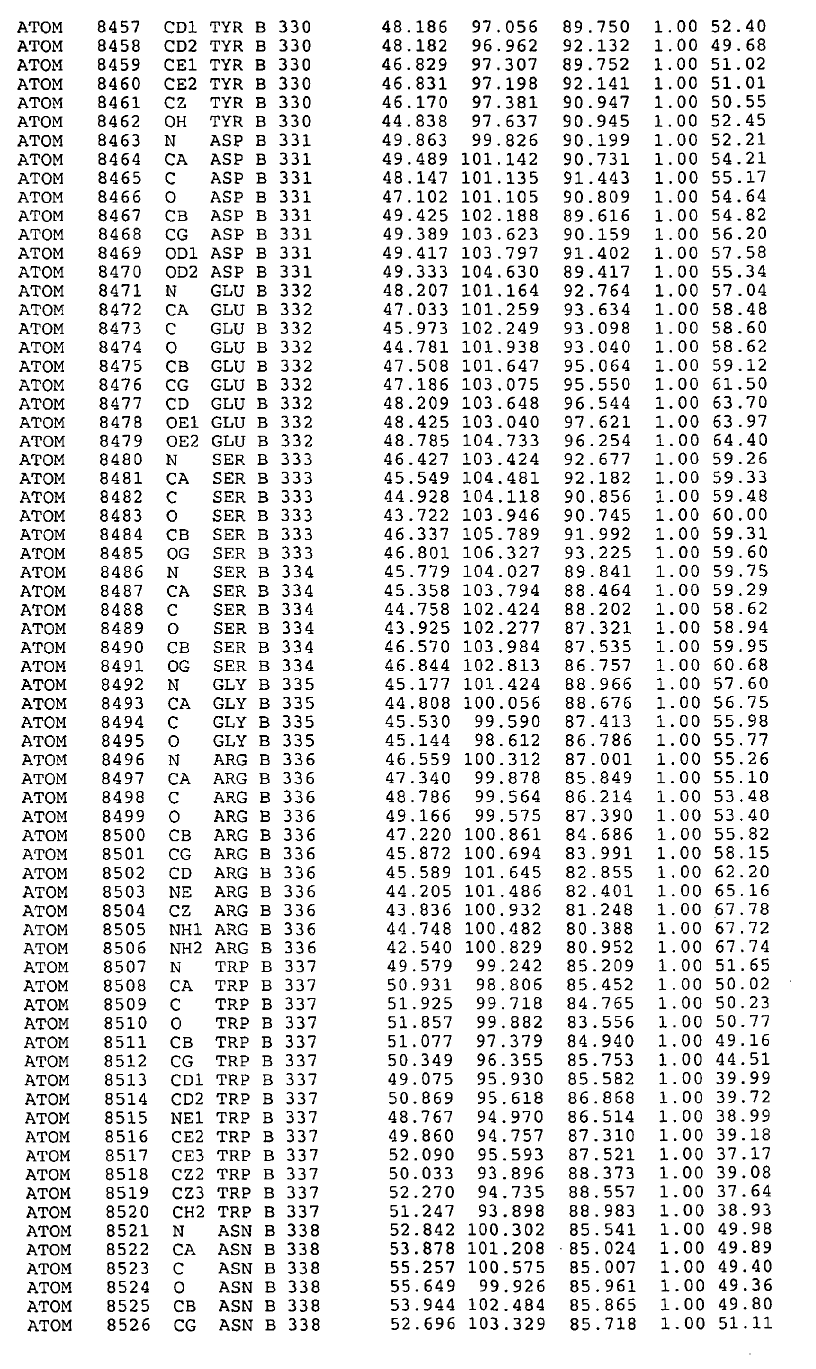

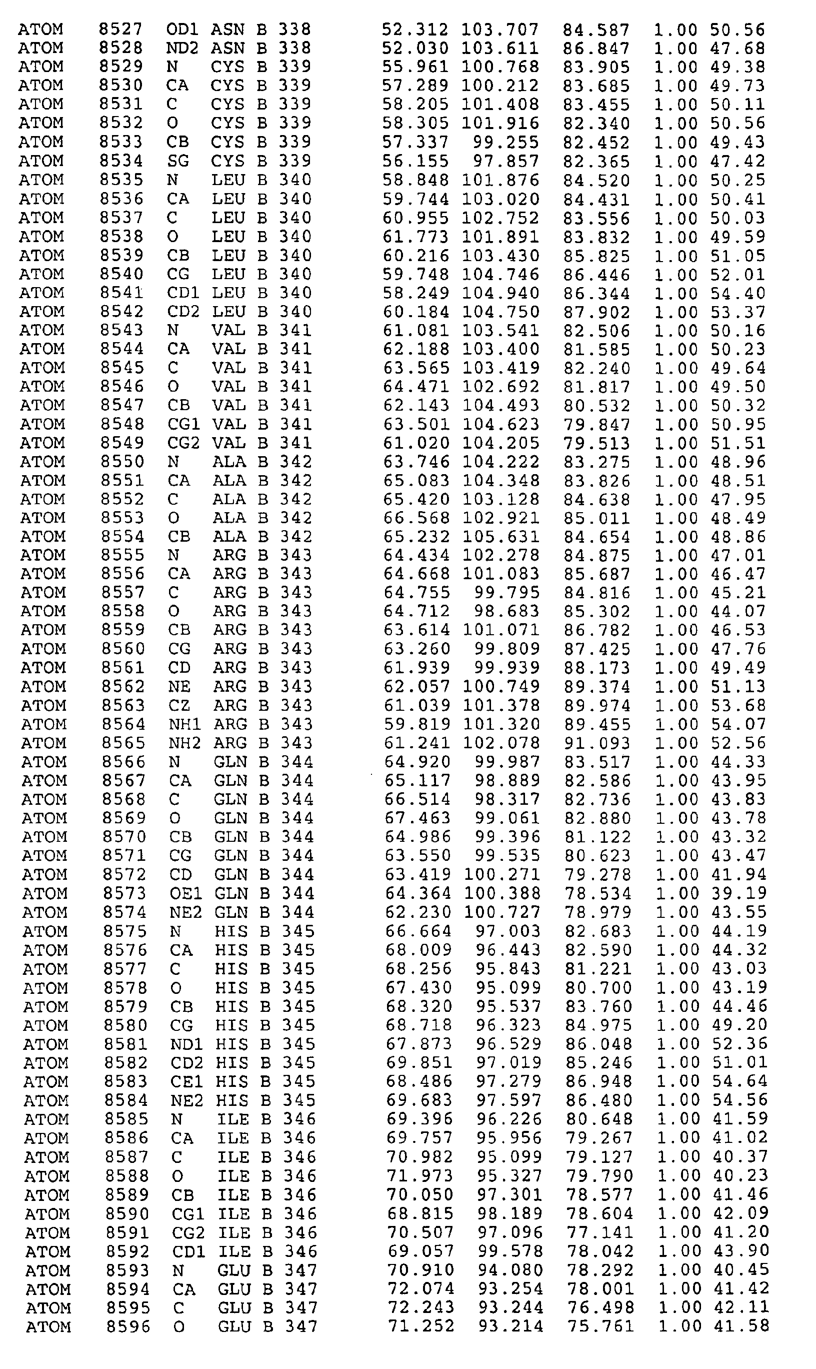

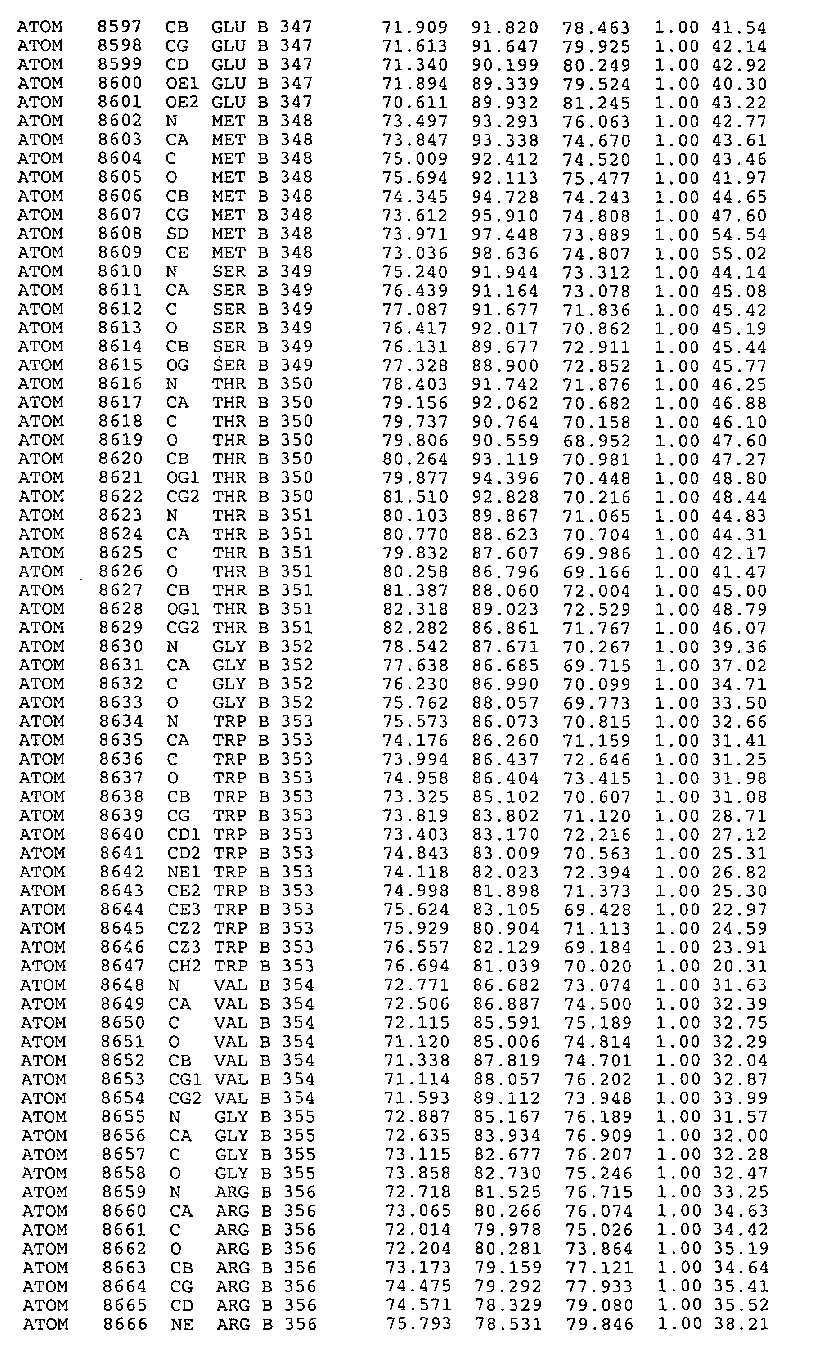

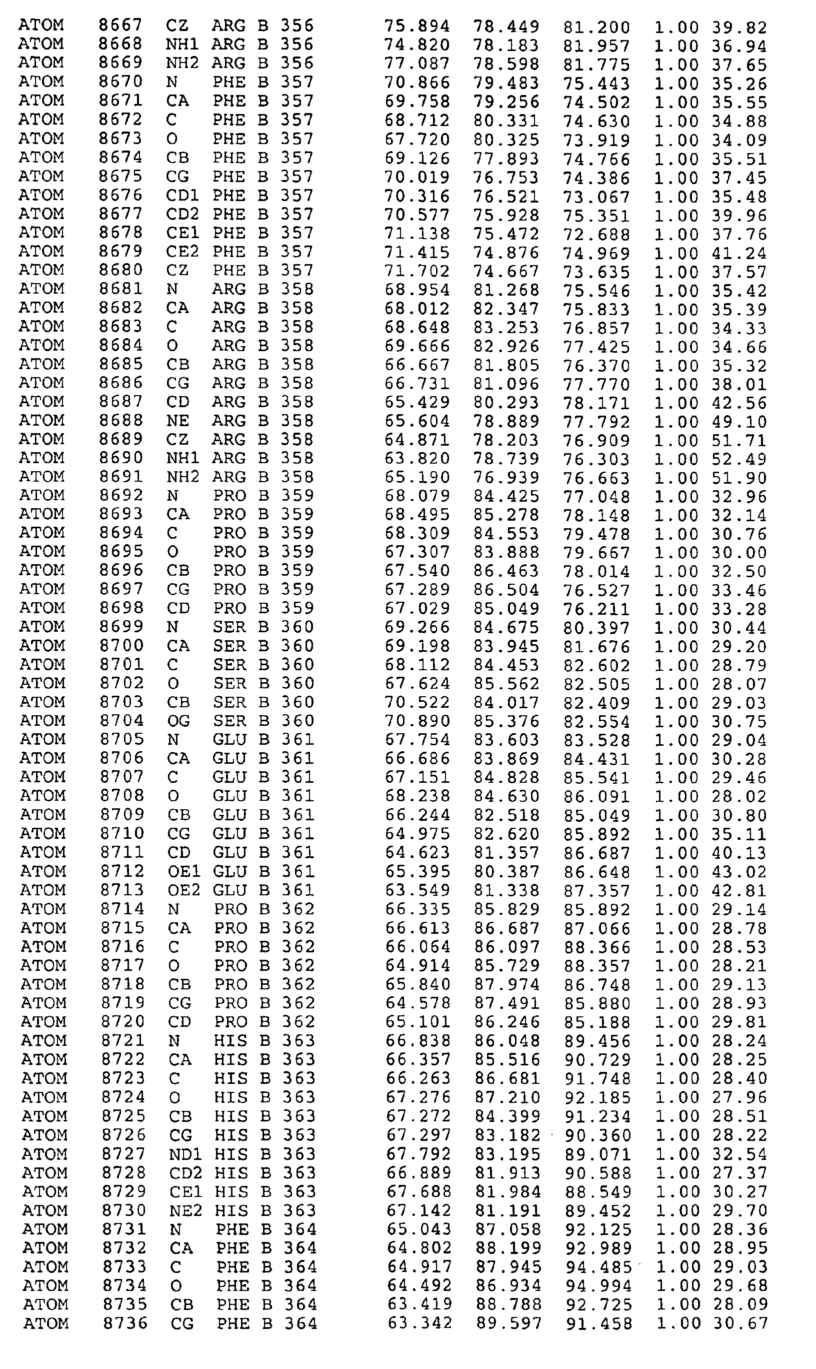

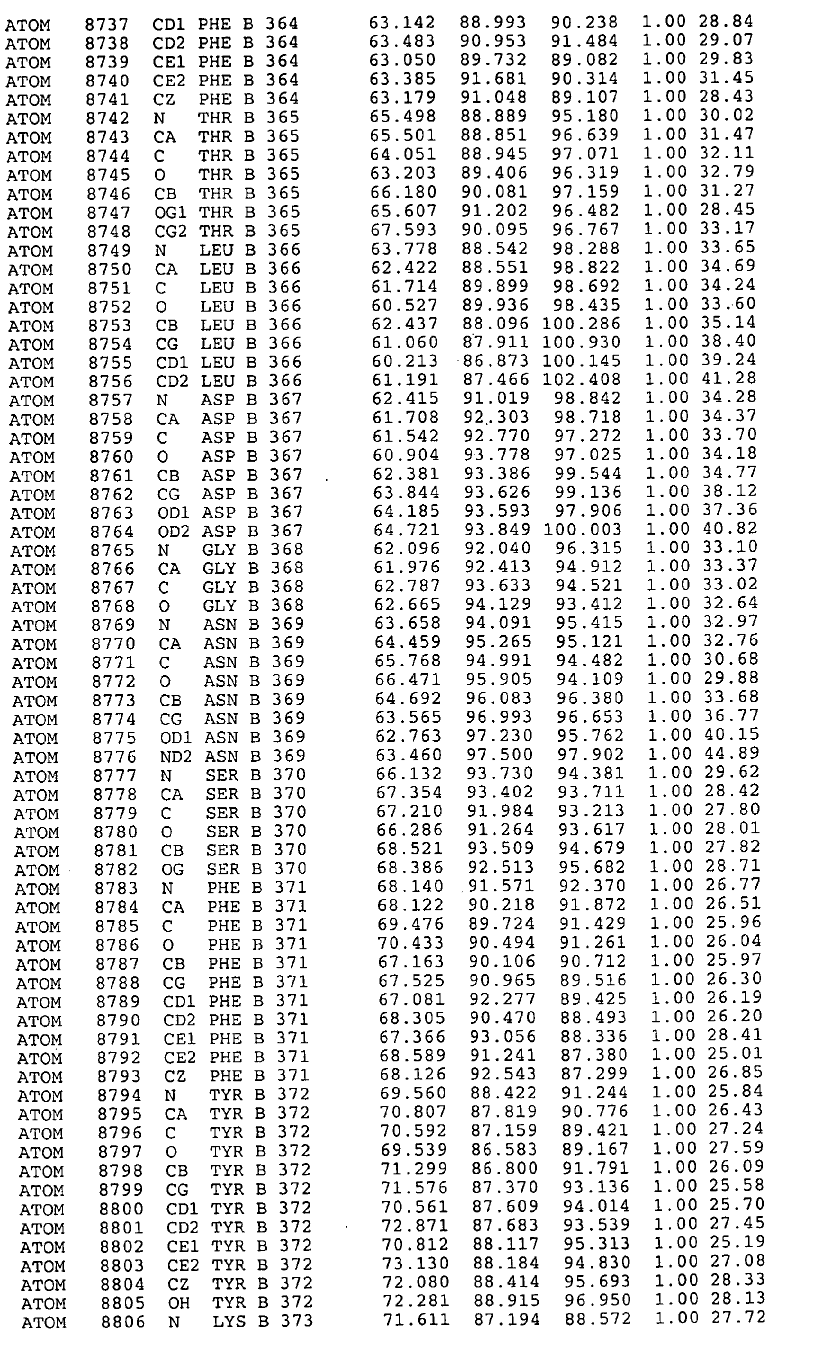

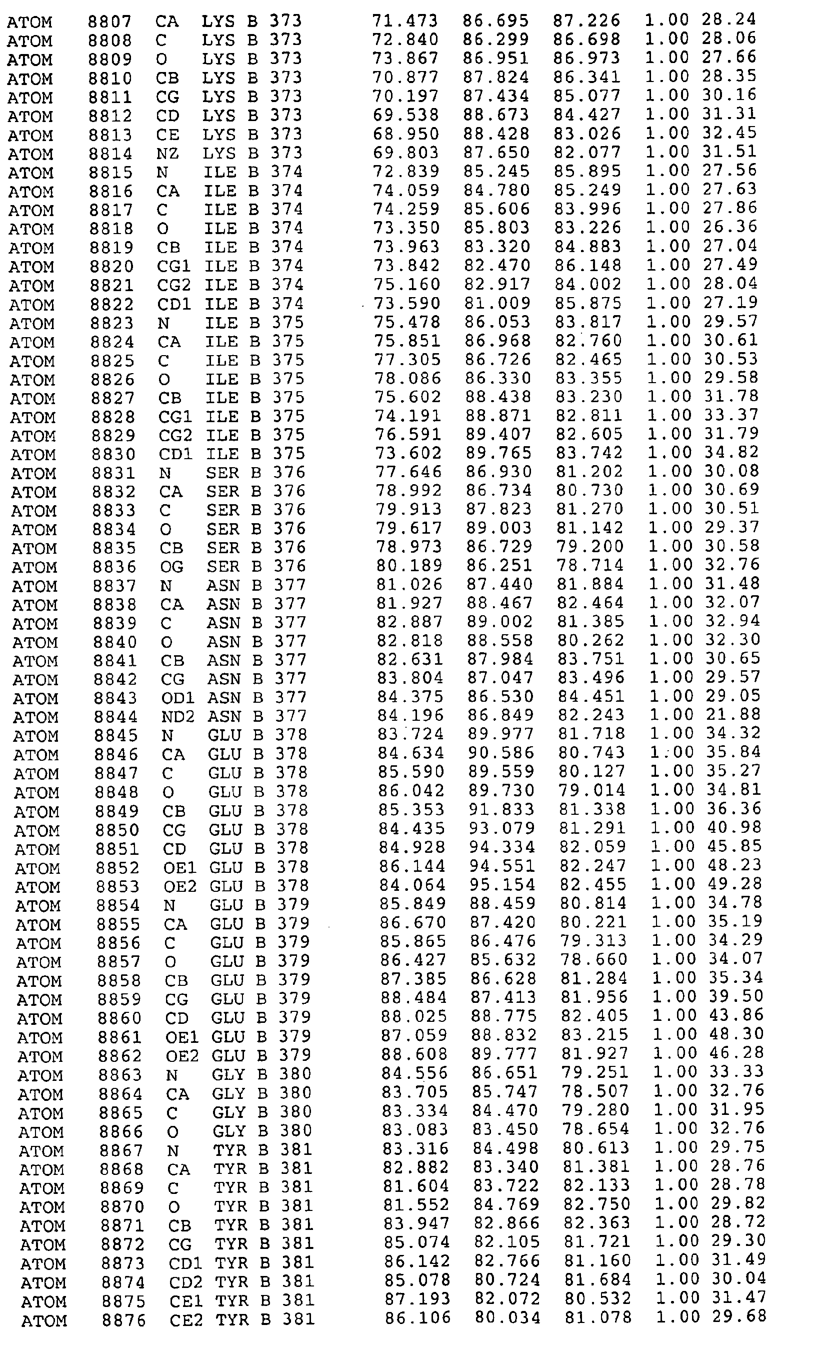

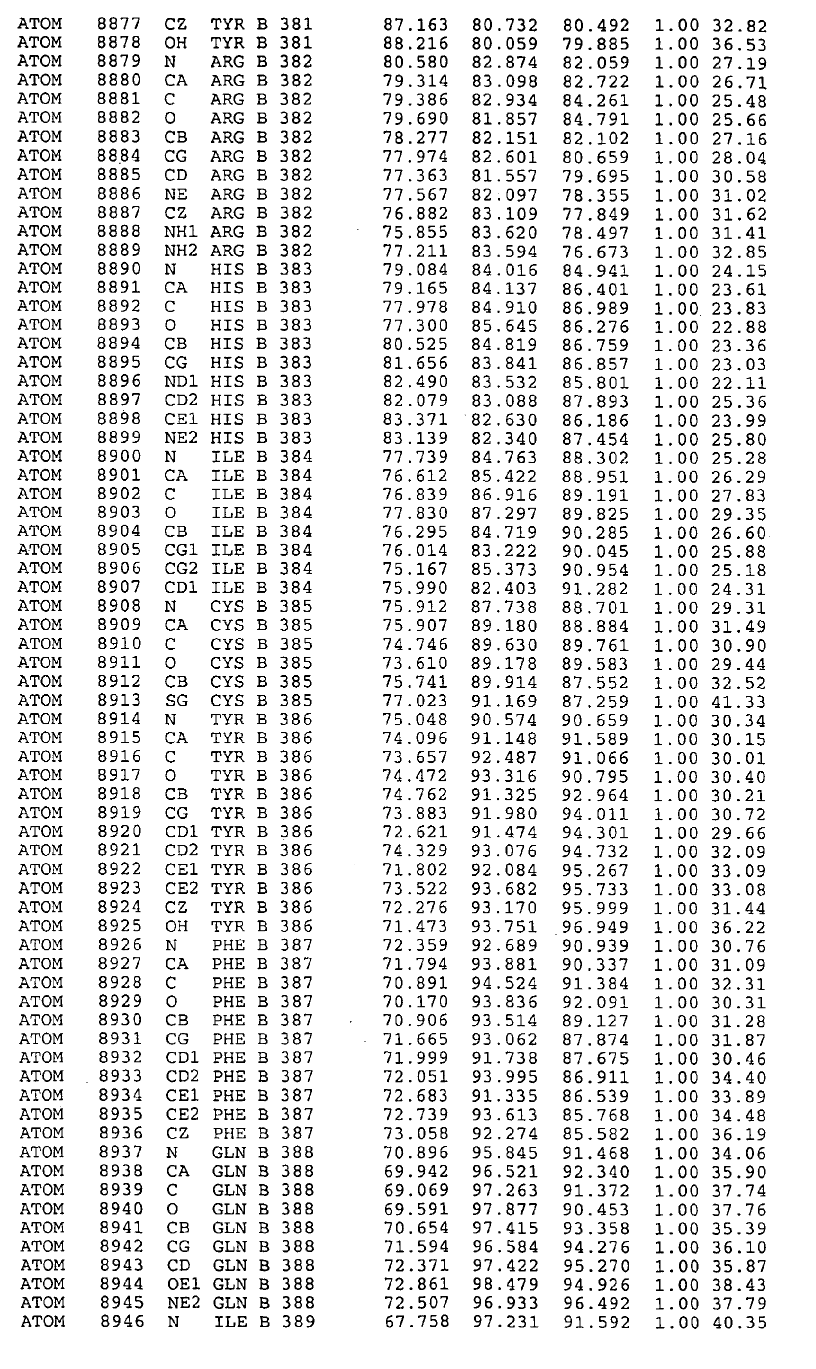

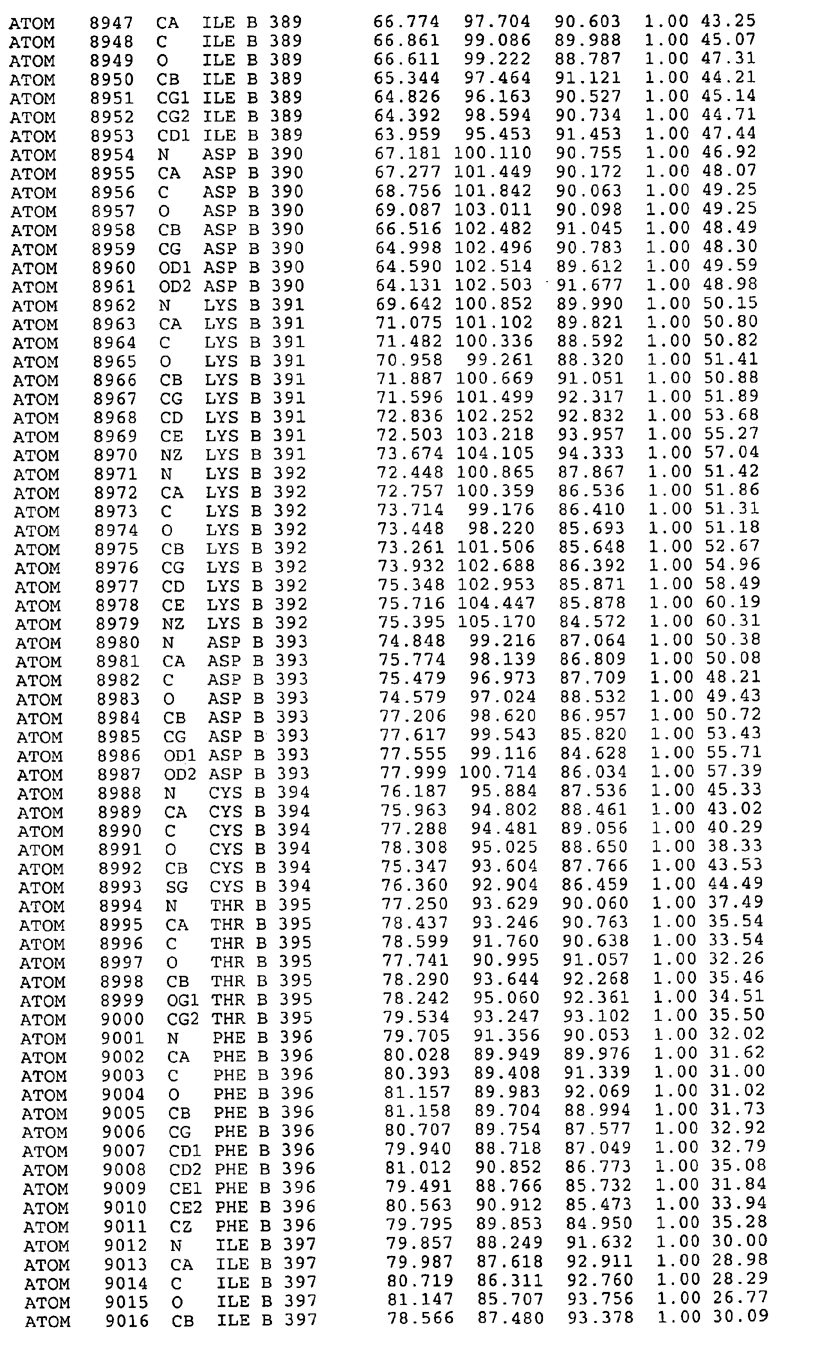

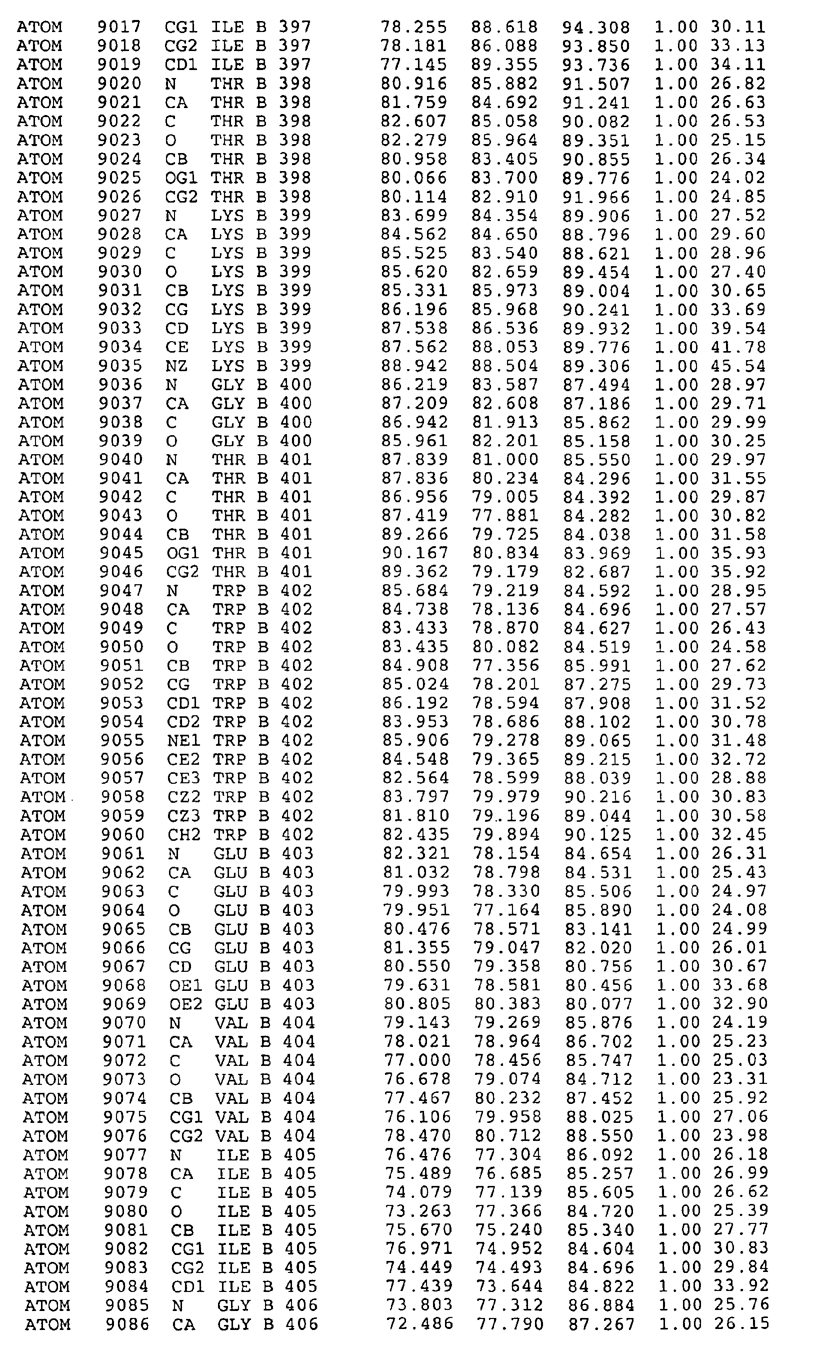

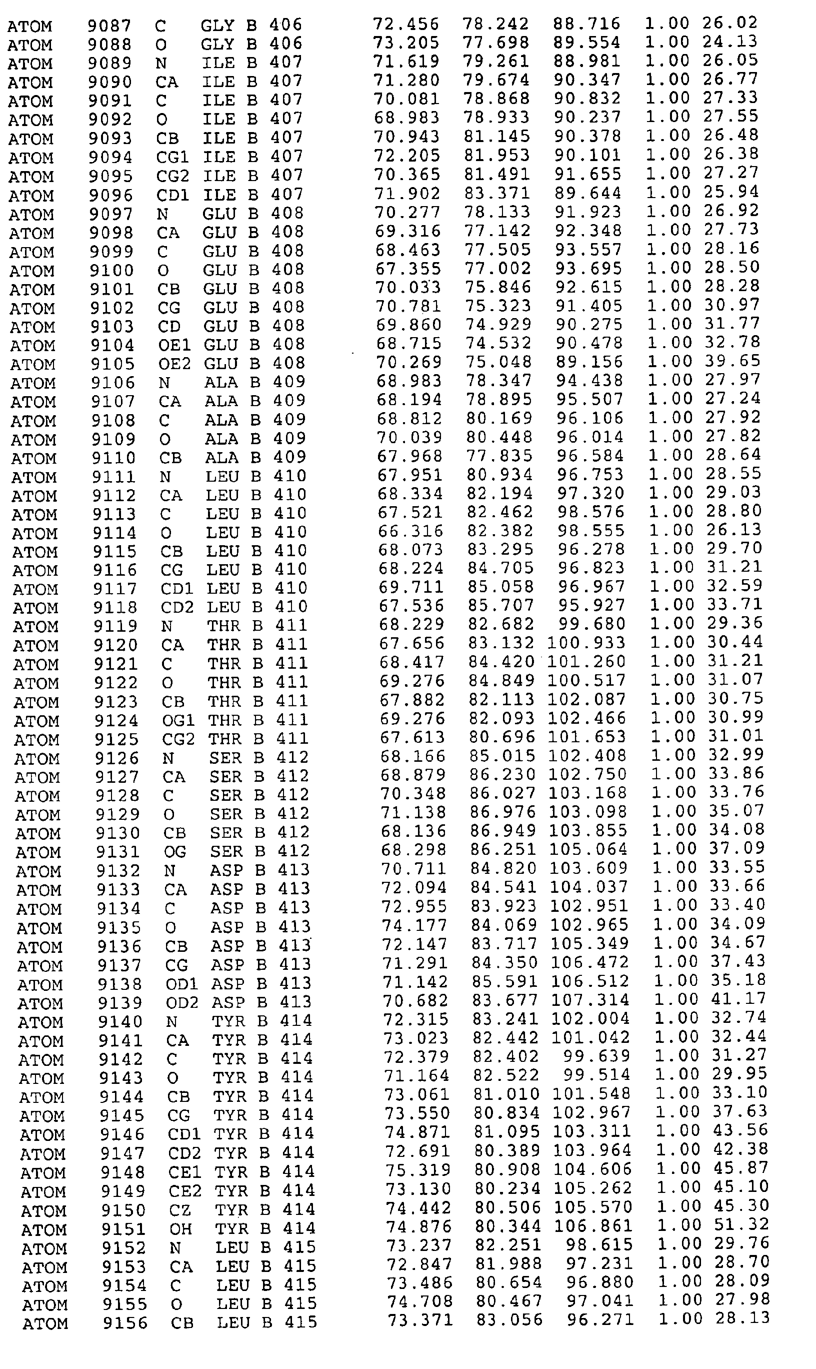

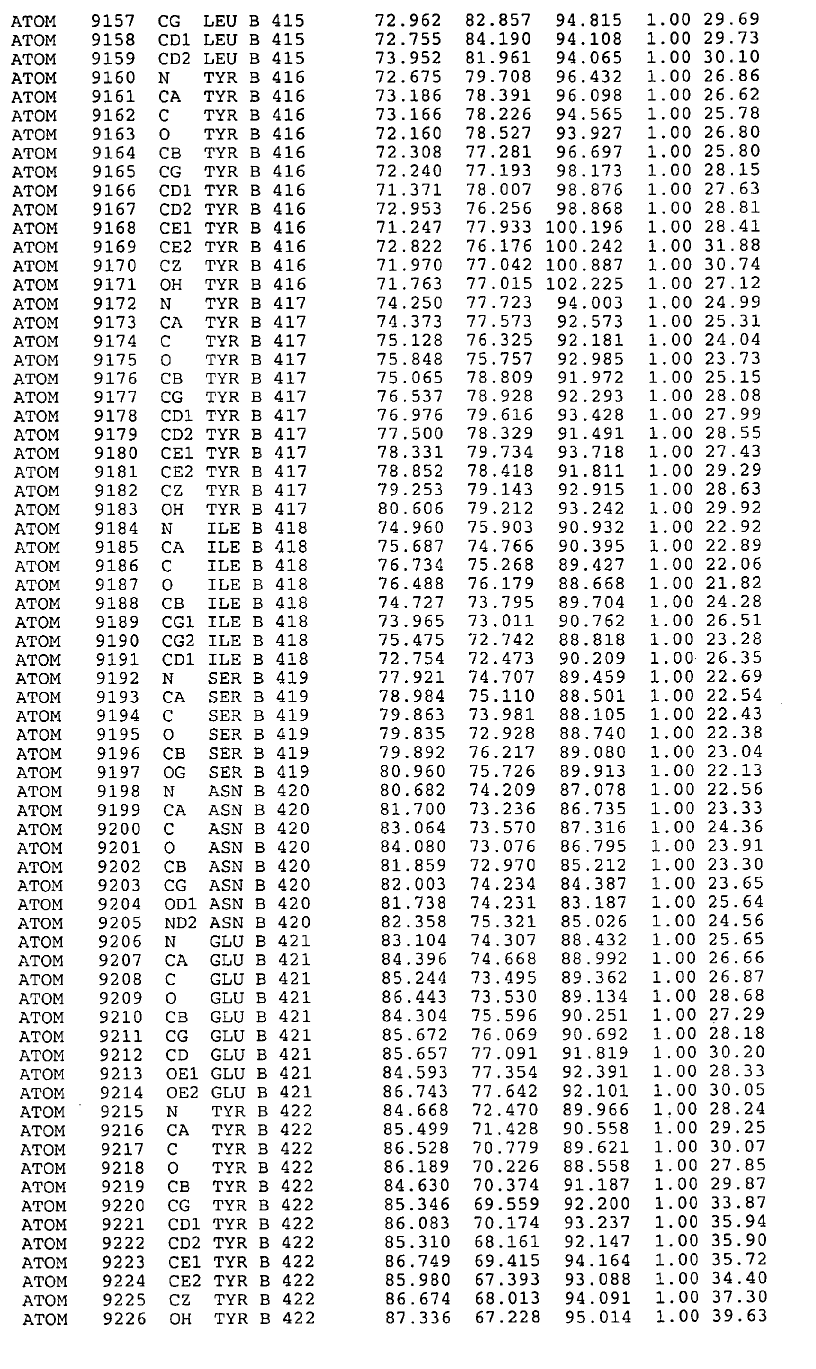

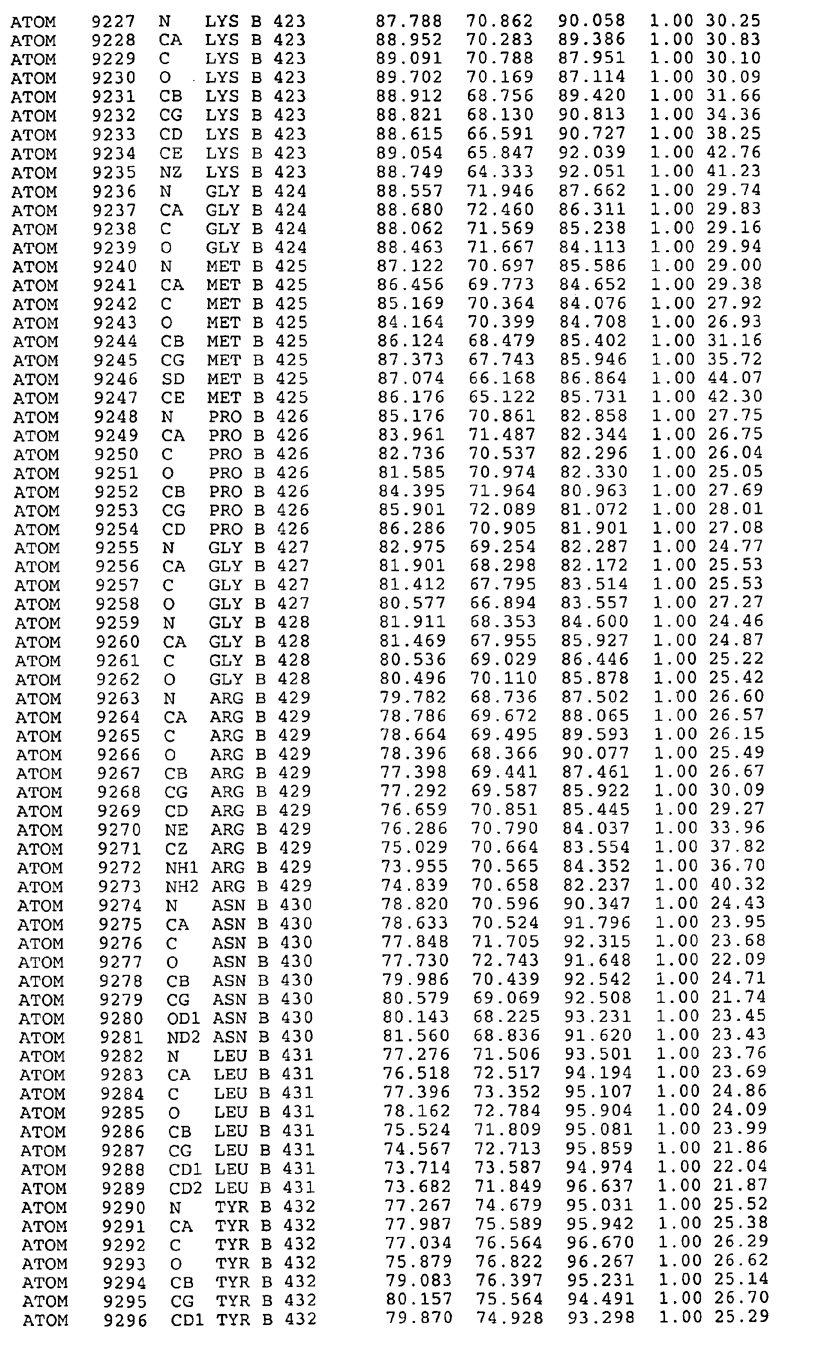

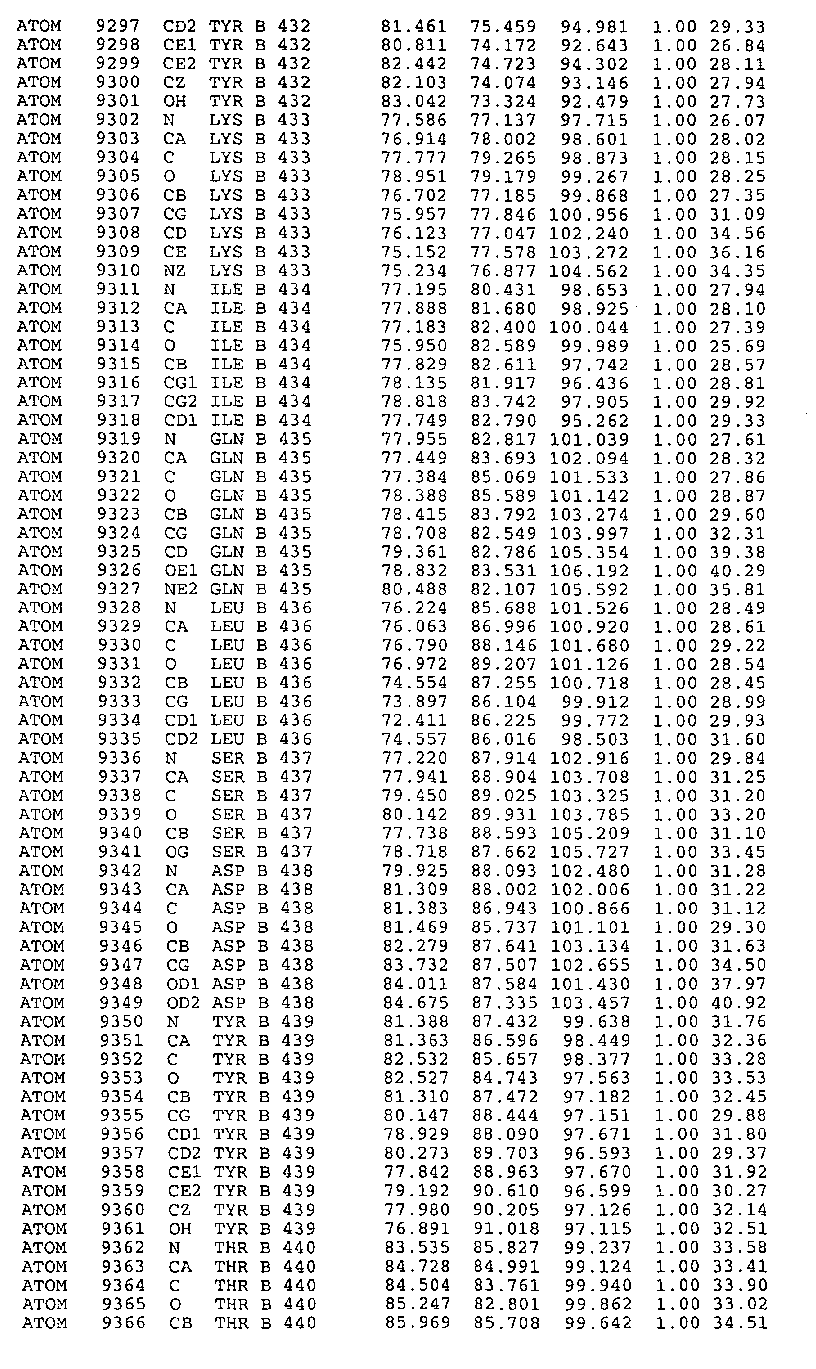

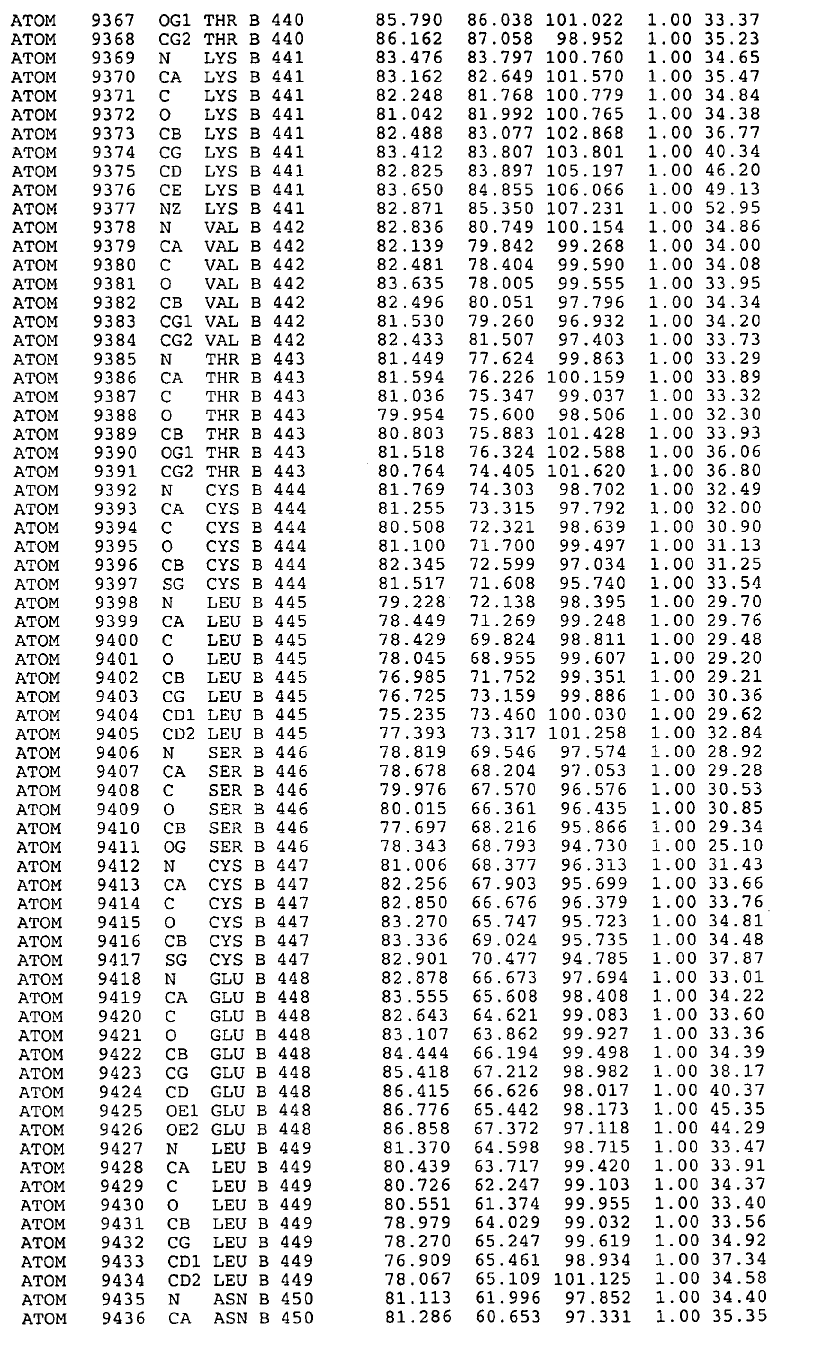

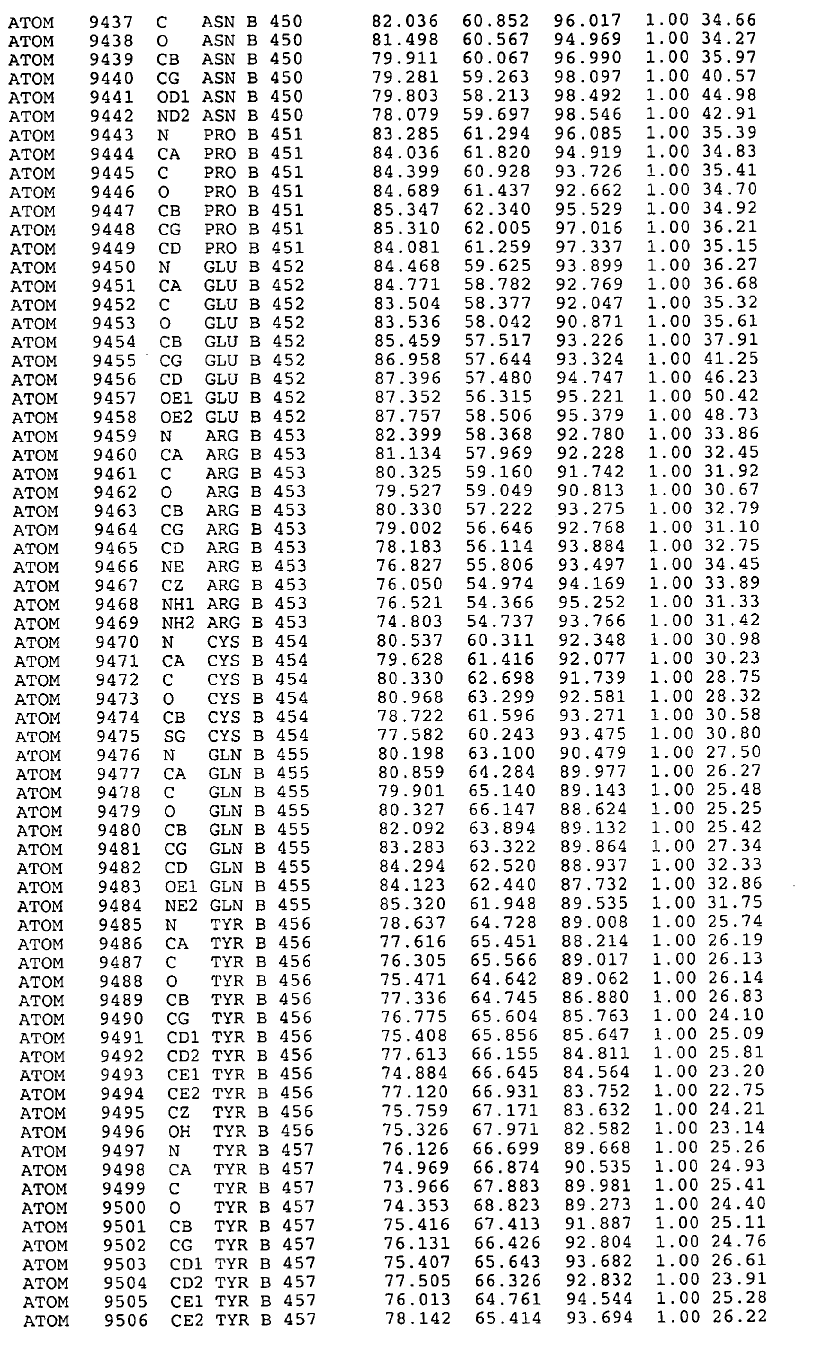

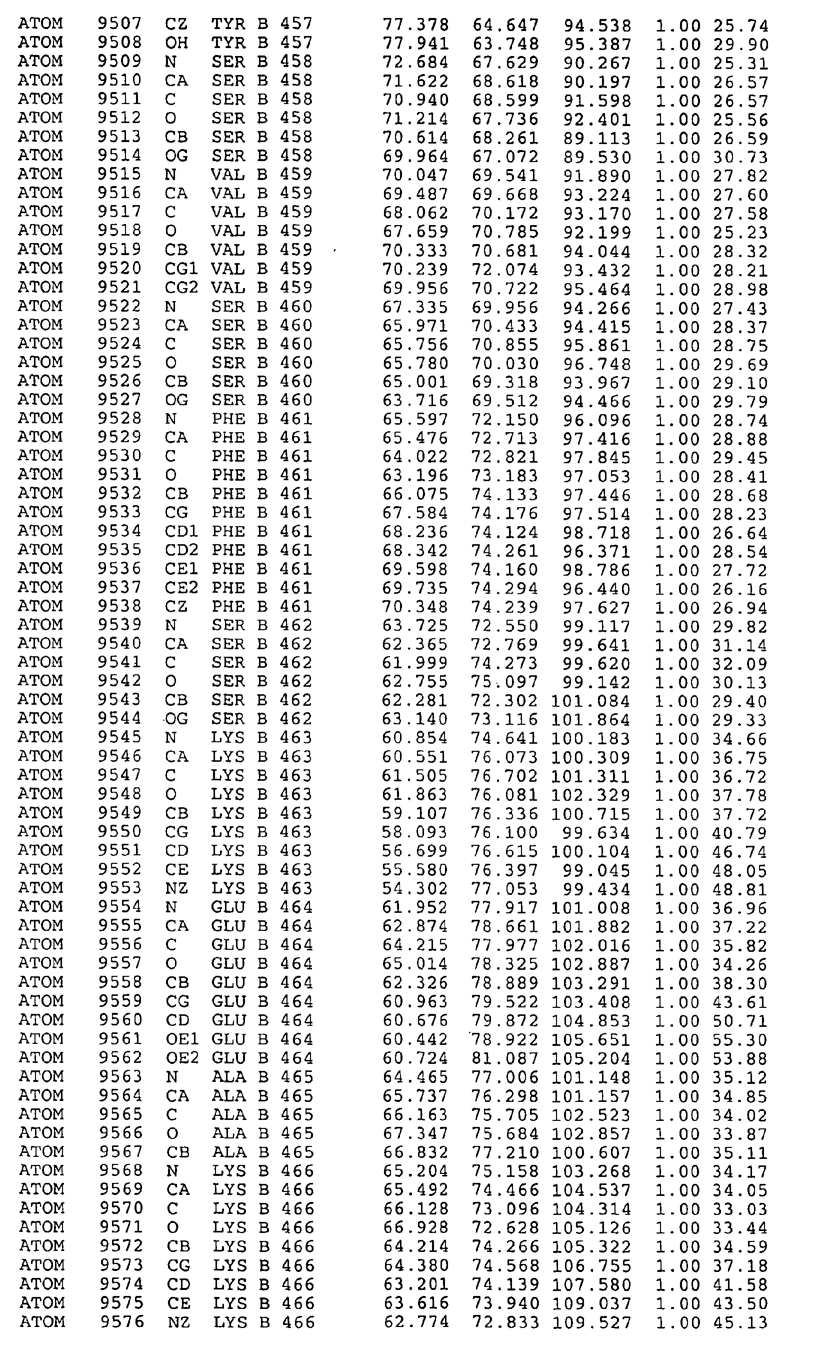

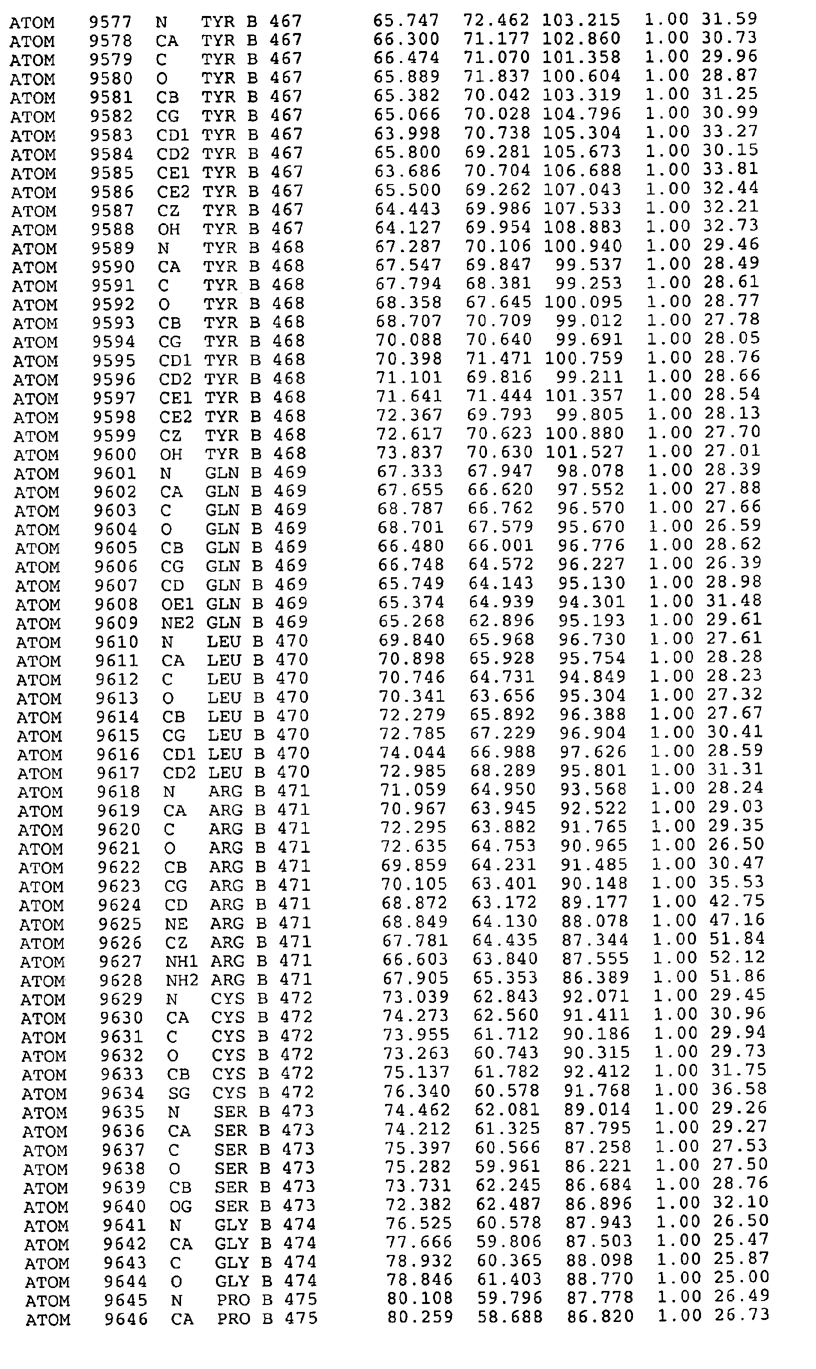

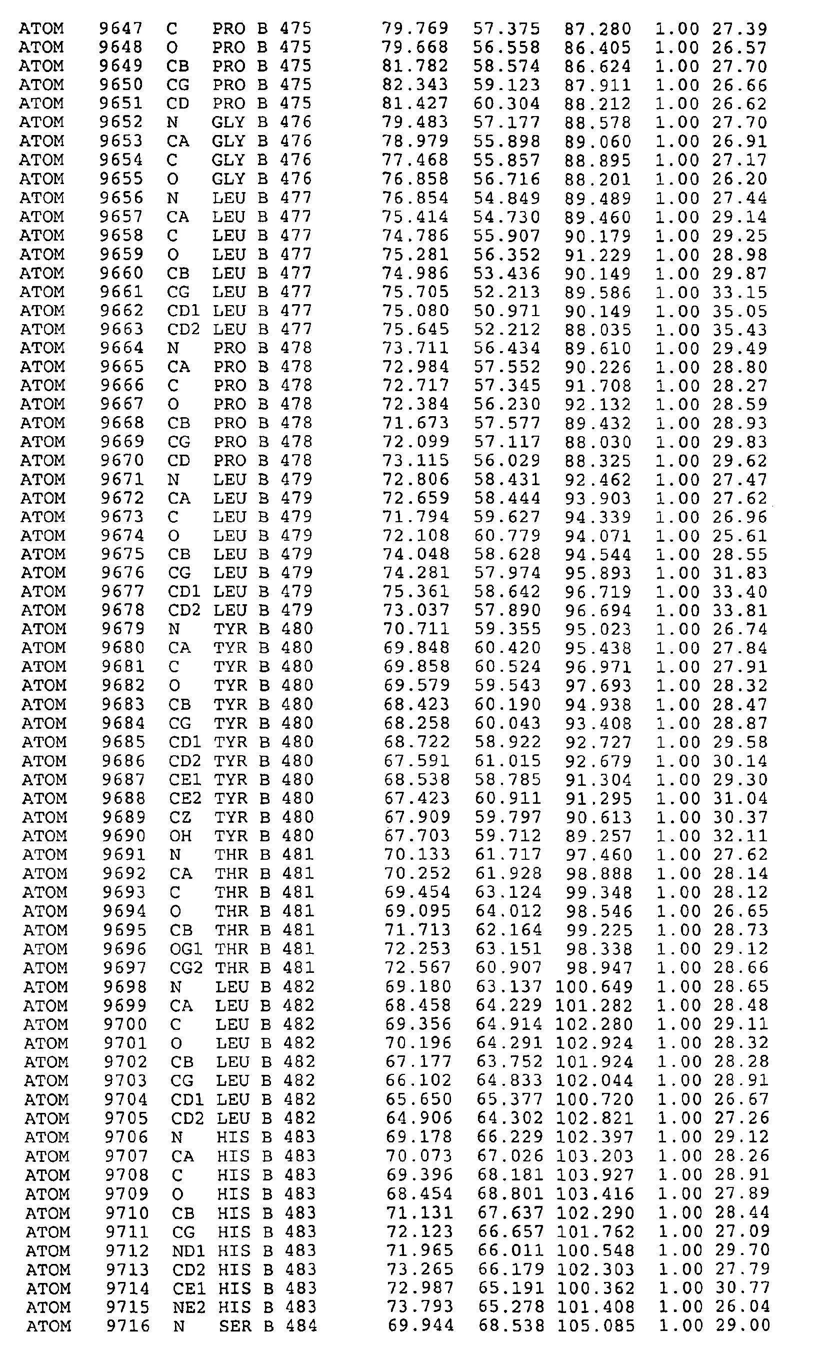

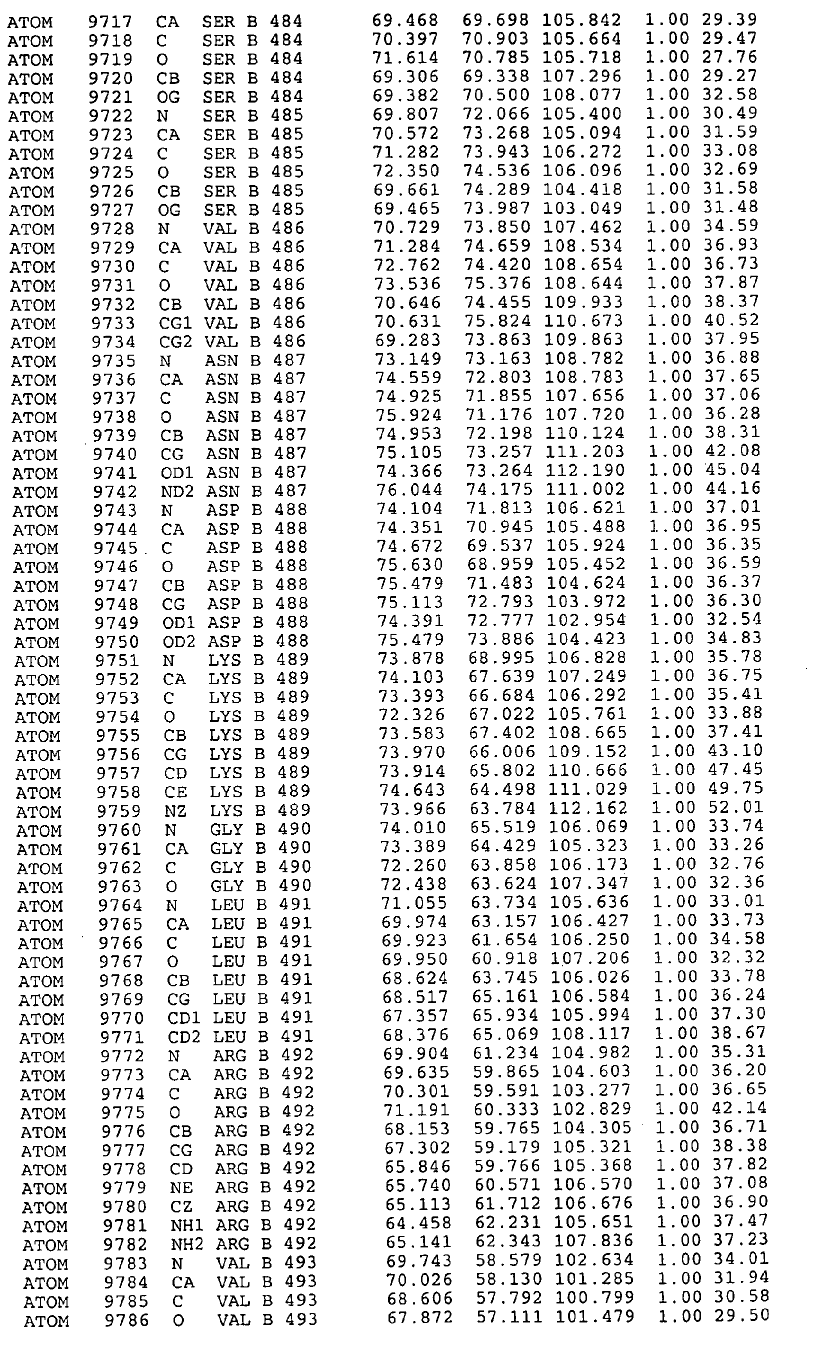

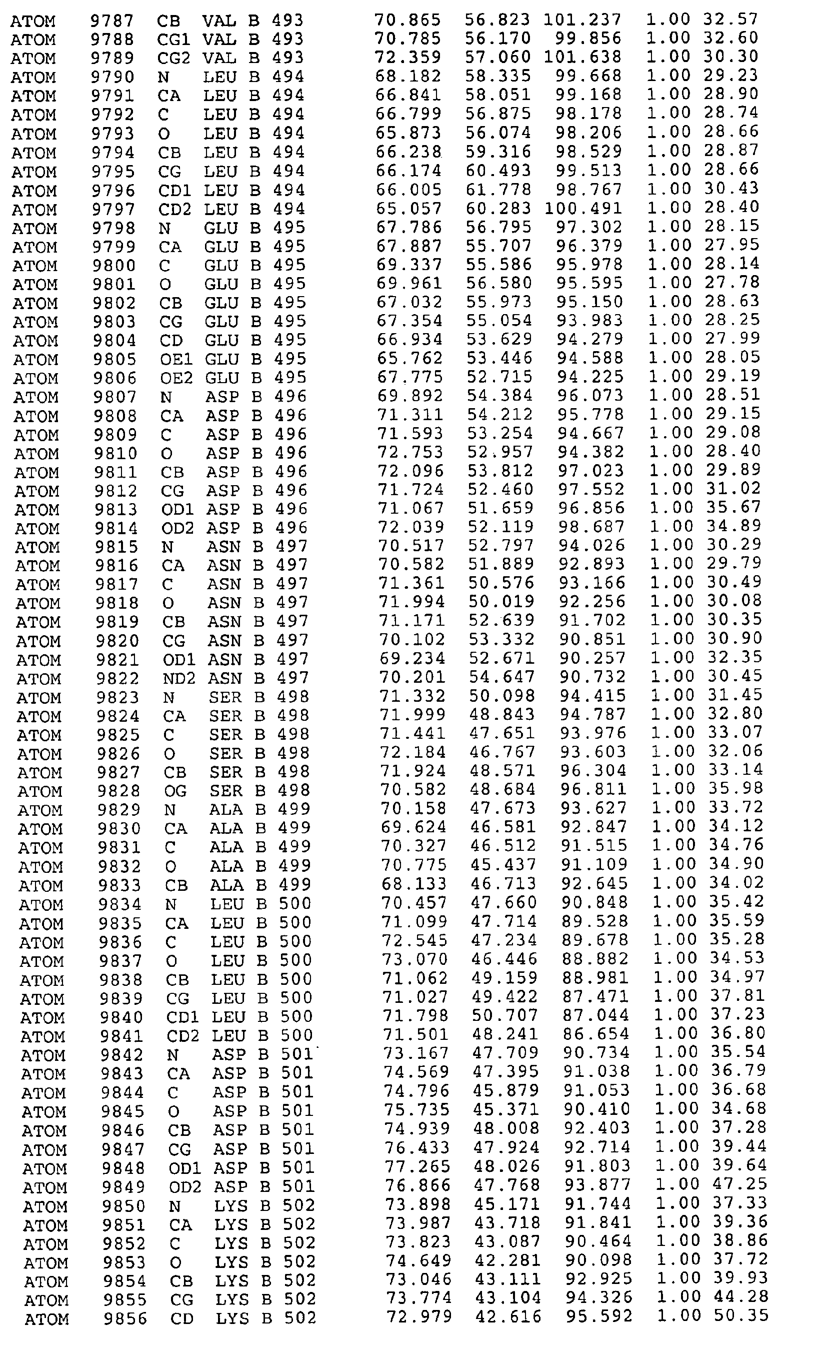

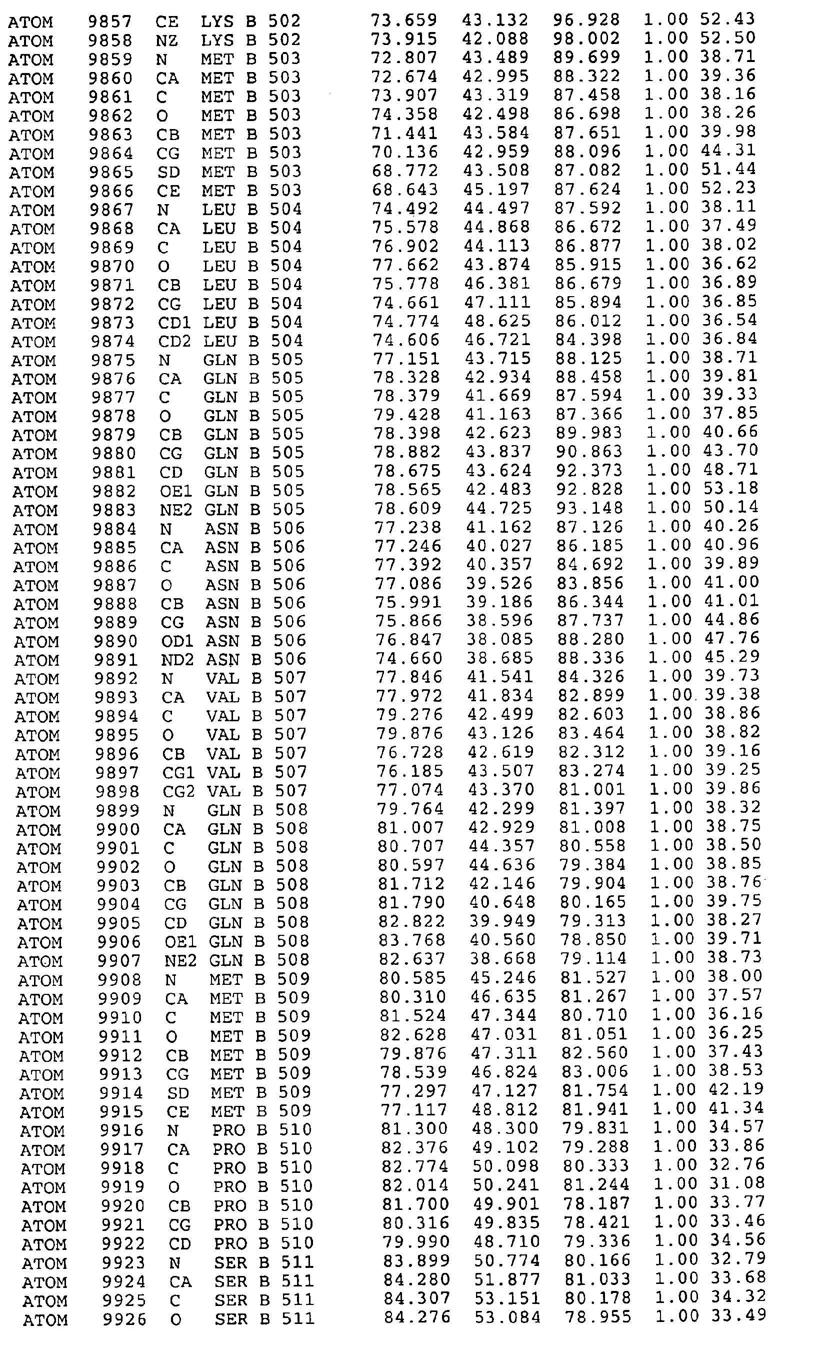

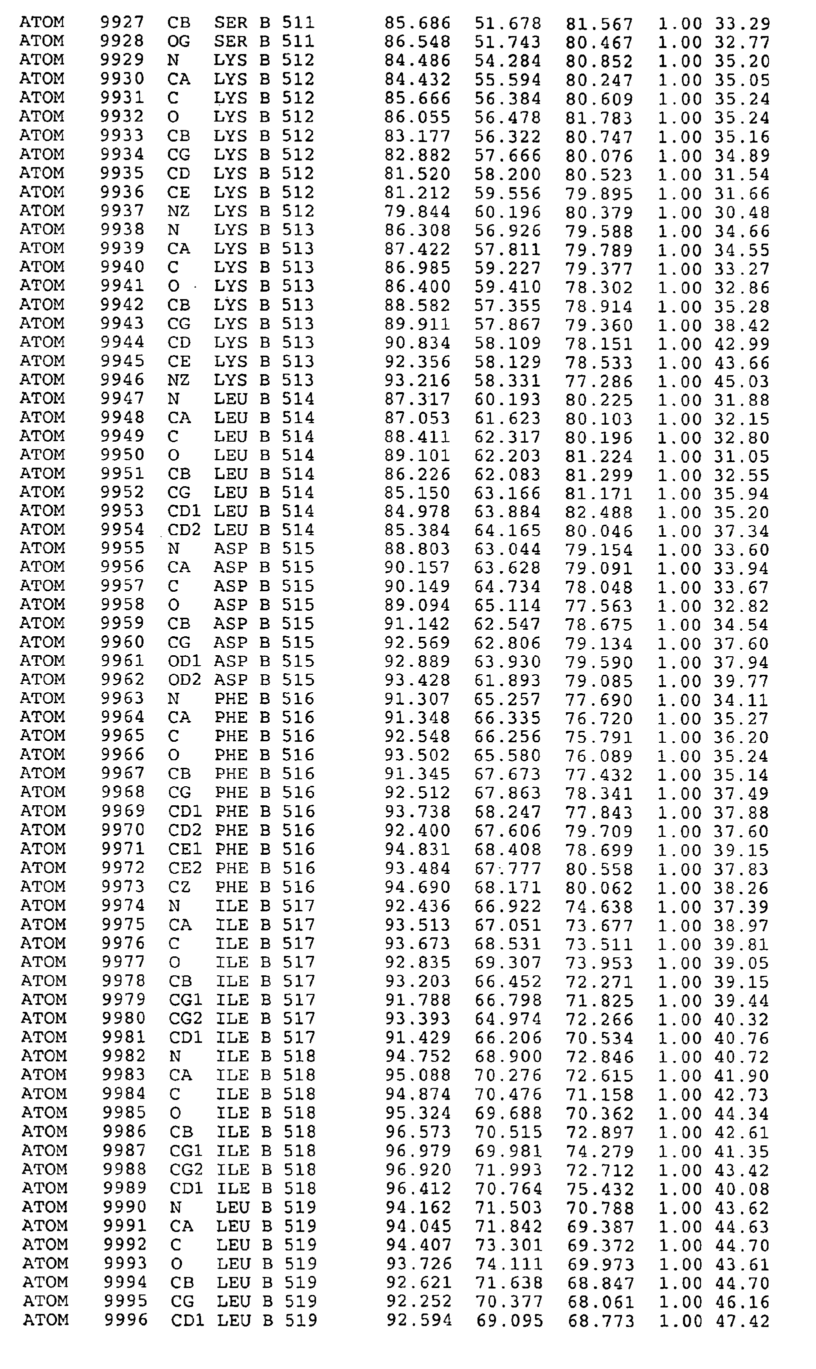

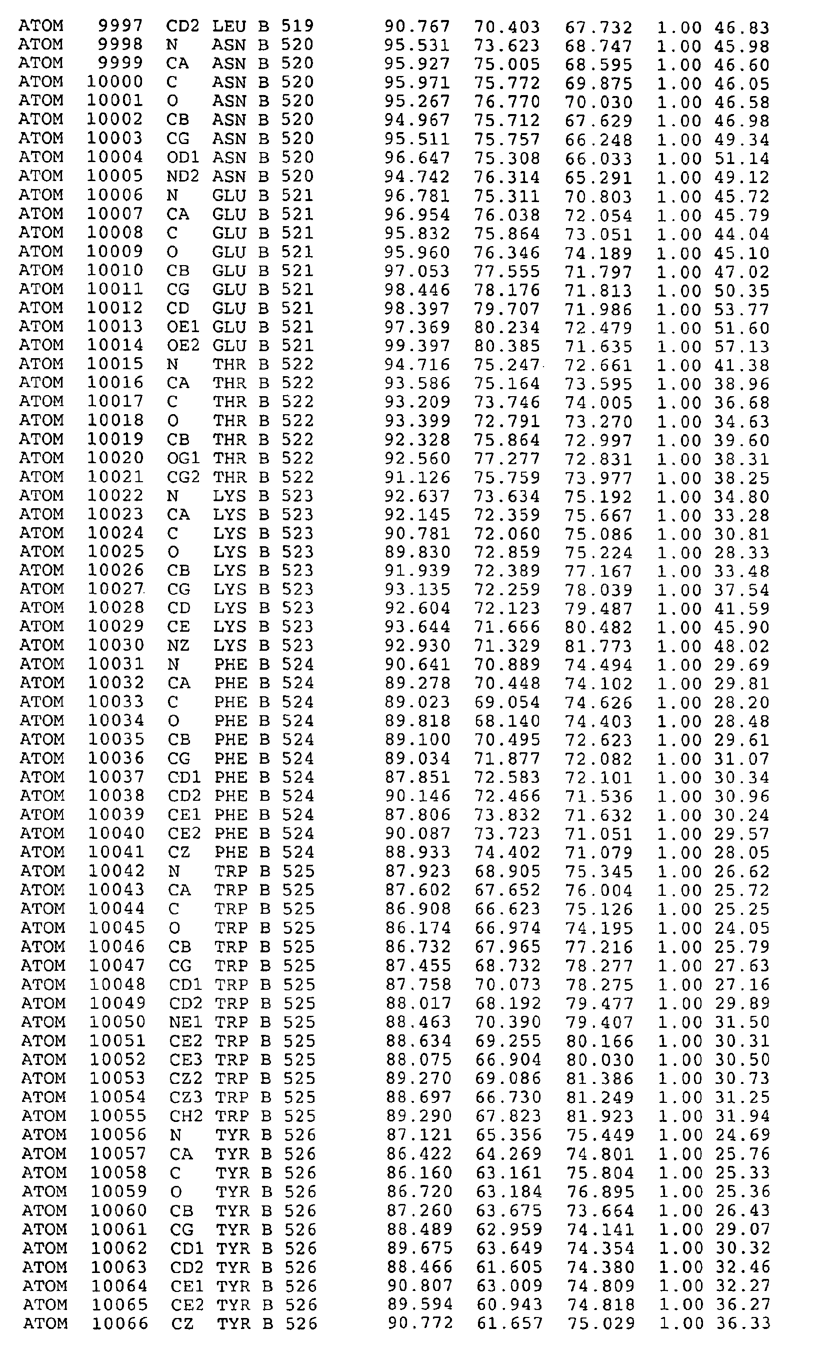

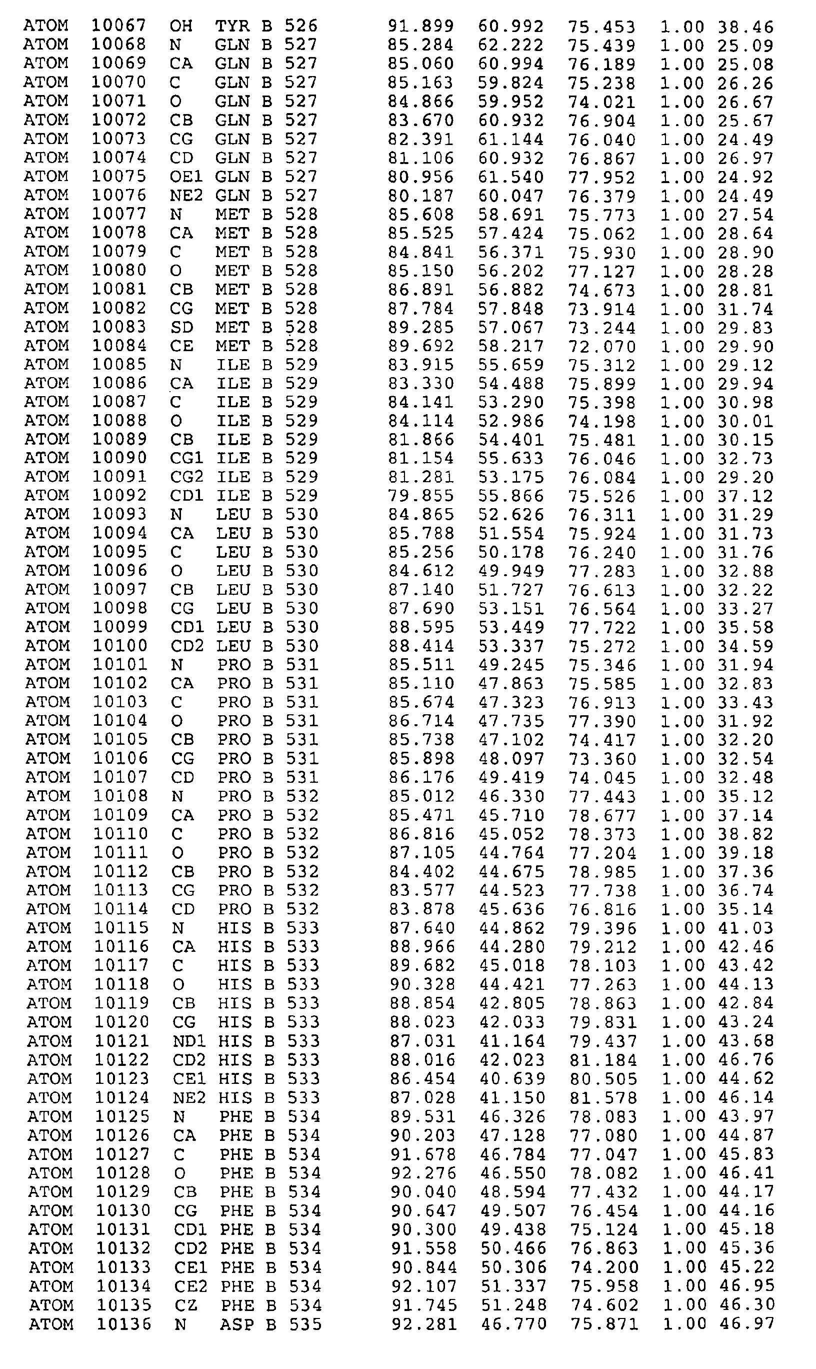

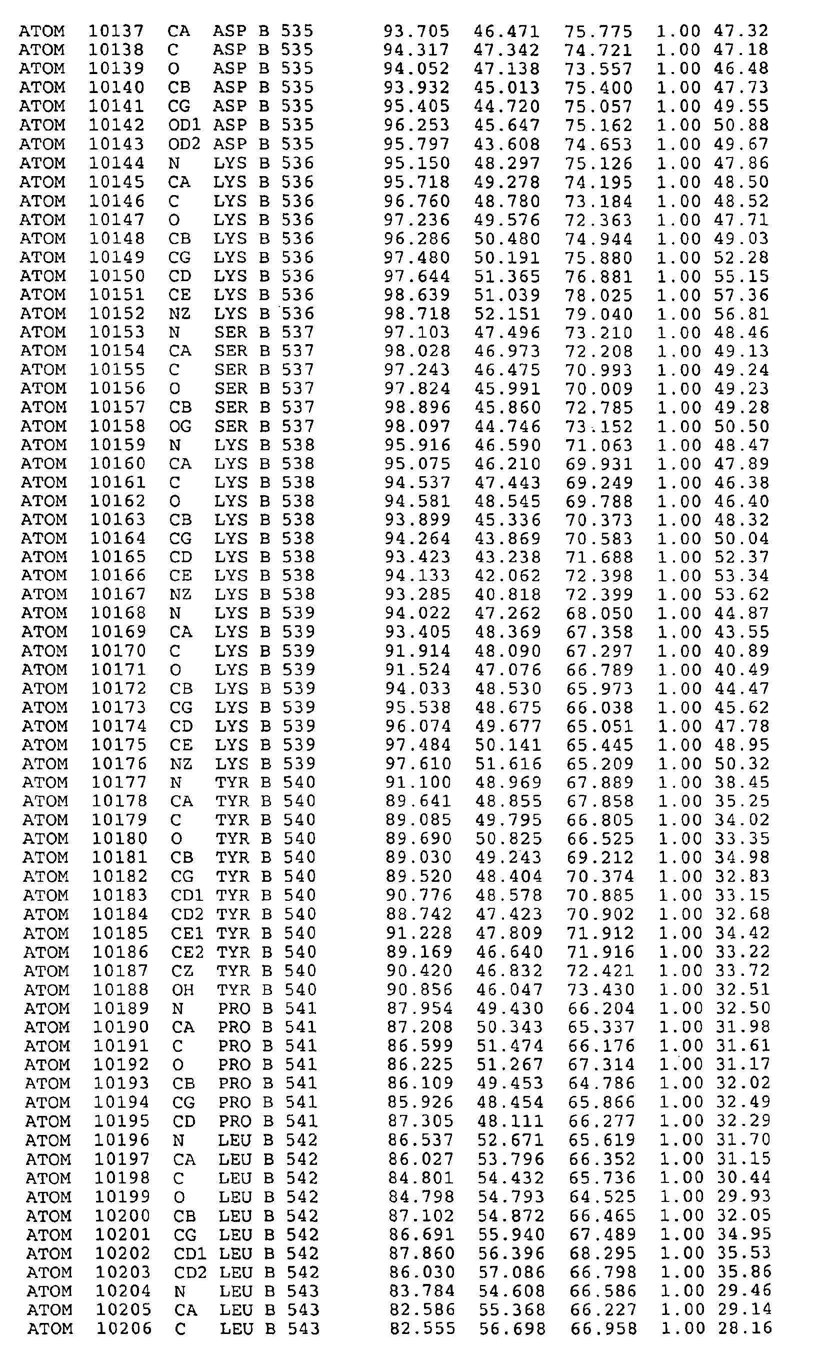

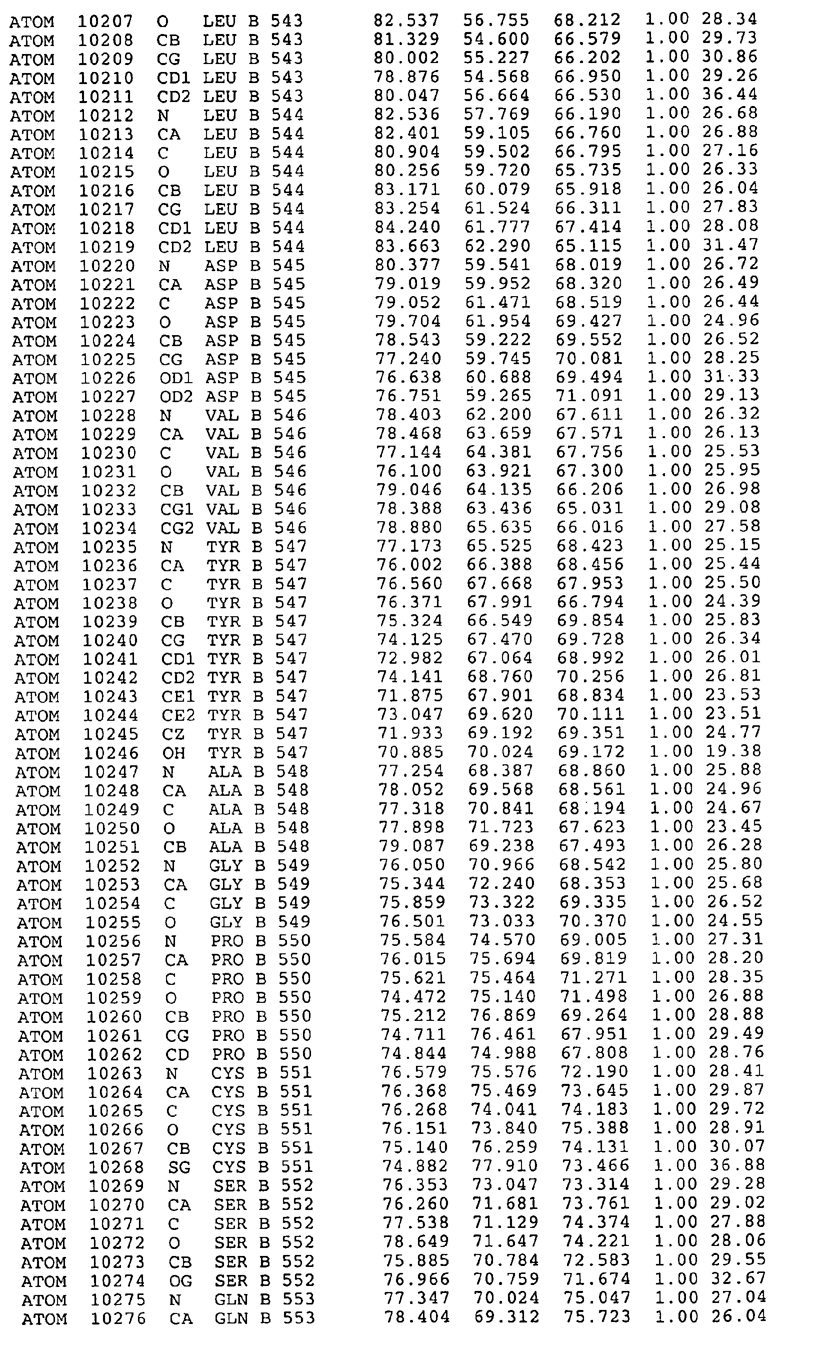

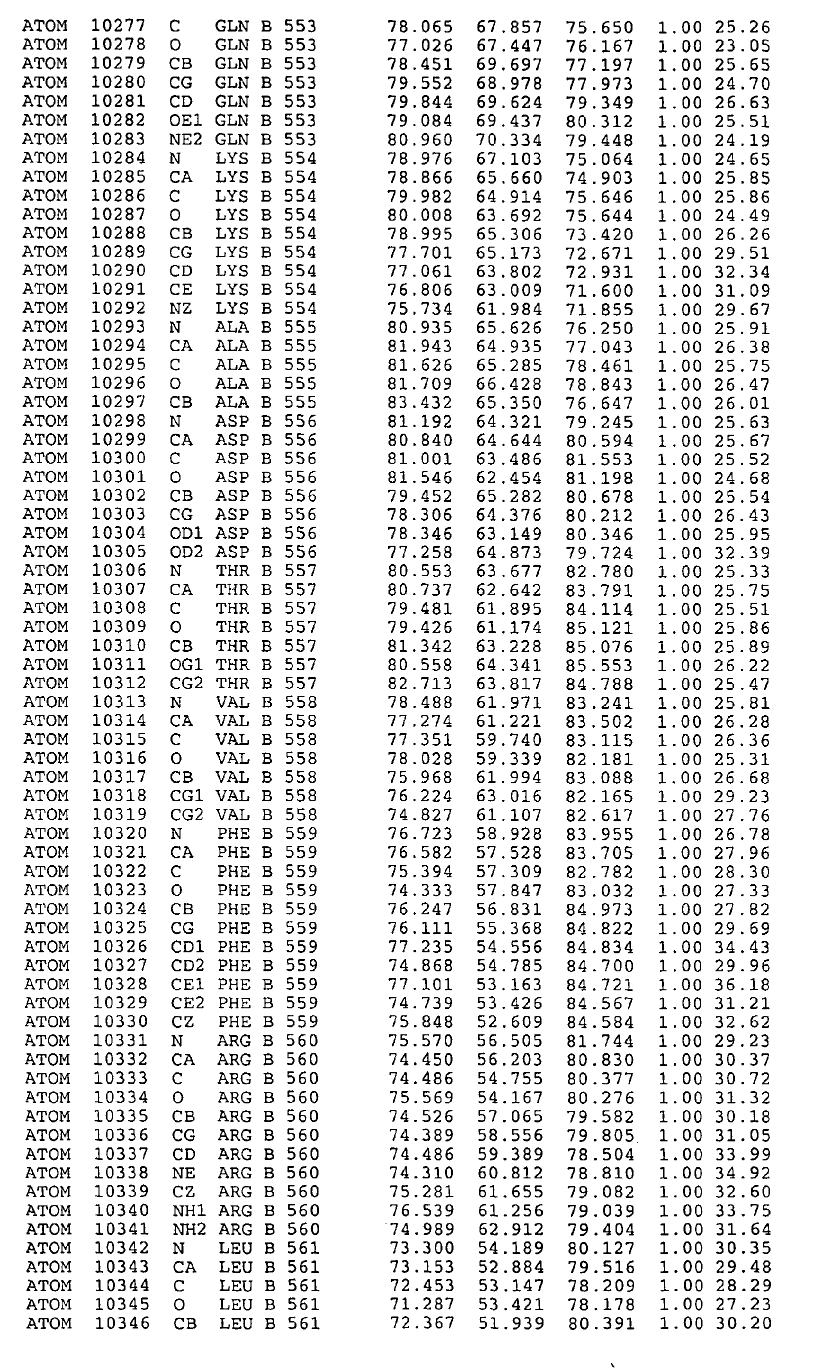

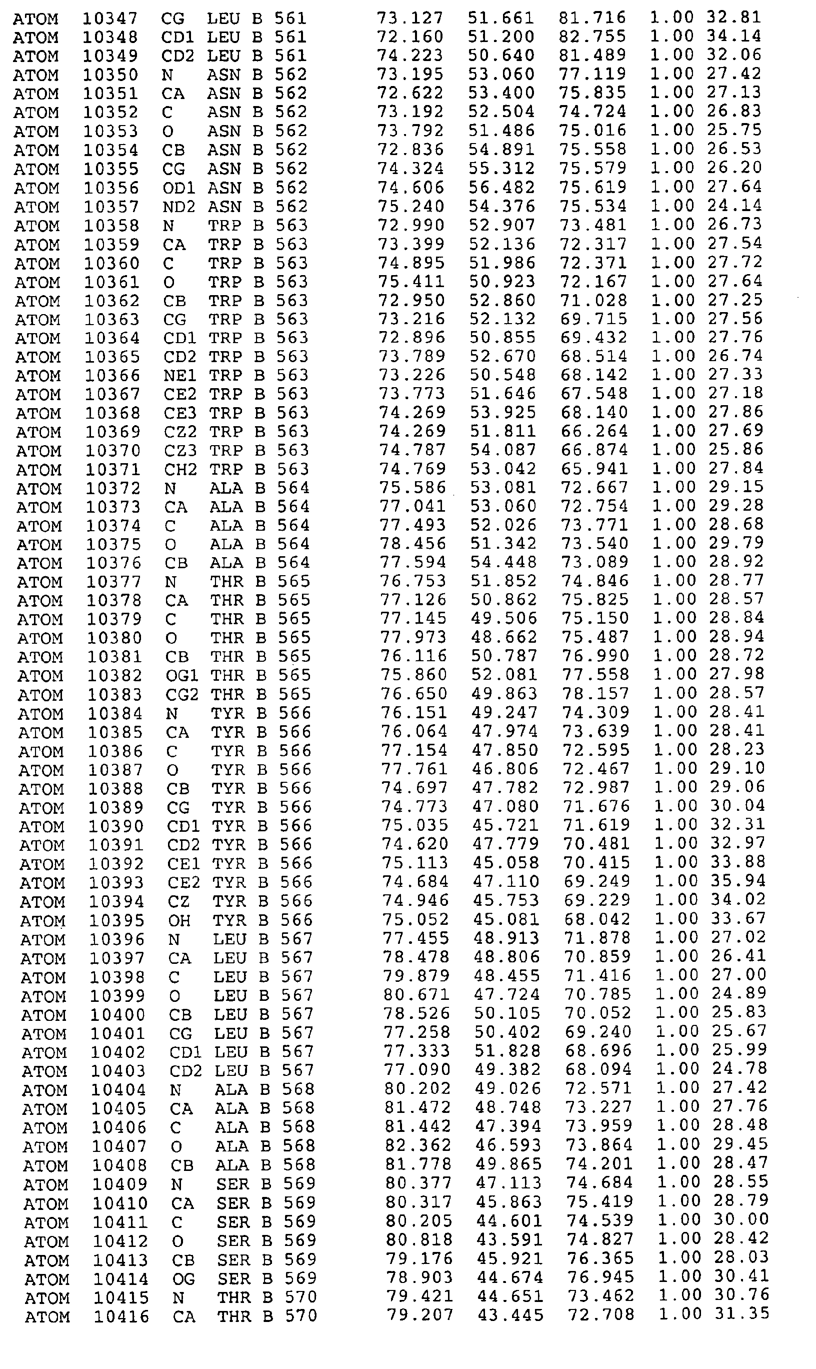

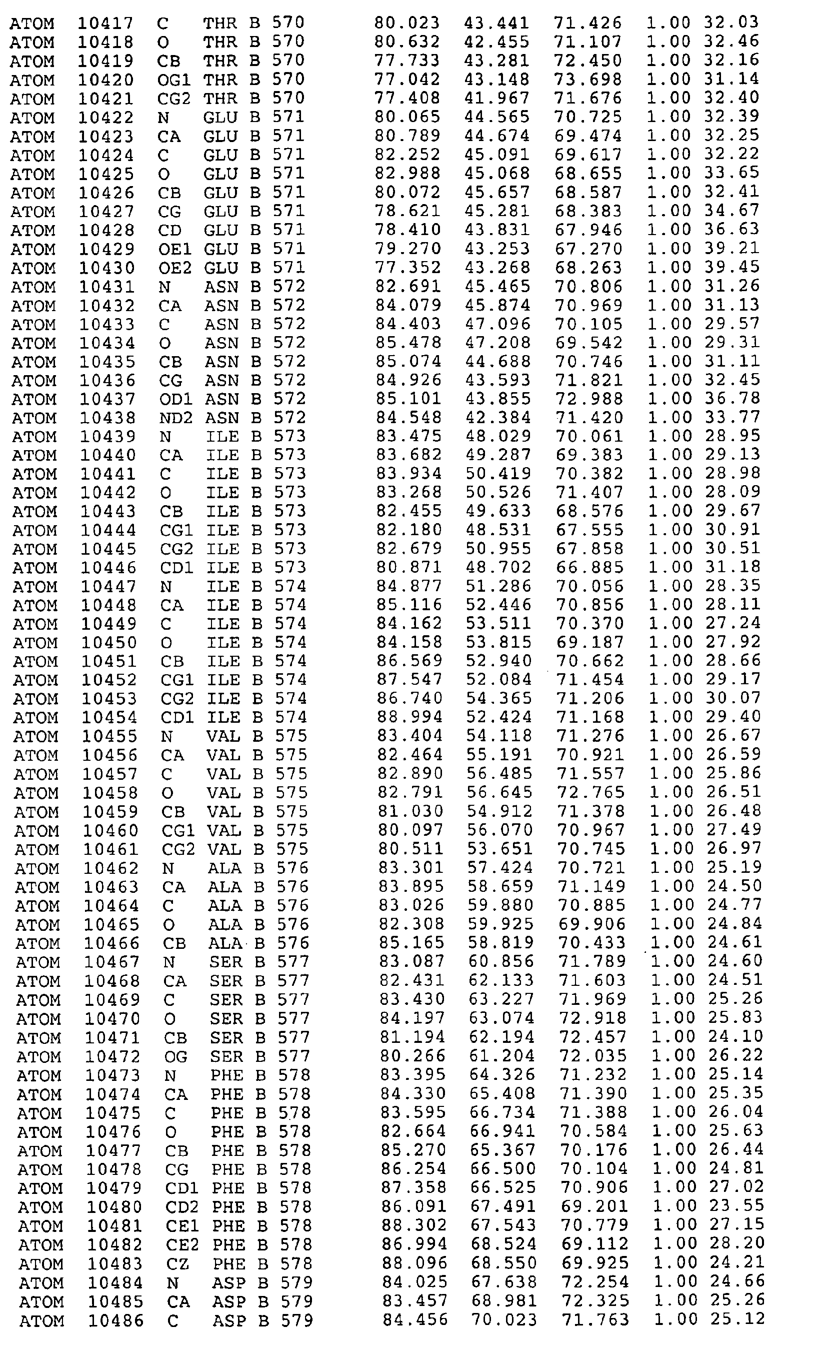

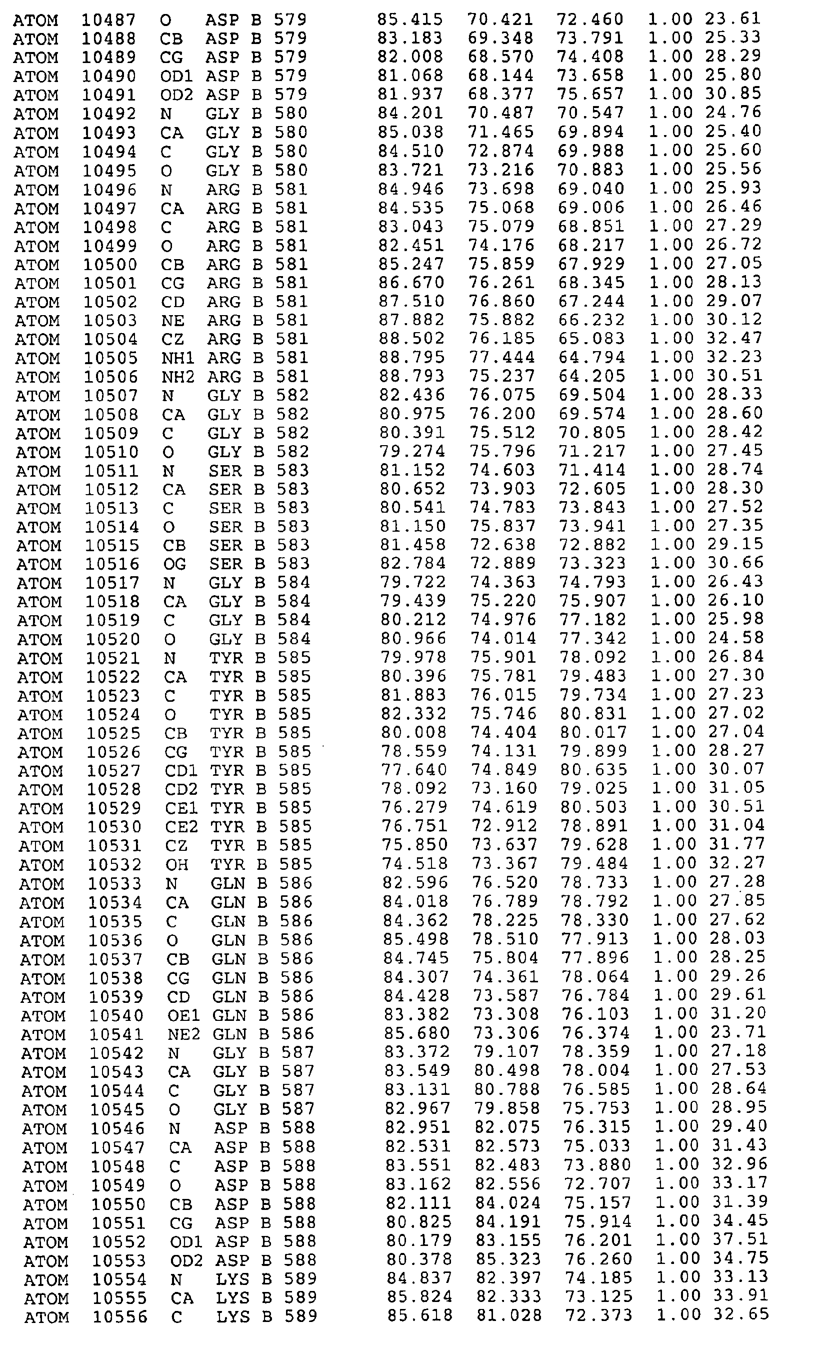

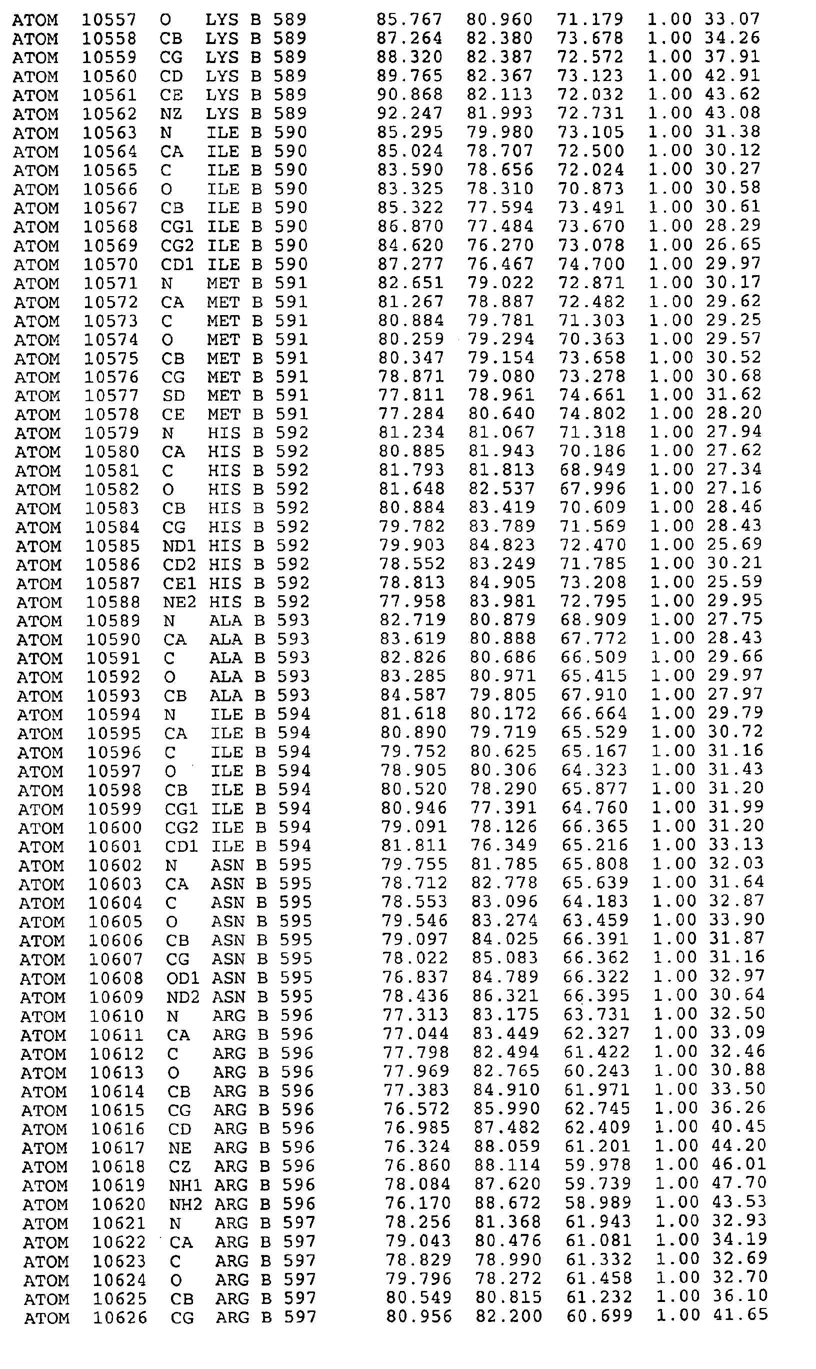

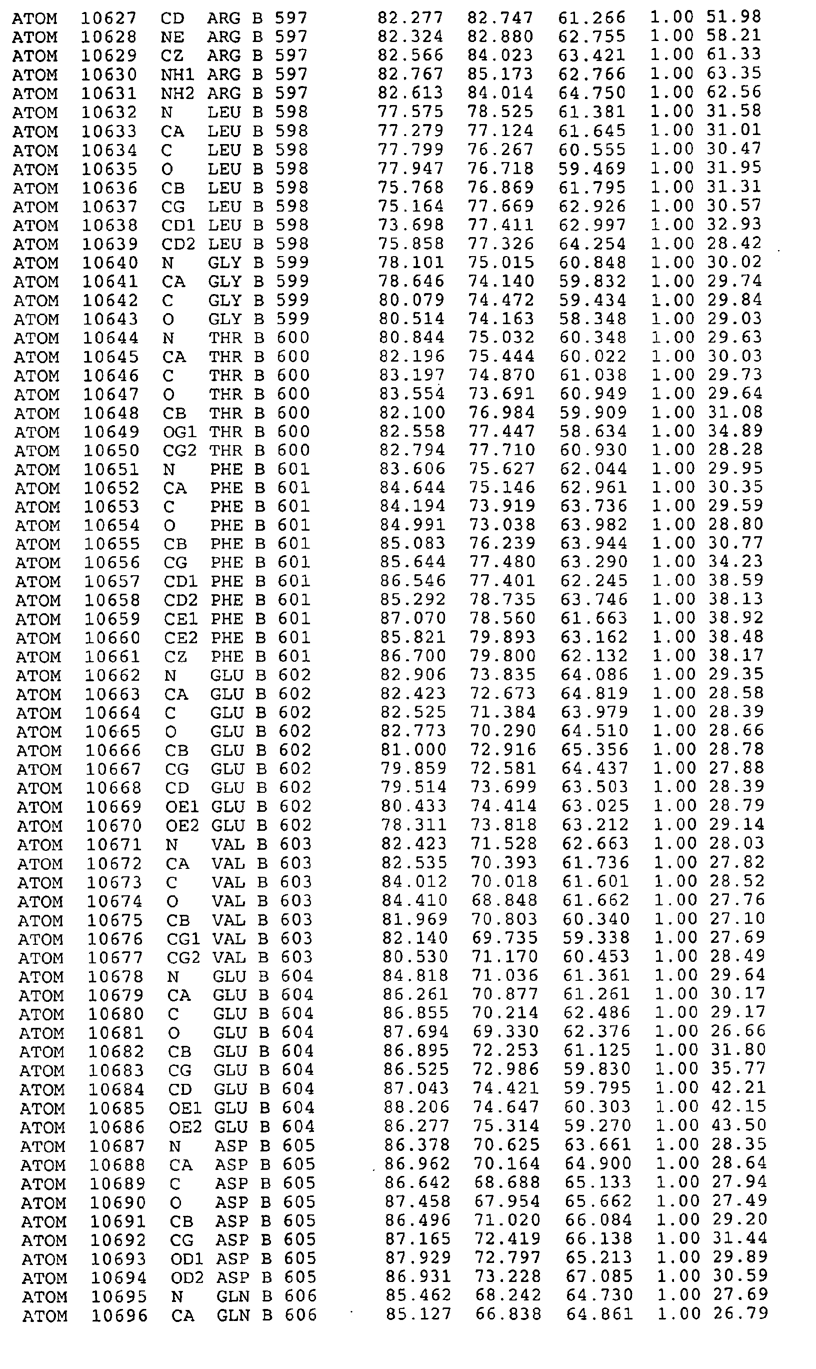

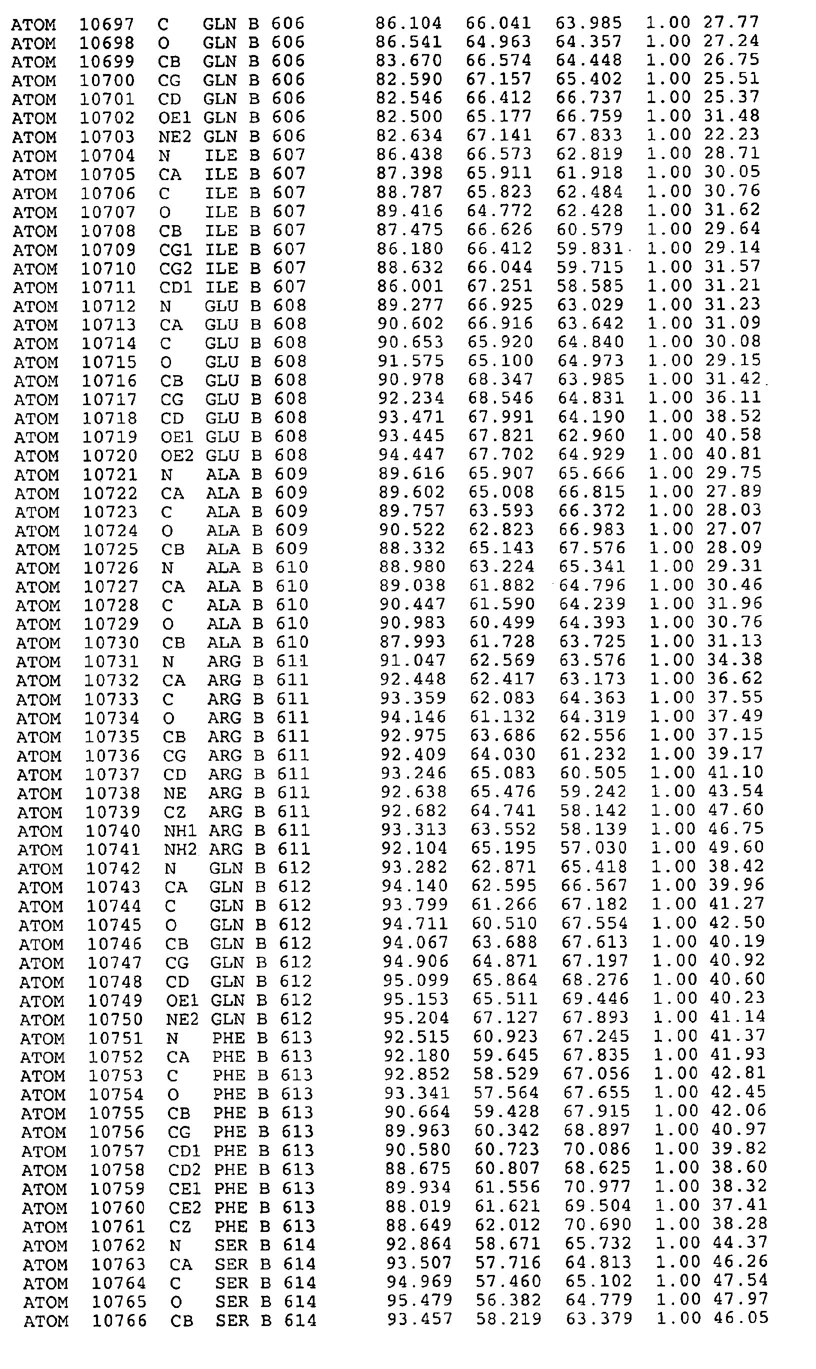

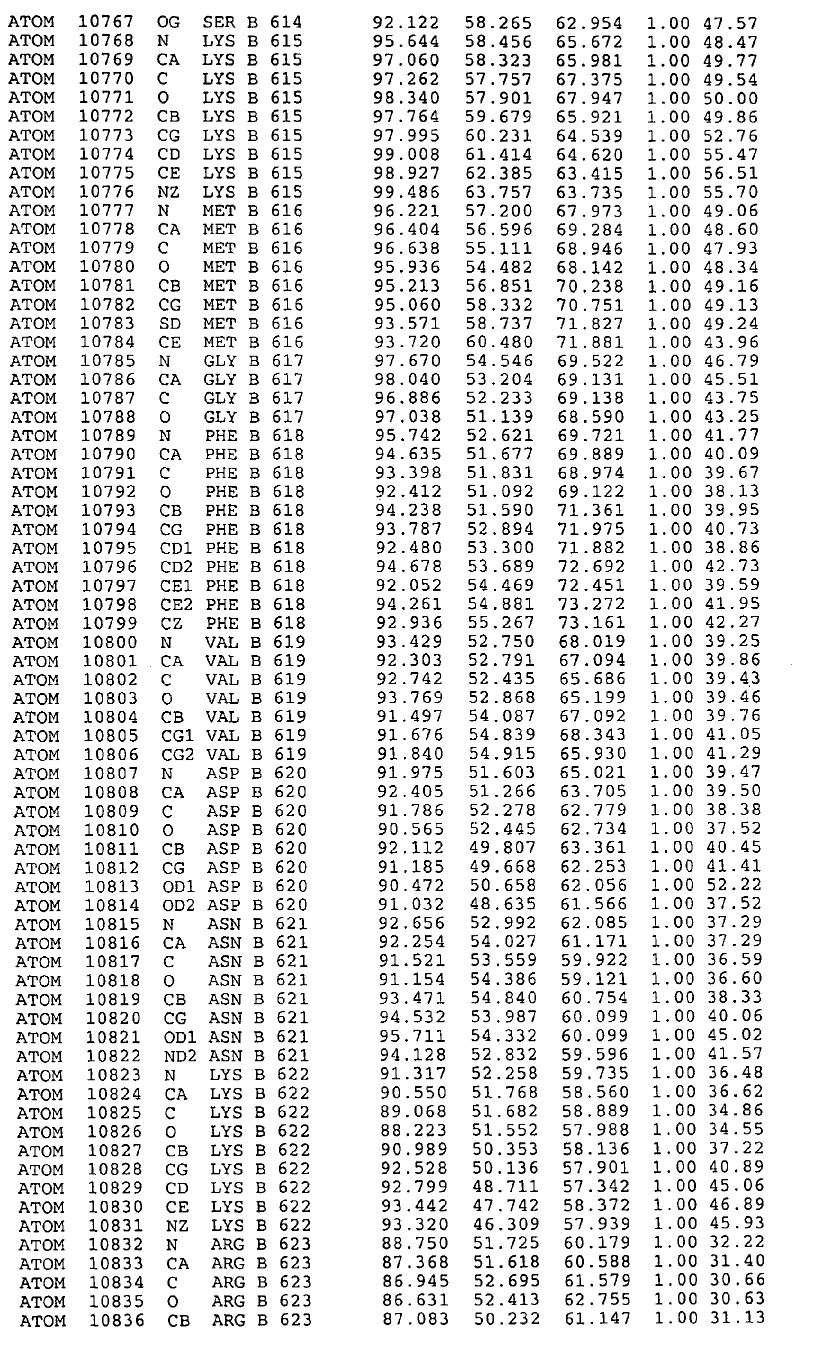

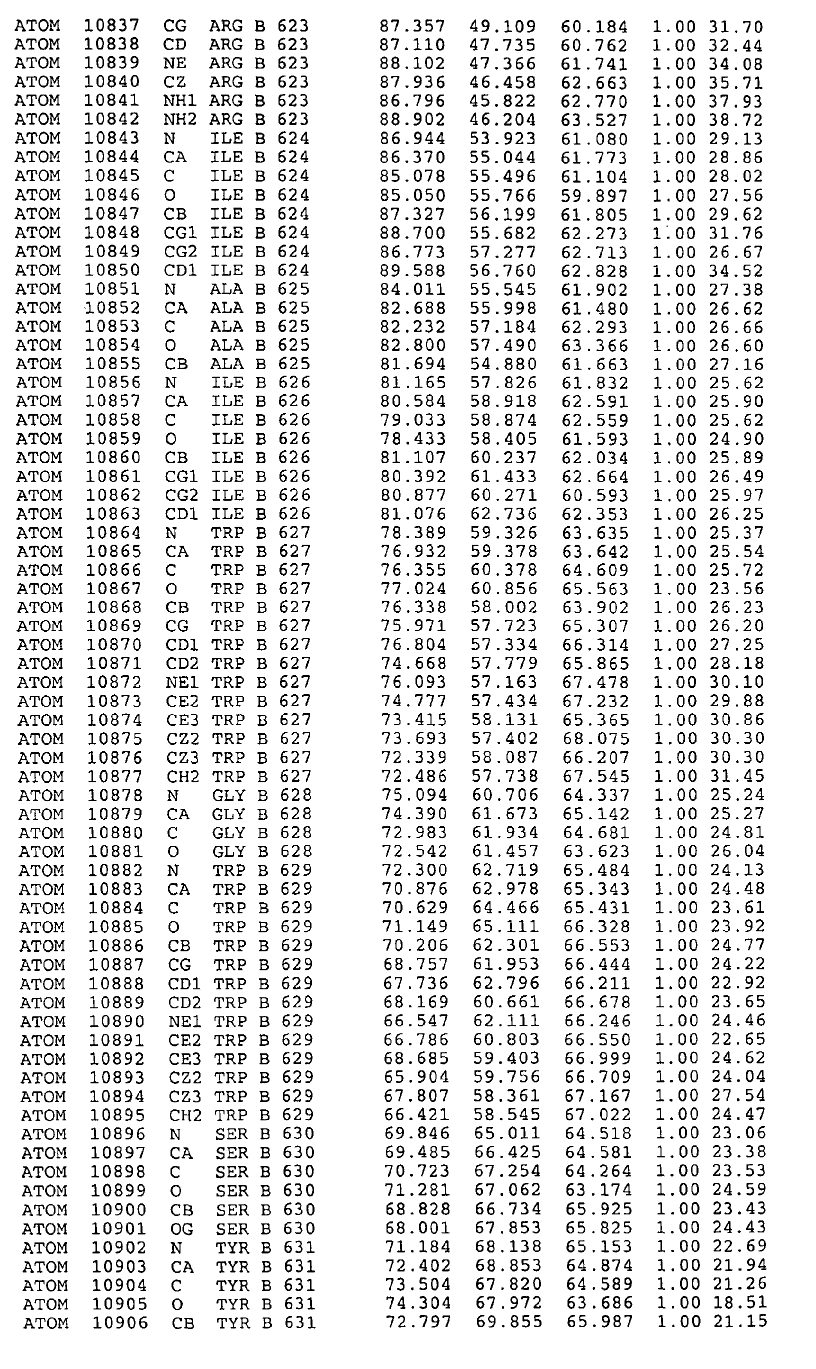

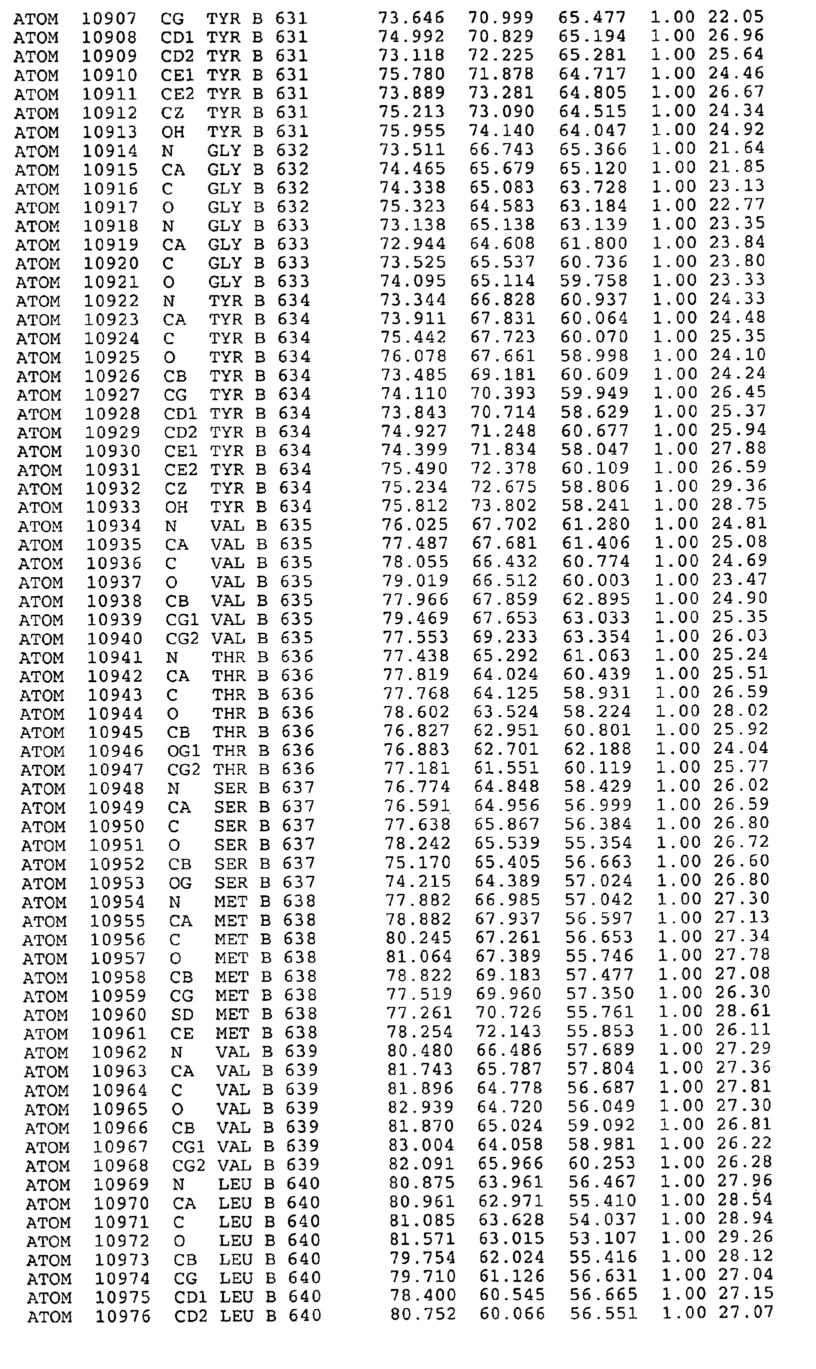

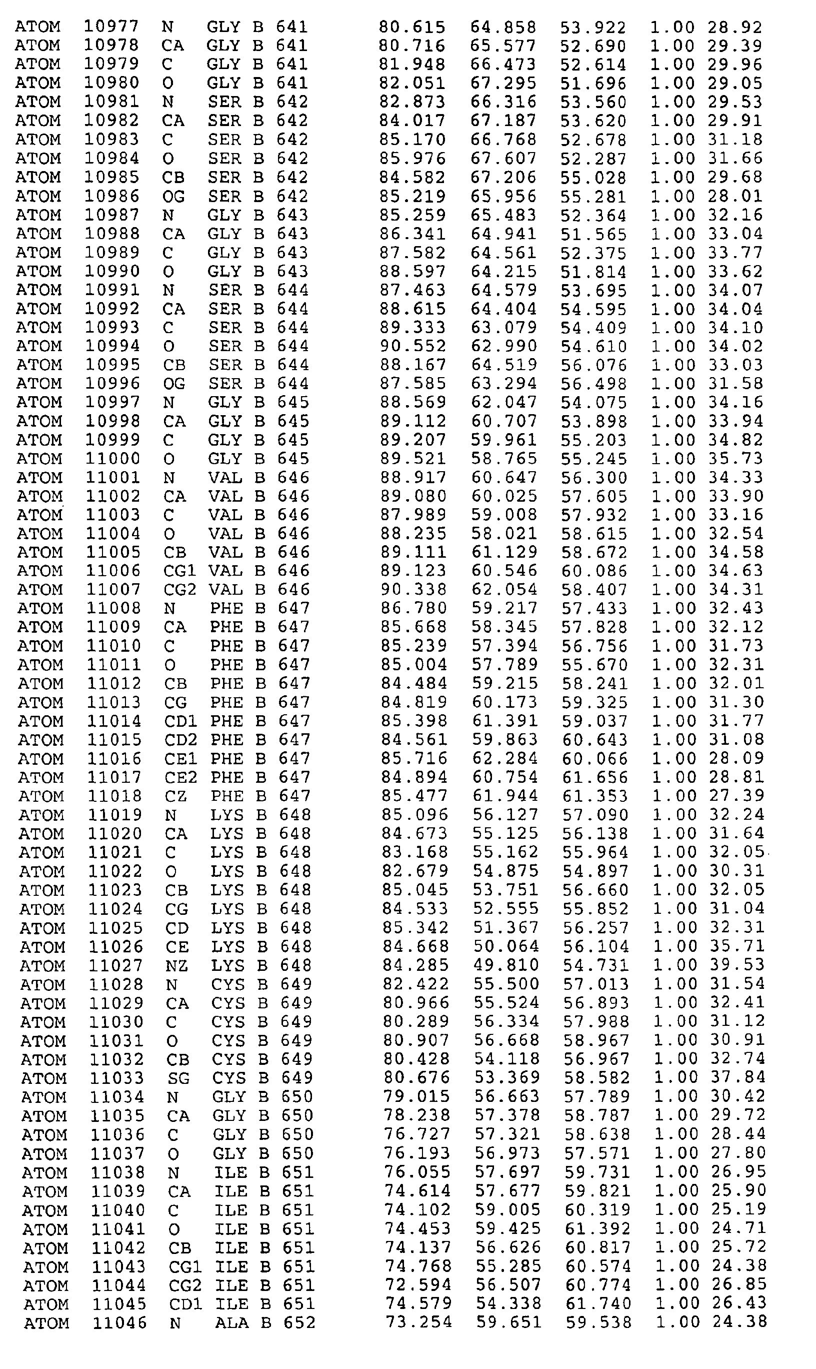

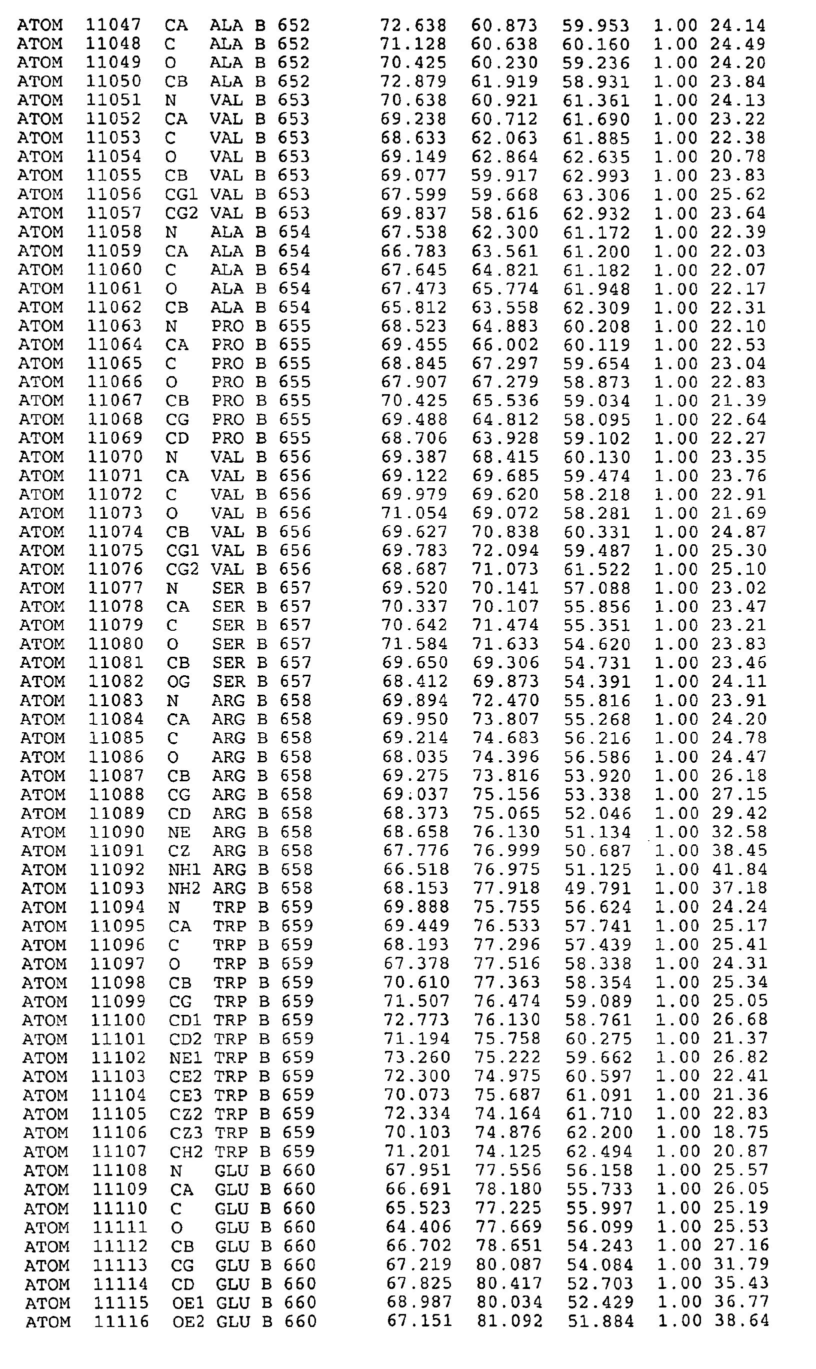

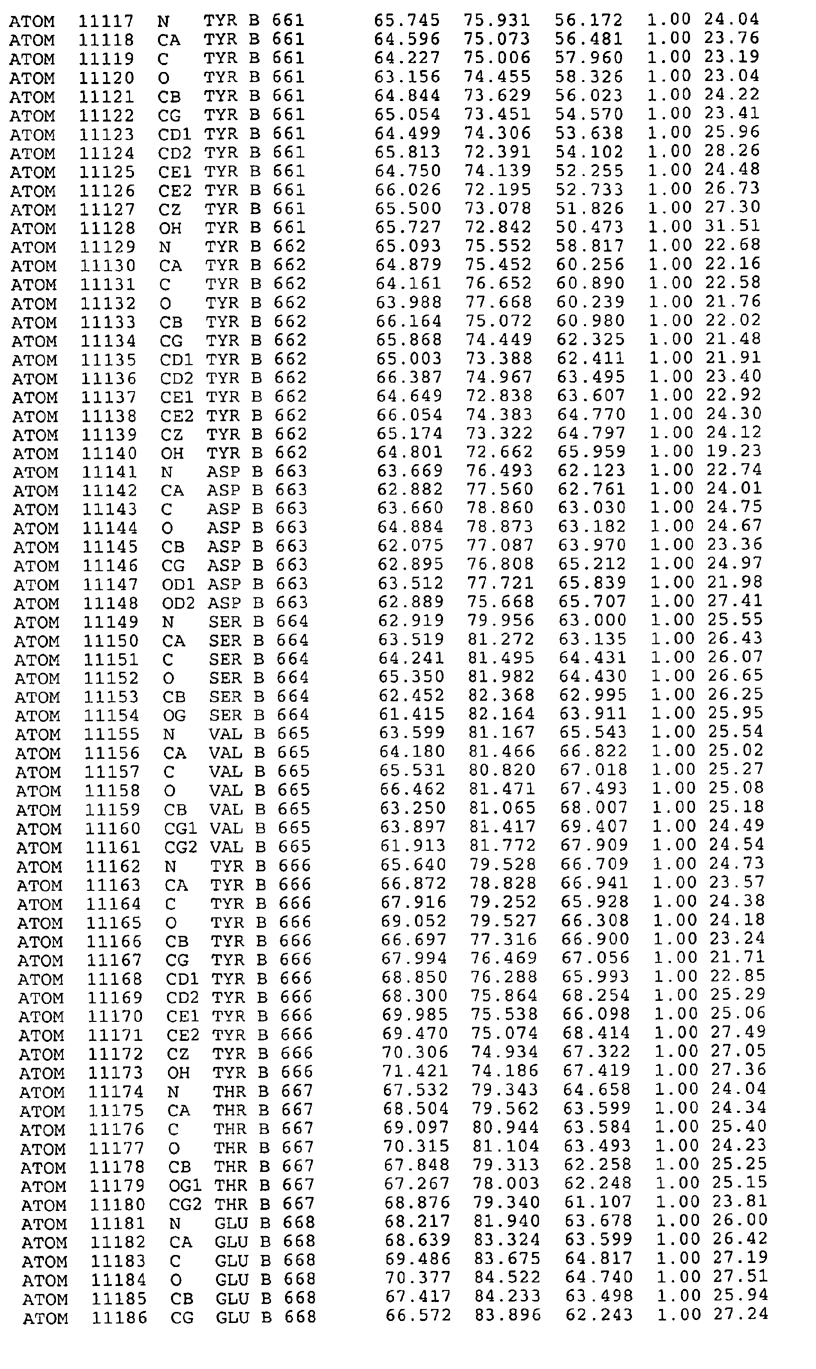

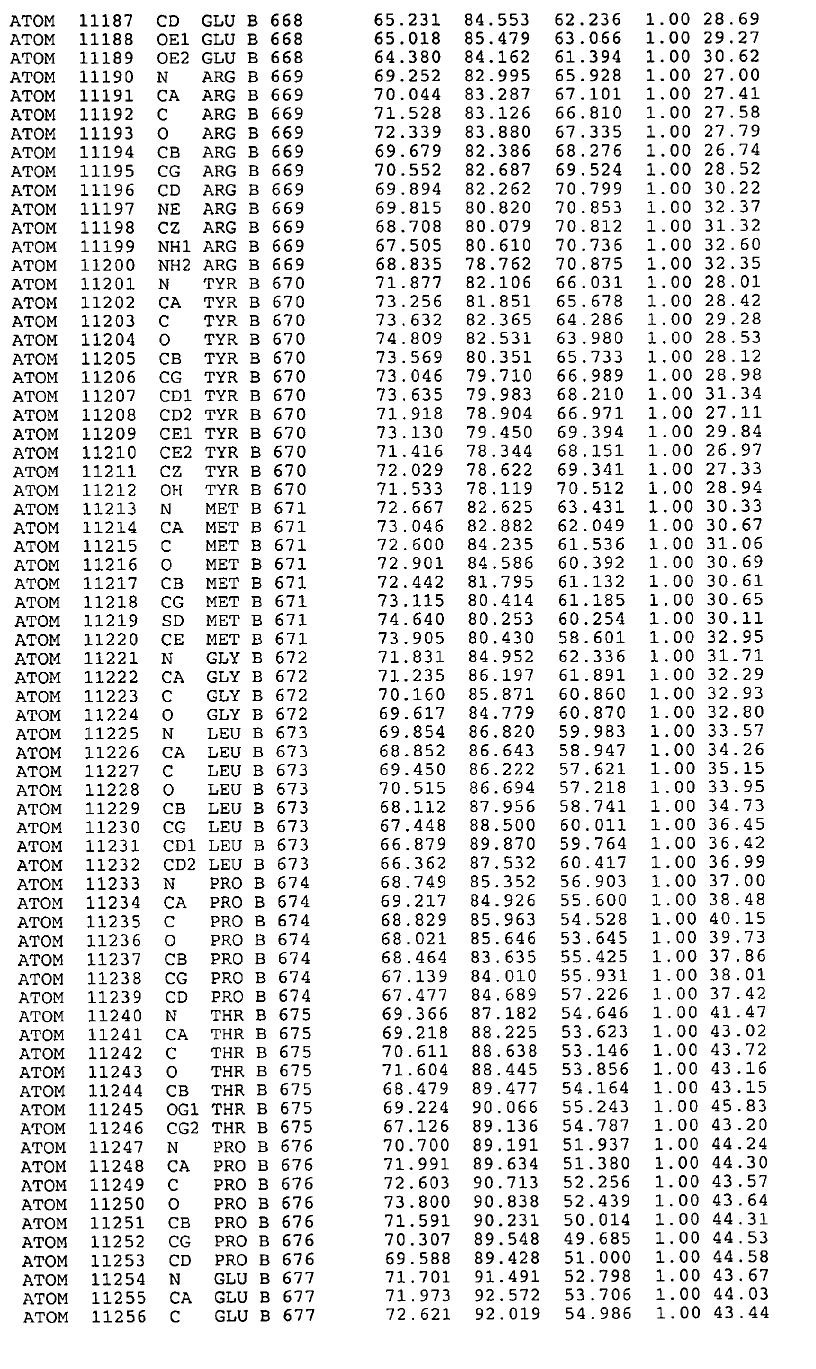

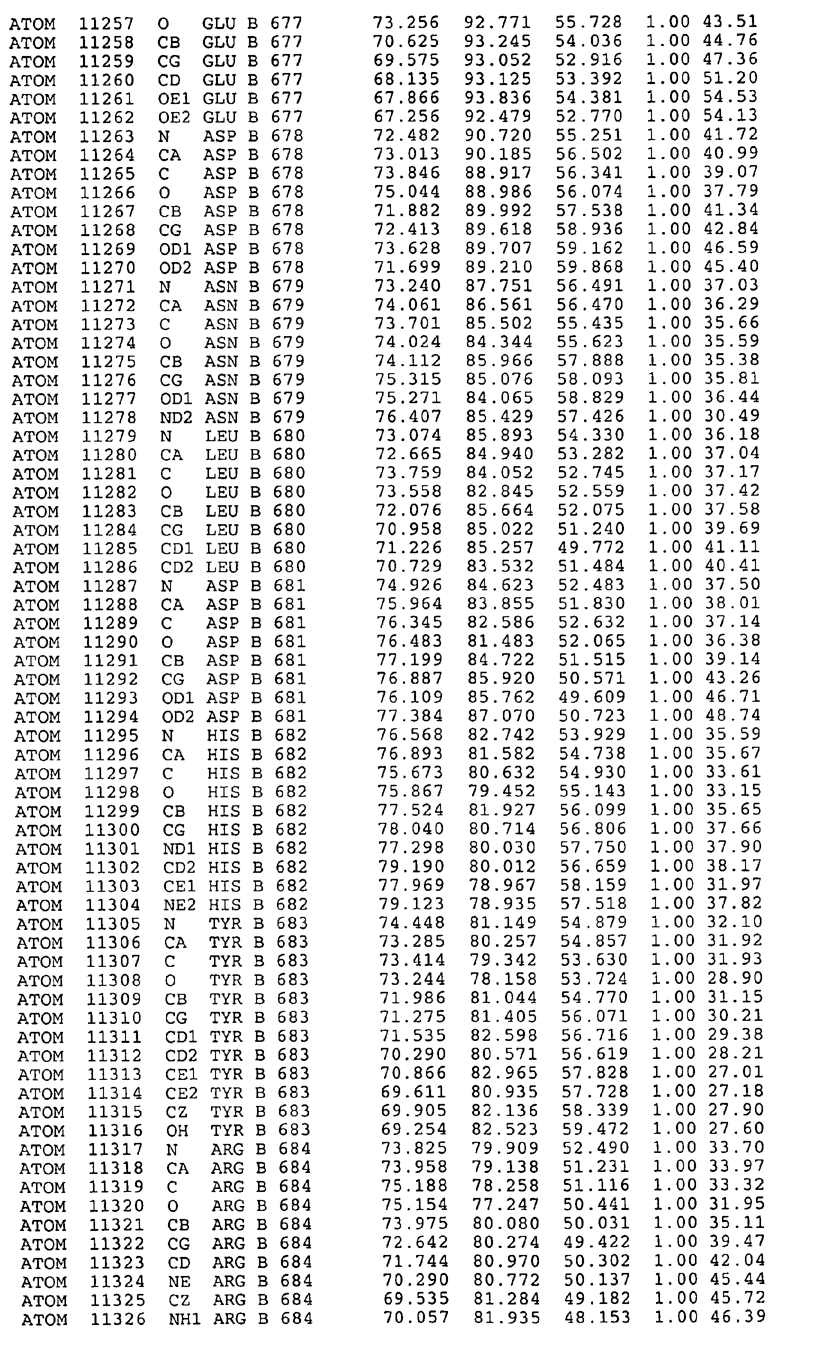

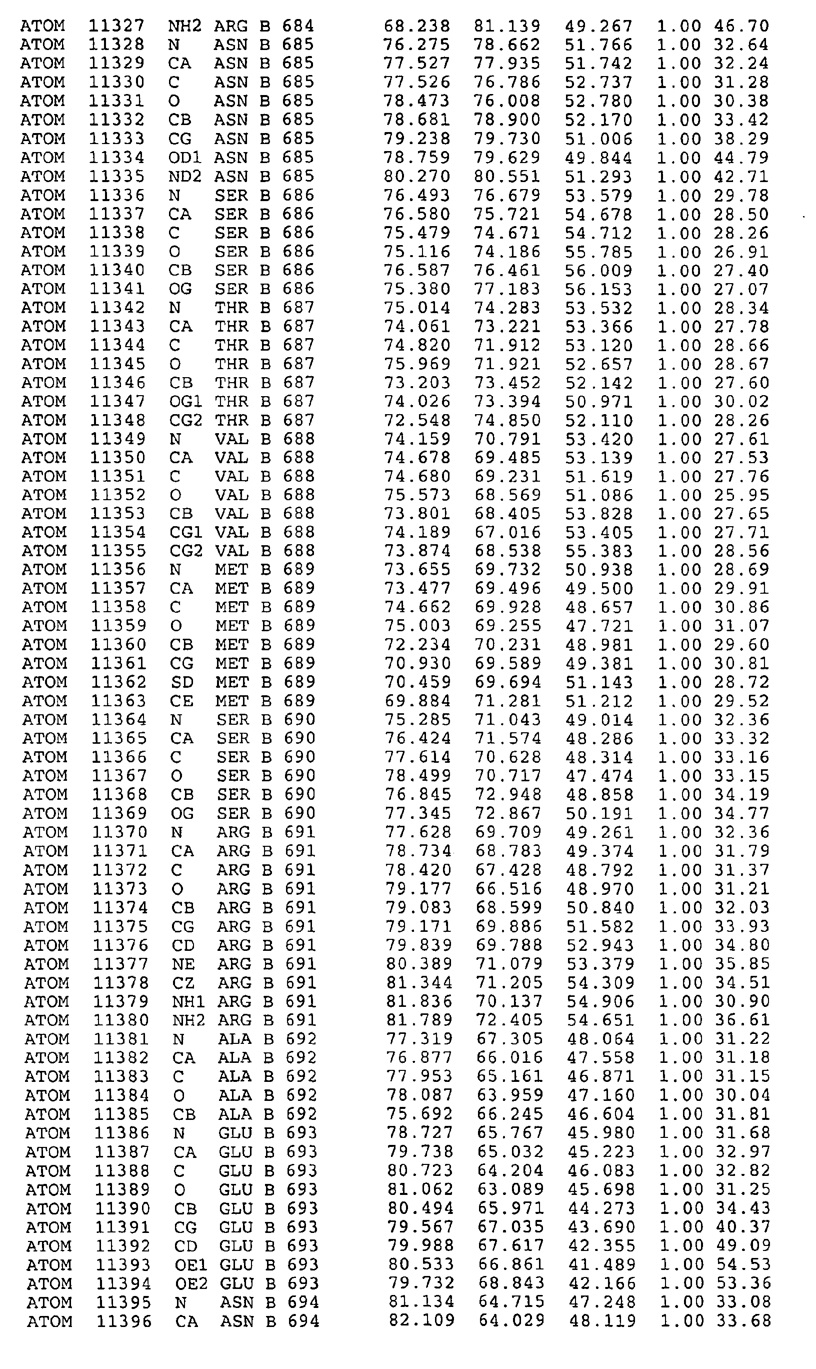

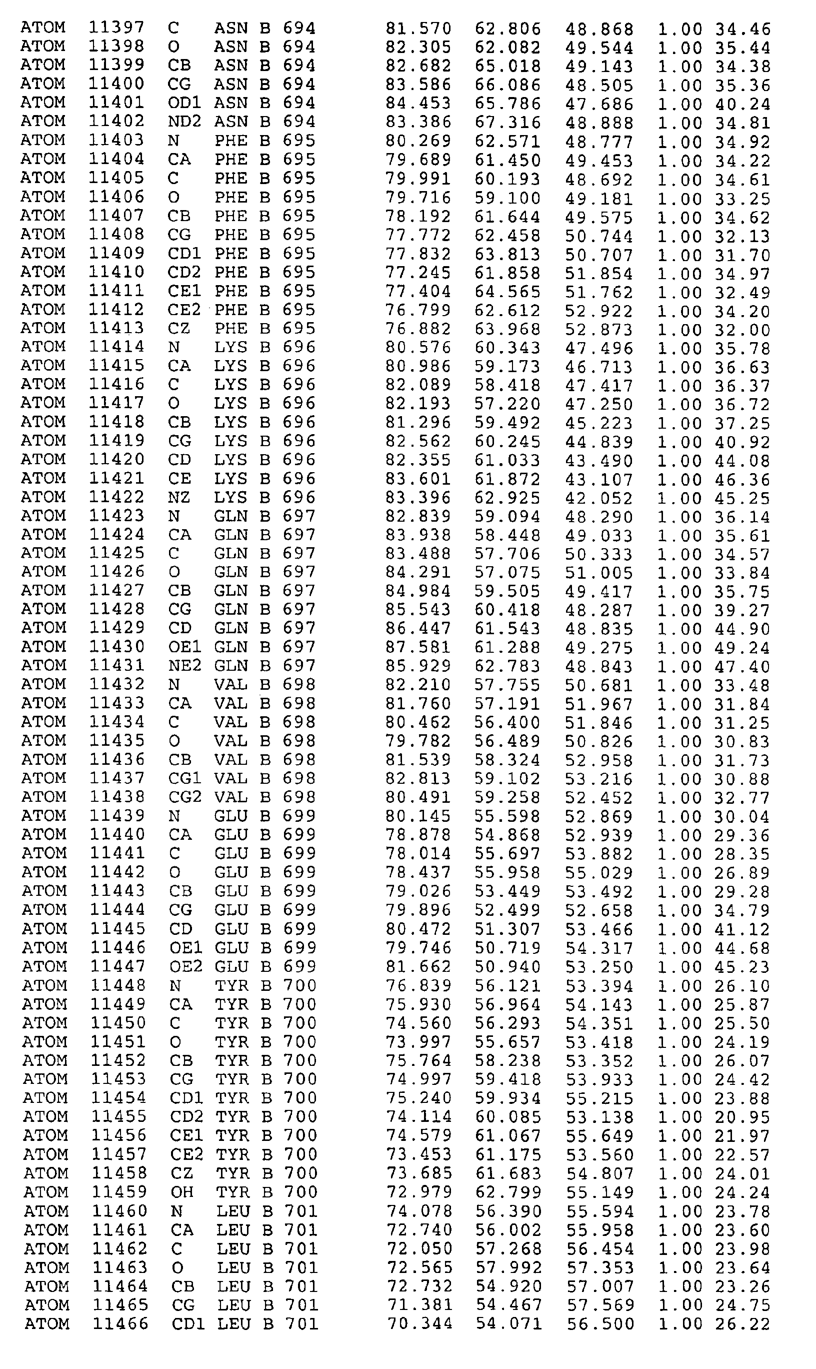

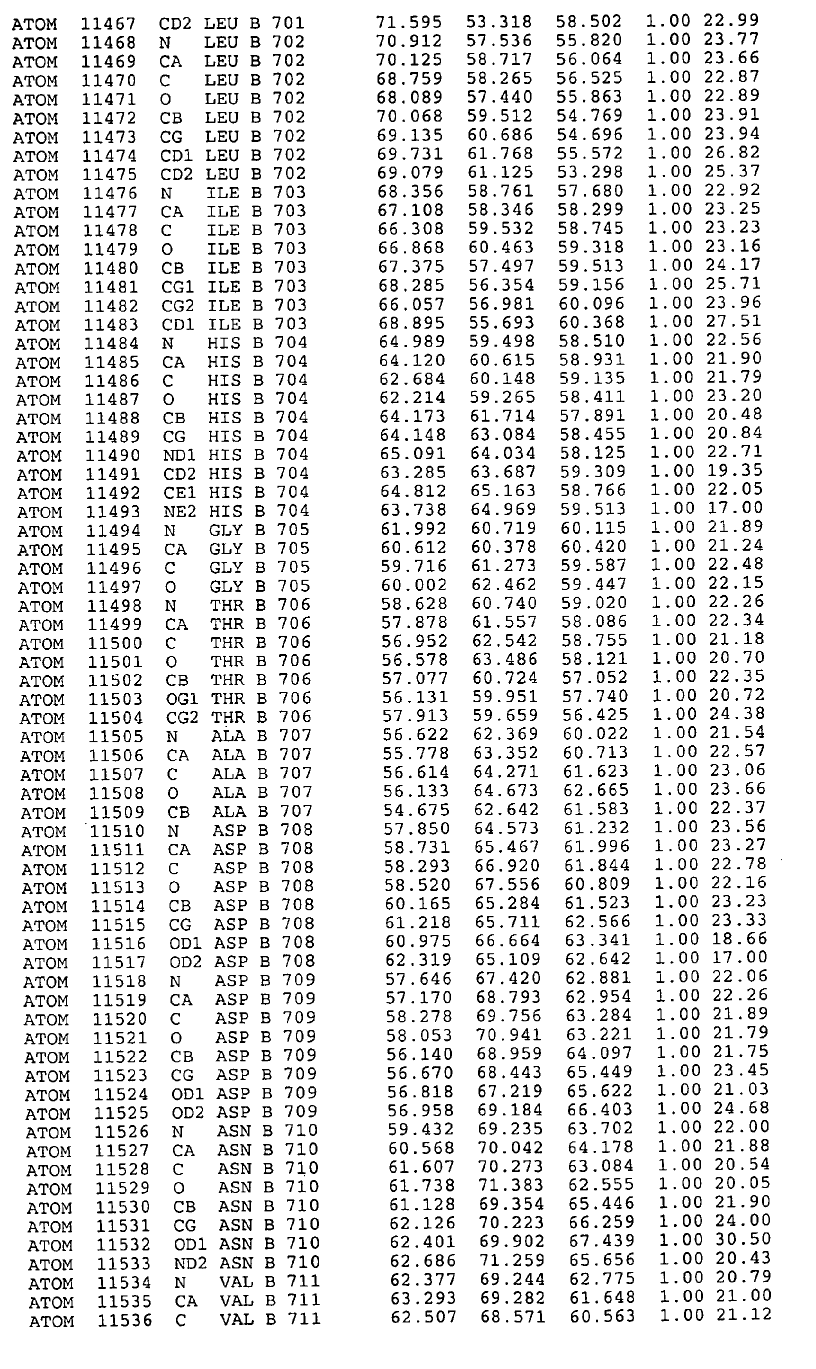

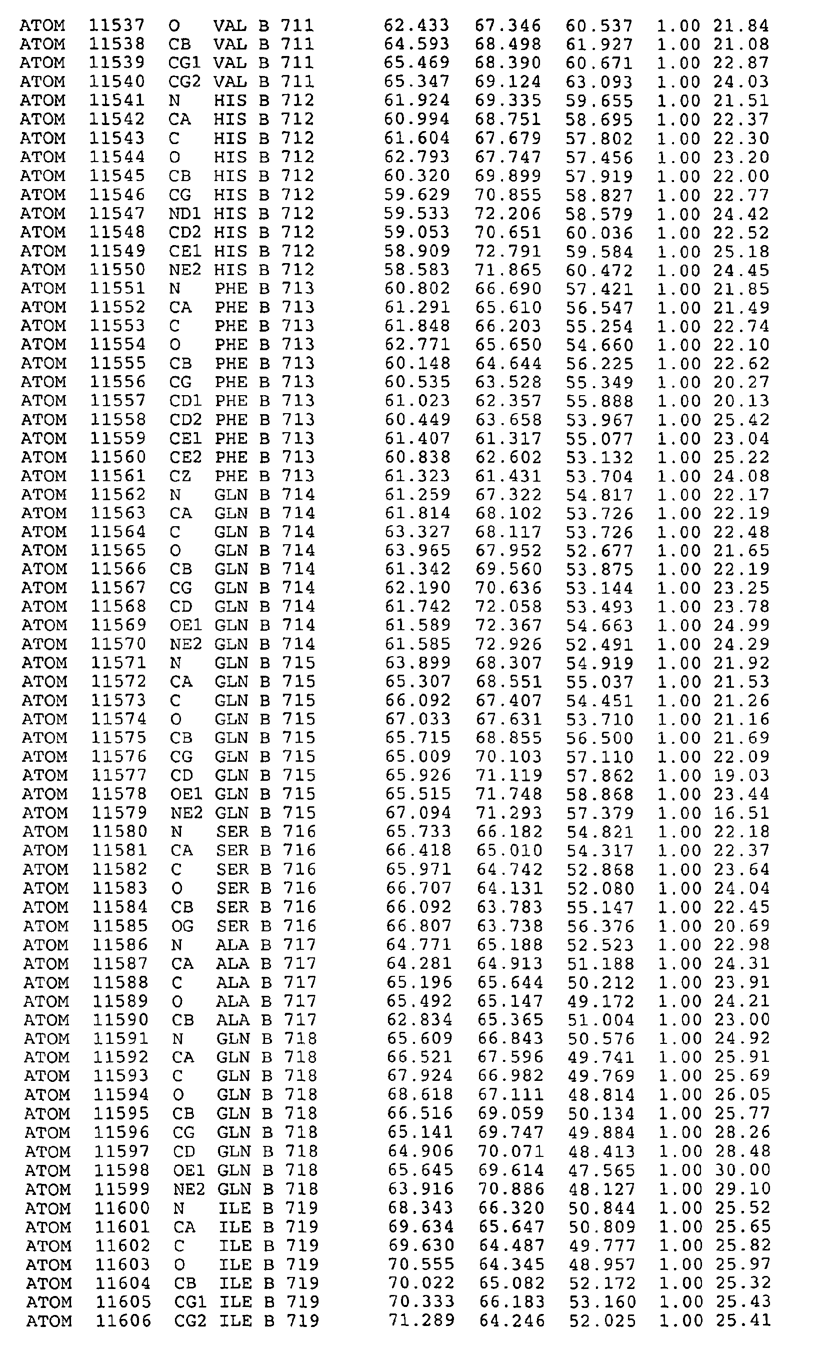

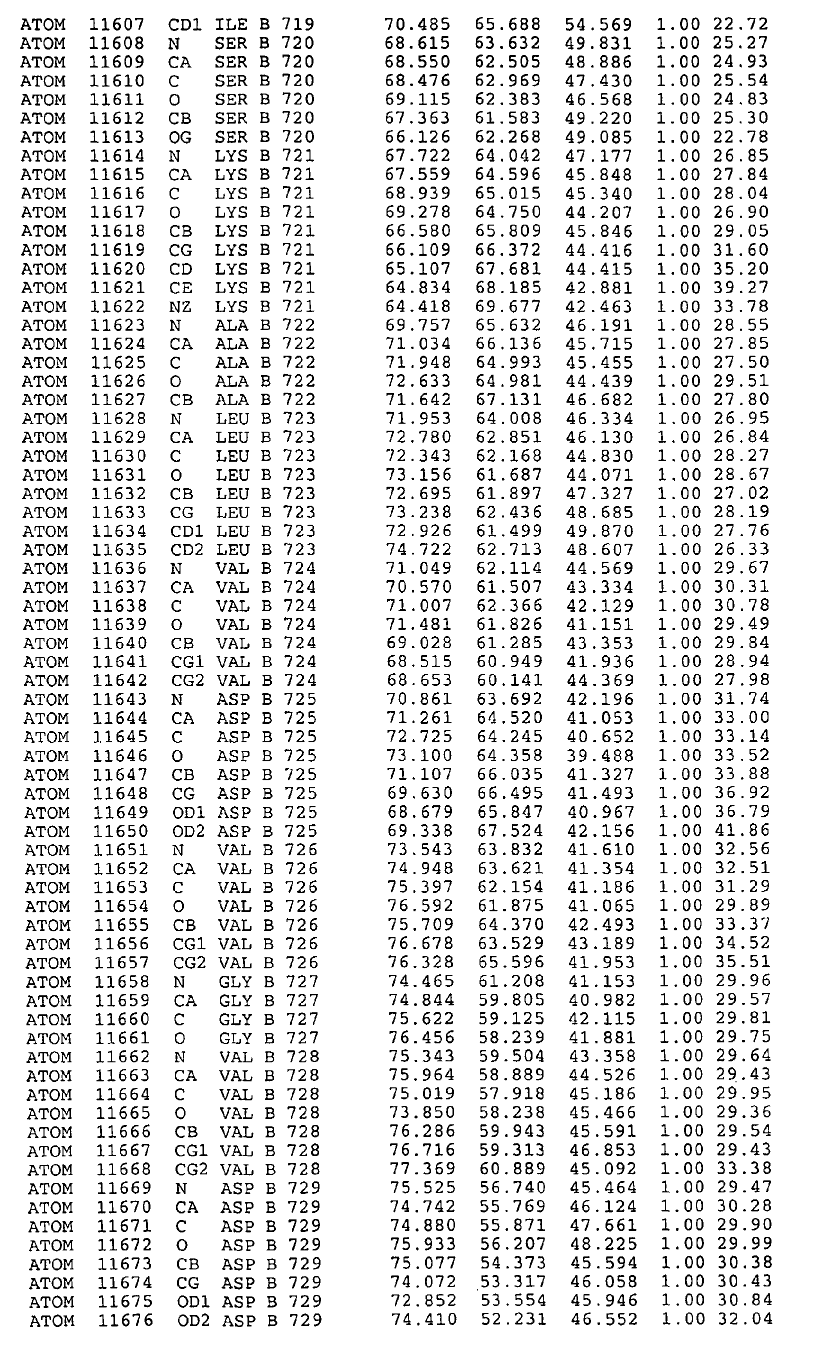

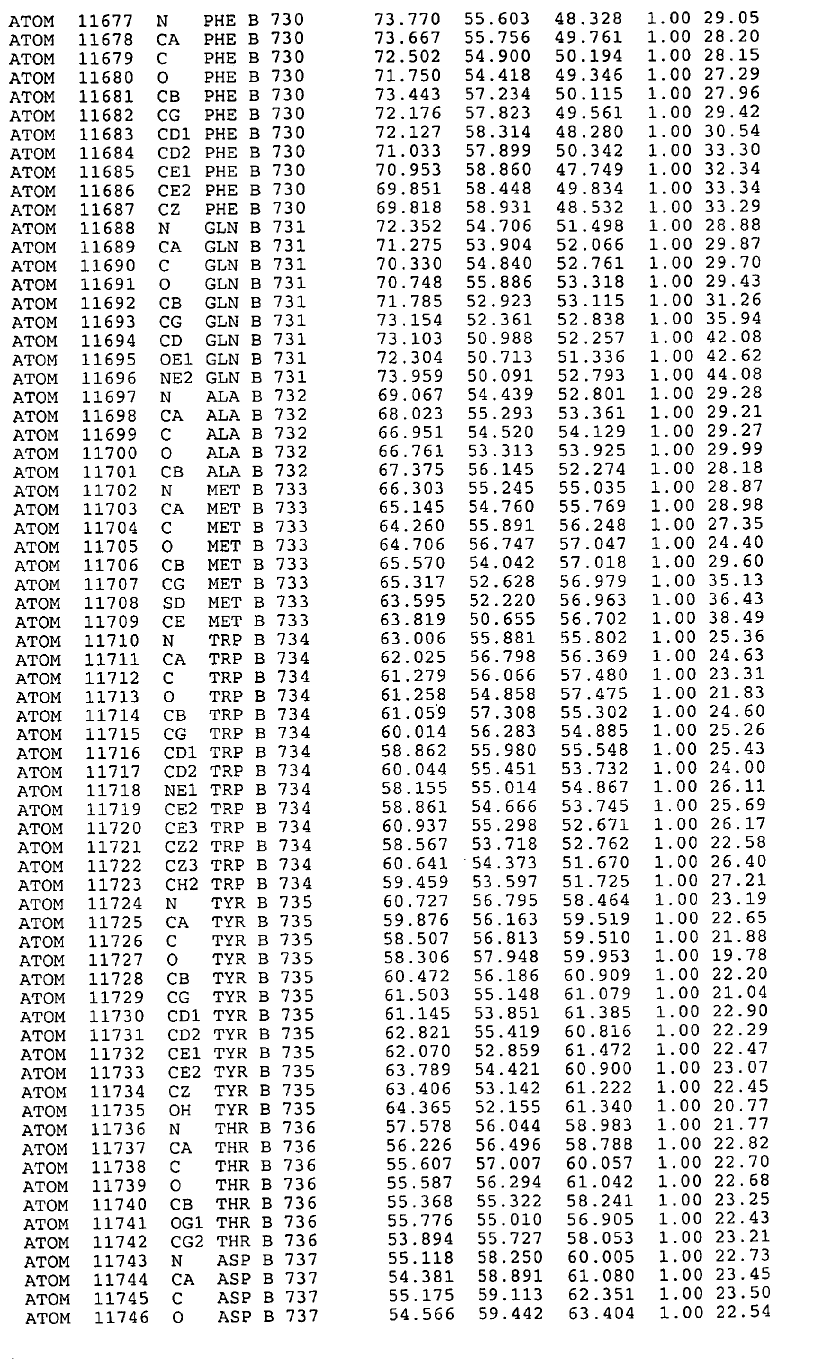

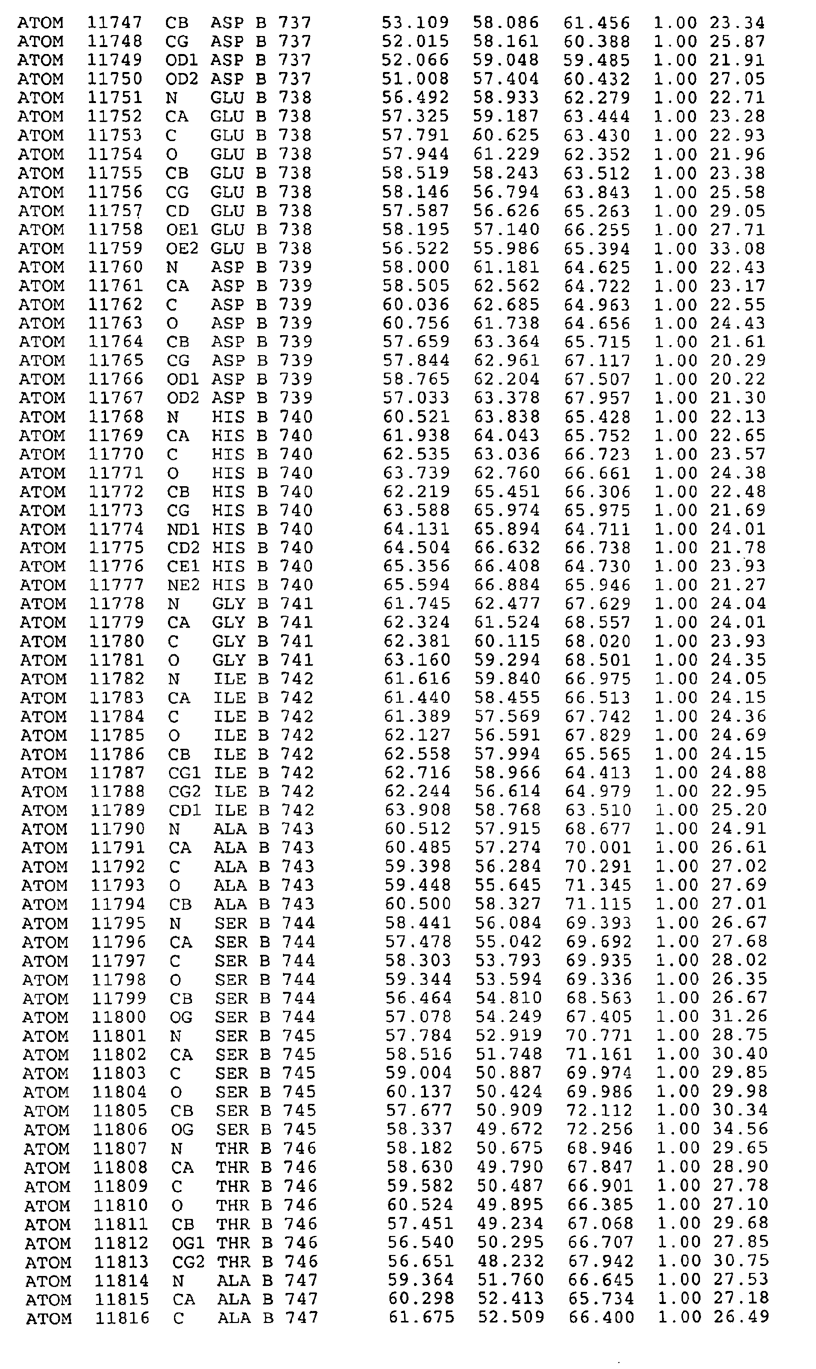

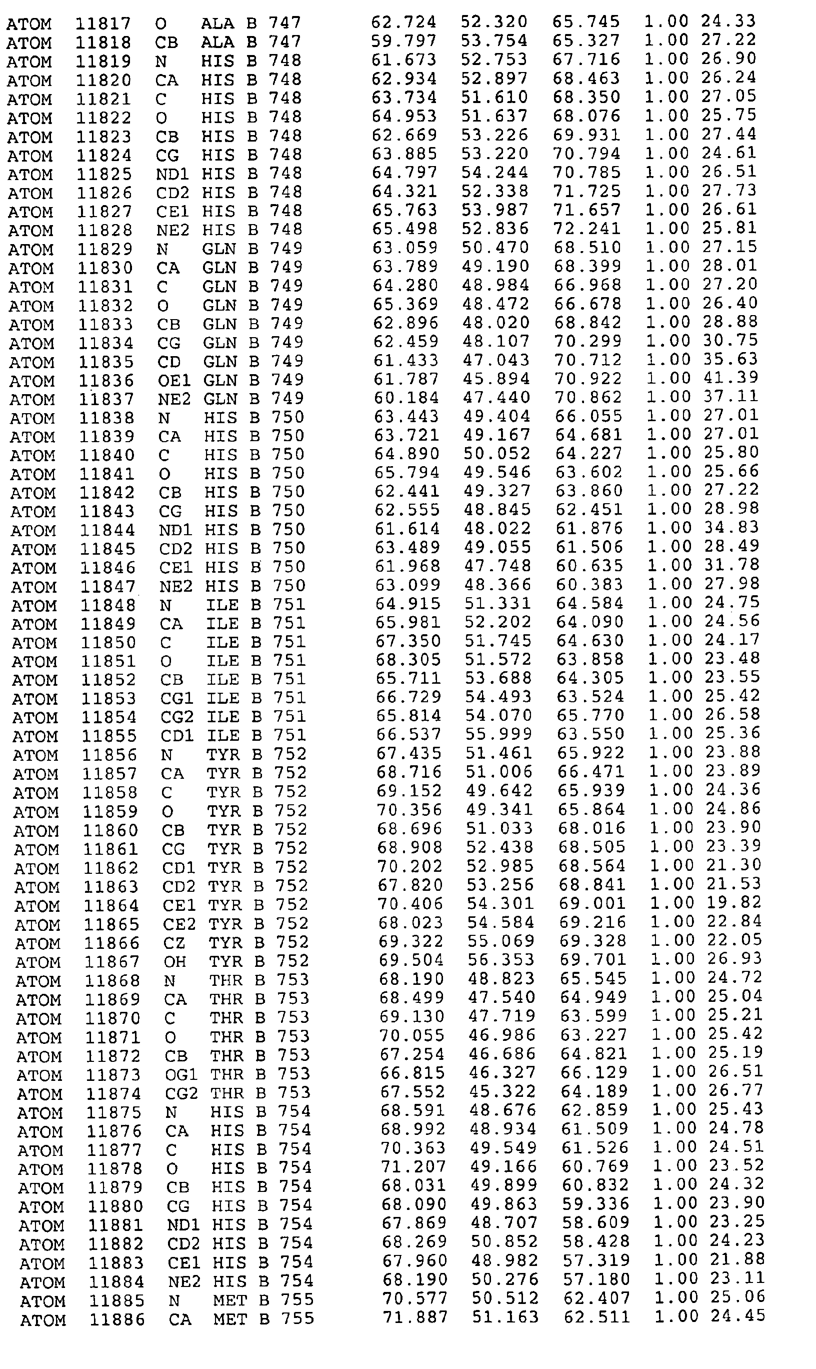

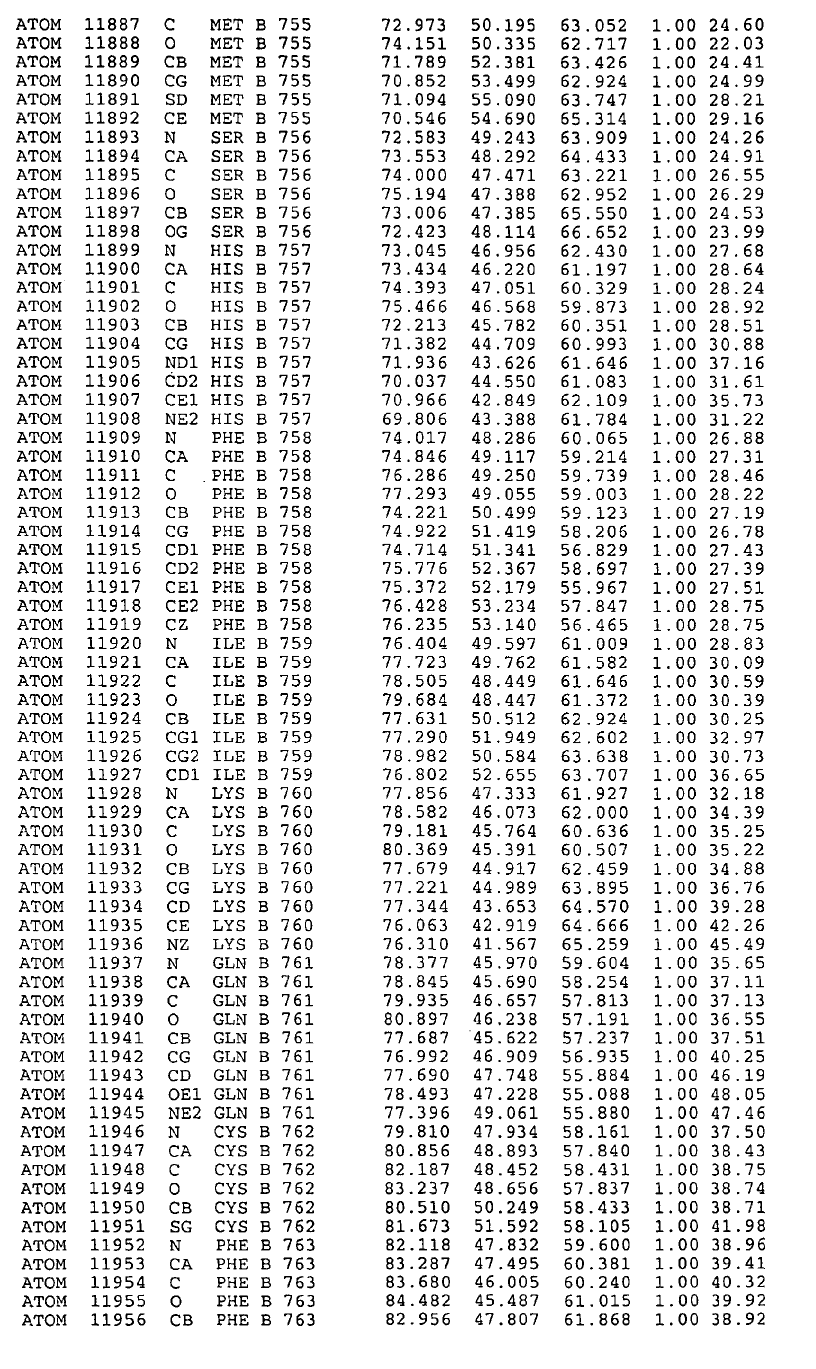

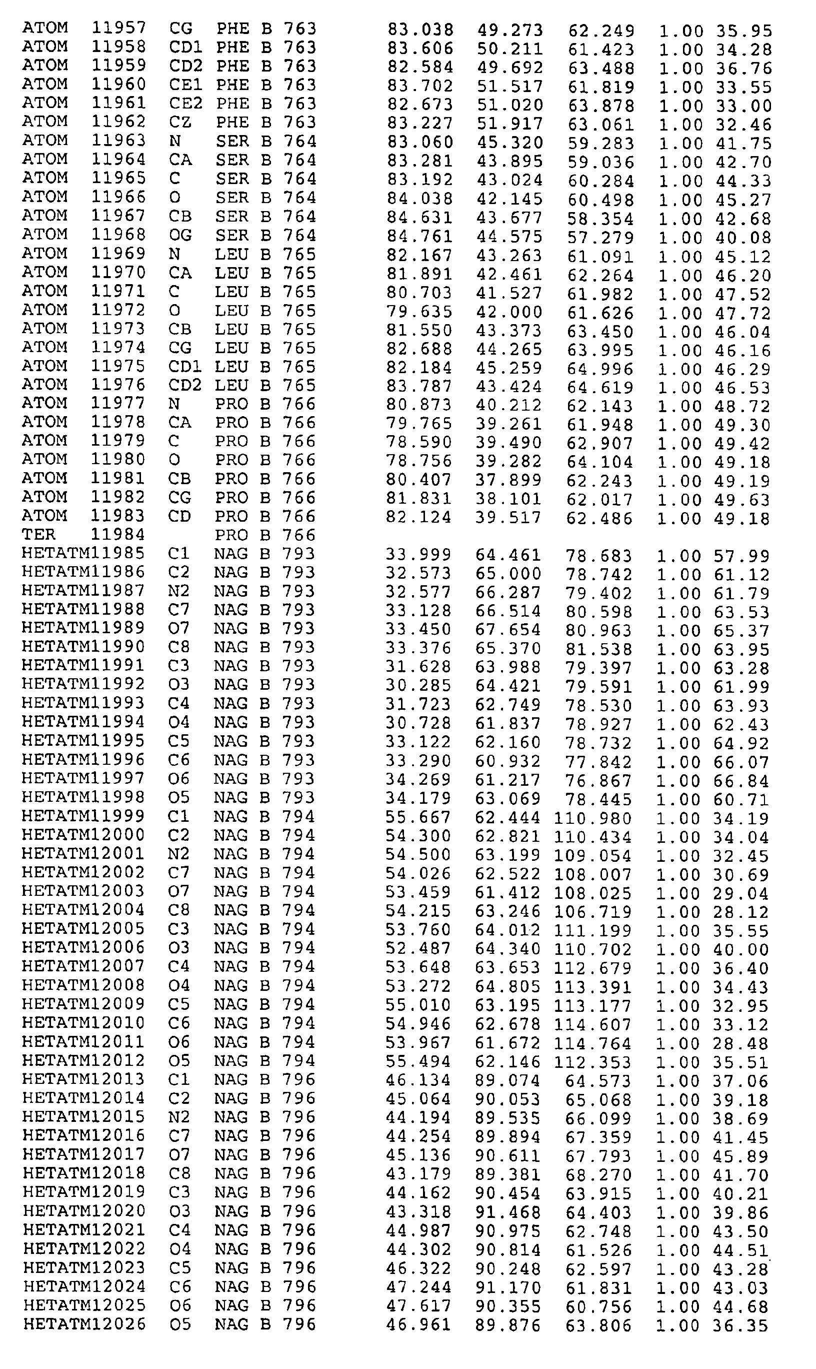

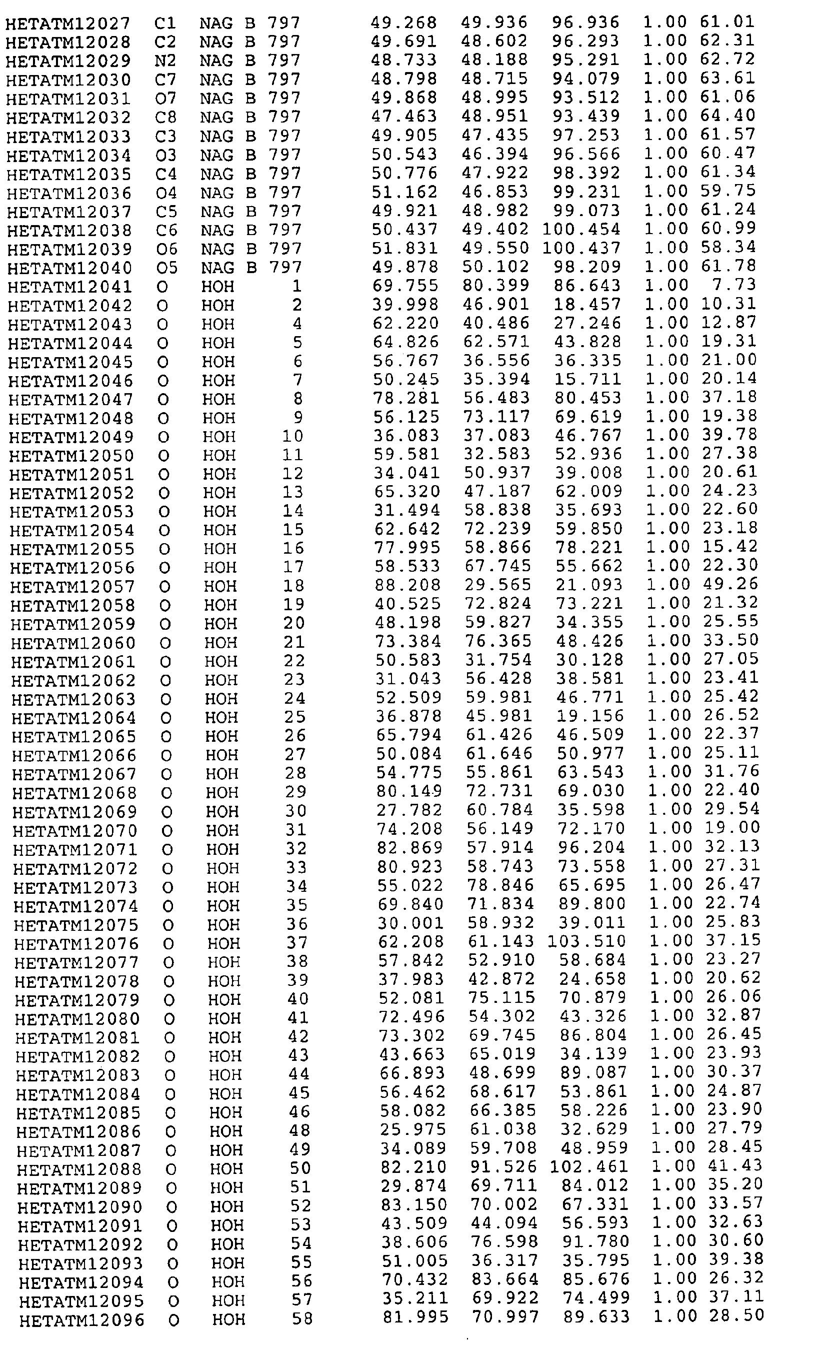

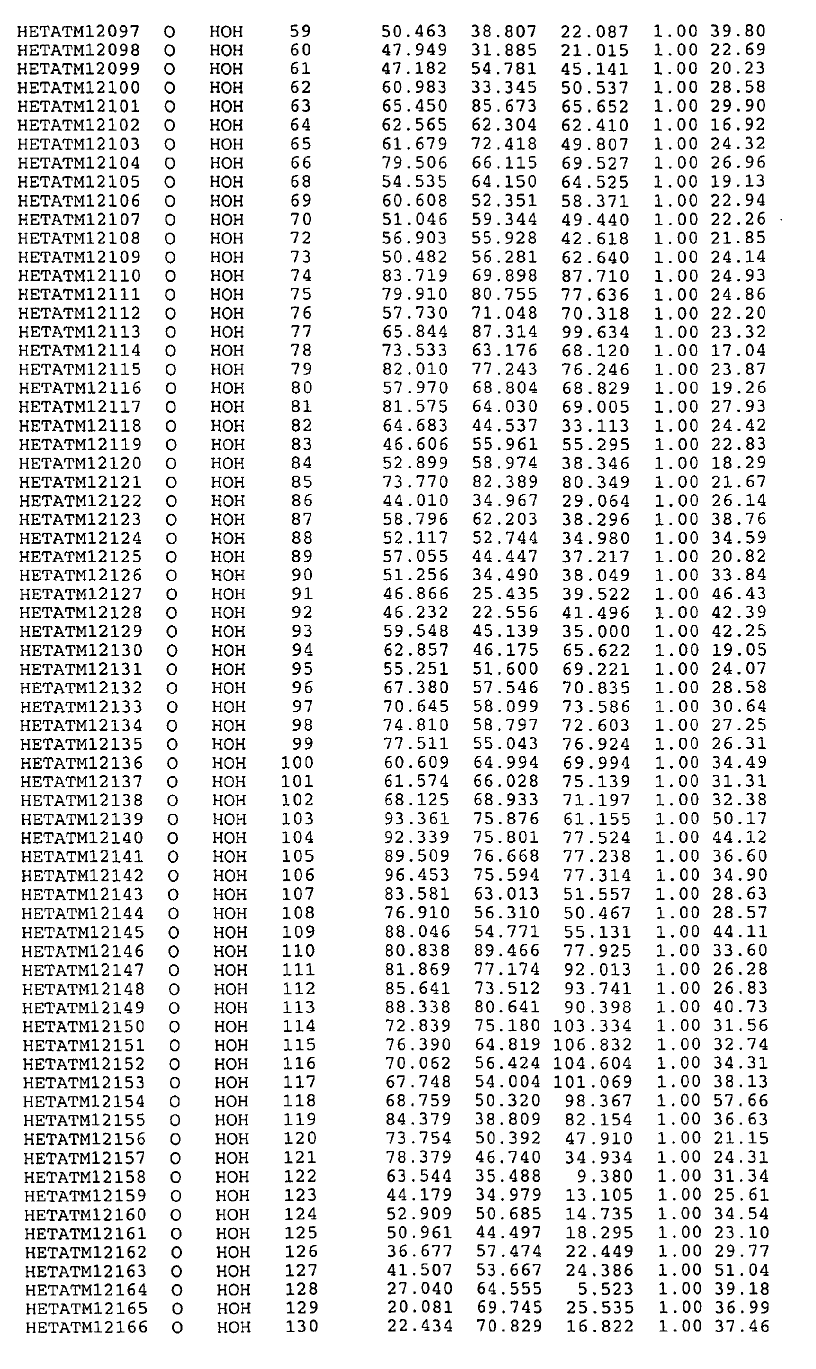

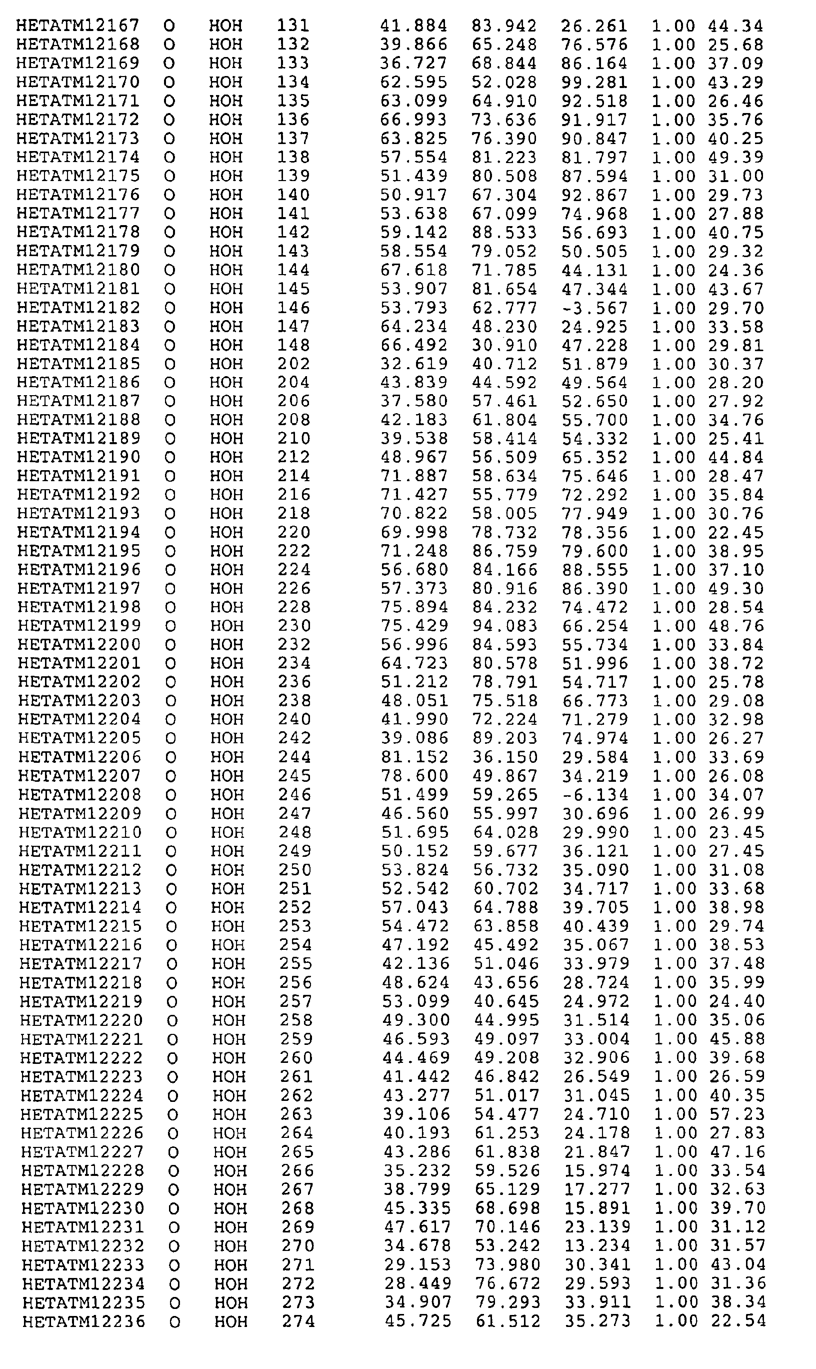

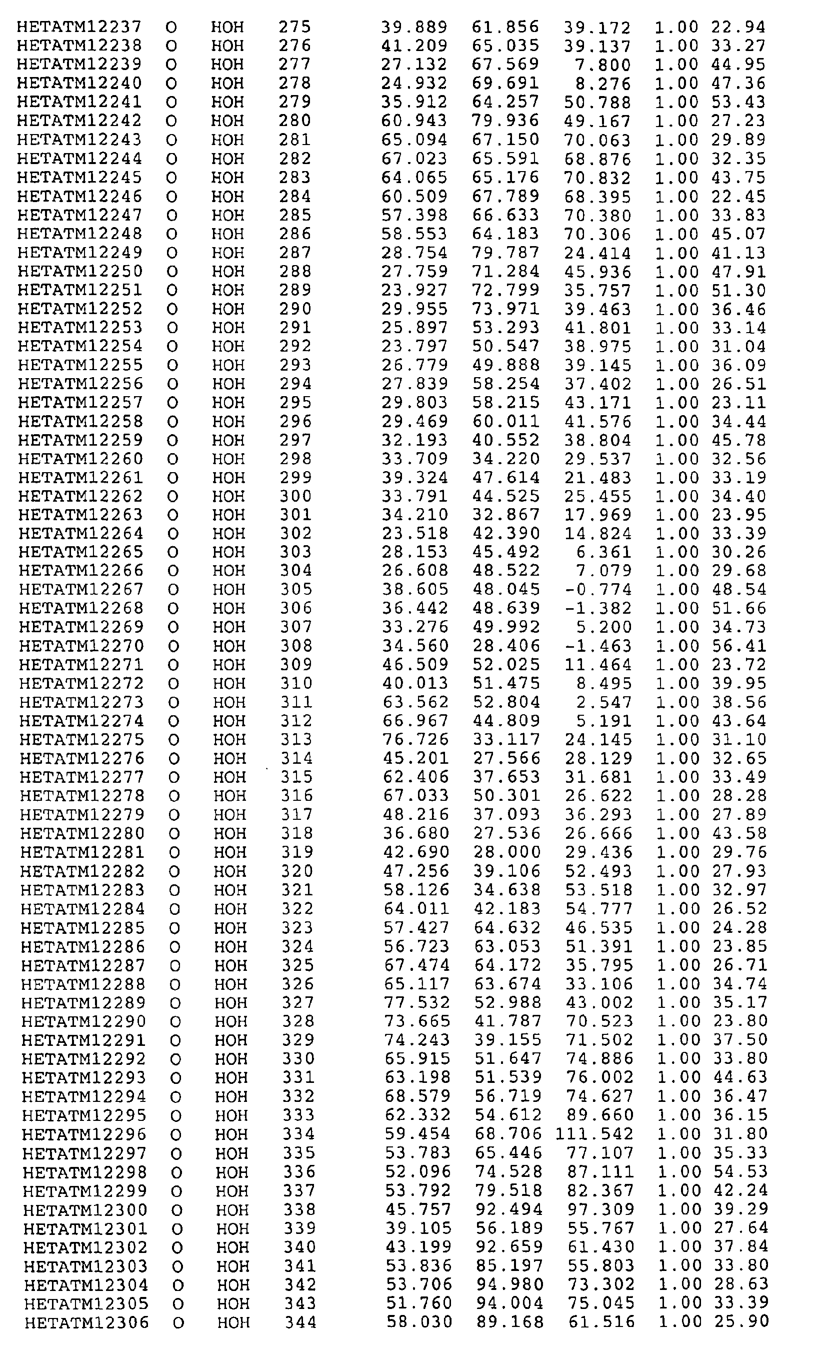

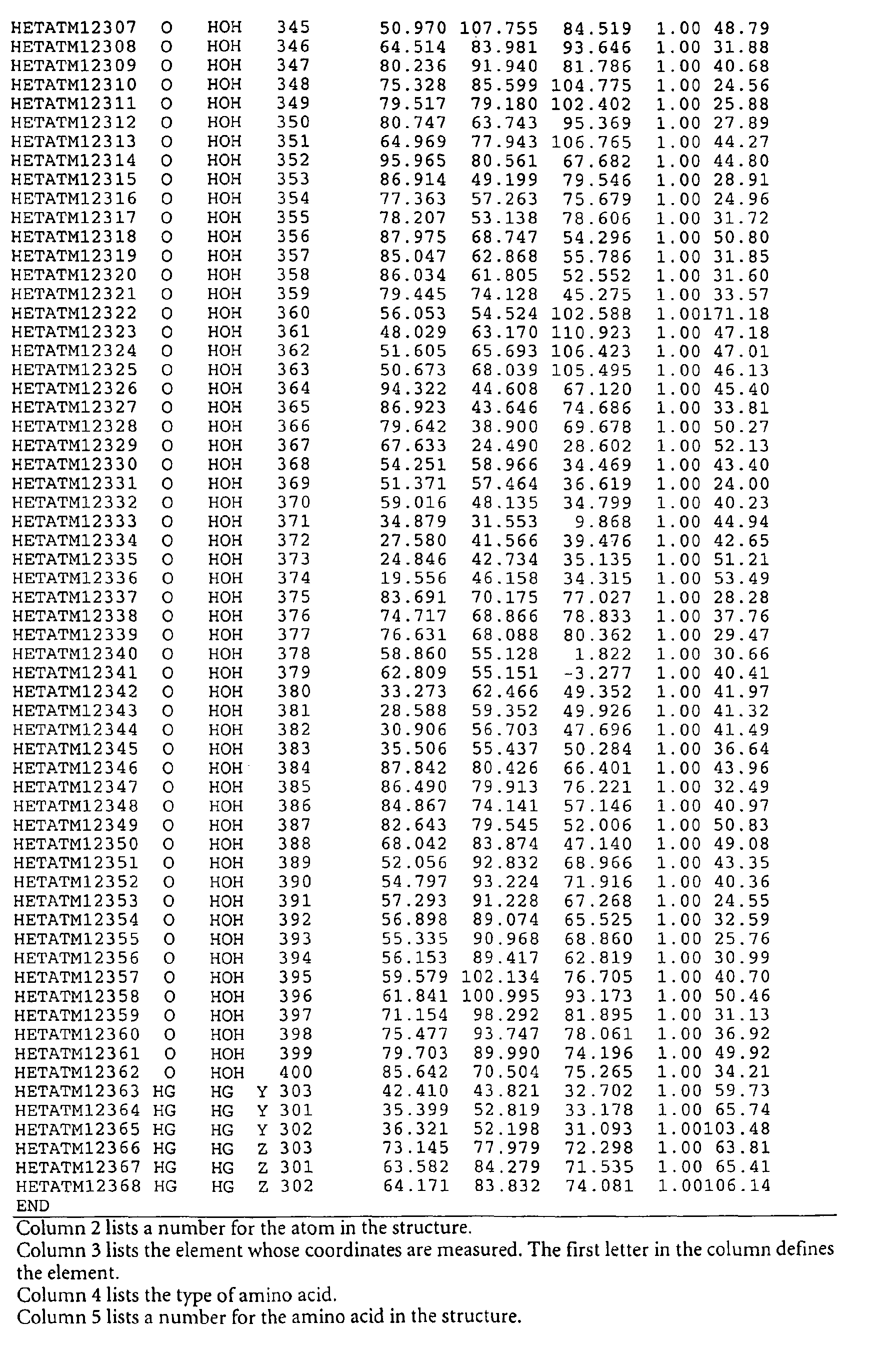

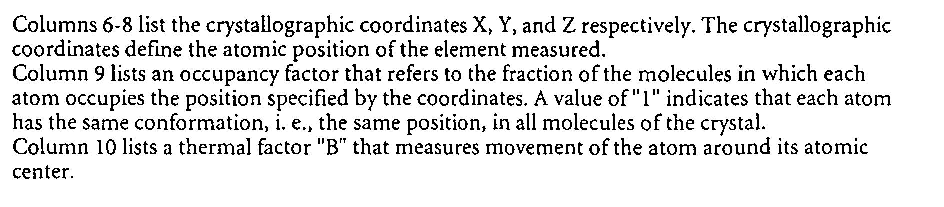

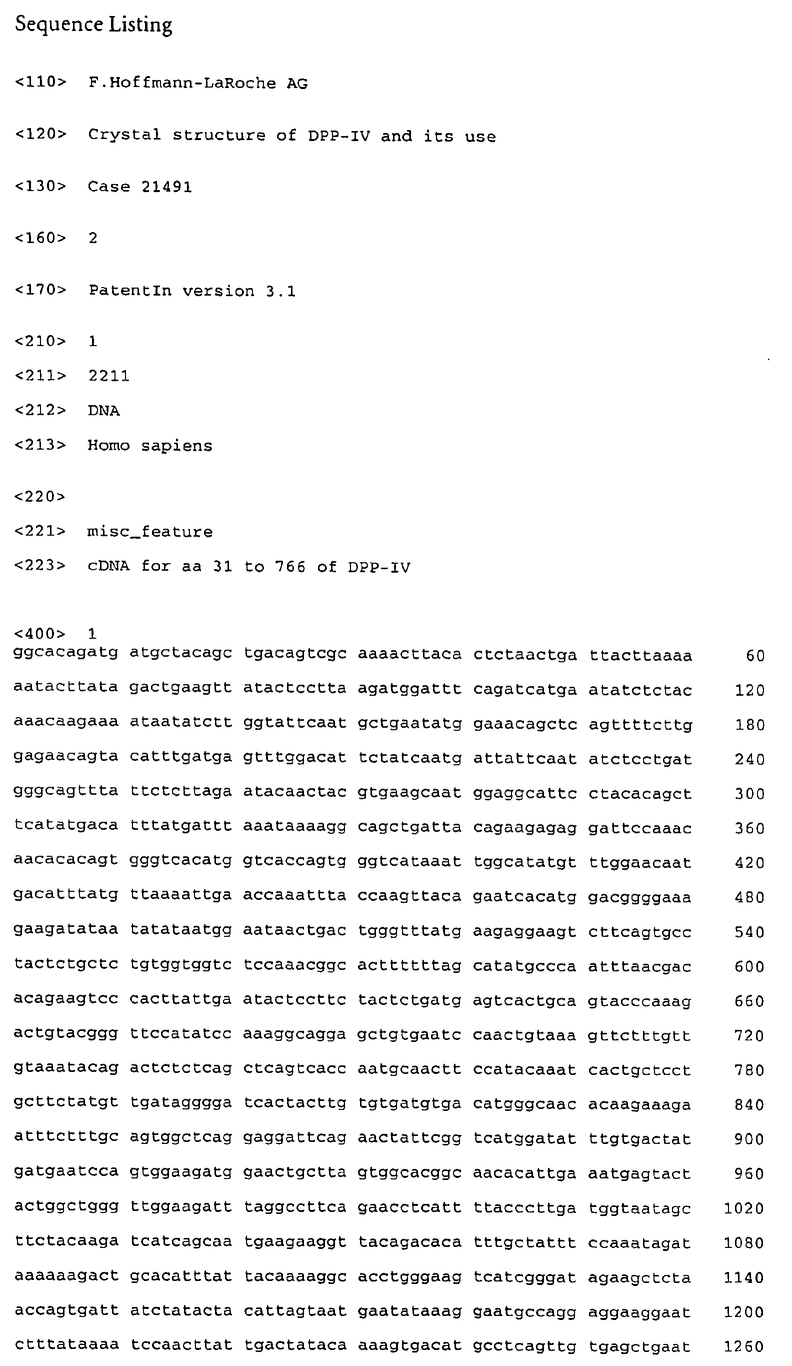

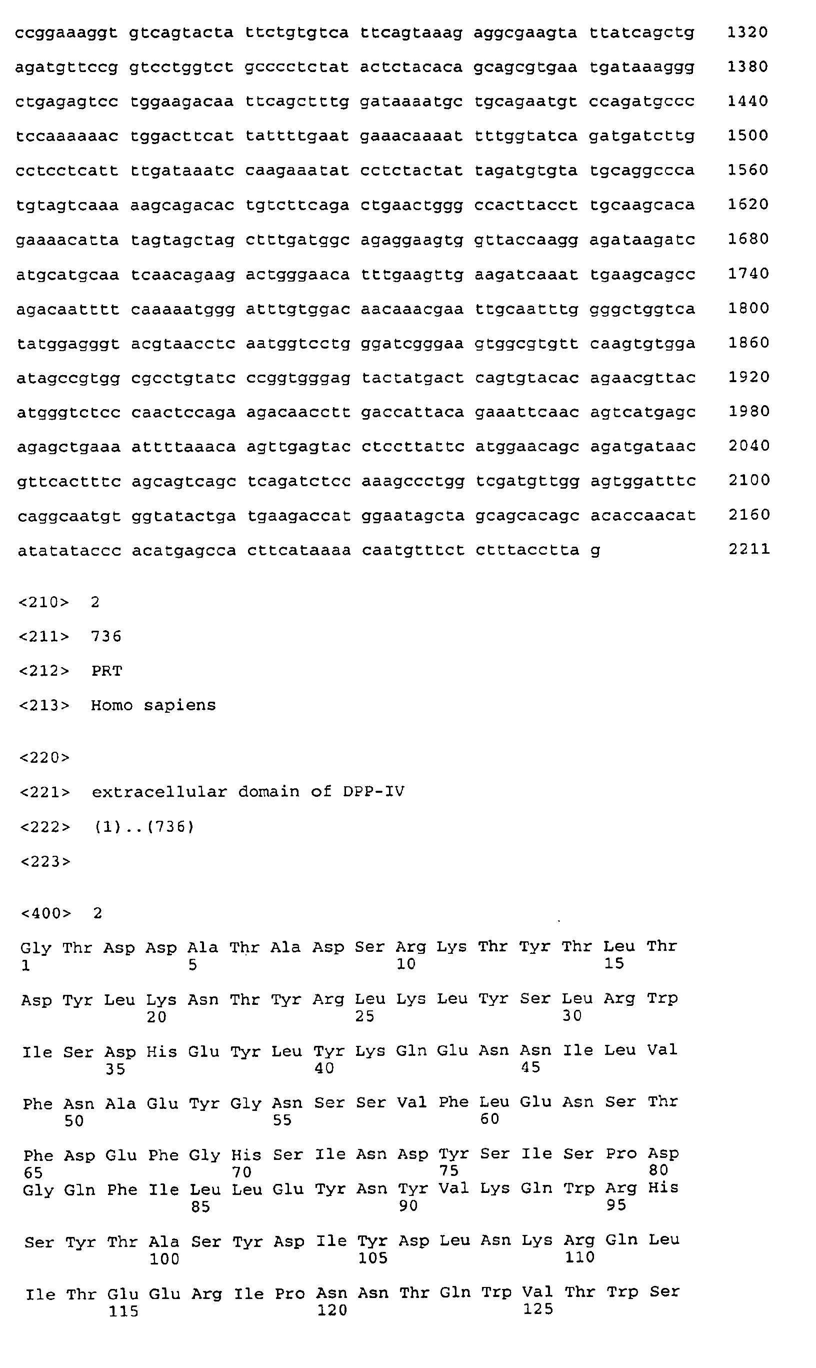

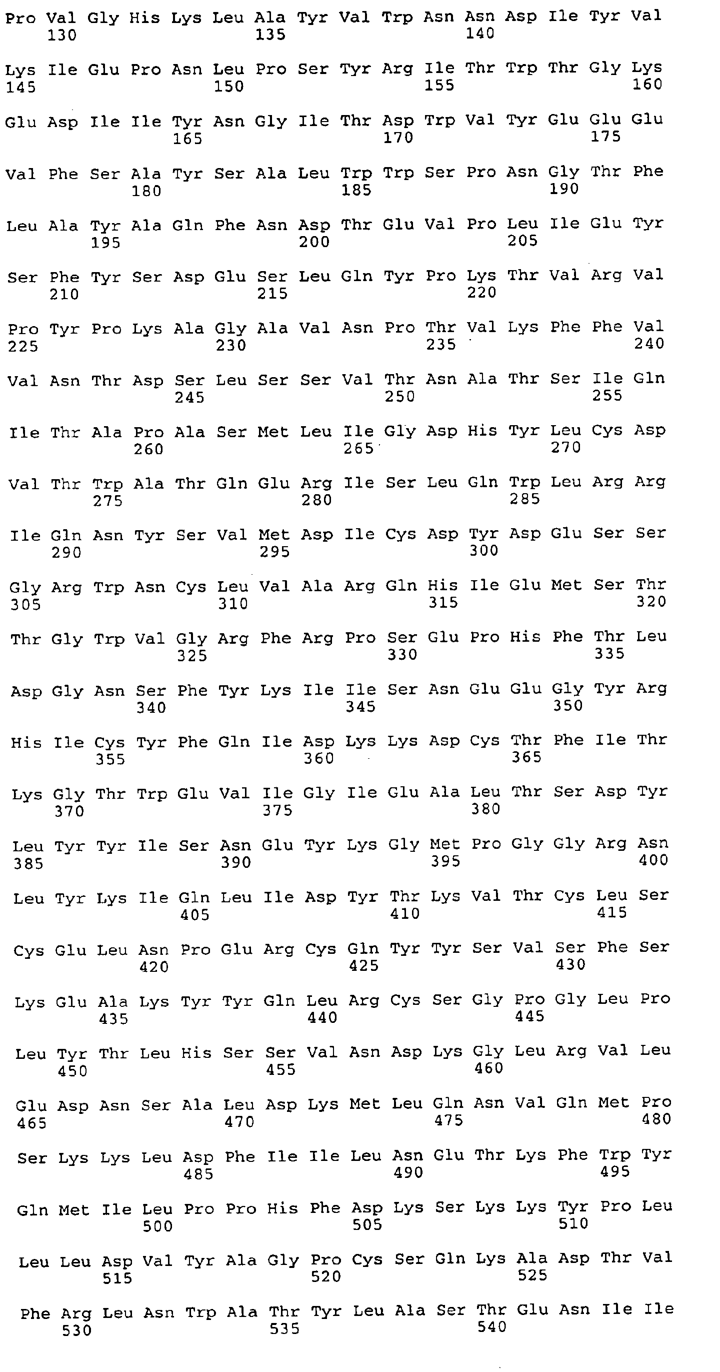

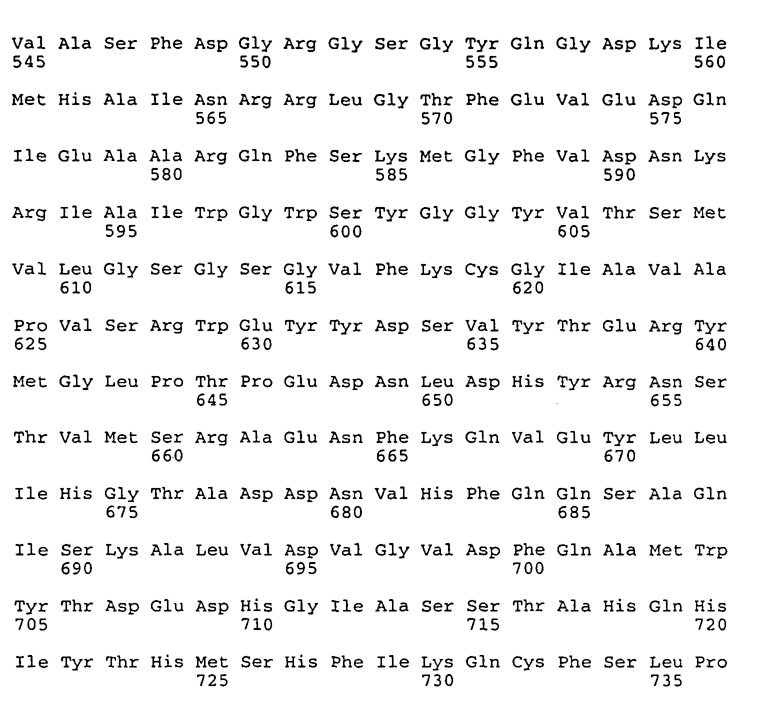

## Claims

1. A crystal of the extracellular domain of mammalian DPP-IV.

2. The crystal of claim 1, characterized as having an orthorhombic space group of P2₁2₁2₁ and one homodimer of DPP-IV in the asymmetric unit.

3. The crystal of claims 1 and 2, wherein the crystal has unit cell dimensions of:
a is from 63 Å to 70 Å;
b is from 66 Å to 70 Å;
c is from 416 Å to 424 Å;
and a P2₁2₁2₁ symmetry.

4. The crystal of claims 1 to 3, **characterized by** the atomic structure coordinates of Table 4.

5. A co-crystal of the extracellular domain of mammalian DPP-IV and a ligand bound to its active site.

6. The crystal of claim 5, characterized as having an orthorhombic space group of P2₁2₁2₁ and one homodimer of DPP-IV in the asymmetric unit.

7. The co-crystal of claim 6, wherein the co-crystal has unit cell dimensions of:
a is from 63 Å to 70 Å;
b is from 66 Å to 70 Å;
c is from 416 Å to 424 Å;
and a P2₁2₁2₁ symmetry.

8. A co-crystal of the extracellular domain of mammalian DPP-IV and a ligand bound to an allosteric binding site.

9. A co-crystal of the extracellular domain of mammalian DPP-IV and HgCl₂.

10. A method for crystallizing mammalian DPP-IV, the method comprising
(a) providing a buffered, aqueous solution of pH 7 to 8.5 with a concentration of 7 mg/ml to 22 mg/ml of the extracellular domain of mammalian DPP-IV; and
(b) growing crystals by vapor diffusion using a buffered reservoir solution with between 10% and 30% PEG, between 10% and 20% glycerol, wherein PEG has an average molecular weight between 1000 and 20000.

11. The method according to claim 10, wherein the extracellular domain of mammalian DPP-IV of step (a) is produced in P. pastoris and then deglycosylated.

12. A method for co-crystallizing mammalian DPP-IV and an active site ligand, the method comprising
(a) providing a buffered, aqueous solution of pH 7 to 8.5 with a concentration of 7 mg/ml to 22 mg/ml of the extracellular domain of mammalian DPP-IV;
(b) adding a molar excess of the active site ligand to the aqueous solution of mammalian DPP-IV;
(c) growing crystals by vapor diffusion using a buffered reservoir solution with between 10% and 30% PEG, between 10% and 20% glycerol, wherein PEG has an average molecular weight between 1000 and 20000.

13. The method according to claim 12, wherein the extracellular domain of mammalian DPP-IV of step (a) is produced in P. pastoris and then deglycosylated.

14. A crystal produced by the methods according to claims 10 to 13.

15. A method for determining the three-dimensional structure of a crystallized extracellular domain of mammalian DPP-IV to a resolution of 3.5Å to 2.1Å or better, the method comprising
(a) crystallizing an extracellular domain of mammalian DPP-IV; and
(b) analysing the extracellular domain of mammalian DPP-IV by X-ray diffraction to determine the three-dimensional structure of the crystallized extracellular domain of mammalian DPP-IV, whereby the three-dimensional structure of a crystallized extracellular domain of mammalian DPP-IV is determined to a resolution of 3.5Å to 2.1Å or better.

16. A machine-readable data storage medium comprising a data storage material encoded with machine readable data which, when using a machine programmed with instructions for using said data, displays a graphical three-dimensional representation of a molecule or molecular complex comprising at least a portion of the extracellular domain of mammalian DPP-IV comprising the amino acids of SEQ ID NO:2, the extracellular domain comprising the ligand binding active site being defined by a set of points having a root mean square deviation of less than about 1.5Å from points representing the backbone atoms of said amino acids as represented by structure coordinates listed in Table 4.

17. A method for identifying a compound that interacts with DPP-IV, comprising the steps of
(a) generating a three-dimensional model of DPP-IV using the structure coordinates listed in Table 4, a root mean square deviation from the backbone atoms of said amino acids of less than 1.5Å; and
(b) employing said three-dimensional model to design or select a compound that interacts with DPP-IV.

18. The method according to claim 17, further comprising the steps of
(c) obtaining the identified compound; and
(d) contacting the obtained compound with DPP-IV in order to determine the effect the compound has on DPP-IV activity.

19. The method according to claims 17 and 18, wherein the compound interacts with the active site of DPP-IV.

20. The method according to claims 17 and 18, wherein the compound interacts with an allosteric binding site of DPP-IV.

21. The method according to claims 17 and 20, wherein the compound is an inhibitor of DPP-IV activity.

22. The method according to claims 17 to 21, wherein the method is a computer-assisted method.

23. A compound identified by the methods according to claims 17 to 22.

24. A pharmaceutical composition comprising the compound of claim 23 and a pharmaceutically acceptable carrier.

25. A compound according to claim 23 for use as a therapeutic active substance, in particular for the treatment of diabetes type I, diabetes type II, IGT , obesity and cancer.

26. An isolated nucleic acid sequence encoding the soluble extracellular domain of DPP-IV comprising the nucleotide sequence of SEQ ID NO:1.

27. A nucleic acid construct comprising an expression vector and the nucleic acid sequence according to claim 26.

28. A host cell transformed with the nucleic acid construct according to claim 27.

29. A method of producing the soluble extracellular domain of DPP-IV comprising culturing the host cell of claim 28 under conditions permitting the expression of the soluble extracellular domain of DPP-IV by the host cell.

30. The method according to claim 29, wherein the host cell is P. pastoris.

31. A polypeptide comprising the soluble extracellular domain of DPP-IV as set forth in SEQ ID NO:2.

32. Use of a compound according to claim 23 for the manufacture of a medicament for the treatment of diabetes type-I, diabetes type-II, IGT, obesity and cancer.

33. Use of a crystal or a co-crystal according to claims 1 to 9 for the identification and/or design of inhibitors of DPP-IV activity.

34. The novel crystals, methods, compounds, compositions and uses substantially as herein before described especially with reference to the foregoing Examples.
